# EUROPEAN PATENT SPECIFICATION

(11) **EP 3 317 427 B1**
(45) Date of publication and mention of the grant of the patent: **23.12.2020**
(21) Application number: 16818507.2
(22) Date of filing: 24.06.2016
(51) Int. Cl.: C12Q 1/6883

(54) **BIOMARKERS OF RESPONSE TO SELECTIVE INHIBITORS OF AURORA A KINASE**
BIOMARKER DER REAKTION AUF SELEKTIVE INHIBITOREN DER AURORA-A-KINASE
BIOMARQUEURS DE RÉPONSE À DES INHIBITEURS SÉLECTIFS D'AURORA A KINASE

(30) Priority: 02.07.2015 US 201562188113 P
(43) Date of publication of application: 09.05.2018
(73) Proprietor: Millennium Pharmaceuticals, Inc., Cambridge, MA 02139 (US)
(72) Inventor: GAO, Feng, Framingham, MA 01701 (US); NIU, Huifeng, Cambridge, MA 02141 (US); SHIN, Hyunjin, Brookline, MA 02445 (US); ZHANG, Zhongfa, Waltham, MA 02452 (US)
(74) Representative: Cornish, Kristina Victoria Joy
(86) International application number: PCT/US2016/039195
(87) International publication number: WO 2017/003845

(56) References cited:
- WO-A1-2013/142491
- WO-A2-2013/083098
- WO-A2-2013/083098
- K. E. HOOK ET AL: "An Integrated Genomic Approach to Identify Predictive Biomarkers of Response to the Aurora Kinase Inhibitor PF-03814735", MOLECULAR CANCER THERAPEUTICS, vol. 11, no. 3, 5 January 2012 (2012-01-05), pages 710-719, XP055343100, US ISSN: 1535-7163, DOI: 10.1158/1535-7163.MCT-11-0184
- F Sootome ET AL: "Genomic predictors of therapeutic sensitivity to TAS- 119, a selective inhibitor of Aurora-A kinase.", European Journal of Cancer, 1 November 2014 (2014-11-01), pages 142-143, XP055343106, DOI: 10.1016/S0959-8049(14)70559-3 Retrieved from the Internet: URL:https://www.sciencedirect.com/science/ article/pii/S0959804914705593?via%3Dihub [retrieved on 2017-02-07]
- AHMED KATSHA ET AL: "Aurora kinase A in gastrointestinal cancers: time to target", MOLECULAR CANCER, BIOMED CENTRAL, LONDON, GB, vol. 14, no. 1, 20 May 2015 (2015-05-20), page 106, XP021223022, ISSN: 1476-4598, DOI: 10.1186/S12943-015-0375-4
- YA-PING CHEN ET AL: "CDKN1A-mediated responsiveness of MLL-AF4 -positive acute lymphoblastic leukemia to Aurora kinase-A inhibitors", INTERNATIONAL JOURNAL OF CANCER, vol. 135, no. 3, 13 January 2014 (2014-01-13), pages 751-762, XP055343109, US ISSN: 0020-7136, DOI: 10.1002/ijc.28708
- J. TENTLER ET AL: "255 Molecular Markers of Sensitivity to the Aurora and Angiogenic Kinase Inhibitor ENMD-2076 in Human Colorectal Cancer (CRC) Models", EUROPEAN JOURNAL OF CANCER, vol. 48, 1 November 2012 (2012-11-01), page 78, XP055343112, AMSTERDAM, NL ISSN: 0959-8049, DOI: 10.1016/S0959-8049(12)72053-1
- MARTA GOMEZ ET AL: "The Hippo pathway in disease and therapy: cancer and beyond", CLINICAL AND TRANSLATIONAL MEDICINE, vol. 3, no. 1, 1 January 2014 (2014-01-01) , page 22, XP055343115, SE ISSN: 2001-1326, DOI: 10.1186/2001-1326-3-22
- POLAKIS P: "Wnt signaling and cancer", GENES AND DEVELOPMENT, COLD SPRING HARBOR LABORATORY PRESS, PLAINVIEW, NY, US, vol. 14, no. 15, 1 August 2000 (2000-08-01), pages 1837-1851, XP002240708, ISSN: 0890-9369
- HOOK, K.E. ET AL.: 'An Integrated Genomic Approach to Identify Predictive Biomarkers of Response to the Aurora Kinase Inhibitor PF-03814735.' MOL CANCER THER vol. 11, no. 3, 05 January 2012, pages 710 - 719, XP055343100
- SOOTOME, H. ET AL.: 'Genomic predictors of therapeutic sensitivity to TAS- 119, a selective inhibitor of Aurora-A kinase.' EUROPEAN JOURNAL OF CANCER vol. 50, no. SUPPLE, 01 December 2014, pages 142 - 143, XP055343106
- KATSHA, A. ET AL.: 'Aurora kinase A in gastrointestinal cancers: time to target.' MOL CANCER vol. 14, 20 May 2015, pages 106.1 - 106.13, XP021223022
- CHEN, Y-P. ET AL.: 'CDKN1A-mediated responsiveness of MLL-AF4-positive acute lymphoblastic leukemia to Aurora kinase-A inhibitors.' INTERNATIONAL JOURNAL OF CANCER vol. 135, no. 3, 31 December 2013, pages 751 - 762, XP055343109
- TENTLER, J. ET AL.: 'Molecular markers of sensitivity to Aurora and angiogenic kinase inhibitor EMMD-2076 in human colorectal cancer (CRC) models.' EUROPEAN JOURNAL OF CANCER vol. 48, no. SUPPLE, 30 November 2012, page 78, XP055343112
- GOMEZ, M. ET AL.: 'The Hippo pathway in disease and therapy: cancer and beyond.' CLIN TRANSL MED vol. 3, 10 July 2014, pages 22.1 - 22.12, XP055343115
- POLAKIS, P.: 'Wnt signaling and cancer.' GENES DEV vol. 14, no. 15, 01 August 2000, pages 1837 - 1851, XP002240708

### SEQUENCE LISTING

<110> Gao, Feng
   Niu, Huifeng
   Shin, Hyunjin
   Zhang, Zhongfa
   Millennium Pharmaceuticals, Inc.
<120> BIOMARKERS OF RESPONSE TO SELECTIVE
   INHIBITORS OF AURORA A KINASE
<130> MPI15-008P1WOM
<140> 62/188,113
   <141> 2015-07-02
<160> 46
<170> FastSEQ for Windows Version 4.0
<210> 1
   <211> 3620
   <212> DNA
   <213> Homo sapiens
<400> 1
   agcgccggcg aggcgcggga ggaggagaag cagtggggag gcgcagccgc tcacctgcgg 60
   ggcagggcgc ggaggaggga cccgggctgc gcgctctcgg gccgaggaac caggacgcgc 120
   ccggagcctc gcacgcggcc aagctcgggg cgtcccctcc cctcggccgg gcgaactcaa 180
   ggggcgcagc tctttgcttt gacagagctg gccggcggag gcgtgcagag cggcgagccg 240
   gcgagccagg ctgagaaact cgagccggga acaaagaggg gtcggactga gtgtgtgtgt 300
   cggctcgagc tccgggcaga ggcatttggg cccgaggccc ccgctgtgac tccccgagac 360
   tccgcagtgc cctccactgc ggagtccccg cgcttgccgg caaaaacttt attcttggca 420
   aacttctctt tctcttcccc tcctcctcgg cccccatctt ctgctcctcc tccttctcta 480
   gcagattaaa tgagcctcga gaagaaaaac cgaagcgaaa gggaagaaaa taagaagatc 540
   taaaacggac atctccagcg tgggtggctc ctttttcttt ttcttttttt cccacccttc 600
   aggaagtgga cgtttcgtta tcttctgatc cttgcacctt cttttggggc aaacggggcc 660
   cttctgccca gatcccctct cttttctcgg aaaacaaact actaagtcgg catccggggt 720
   aactacagtg gagagggttt ccgcggagac gcgccgccgg accctcctct gcactttggg 780
   gaggcgtgct ccctccagaa ccggcgttct ccgcgcgcaa atcccggcga cgcggggtcg 840
   cggggtggcc gccggggcag cctcgtctag cgcgcgccgc gcagacgccc ccggagtcgc 900
   cagctaccgc agccctcgcc gcccagtgcc cttcggcctc gggggcgggc gcctgcgtcg 960
   gtctccgcga agcgggaaag cgcggcggcc gccgggattc gggcgccgcg gcagctgctc 1020
   cggctgccgg ccggcggccc cgcgctcgcc cgccccgctt ccgcccgctg tcctgctgca 1080
   cgaacccttc caactctcct ttcctccccc acccttgagt tacccctctg tctttcctgc 1140
   tgttgcgcgg gtgctcccac agcggagcgg agattacaga gccgccggga tgccccaact 1200
   ctccggagga ggtggcggcg gcggggggga cccggaactc tgcgccacgg acgagatgat 1260
   ccccttcaag gacgagggcg atcctcagaa ggaaaagatc ttcgccgaga tcagtcatcc 1320
   cgaagaggaa ggcgatttag ctgacatcaa gtcttccttg gtgaacgagt ctgaaatcat 1380
   cccggccagc aacggacacg aggtggccag acaagcacaa acctctcagg agccctacca 1440
   cgacaaggcc agagaacacc ccgatgacgg aaagcatcca gatggaggcc tctacaacaa 1500
   gggaccctcc tactcgagtt attccgggta cataatgatg ccaaatatga ataacgaccc 1560
   atacatgtca aatggatctc tttctccacc catcccgaga acatcaaata aagtgcccgt 1620
   ggtgcagcca tcccatgcgg tccatcctct cacccccctc atcacttaca gtgacgagca 1680
   cttttctcca ggatcacacc cgtcacacat cccatcagat gtcaactcca aacaaggcat 1740
   gtccagacat cctccagctc ctgatatccc tactttttat cccttgtctc cgggtggtgt 1800
   tggacagatc accccacctc ttggctggca aggtcagcct gtatatccca tcacgggtgg 1860
   attcaggcaa ccctacccat cctcactgtc agtcgacact tccatgtcca ggttttccca 1920
   tcatatgatt cccggtcctc ctggtcccca cacaactggc atccctcatc cagctattgt 1980
   aacacctcag gtcaaacagg aacatcccca cactgacagt gacctaatgc acgtgaagcc 2040
   tcagcatgaa cagagaaagg agcaggagcc aaaaagacct cacattaaga agcctctgaa 2100
   tgcttttatg ttatacatga aagaaatgag agcgaatgtc gttgctgagt gtactctaaa 2160
   agaaagtgca gctatcaacc agattcttgg cagaaggtgg catgccctct cccgtgaaga 2220
   gcaggctaaa tattatgaat tagcacggaa agaaagacag ctacatatgc agctttatcc 2280
   aggctggtct gcaagagaca attatggtaa gaaaaagaag aggaagagag agaaactaca 2340
   ggaatctgca tcaggtacag gtccaagaat gacagctgcc tacatctgaa acatggtgga 2400
   aaacgaagct cattcccaac gtgcaaagcc aaggcagcga ccccaggacc tcttctggag 2460
   atggaagctt gttgaaaacc cagactgtct ccacggcctg cccagtcgac cccaaaggaa 2520
   cactgacatc aattttaccc tgaggtcact gctagagacg ctgatccata aagacaatca 2580
   ctgccaaccc ctctttcgtc tactgcaaga gccaagttcc aaaataaagc ataaaaaggt 2640
   tttttaaaag gaaatgtaaa agcacatgag aatgctagca ggctgtgggg cagctgagca 2700
   gcttttctcc cctcatatct gcgtgcactt cccagagcat cttgcatcca aacctgtaac 2760
   ctttcggcaa ggacggtaac ttggctgcat ttgcctgtca tgcgcaactg gagccagcaa 2820
   ccagcacatc catcagcacc ccagtggagg agttcatgga agagttccct ctttgtttct 2880
   gcttcatttt tctttctttt cttttctcct aaagctttta tttaacagtg caaaaggatc 2940
   gttttttttt gcttttttaa acttgaattt ttttaattta cactttttag ttttaatttt 3000
   cttgtatatt ttgctagcta tgagctttta aataaaattg aaagttctgg aaaagtttga 3060
   aataatgaca taaaaagaag ccttcttttt ctgagacagc ttgtctggta agtggcttct 3120
   ctgtgaattg cctgtaacac atagtggctt ctccgccctt gtaaggtgtt cagtagagct 3180
   aaataaatgt aatagccaaa cccactctgt tggtagcaat tggcagccct atttcagttt 3240
   attttttctt ctgttttctt cttttctttt tttaaacagt aaaccttaac agatgcgttc 3300
   agcagactgg tttgcagtga attttcattt ctttccttat cacccccttg ttgtaaaaag 3360
   cccagcactt gaattgttat tactttaaat gttctgtatt tgtatctgtt tttattagcc 3420
   aattagtggg attttatgcc agttgttaaa atgagcattg atgtacccat tttttaaaaa 3480
   agcaaggcac agcctttgcc caaaactgtc atcctaacgt ttgtcattcc agtttgagtt 3540
   aatgtgctga gcattttttt aaaagaagct ttgtaataaa acatttttaa aaattgtcat 3600
   ttaaaaaaaa aaaaaaaaaa                                             3620
<210> 2
   <211> 399
   <212> PRT
   <213> Homo sapiens
<400> 2
   Met Pro Gln Leu Ser Gly Gly Gly Gly Gly Gly Gly Gly Asp Pro Glu
    1               5                  10                  15
   Leu Cys Ala Thr Asp Glu Met Ile Pro Phe Lys Asp Glu Gly Asp Pro
               20                  25                  30
   Gln Lys Glu Lys Ile Phe Ala Glu Ile Ser His Pro Glu Glu Glu Gly
           35                  40                  45
   Asp Leu Ala Asp Ile Lys Ser Ser Leu Val Asn Glu Ser Glu Ile Ile
       50                  55                  60
   Pro Ala Ser Asn Gly His Glu Val Ala Arg Gln Ala Gln Thr Ser Gln
   65                  70                  75                  80
   Glu Pro Tyr His Asp Lys Ala Arg Glu His Pro Asp Asp Gly Lys His
                   85                  90                  95
   Pro Asp Gly Gly Leu Tyr Asn Lys Gly Pro Ser Tyr Ser Ser Tyr Ser
               100                 105                 110
   Gly Tyr Ile Met Met Pro Asn Met Asn Asn Asp Pro Tyr Met Ser Asn
           115                 120                 125
   Gly Ser Leu Ser Pro Pro Ile Pro Arg Thr Ser Asn Lys Val Pro Val
       130                 135                 140
   Val Gln Pro Ser His Ala Val His Pro Leu Thr Pro Leu Ile Thr Tyr
   145                 150                 155                 160
   Ser Asp Glu His Phe Ser Pro Gly Ser His Pro Ser His Ile Pro Ser
                   165                 170                 175
   Asp Val Asn Ser Lys Gln Gly Met Ser Arg His Pro Pro Ala Pro Asp
               180                 185                 190
   Ile Pro Thr Phe Tyr Pro Leu Ser Pro Gly Gly Val Gly Gln Ile Thr
           195                 200                 205
   Pro Pro Leu Gly Trp Gln Gly Gln Pro Val Tyr Pro Ile Thr Gly Gly
       210                 215                 220
   Phe Arg Gln Pro Tyr Pro Ser Ser Leu Ser Val Asp Thr Ser Met Ser
   225                 230                 235                 240
   Arg Phe Ser His His Met Ile Pro Gly Pro Pro Gly Pro His Thr Thr
                   245                 250                 255
   Gly Ile Pro His Pro Ala Ile Val Thr Pro Gln Val Lys Gln Glu His
               260                 265                 270
   Pro His Thr Asp Ser Asp Leu Met His Val Lys Pro Gln His Glu Gln
           275                 280                 285
   Arg Lys Glu Gln Glu Pro Lys Arg Pro His Ile Lys Lys Pro Leu Asn
       290                 295                 300
   Ala Phe Met Leu Tyr Met Lys Glu Met Arg Ala Asn Val Val Ala Glu
   305                 310                 315                 320
   Cys Thr Leu Lys Glu Ser Ala Ala Ile Asn Gln Ile Leu Gly Arg Arg
                   325                 330                 335
   Trp His Ala Leu Ser Arg Glu Glu Gln Ala Lys Tyr Tyr Glu Leu Ala
               340                 345                 350
   Arg Lys Glu Arg Gln Leu His Met Gln Leu Tyr Pro Gly Trp Ser Ala
           355                 360                 365
   Arg Asp Asn Tyr Gly Lys Lys Lys Lys Arg Lys Arg Glu Lys Leu Gln
       370                 375                 380
   Glu Ser Ala Ser Gly Thr Gly Pro Arg Met Thr Ala Ala Tyr Ile
   385                 390                 395
<210> 3
   <211> 5172
   <212> DNA
   <213> Homo sapiens
<400> 3
   gtcctctctc gcggtatcat ccggttgctg aggccctgta ataaaggtct cgcgaaattt 60
   gttctagagg tccaagtttg cttcttagct tactccaccc cacccccaac ctgtccctcc 120
   ttttctttcc aagtcacaaa attctcccct cccctacccc ggagtttacg gccctcctcc 180
   tgtttccgat ttcagcccgg aaccggaagt gtagtgggcg gggcccgtcg gcggaaaacg 240
   cagcggagcc agagccggac acggctgtgg ccgctgcctc tacccccgcc acggatcgcc 300
   gggtagtagg actgcgcggc tccaggctga gggtcggtcc ggaggcgggt gggcgcgggt 360
   ctcacccgga ttgtccgggt ggcaccgttc ccggccccac cgggcgccgc gagggatcat 420
   gtctacagcc tctgccgcct cctcctcctc ctcgtcttcg gccggtgaga tgatcgaagc 480
   cccttcccag gtcctcaact ttgaagagat cgactacaag gagatcgagg tggaagaggt 540
   tgttggaaga ggagcctttg gagttgtttg caaagctaag tggagagcaa aagatgttgc 600
   tattaaacaa atagaaagtg aatctgagag gaaagcgttt attgtagagc ttcggcagtt 660
   atcccgtgtg aaccatccta atattgtaaa gctttatgga gcctgcttga atccagtgtg 720
   tcttgtgatg gaatatgctg aagggggctc tttatataat gtgctgcatg gtgctgaacc 780
   attgccatat tatactgctg cccacgcaat gagttggtgt ttacagtgtt cccaaggagt 840
   ggcttatctt cacagcatgc aacccaaagc gctaattcac agggacctga aaccaccaaa 900
   cttactgctg gttgcagggg ggacagttct aaaaatttgt gattttggta cagcctgtga 960
   cattcagaca cacatgacca ataacaaggg gagtgctgct tggatggcac ctgaagtttt 1020
   tgaaggtagt aattacagtg aaaaatgtga cgtcttcagc tggggtatta ttctttggga 1080
   agtgataacg cgtcggaaac cctttgatga gattggtggc ccagctttcc gaatcatgtg 1140
   ggctgttcat aatggtactc gaccaccact gataaaaaat ttacctaagc ccattgagag 1200
   cctgatgact cgttgttggt ctaaagatcc ttcccagcgc ccttcaatgg aggaaattgt 1260
   gaaaataatg actcacttga tgcggtactt tccaggagca gatgagccat tacagtatcc 1320
   ttgtcagtat tcagatgaag gacagagcaa ctctgccacc agtacaggct cattcatgga 1380
   cattgcttct acaaatacga gtaacaaaag tgacactaat atggagcaag ttcctgccac 1440
   aaatgatact attaagcgct tagaatcaaa attgttgaaa aatcaggcaa agcaacagag 1500
   tgaatctgga cgtttaagct tgggagcctc ccgtgggagc agtgtggaga gcttgccccc 1560
   aacctctgag ggcaagagga tgagtgctga catgtctgaa atagaagcta ggatcgccgc 1620
   aaccacagcc tattccaagc ctaaacgggg ccaccgtaaa actgcttcat ttggcaacat 1680
   tctggatgtc cctgagatcg tcatatcagg caacggacag ccaagacgta gatccatcca 1740
   agacttgact gtaactggaa cagaacctgg tcaggtgagc agtaggtcat ccagtcccag 1800
   tgtcagaatg attactacct caggaccaac ctcagaaaag ccaactcgaa gtcatccatg 1860
   gacccctgat gattccacag ataccaatgg atcagataac tccatcccaa tggcttatct 1920
   tacactggat caccaactac agcctctagc accgtgccca aactccaaag aatctatggc 1980
   agtgtttgaa cagcattgta aaatggcaca agaatatatg aaagttcaaa cagaaattgc 2040
   attgttatta cagagaaagc aagaactagt tgcagaactg gaccaggatg aaaaggacca 2100
   gcaaaataca tctcgcctgg tacaggaaca taaaaagctt ttagatgaaa acaaaagcct 2160
   ttctacttac taccagcaat gcaaaaaaca actagaggtc atcagaagtc agcagcagaa 2220
   acgacaaggc acttcatgat tctctgggac cgttacattt tgaaatatgc aaagaaagac 2280
   ttttttttta aggaaaggaa aaccttataa tgacgattca tgagtgttag ctttttggcg 2340
   tgttctgaat gccaactgcc tatatttgct gcattttttt cattgtttat tttccttttc 2400
   tcatggtgga catacaattt tactgtttca ttgcataaca tggtagcatc tgtgacttga 2460
   atgagcagca ctttgcaact tcaaaacaga tgcagtgaac tgtggctgta tatgcatgct 2520
   cattgtgtga aggctagcct aacagaacag gaggtatcaa actagctgct atgtgcaaac 2580
   agcgtccatt ttttcatatt agaggtggaa cctcaagaat gactttattc ttgtatctca 2640
   tctcaaaata ttaataattt ttttcccaaa agatggtata taccaagtta aagacagggt 2700
   attataaatt tagagtgatt ggtggtatat tacggaaata cggaaccttt agggatagtt 2760
   ccgtgtaagg gctttgatgc cagcatcctt ggatcagtac tgaactcagt tccatccgta 2820
   aaatatgtaa aggtaagtgg cagctgctct atttaatgaa agcagtttta ccggattttg 2880
   ttagactaaa atttgattgt gatacattga acaaaatgga actcattttt ttttaaggag 2940
   taaagatttt taattctgtg attgtgtgta tgtgtgttga aactgtaaag cttttatgac 3000
   tctaatatta atctcttaaa tgaaattaaa aggcaaaaga acatgattga gcttaaatga 3060
   tcatttcttc ctgcagtgat tcttggattg ttttctcatg tatttgaaaa aaaaaaaatg 3120
   aagaaaaata atggaaaatg gaagtaatta ctccagctaa aaaaagcttg gacttagatt 3180
   tctttttatg ataccaaatg agaaataaac caggcaaatc agaaggaagt taaagaagca 3240
   aatataaatt caacaagtgt cctaattatc ctggatattg gaatatttga ttttccttac 3300
   aatcccgttc tagaatgcct gccgcctttc aacactttca agagaatttc aacacttaca 3360
   gagtatttat attgttaaca gtaattttgc taccaaaacc ttcagaataa ctttaataat 3420
   aaaatgacac tgaaataata gactatacaa actctatatt ttttcagttg gtgttaaaat 3480
   aagtcacatt ttgataccag tacaagatgt cttttaaata aggatgtcat cagtctgatt 3540
   tttatagcat taatgtttta tgaagaaaaa gttcaaaatg aaagcattaa ttgctgtgat 3600
   tattagaatt ctatcatgac tgtattgtag tttttgctct atttcagata agcaagatct 3660
   aagaagttat caaaactatt ctttaaaatg ctaaagcagg taactttttc ttccattatt 3720
   ttttcctcct accactgagt tttgtaatga attccttgtg tatacaagca atacaggtga 3780
   atactaaact gttattttta gcttcttcaa aagctatttt agaaagcttc ctggaaataa 3840
   atgtcttctg tcatttaatt taaataaaag gagtgttaat tgttcccaaa tttgattacc 3900
   tagctgaaac agatattgga ttcagcatag taatagtaaa ataaaattgg cagagaaaat 3960
   aactgtagac taggataaag catgactcct ccatagcatt gttgcttgta tgtacatctg 4020
   tcactccact gaaagggacc tagtcacttt aaagaggcct tttatctcag cacgagatat 4080
   ccaagcgtga atgctaaggc ttgatgtttc cattaggatt tagcattcga gatttcagat 4140
   tttatttata gtcattgatg tgttttgctg tattataaca catttaaggg aaattttatt 4200
   atggttttta catgtagtca tttcataaaa atatccagat aggtaaatgg aagaaaatag 4260
   tagattttag tcatggtacc aaatgcaaga ggctgttgaa cagttgcttg tttgtttaca 4320
   gtaagttcct tgagattttc agtactgtag ttgccctgga cattgagtac agttcagcct 4380
   tactctgttt ttaagtagtt gtgttgtcaa tttcatgctt ttaagtcctg atgatccttg 4440
   gagatgggag aaaaagatac cagggataat taaaatccac agccagtttc atcagtttcc 4500
   tcatccatcc tgcaaataaa aatgttaaca aggagccaaa cttattcgtt ctggttttac 4560
   aatttatttt gaccgttttt tgggtgaaaa ttgcaaacag ccaagcaagt ggctggaatg 4620
   ccccagtcta agaaattcaa aaacaatcac tgagtaagcc ctaattacat taattacatt 4680
   ttactcttga ctccaggaaa agaaagcaag cttttatcta atctagaggg aagtattcgt 4740
   gagcaataaa aatcactttt ttgagttgaa taataatcag ttaagaatat ttaccgcata 4800
   gcaacacaac atataaagat ttgttacaag ttgtttacgg atgtttgact atttttgctg 4860
   aagtatttta gagtattgaa tgtcttctct cttcaagttt ctttcatgtt cctaatttca 4920
   gctcctgtag ccagagatca caggtcttcc ctgtgaaact ttggtttctt tctataaatg 4980
   tgtgtggttt tcagcgctca actcctgtct tcaaatggta gtaagttcta cttctacttc 5040
   tgtcattcag aacattttat gtcaaatgat gtaatgcaga aattcttgtg catatttgta 5100
   actgaaggaa gctttttaga tttatttttg tttttaataa aattcagatt cctattctaa 5160
   actggtaaaa aa                                                      5172
<210> 4
   <211> 606
   <212> PRT
   <213> Homo sapiens
<400> 4
<210> 5
   <211> 10740
   <212> DNA
   <213> Homo sapiens
<400> 5
   gtattggtgc agcccgccag ggtgtcactg gagacagaat ggaggtgctg ccggactcgg 60
   aaatggggtc caagggtagc caaggatggc tgcagcttca tatgatcagt tgttaaagca 120
   agttgaggca ctgaagatgg agaactcaaa tcttcgacaa gagctagaag ataattccaa 180
   tcatcttaca aaactggaaa ctgaggcatc taatatgaag gaagtactta aacaactaca 240
   aggaagtatt gaagatgaag ctatggcttc ttctggacag attgatttat tagagcgtct 300
   taaagagctt aacttagata gcagtaattt ccctggagta aaactgcggt caaaaatgtc 360
   cctccgttct tatggaagcc gggaaggatc tgtatcaagc cgttctggag agtgcagtcc 420
   tgttcctatg ggttcatttc caagaagagg gtttgtaaat ggaagcagag aaagtactgg 480
   atatttagaa gaacttgaga aagagaggtc attgcttctt gctgatcttg acaaagaaga 540
   aaaggaaaaa gactggtatt acgctcaact tcagaatctc actaaaagaa tagatagtct 600
   tcctttaact gaaaattttt ccttacaaac agatatgacc agaaggcaat tggaatatga 660
   agcaaggcaa atcagagttg cgatggaaga acaactaggt acctgccagg atatggaaaa 720
   acgagcacag cgaagaatag ccagaattca gcaaatcgaa aaggacatac ttcgtatacg 780
   acagctttta cagtcccaag caacagaagc agagaggtca tctcagaaca agcatgaaac 840
   cggctcacat gatgctgagc ggcagaatga aggtcaagga gtgggagaaa tcaacatggc 900
   aacttctggt aatggtcagg gttcaactac acgaatggac catgaaacag ccagtgtttt 960
   gagttctagt agcacacact ctgcacctcg aaggctgaca agtcatctgg gaaccaaggt 1020
   ggaaatggtg tattcattgt tgtcaatgct tggtactcat gataaggatg atatgtcgcg 1080
   aactttgcta gctatgtcta gctcccaaga cagctgtata tccatgcgac agtctggatg 1140
   tcttcctctc ctcatccagc ttttacatgg caatgacaaa gactctgtat tgttgggaaa 1200
   ttcccggggc agtaaagagg ctcgggccag ggccagtgca gcactccaca acatcattca 1260
   ctcacagcct gatgacaaga gaggcaggcg tgaaatccga gtccttcatc ttttggaaca 1320
   gatacgcgct tactgtgaaa cctgttggga gtggcaggaa gctcatgaac caggcatgga 1380
   ccaggacaaa aatccaatgc cagctcctgt tgaacatcag atctgtcctg ctgtgtgtgt 1440
   tctaatgaaa ctttcatttg atgaagagca tagacatgca atgaatgaac tagggggact 1500
   acaggccatt gcagaattat tgcaagtgga ctgtgaaatg tatgggctta ctaatgacca 1560
   ctacagtatt acactaagac gatatgctgg aatggctttg acaaacttga cttttggaga 1620
   tgtagccaac aaggctacgc tatgctctat gaaaggctgc atgagagcac ttgtggccca 1680
   actaaaatct gaaagtgaag acttacagca ggttattgcg agtgttttga ggaatttgtc 1740
   ttggcgagca gatgtaaata gtaaaaagac gttgcgagaa gttggaagtg tgaaagcatt 1800
   gatggaatgt gctttagaag ttaaaaagga atcaaccctc aaaagcgtat tgagtgcctt 1860
   atggaatttg tcagcacatt gcactgagaa taaagctgat atatgtgctg tagatggtgc 1920
   acttgcattt ttggttggca ctcttactta ccggagccag acaaacactt tagccattat 1980
   tgaaagtgga ggtgggatat tacggaatgt gtccagcttg atagctacaa atgaggacca 2040
   caggcaaatc ctaagagaga acaactgtct acaaacttta ttacaacact taaaatctca 2100
   tagtttgaca atagtcagta atgcatgtgg aactttgtgg aatctctcag caagaaatcc 2160
   taaagaccag gaagcattat gggacatggg ggcagttagc atgctcaaga acctcattca 2220
   ttcaaagcac aaaatgattg ctatgggaag tgctgcagct ttaaggaatc tcatggcaaa 2280
   taggcctgcg aagtacaagg atgccaatat tatgtctcct ggctcaagct tgccatctct 2340
   tcatgttagg aaacaaaaag ccctagaagc agaattagat gctcagcact tatcagaaac 2400
   ttttgacaat atagacaatt taagtcccaa ggcatctcat cgtagtaagc agagacacaa 2460
   gcaaagtctc tatggtgatt atgtttttga caccaatcga catgatgata ataggtcaga 2520
   caattttaat actggcaaca tgactgtcct ttcaccatat ttgaatacta cagtgttacc 2580
   cagctcctct tcatcaagag gaagcttaga tagttctcgt tctgaaaaag atagaagttt 2640
   ggagagagaa cgcggaattg gtctaggcaa ctaccatcca gcaacagaaa atccaggaac 2700
   ttcttcaaag cgaggtttgc agatctccac cactgcagcc cagattgcca aagtcatgga 2760
   agaagtgtca gccattcata cctctcagga agacagaagt tctgggtcta ccactgaatt 2820
   acattgtgtg acagatgaga gaaatgcact tagaagaagc tctgctgccc atacacattc 2880
   aaacacttac aatttcacta agtcggaaaa ttcaaatagg acatgttcta tgccttatgc 2940
   caaattagaa tacaagagat cttcaaatga tagtttaaat agtgtcagta gtagtgatgg 3000
   ttatggtaaa agaggtcaaa tgaaaccctc gattgaatcc tattctgaag atgatgaaag 3060
   taagttttgc agttatggtc aatacccagc cgacctagcc cataaaatac atagtgcaaa 3120
   tcatatggat gataatgatg gagaactaga tacaccaata aattatagtc ttaaatattc 3180
   agatgagcag ttgaactctg gaaggcaaag tccttcacag aatgaaagat gggcaagacc 3240
   caaacacata atagaagatg aaataaaaca aagtgagcaa agacaatcaa ggaatcaaag 3300
   tacaacttat cctgtttata ctgagagcac tgatgataaa cacctcaagt tccaaccaca 3360
   ttttggacag caggaatgtg tttctccata caggtcacgg ggagccaatg gttcagaaac 3420
   aaatcgagtg ggttctaatc atggaattaa tcaaaatgta agccagtctt tgtgtcaaga 3480
   agatgactat gaagatgata agcctaccaa ttatagtgaa cgttactctg aagaagaaca 3540
   gcatgaagaa gaagagagac caacaaatta tagcataaaa tataatgaag agaaacgtca 3600
   tgtggatcag cctattgatt atagtttaaa atatgccaca gatattcctt catcacagaa 3660
   acagtcattt tcattctcaa agagttcatc tggacaaagc agtaaaaccg aacatatgtc 3720
   ttcaagcagt gagaatacgt ccacaccttc atctaatgcc aagaggcaga atcagctcca 3780
   tccaagttct gcacagagta gaagtggtca gcctcaaaag gctgccactt gcaaagtttc 3840
   ttctattaac caagaaacaa tacagactta ttgtgtagaa gatactccaa tatgtttttc 3900
   aagatgtagt tcattatcat ctttgtcatc agctgaagat gaaataggat gtaatcagac 3960
   gacacaggaa gcagattctg ctaataccct gcaaatagca gaaataaaag aaaagattgg 4020
   aactaggtca gctgaagatc ctgtgagcga agttccagca gtgtcacagc accctagaac 4080
   caaatccagc agactgcagg gttctagttt atcttcagaa tcagccaggc acaaagctgt 4140
   tgaattttct tcaggagcga aatctccctc caaaagtggt gctcagacac ccaaaagtcc 4200
   acctgaacac tatgttcagg agaccccact catgtttagc agatgtactt ctgtcagttc 4260
   acttgatagt tttgagagtc gttcgattgc cagctccgtt cagagtgaac catgcagtgg 4320
   aatggtaagt ggcattataa gccccagtga tcttccagat agccctggac aaaccatgcc 4380
   accaagcaga agtaaaacac ctccaccacc tcctcaaaca gctcaaacca agcgagaagt 4440
   acctaaaaat aaagcaccta ctgctgaaaa gagagagagt ggacctaagc aagctgcagt 4500
   aaatgctgca gttcagaggg tccaggttct tccagatgct gatactttat tacattttgc 4560
   cacggaaagt actccagatg gattttcttg ttcatccagc ctgagtgctc tgagcctcga 4620
   tgagccattt atacagaaag atgtggaatt aagaataatg cctccagttc aggaaaatga 4680
   caatgggaat gaaacagaat cagagcagcc taaagaatca aatgaaaacc aagagaaaga 4740
   ggcagaaaaa actattgatt ctgaaaagga cctattagat gattcagatg atgatgatat 4800
   tgaaatacta gaagaatgta ttatttctgc catgccaaca aagtcatcac gtaaagcaaa 4860
   aaagccagcc cagactgctt caaaattacc tccacctgtg gcaaggaaac caagtcagct 4920
   gcctgtgtac aaacttctac catcacaaaa caggttgcaa ccccaaaagc atgttagttt 4980
   tacaccgggg gatgatatgc cacgggtgta ttgtgttgaa gggacaccta taaacttttc 5040
   cacagctaca tctctaagtg atctaacaat cgaatcccct ccaaatgagt tagctgctgg 5100
   agaaggagtt agaggagggg cacagtcagg tgaatttgaa aaacgagata ccattcctac 5160
   agaaggcaga agtacagatg aggctcaagg aggaaaaacc tcatctgtaa ccatacctga 5220
   attggatgac aataaagcag aggaaggtga tattcttgca gaatgcatta attctgctat 5280
   gcccaaaggg aaaagtcaca agcctttccg tgtgaaaaag ataatggacc aggtccagca 5340
   agcatctgcg tcttcttctg cacccaacaa aaatcagtta gatggtaaga aaaagaaacc 5400
   aacttcacca gtaaaaccta taccacaaaa tactgaatat aggacacgtg taagaaaaaa 5460
   tgcagactca aaaaataatt taaatgctga gagagttttc tcagacaaca aagattcaaa 5520
   gaaacagaat ttgaaaaata attccaaggt cttcaatgat aagctcccaa ataatgaaga 5580
   tagagtcaga ggaagttttg cttttgattc acctcatcat tacacgccta ttgaaggaac 5640
   tccttactgt ttttcacgaa atgattcttt gagttctcta gattttgatg atgatgatgt 5700
   tgacctttcc agggaaaagg ctgaattaag aaaggcaaaa gaaaataagg aatcagaggc 5760
   taaagttacc agccacacag aactaacctc caaccaacaa tcagctaata agacacaagc 5820
   tattgcaaag cagccaataa atcgaggtca gcctaaaccc atacttcaga aacaatccac 5880
   ttttccccag tcatccaaag acataccaga cagaggggca gcaactgatg aaaagttaca 5940
   gaattttgct attgaaaata ctccggtttg cttttctcat aattcctctc tgagttctct 6000
   cagtgacatt gaccaagaaa acaacaataa agaaaatgaa cctatcaaag agactgagcc 6060
   ccctgactca cagggagaac caagtaaacc tcaagcatca ggctatgctc ctaaatcatt 6120
   tcatgttgaa gataccccag tttgtttctc aagaaacagt tctctcagtt ctcttagtat 6180
   tgactctgaa gatgacctgt tgcaggaatg tataagctcc gcaatgccaa aaaagaaaaa 6240
   gccttcaaga ctcaagggtg ataatgaaaa acatagtccc agaaatatgg gtggcatatt 6300
   aggtgaagat ctgacacttg atttgaaaga tatacagaga ccagattcag aacatggtct 6360
   atcccctgat tcagaaaatt ttgattggaa agctattcag gaaggtgcaa attccatagt 6420
   aagtagttta catcaagctg ctgctgctgc atgtttatct agacaagctt cgtctgattc 6480
   agattccatc ctttccctga aatcaggaat ctctctggga tcaccatttc atcttacacc 6540
   tgatcaagaa gaaaaaccct ttacaagtaa taaaggccca cgaattctaa aaccagggga 6600
   gaaaagtaca ttggaaacta aaaagataga atctgaaagt aaaggaatca aaggaggaaa 6660
   aaaagtttat aaaagtttga ttactggaaa agttcgatct aattcagaaa tttcaggcca 6720
   aatgaaacag ccccttcaag caaacatgcc ttcaatctct cgaggcagga caatgattca 6780
   tattccagga gttcgaaata gctcctcaag tacaagtcct gtttctaaaa aaggcccacc 6840
   ccttaagact ccagcctcca aaagccctag tgaaggtcaa acagccacca cttctcctag 6900
   aggagccaag ccatctgtga aatcagaatt aagccctgtt gccaggcaga catcccaaat 6960
   aggtgggtca agtaaagcac cttctagatc aggatctaga gattcgaccc cttcaagacc 7020
   tgcccagcaa ccattaagta gacctataca gtctcctggc cgaaactcaa tttcccctgg 7080
   tagaaatgga ataagtcctc ctaacaaatt atctcaactt ccaaggacat catcccctag 7140
   tactgcttca actaagtcct caggttctgg aaaaatgtca tatacatctc caggtagaca 7200
   gatgagccaa cagaacctta ccaaacaaac aggtttatcc aagaatgcca gtagtattcc 7260
   aagaagtgag tctgcctcca aaggactaaa tcagatgaat aatggtaatg gagccaataa 7320
   aaaggtagaa ctttctagaa tgtcttcaac taaatcaagt ggaagtgaat ctgatagatc 7380
   agaaagacct gtattagtac gccagtcaac tttcatcaaa gaagctccaa gcccaacctt 7440
   aagaagaaaa ttggaggaat ctgcttcatt tgaatctctt tctccatcat ctagaccagc 7500
   ttctcccact aggtcccagg cacaaactcc agttttaagt ccttcccttc ctgatatgtc 7560
   tctatccaca cattcgtctg ttcaggctgg tggatggcga aaactcccac ctaatctcag 7620
   tcccactata gagtataatg atggaagacc agcaaagcgc catgatattg cacggtctca 7680
   ttctgaaagt ccttctagac ttccaatcaa taggtcagga acctggaaac gtgagcacag 7740
   caaacattca tcatcccttc ctcgagtaag cacttggaga agaactggaa gttcatcttc 7800
   aattctttct gcttcatcag aatccagtga aaaagcaaaa agtgaggatg aaaaacatgt 7860
   gaactctatt tcaggaacca aacaaagtaa agaaaaccaa gtatccgcaa aaggaacatg 7920
   gagaaaaata aaagaaaatg aattttctcc cacaaatagt acttctcaga ccgtttcctc 7980
   aggtgctaca aatggtgctg aatcaaagac tctaatttat caaatggcac ctgctgtttc 8040
   taaaacagag gatgtttggg tgagaattga ggactgtccc attaacaatc ctagatctgg 8100
   aagatctccc acaggtaata ctcccccggt gattgacagt gtttcagaaa aggcaaatcc 8160
   aaacattaaa gattcaaaag ataatcaggc aaaacaaaat gtgggtaatg gcagtgttcc 8220
   catgcgtacc gtgggtttgg aaaatcgcct gaactccttt attcaggtgg atgcccctga 8280
   ccaaaaagga actgagataa aaccaggaca aaataatcct gtccctgtat cagagactaa 8340
   tgaaagttct atagtggaac gtaccccatt cagttctagc agctcaagca aacacagttc 8400
   acctagtggg actgttgctg ccagagtgac tccttttaat tacaacccaa gccctaggaa 8460
   aagcagcgca gatagcactt cagctcggcc atctcagatc ccaactccag tgaataacaa 8520
   cacaaagaag cgagattcca aaactgacag cacagaatcc agtggaaccc aaagtcctaa 8580
   gcgccattct gggtcttacc ttgtgacatc tgtttaaaag agaggaagaa tgaaactaag 8640
   aaaattctat gttaattaca actgctatat agacattttg tttcaaatga aactttaaaa 8700
   gactgaaaaa ttttgtaaat aggtttgatt cttgttagag ggtttttgtt ctggaagcca 8760
   tatttgatag tatactttgt cttcactggt cttattttgg gaggcactct tgatggttag 8820
   gaaaaaaata gtaaagccaa gtatgtttgt acagtatgtt ttacatgtat ttaaagtagc 8880
   atcccatccc aacttccttt aattattgct tgtcttaaaa taatgaacac tacagataga 8940
   aaatatgata tattgctgtt atcaatcatt tctagattat aaactgacta aacttacatc 9000
   agggaaaaat tggtatttat gcaaaaaaaa atgtttttgt ccttgtgagt ccatctaaca 9060
   tcataattaa tcatgtggct gtgaaattca cagtaatatg gttcccgatg aacaagttta 9120
   cccagcctgc tttgctttac tgcatgaatg aaactgatgg ttcaatttca gaagtaatga 9180
   ttaacagtta tgtggtcaca tgatgtgcat agagatagct acagtgtaat aatttacact 9240
   attttgtgct ccaaacaaaa caaaaatctg tgtaactgta aaacattgaa tgaaactatt 9300
   ttacctgaac tagattttat ctgaaagtag gtagaatttt tgctatgctg taatttgttg 9360
   tatattctgg tatttgaggt gagatggctg ctcttttatt aatgagacat gaattgtgtc 9420
   tcaacagaaa ctaaatgaac atttcagaat aaattattgc tgtatgtaaa ctgttactga 9480
   aattggtatt tgtttgaagg gtcttgtttc acatttgtat taataattgt ttaaaatgcc 9540
   tcttttaaaa gcttatataa attttttctt tcagcttcta tgcattaaga gtaaaattcc 9600
   tcttactgta ataaaaacaa ttgaagaaga ctgttgccac ttaaccattc catgcgttgg 9660
   cacttatcta ttcctgaaat ttcttttatg tgattagctc atcttgattt ttaatatttt 9720
   tccacttaaa cttttttttc ttactccact ggagctcagt aaaagtaaat tcatgtaata 9780
   gcaatgcaag cagcctagca cagactaagc attgagcata ataggcccac ataatttcct 9840
   ctttcttaat attatagaat tctgtacttg aaattgattc ttagacattg cagtctcttc 9900
   gaggctttac agtgtaaact gtcttgcccc ttcatcttct tgttgcaact gggtctgaca 9960
   tgaacacttt ttatcaccct gtatgttagg gcaagatctc agcagtgaag tataatcagc 10020
   actttgccat gctcagaaaa ttcaaatcac atggaacttt agaggtagat ttaatacgat 10080
   taagatattc agaagtatat tttagaatcc ctgcctgtta aggaaacttt atttgtggta 10140
   ggtacagttc tggggtacat gttaagtgtc cccttataca gtggagggaa gtcttccttc 10200
   ctgaaggaaa ataaactgac acttattaac taagataatt tacttaatat atcttccctg 10260
   atttgtttta aaagatcaga gggtgactga tgatacatgc atacatattt gttgaataaa 10320
   tgaaaattta tttttagtga taagattcat acactctgta tttggggagg gaaaaccttt 10380
   ttaagcatgg tggggcactc agataggagt gaatacacct acctggtgcc ttgaaaatca 10440
   catcaagtag ttaattatct accccttacc tgtgtttata acttccaggt aatgagaatg 10500
   atttttttta aagctaaaat gccagtaaat aaaagtgcta tgacttgagc taagatattt 10560
   gactccaatg cctgtactgt gtctactgca ccactttgta aacacttcaa tttactatct 10620
   ttgaaatgat tgacctttaa atttttgcca aatgttatct gaaattgtct atgaatacca 10680
   tctacttctg ttgttttccc aggcttccat aaacaatgga gatacatgca aaaaaaaaaa 10740
<210> 6
   <211> 2843
   <212> PRT
   <213> Homo sapiens
<400> 6
   Met Ala Ala Ala Ser Tyr Asp Gln Leu Leu Lys Gln Val Glu Ala Leu
    1               5                  10                  15
   Lys Met Glu Asn Ser Asn Leu Arg Gln Glu Leu Glu Asp Asn Ser Asn
               20                  25                  30
   His Leu Thr Lys Leu Glu Thr Glu Ala Ser Asn Met Lys Glu Val Leu
           35                  40                  45
   Lys Gln Leu Gln Gly Ser Ile Glu Asp Glu Ala Met Ala Ser Ser Gly
       50                  55                  60
   Gln Ile Asp Leu Leu Glu Arg Leu Lys Glu Leu Asn Leu Asp Ser Ser
   65                  70                  75                  80
   Asn Phe Pro Gly Val Lys Leu Arg Ser Lys Met Ser Leu Arg Ser Tyr
                   85                  90                  95
   Gly Ser Arg Glu Gly Ser Val Ser Ser Arg Ser Gly Glu Cys Ser Pro
               100                 105                 110
   Val Pro Met Gly Ser Phe Pro Arg Arg Gly Phe Val Asn Gly Ser Arg
           115                 120                 125
   Glu Ser Thr Gly Tyr Leu Glu Glu Leu Glu Lys Glu Arg Ser Leu Leu
       130                 135                 140
   Leu Ala Asp Leu Asp Lys Glu Glu Lys Glu Lys Asp Trp Tyr Tyr Ala
   145                 150                 155                 160
   Gln Leu Gln Asn Leu Thr Lys Arg Ile Asp Ser Leu Pro Leu Thr Glu
                   165                 170                 175
   Asn Phe Ser Leu Gln Thr Asp Met Thr Arg Arg Gln Leu Glu Tyr Glu
               180                 185                 190
   Ala Arg Gln Ile Arg Val Ala Met Glu Glu Gln Leu Gly Thr Cys Gln
           195                 200                 205
   Asp Met Glu Lys Arg Ala Gln Arg Arg Ile Ala Arg Ile Gln Gln Ile
       210                 215                 220
   Glu Lys Asp Ile Leu Arg Ile Arg Gln Leu Leu Gln Ser Gln Ala Thr
   225                 230                 235                 240
   Glu Ala Glu Arg Ser Ser Gln Asn Lys His Glu Thr Gly Ser His Asp
                   245                 250                 255
   Ala Glu Arg Gln Asn Glu Gly Gln Gly Val Gly Glu Ile Asn Met Ala
               260                 265                 270
   Thr Ser Gly Asn Gly Gln Gly Ser Thr Thr Arg Met Asp His Glu Thr
           275                 280                 285
   Ala Ser Val Leu Ser Ser Ser Ser Thr His Ser Ala Pro Arg Arg Leu
       290                 295                 300
   Thr Ser His Leu Gly Thr Lys Val Glu Met Val Tyr Ser Leu Leu Ser
   305                 310                 315                 320
   Met Leu Gly Thr His Asp Lys Asp Asp Met Ser Arg Thr Leu Leu Ala
                   325                 330                 335
   Met Ser Ser Ser Gln Asp Ser Cys Ile Ser Met Arg Gln Ser Gly Cys
               340                 345                 350
   Leu Pro Leu Leu Ile Gln Leu Leu His Gly Asn Asp Lys Asp Ser Val
           355                 360                 365
   Leu Leu Gly Asn Ser Arg Gly Ser Lys Glu Ala Arg Ala Arg Ala Ser
       370                 375                 380
   Ala Ala Leu His Asn Ile Ile His Ser Gln Pro Asp Asp Lys Arg Gly
   385                 390                 395                 400
   Arg Arg Glu Ile Arg Val Leu His Leu Leu Glu Gln Ile Arg Ala Tyr
   405 410 415
   Cys Glu Thr Cys Trp Glu Trp Gln Glu Ala His Glu Pro Gly Met Asp
   420 425 430
   Gln Asp Lys Asn Pro Met Pro Ala Pro Val Glu His Gln Ile Cys Pro
   435 440 445
   Ala Val Cys Val Leu Met Lys Leu Ser Phe Asp Glu Glu His Arg His
   450 455 460
   Ala Met Asn Glu Leu Gly Gly Leu Gln Ala Ile Ala Glu Leu Leu Gln
   465 470 475 480
   Val Asp Cys Glu Met Tyr Gly Leu Thr Asn Asp His Tyr Ser Ile Thr
   485 490 495
   Leu Arg Arg Tyr Ala Gly Met Ala Leu Thr Asn Leu Thr Phe Gly Asp
   500 505 510
   Val Ala Asn Lys Ala Thr Leu Cys Ser Met Lys Gly Cys Met Arg Ala
   515 520 525
   Leu Val Ala Gln Leu Lys Ser Glu Ser Glu Asp Leu Gln Gln Val Ile
   530 535 540
   Ala Ser Val Leu Arg Asn Leu Ser Trp Arg Ala Asp Val Asn Ser Lys
   545 550 555 560
   Lys Thr Leu Arg Glu Val Gly Ser Val Lys Ala Leu Met Glu Cys Ala
   565 570 575
   Leu Glu Val Lys Lys Glu Ser Thr Leu Lys Ser Val Leu Ser Ala Leu
   580 585 590
   Trp Asn Leu Ser Ala His Cys Thr Glu Asn Lys Ala Asp Ile Cys Ala
   595 600 605
   Val Asp Gly Ala Leu Ala Phe Leu Val Gly Thr Leu Thr Tyr Arg Ser
   610 615 620
   Gln Thr Asn Thr Leu Ala Ile Ile Glu Ser Gly Gly Gly Ile Leu Arg
   625 630 635 640
   Asn Val Ser Ser Leu Ile Ala Thr Asn Glu Asp His Arg Gln Ile Leu
   645 650 655
   Arg Glu Asn Asn Cys Leu Gln Thr Leu Leu Gln His Leu Lys Ser His
   660 665 670
   Ser Leu Thr Ile Val Ser Asn Ala Cys Gly Thr Leu Trp Asn Leu Ser
   675 680 685
   Ala Arg Asn Pro Lys Asp Gln Glu Ala Leu Trp Asp Met Gly Ala Val
   690 695 700
   Ser Met Leu Lys Asn Leu Ile His Ser Lys His Lys Met Ile Ala Met
   705 710 715 720
   Gly Ser Ala Ala Ala Leu Arg Asn Leu Met Ala Asn Arg Pro Ala Lys
   725 730 735
   Tyr Lys Asp Ala Asn Ile Met Ser Pro Gly Ser Ser Leu Pro Ser Leu
   740 745 750
   His Val Arg Lys Gln Lys Ala Leu Glu Ala Glu Leu Asp Ala Gln His
   755 760 765
   Leu Ser Glu Thr Phe Asp Asn Ile Asp Asn Leu Ser Pro Lys Ala Ser
   770 775 780
   His Arg Ser Lys Gln Arg His Lys Gln Ser Leu Tyr Gly Asp Tyr Val
   785 790 795 800
   Phe Asp Thr Asn Arg His Asp Asp Asn Arg Ser Asp Asn Phe Asn Thr
   805 810 815
   Gly Asn Met Thr Val Leu Ser Pro Tyr Leu Asn Thr Thr Val Leu Pro
   820 825 830
   Ser Ser Ser Ser Ser Arg Gly Ser Leu Asp Ser Ser Arg Ser Glu Lys
   835 840 845
   Asp Arg Ser Leu Glu Arg Glu Arg Gly Ile Gly Leu Gly Asn Tyr His
   850 855 860
   Pro Ala Thr Glu Asn Pro Gly Thr Ser Ser Lys Arg Gly Leu Gln Ile
   865 870 875 880
   Ser Thr Thr Ala Ala Gln Ile Ala Lys Val Met Glu Glu Val Ser Ala
   885 890 895
   Ile His Thr Ser Gln Glu Asp Arg Ser Ser Gly Ser Thr Thr Glu Leu
   900 905 910
   His Cys Val Thr Asp Glu Arg Asn Ala Leu Arg Arg Ser Ser Ala Ala
           915                 920                 925
   His Thr His Ser Asn Thr Tyr Asn Phe Thr Lys Ser Glu Asn Ser Asn
       930                 935                 940
   Arg Thr Cys Ser Met Pro Tyr Ala Lys Leu Glu Tyr Lys Arg Ser Ser
   945                 950                 955                 960
   Asn Asp Ser Leu Asn Ser Val Ser Ser Ser Asp Gly Tyr Gly Lys Arg
                   965                 970                 975
   Gly Gln Met Lys Pro Ser Ile Glu Ser Tyr Ser Glu Asp Asp Glu Ser
               980                 985                 990
   Lys Phe Cys Ser Tyr Gly Gln Tyr Pro Ala Asp Leu Ala His Lys Ile
           995                 1000                1005
   His Ser Ala Asn His Met Asp Asp Asn Asp Gly Glu Leu Asp Thr Pro
       1010                1015                1020
   Ile Asn Tyr Ser Leu Lys Tyr Ser Asp Glu Gln Leu Asn Ser Gly Arg
   1025                1030                1035                1040
   Gln Ser Pro Ser Gln Asn Glu Arg Trp Ala Arg Pro Lys His Ile Ile
                   1045                1050                1055
   Glu Asp Glu Ile Lys Gln Ser Glu Gln Arg Gln Ser Arg Asn Gln Ser
               1060                1065                1070
   Thr Thr Tyr Pro Val Tyr Thr Glu Ser Thr Asp Asp Lys His Leu Lys
           1075                1080                1085
   Phe Gln Pro His Phe Gly Gln Gln Glu Cys Val Ser Pro Tyr Arg Ser
       1090                1095                1100
   Arg Gly Ala Asn Gly Ser Glu Thr Asn Arg Val Gly Ser Asn His Gly
   1105                1110                1115                1120
   Ile Asn Gln Asn Val Ser Gln Ser Leu Cys Gln Glu Asp Asp Tyr Glu
                   1125                1130                1135
   Asp Asp Lys Pro Thr Asn Tyr Ser Glu Arg Tyr Ser Glu Glu Glu Gln
               1140                1145                1150
   His Glu Glu Glu Glu Arg Pro Thr Asn Tyr Ser Ile Lys Tyr Asn Glu
           1155                1160                1165
   Glu Lys Arg His Val Asp Gln Pro Ile Asp Tyr Ser Leu Lys Tyr Ala
       1170                1175                1180
   Thr Asp Ile Pro Ser Ser Gln Lys Gln Ser Phe Ser Phe Ser Lys Ser
   1185                1190                1195                1200
   Ser Ser Gly Gln Ser Ser Lys Thr Glu His Met Ser Ser Ser Ser Glu
                   1205                1210                1215
   Asn Thr Ser Thr Pro Ser Ser Asn Ala Lys Arg Gln Asn Gln Leu His
               1220                1225                1230
   Pro Ser Ser Ala Gln Ser Arg Ser Gly Gln Pro Gln Lys Ala Ala Thr
           1235                1240                1245
   Cys Lys Val Ser Ser Ile Asn Gln Glu Thr Ile Gln Thr Tyr Cys Val
       1250                1255                1260
   Glu Asp Thr Pro Ile Cys Phe Ser Arg Cys Ser Ser Leu Ser Ser Leu
   1265                1270                1275                1280
   Ser Ser Ala Glu Asp Glu Ile Gly Cys Asn Gln Thr Thr Gln Glu Ala
                   1285                1290                1295
   Asp Ser Ala Asn Thr Leu Gln Ile Ala Glu Ile Lys Glu Lys Ile Gly
               1300                1305                1310
   Thr Arg Ser Ala Glu Asp Pro Val Ser Glu Val Pro Ala Val Ser Gln
           1315                1320                1325
   His Pro Arg Thr Lys Ser Ser Arg Leu Gln Gly Ser Ser Leu Ser Ser
       1330                1335                1340
   Glu Ser Ala Arg His Lys Ala Val Glu Phe Ser Ser Gly Ala Lys Ser
   1345                1350                1355                1360
   Pro Ser Lys Ser Gly Ala Gln Thr Pro Lys Ser Pro Pro Glu His Tyr
                   1365                1370                1375
   Val Gln Glu Thr Pro Leu Met Phe Ser Arg Cys Thr Ser Val Ser Ser
               1380                1385                1390
   Leu Asp Ser Phe Glu Ser Arg Ser Ile Ala Ser Ser Val Gln Ser Glu
           1395                1400                1405
   Pro Cys Ser Gly Met Val Ser Gly Ile Ile Ser Pro Ser Asp Leu Pro
   1410 1415 1420
   Asp Ser Pro Gly Gln Thr Met Pro Pro Ser Arg Ser Lys Thr Pro Pro
   1425 1430 1435 1440
   Pro Pro Pro Gln Thr Ala Gln Thr Lys Arg Glu Val Pro Lys Asn Lys
   1445 1450 1455
   Ala Pro Thr Ala Glu Lys Arg Glu Ser Gly Pro Lys Gln Ala Ala Val
   1460 1465 1470
   Asn Ala Ala Val Gln Arg Val Gln Val Leu Pro Asp Ala Asp Thr Leu
   1475 1480 1485
   Leu His Phe Ala Thr Glu Ser Thr Pro Asp Gly Phe Ser Cys Ser Ser
   1490 1495 1500
   Ser Leu Ser Ala Leu Ser Leu Asp Glu Pro Phe Ile Gln Lys Asp Val
   1505 1510 1515 1520
   Glu Leu Arg Ile Met Pro Pro Val Gln Glu Asn Asp Asn Gly Asn Glu
   1525 1530 1535
   Thr Glu Ser Glu Gln Pro Lys Glu Ser Asn Glu Asn Gln Glu Lys Glu
   1540 1545 1550
   Ala Glu Lys Thr Ile Asp Ser Glu Lys Asp Leu Leu Asp Asp Ser Asp
   1555 1560 1565
   Asp Asp Asp Ile Glu Ile Leu Glu Glu Cys Ile Ile Ser Ala Met Pro
   1570 1575 1580
   Thr Lys Ser Ser Arg Lys Ala Lys Lys Pro Ala Gln Thr Ala Ser Lys
   1585 1590 1595 1600
   Leu Pro Pro Pro Val Ala Arg Lys Pro Ser Gln Leu Pro Val Tyr Lys
   1605 1610 1615
   Leu Leu Pro Ser Gln Asn Arg Leu Gln Pro Gln Lys His Val Ser Phe
   1620 1625 1630
   Thr Pro Gly Asp Asp Met Pro Arg Val Tyr Cys Val Glu Gly Thr Pro
   1635 1640 1645
   Ile Asn Phe Ser Thr Ala Thr Ser Leu Ser Asp Leu Thr Ile Glu Ser
   1650 1655 1660
   Pro Pro Asn Glu Leu Ala Ala Gly Glu Gly Val Arg Gly Gly Ala Gln
   1665 1670 1675 1680
   Ser Gly Glu Phe Glu Lys Arg Asp Thr Ile Pro Thr Glu Gly Arg Ser
   1685 1690 1695
   Thr Asp Glu Ala Gln Gly Gly Lys Thr Ser Ser Val Thr Ile Pro Glu
   1700 1705 1710
   Leu Asp Asp Asn Lys Ala Glu Glu Gly Asp Ile Leu Ala Glu Cys Ile
   1715 1720 1725
   Asn Ser Ala Met Pro Lys Gly Lys Ser His Lys Pro Phe Arg Val Lys
   1730 1735 1740
   Lys Ile Met Asp Gln Val Gln Gln Ala Ser Ala Ser Ser Ser Ala Pro
   1745 1750 1755 1760
   Asn Lys Asn Gln Leu Asp Gly Lys Lys Lys Lys Pro Thr Ser Pro Val
   1765 1770 1775
   Lys Pro Ile Pro Gln Asn Thr Glu Tyr Arg Thr Arg Val Arg Lys Asn
   1780 1785 1790
   Ala Asp Ser Lys Asn Asn Leu Asn Ala Glu Arg Val Phe Ser Asp Asn
   1795 1800 1805
   Lys Asp Ser Lys Lys Gln Asn Leu Lys Asn Asn Ser Lys Val Phe Asn
   1810 1815 1820
   Asp Lys Leu Pro Asn Asn Glu Asp Arg Val Arg Gly Ser Phe Ala Phe
   1825 1830 1835 1840
   Asp Ser Pro His His Tyr Thr Pro Ile Glu Gly Thr Pro Tyr Cys Phe
   1845 1850 1855
   Ser Arg Asn Asp Ser Leu Ser Ser Leu Asp Phe Asp Asp Asp Asp Val
   1860 1865 1870
   Asp Leu Ser Arg Glu Lys Ala Glu Leu Arg Lys Ala Lys Glu Asn Lys
   1875 1880 1885
   Glu Ser Glu Ala Lys Val Thr Ser His Thr Glu Leu Thr Ser Asn Gln
   1890 1895 1900
   Gln Ser Ala Asn Lys Thr Gln Ala Ile Ala Lys Gln Pro Ile Asn Arg
   1905 1910 1915 1920
   Gly Gln Pro Lys Pro Ile Leu Gln Lys Gln Ser Thr Phe Pro Gln Ser
   1925 1930 1935
   Ser Lys Asp Ile Pro Asp Arg Gly Ala Ala Thr Asp Glu Lys Leu Gln
   1940 1945 1950
   Asn Phe Ala Ile Glu Asn Thr Pro Val Cys Phe Ser His Asn Ser Ser
   1955 1960 1965
   Leu Ser Ser Leu Ser Asp Ile Asp Gln Glu Asn Asn Asn Lys Glu Asn
   1970 1975 1980
   Glu Pro Ile Lys Glu Thr Glu Pro Pro Asp Ser Gln Gly Glu Pro Ser
   1985 1990 1995 2000
   Lys Pro Gln Ala Ser Gly Tyr Ala Pro Lys Ser Phe His Val Glu Asp
   2005 2010 2015
   Thr Pro Val Cys Phe Ser Arg Asn Ser Ser Leu Ser Ser Leu Ser Ile
   2020 2025 2030
   Asp Ser Glu Asp Asp Leu Leu Gln Glu Cys Ile Ser Ser Ala Met Pro
   2035 2040 2045
   Lys Lys Lys Lys Pro Ser Arg Leu Lys Gly Asp Asn Glu Lys His Ser
   2050 2055 2060
   Pro Arg Asn Met Gly Gly Ile Leu Gly Glu Asp Leu Thr Leu Asp Leu
   2065 2070 2075 2080
   Lys Asp Ile Gln Arg Pro Asp Ser Glu His Gly Leu Ser Pro Asp Ser
   2085 2090 2095
   Glu Asn Phe Asp Trp Lys Ala Ile Gln Glu Gly Ala Asn Ser Ile Val
   2100 2105 2110
   Ser Ser Leu His Gln Ala Ala Ala Ala Ala Cys Leu Ser Arg Gln Ala
   2115 2120 2125
   Ser Ser Asp Ser Asp Ser Ile Leu Ser Leu Lys Ser Gly Ile Ser Leu
   2130 2135 2140
   Gly Ser Pro Phe His Leu Thr Pro Asp Gln Glu Glu Lys Pro Phe Thr
   2145 2150 2155 2160
   Ser Asn Lys Gly Pro Arg Ile Leu Lys Pro Gly Glu Lys Ser Thr Leu
   2165 2170 2175
   Glu Thr Lys Lys Ile Glu Ser Glu Ser Lys Gly Ile Lys Gly Gly Lys
   2180 2185 2190
   Lys Val Tyr Lys Ser Leu Ile Thr Gly Lys Val Arg Ser Asn Ser Glu
   2195 2200 2205
   Ile Ser Gly Gln Met Lys Gln Pro Leu Gln Ala Asn Met Pro Ser Ile
   2210 2215 2220
   Ser Arg Gly Arg Thr Met Ile His Ile Pro Gly Val Arg Asn Ser Ser
   2225 2230 2235 2240
   Ser Ser Thr Ser Pro Val Ser Lys Lys Gly Pro Pro Leu Lys Thr Pro
   2245 2250 2255
   Ala Ser Lys Ser Pro Ser Glu Gly Gln Thr Ala Thr Thr Ser Pro Arg
   2260 2265 2270
   Gly Ala Lys Pro Ser Val Lys Ser Glu Leu Ser Pro Val Ala Arg Gln
   2275 2280 2285
   Thr Ser Gln Ile Gly Gly Ser Ser Lys Ala Pro Ser Arg Ser Gly Ser
   2290 2295 2300
   Arg Asp Ser Thr Pro Ser Arg Pro Ala Gln Gln Pro Leu Ser Arg Pro
   2305 2310 2315 2320
   Ile Gln Ser Pro Gly Arg Asn Ser Ile Ser Pro Gly Arg Asn Gly Ile
   2325 2330 2335
   Ser Pro Pro Asn Lys Leu Ser Gln Leu Pro Arg Thr Ser Ser Pro Ser
   2340 2345 2350
   Thr Ala Ser Thr Lys Ser Ser Gly Ser Gly Lys Met Ser Tyr Thr Ser
   2355 2360 2365
   Pro Gly Arg Gln Met Ser Gln Gln Asn Leu Thr Lys Gln Thr Gly Leu
   2370 2375 2380
   Ser Lys Asn Ala Ser Ser Ile Pro Arg Ser Glu Ser Ala Ser Lys Gly
   2385 2390 2395 2400
   Leu Asn Gln Met Asn Asn Gly Asn Gly Ala Asn Lys Lys Val Glu Leu
   2405 2410 2415
   Ser Arg Met Ser Ser Thr Lys Ser Ser Gly Ser Glu Ser Asp Arg Ser
                   2420                2425                2430
   Glu Arg Pro Val Leu Val Arg Gln Ser Thr Phe Ile Lys Glu Ala Pro
               2435                2440                2445
   Ser Pro Thr Leu Arg Arg Lys Leu Glu Glu Ser Ala Ser Phe Glu Ser
           2450                2455                2460
   Leu Ser Pro Ser Ser Arg Pro Ala Ser Pro Thr Arg Ser Gln Ala Gln
   2465                2470                2475                2480
   Thr Pro Val Leu Ser Pro Ser Leu Pro Asp Met Ser Leu Ser Thr His
                       2485                2490                2495
   Ser Ser Val Gln Ala Gly Gly Trp Arg Lys Leu Pro Pro Asn Leu Ser
                   2500                2505                2510
   Pro Thr Ile Glu Tyr Asn Asp Gly Arg Pro Ala Lys Arg His Asp Ile
               2515                2520                2525
   Ala Arg Ser His Ser Glu Ser Pro Ser Arg Leu Pro Ile Asn Arg Ser
           2530                2535                2540
   Gly Thr Trp Lys Arg Glu His Ser Lys His Ser Ser Ser Leu Pro Arg
   2545                2550                2555                2560
   Val Ser Thr Trp Arg Arg Thr Gly Ser Ser Ser Ser Ile Leu Ser Ala
                       2565                2570                2575
   Ser Ser Glu Ser Ser Glu Lys Ala Lys Ser Glu Asp Glu Lys His Val
                   2580                2585                2590
   Asn Ser Ile Ser Gly Thr Lys Gln Ser Lys Glu Asn Gln Val Ser Ala
               2595                2600                2605
   Lys Gly Thr Trp Arg Lys Ile Lys Glu Asn Glu Phe Ser Pro Thr Asn
           2610                2615                2620
   Ser Thr Ser Gln Thr Val Ser Ser Gly Ala Thr Asn Gly Ala Glu Ser
   2625                2630                2635                2640
   Lys Thr Leu Ile Tyr Gln Met Ala Pro Ala Val Ser Lys Thr Glu Asp
                       2645                2650                2655
   Val Trp Val Arg Ile Glu Asp Cys Pro Ile Asn Asn Pro Arg Ser Gly
                   2660                2665                2670
   Arg Ser Pro Thr Gly Asn Thr Pro Pro Val Ile Asp Ser Val Ser Glu
               2675                2680                2685
   Lys Ala Asn Pro Asn Ile Lys Asp Ser Lys Asp Asn Gln Ala Lys Gln
           2690                2695                2700
   Asn Val Gly Asn Gly Ser Val Pro Met Arg Thr Val Gly Leu Glu Asn
   2705                2710                2715                2720
   Arg Leu Asn Ser Phe Ile Gln Val Asp Ala Pro Asp Gln Lys Gly Thr
                       2725                2730                2735
   Glu Ile Lys Pro Gly Gln Asn Asn Pro Val Pro Val Ser Glu Thr Asn
                   2740                2745                2750
   Glu Ser Ser Ile Val Glu Arg Thr Pro Phe Ser Ser Ser Ser Ser Ser
               2755                2760                2765
   Lys His Ser Ser Pro Ser Gly Thr Val Ala Ala Arg Val Thr Pro Phe
           2770                2775                2780
   Asn Tyr Asn Pro Ser Pro Arg Lys Ser Ser Ala Asp Ser Thr Ser Ala
   2785                2790                2795                2800
   Arg Pro Ser Gln Ile Pro Thr Pro Val Asn Asn Asn Thr Lys Lys Arg
                       2805                2810                2815
   Asp Ser Lys Thr Asp Ser Thr Glu Ser Ser Gly Thr Gln Ser Pro Lys
                   2820                2825                2830
   Arg His Ser Gly Ser Tyr Leu Val Thr Ser Val
               2835                2840
<210> 7
   <211> 3834
   <212> DNA
   <213> Homo sapiens
<400> 7
   gaggagagag ggcagcagcg cgcggtgtct ccggctgctc agtccgaccg cggcaagcaa 60
   gcgggcaggc gcaccgcccc ctcccccgcc cggcctcccc aactctgcgg ccgcgagtaa 120
   agtttgcaaa gaggcgcggg aggcggcagc cgcagcgagg aggcggcggg gaagaagcgc 180
   agtctccggg ttgggggcgg gggcgggggg ggcgccaagg agccgggtgg ggggcggcgg 240
   ccagcatgcg gccccgcagc gccctgcccc gcctgctgct gccgctgctg ctgctgcccg 300
   ccgccgggcc ggcccagttc cacggggaga agggcatctc catcccggac cacggcttct 360
   gccagcccat ctccatcccg ctgtgcacgg acatcgccta caaccagacc atcatgccca 420
   accttctggg ccacacgaac caggaggacg caggcctaga ggtgcaccag ttctatccgc 480
   tggtgaaggt gcagtgctcg cccgaactgc gcttcttcct gtgctccatg tacgcacccg 540
   tgtgcaccgt gctggaacag gccatcccgc cgtgccgctc tatctgtgag cgcgcgcgcc 600
   agggctgcga agccctcatg aacaagttcg gttttcagtg gcccgagcgc ctgcgctgcg 660
   agcacttccc gcgccacggc gccgagcaga tctgcgtcgg ccagaaccac tccgaggacg 720
   gagctcccgc gctactcacc accgcgccgc cgccgggact gcagccgggt gccgggggca 780
   ccccgggtgg cccgggcggc ggcggcgctc ccccgcgcta cgccacgctg gagcacccct 840
   tccactgccc gcgcgtcctc aaggtgccat cctatctcag ctacaagttt ctgggcgagc 900
   gtgattgtgc tgcgccctgc gaacctgcgc ggcccgatgg ttccatgttc ttctcacagg 960
   aggagacgcg tttcgcgcgc ctctggatcc tcacctggtc ggtgctgtgc tgcgcttcca 1020
   ccttcttcac tgtcaccacg tacttggtag acatgcagcg cttccgctac ccagagcggc 1080
   ctatcatttt tctgtcgggc tgctacacca tggtgtcggt ggcctacatc gcgggcttcg 1140
   tgctccagga gcgcgtggtg tgcaacgagc gcttctccga ggacggttac cgcacggtgg 1200
   tgcagggcac caagaaggag ggctgcacca tcctcttcat gatgctctac ttcttcagca 1260
   tggccagctc catctggtgg gtcatcctgt cgctcacctg gttcctggca gccggcatga 1320
   agtggggcca cgaggccatc gaggccaact ctcagtactt ccacctggcc gcctgggccg 1380
   tgccggccgt caagaccatc accatcctgg ccatgggcca gatcgacggc gacctgctga 1440
   gcggcgtgtg cttcgtaggc ctcaacagcc tggacccgct gcggggcttc gtgctagcgc 1500
   cgctcttcgt gtacctgttc atcggcacgt ccttcctcct ggccggcttc gtgtcgctct 1560
   tccgcatccg caccatcatg aagcacgacg gcaccaagac cgaaaagctg gagcggctca 1620
   tggtgcgcat cggcgtcttc tccgtgctct acacagtgcc cgccaccatc gtcatcgctt 1680
   gctacttcta cgagcaggcc ttccgcgagc actgggagcg ctcgtgggtg agccagcact 1740
   gcaagagcct ggccatcccg tgcccggcgc actacacgcc gcgcatgtcg cccgacttca 1800
   cggtctacat gatcaaatac ctcatgacgc tcatcgtggg catcacgtcg ggcttctgga 1860
   tctggtcggg caagacgctg cactcgtgga ggaagttcta cactcgcctc accaacagcc 1920
   gacacggtga gaccaccgtg tgagggacgc ccccaggccg gaaccgcgcg gcgctttcct 1980
   ccgcccgggg tggggcccct acagactccg tattttattt ttttaaataa aaaacgatcg 2040
   aaaccatttc acttttaggt tgctttttaa aagagaactc tctgcccaac acccccacaa 2100
   ggtttgtaat taaaactgta aatagtcttt gtaaatttaa ttatatatat tttctattta 2160
   aaagaaaaaa ggagaaaaaa aaacaggggt gtgggcgcca ggactgaaga atgaggtgtg 2220
   tgtgtgttgg ggagtgtagt tggggggagg gttctctttc ttaagtgccc ttcaaaaccc 2280
   gccccacttt tggagttttt tggtgatatg gcagggctgg gcctgatggg agaggcaccc 2340
   agcctgagcg ctgtttttgt tggctttgag tcgttaggag tttgttccat tcaactaata 2400
   taaaagccaa atttgtgagc ctcctctctg acgctgggcc tgtggaagcc gttggatatt 2460
   tttgaacagg acttggattc gttttgtttc cttccccctt ttctttccct actcatttgt 2520
   cctgtcttct tcactcactc ttggaaaagt cccaacagag atgaagacgt ggaaaaaaaa 2580
   atcggggtcg gggtgtgctg gtggggagag ggcagagatc cggtgaggaa tggcttccac 2640
   cccctggccc attcccctgc aggctggaag atctttctcc tgtctggctt ctcttctttt 2700
   caattcgctg caccaagtgc ttccagtggc ccaaaaatgc tttttgaagt gtgttttgaa 2760
   acagccccca ccaacataca ccccaccagg agtactgatc ctgcctccct tcatgtctag 2820
   gggaagcatt cgcctttgag cacttgtttg caaatctggg gagtttgaga cctcctagca 2880
   tctcttccct tctttccctg cagtctattc actcccgcag ccaaaaatct ctggcgttca 2940
   ggttagcagt ttctgggttg gtttgtgtgg gtttttgttg ttgtttgggg cttatttttt 3000
   tcaaaaagtg ataaagacgg gtgggttgga gggaggggac tgatgggctg gtgggctttt 3060
   tagttttcct ttgagaaaaa gactggttta tttaaaattt gtccagaaaa aaatgaaaaa 3120
   aaaaaaaaag gtggtatctt tgctttagaa gaagtctctg gataggccct ttgtggctgt 3180
   gggggtgggt tgagtgttta cattacattc tatatcacaa gattgatagg atgtttaaag 3240
   cagatgtttc ttatcttccc ctgagcccct acaaataaag tcaagacttt tctttttttg 3300
   agacggagtc ttgctctgtc gcccaggctg gagtgcagtg gtgcgatctt ggctcactgc 3360
   aacctccact gcagcgtttc tcctgcctca gcctcccaag tagctgggac tacaggcgca 3420
   cgccaccact ccttgctaat ttttgtattt ttagtagaca cagggtttca ccatattggc 3480
   caggctggtc tcgaactcct gacctcgtta tctgcctgcc tcggcctcct aaagtgctgg 3540
   gattacagga gtgagccaca gtgcctggcc tgtcaagact tctcttaagt taacttcctg 3600
   agaagtgatg tctaaaagta tctttgctgg tgtgagaact ccagtttcca acacatatta 3660
   tttccctcaa ctatttggaa tattttagaa ttttaattcc aaaggattag tttgaataca 3720
   agtatgccac ataactcagt tttcgccatc ttccatttct taacagtgta aattaaaagc 3780
   taataatcat aataataaag tgcatttaat tatctttgaa aaaaaaaaaa aaaa       3834
<210> 8
   <211> 565
   <212> PRT
   <213> Homo sapiens
<400> 8
<210> 9
   <211> 8844
   <212> DNA
   <213> Homo sapiens
<400> 9
<210> 10
   <211> 688
   <212> PRT
   <213> Homo sapiens
<400> 10
   Met Ala Asp Val Phe Pro Gly Asn Asp Ser Thr Ala Ser Gln Asp Val
    1               5                  10                  15
   Ala Asn Arg Phe Ala Arg Lys Gly Ala Leu Arg Gln Lys Asn Val His
               20                  25                  30
   Glu Val Lys Asp His Lys Phe Ile Ala Arg Phe Phe Lys Gln Pro Thr
           35                  40                  45
   Phe Cys Ser His Cys Thr Asp Phe Ile Trp Gly Phe Gly Lys Gln Gly
       50                  55                  60
   Phe Gln Cys Gln Val Cys Cys Phe Val Val His Lys Arg Cys His Glu
   65                  70                  75                  80
   Phe Val Thr Phe Ser Cys Pro Gly Ala Asp Lys Gly Pro Asp Thr Asp
                   85                  90                  95
   Asp Pro Arg Ser Lys His Lys Phe Lys Ile His Thr Tyr Gly Ser Pro
               100                 105                 110
   Thr Phe Cys Asp His Cys Gly Ser Leu Leu Tyr Gly Leu Ile His Gln
           115                 120                 125
   Gly Met Lys Cys Asp Thr Cys Asp Met Asn Val His Lys Gln Cys Val
       130                 135                 140
   Ile Asn Val Pro Ser Leu Cys Gly Met Asp His Thr Glu Lys Arg Gly
   145                 150                 155                 160
   Arg Ile Tyr Leu Lys Ala Glu Val Ala Asp Glu Lys Leu His Val Thr
                   165                 170                 175
   Val Arg Asp Ala Lys Asn Leu Ile Pro Met Asp Pro Asn Gly Leu Ser
               180                 185                 190
   Asp Pro Tyr Val Lys Leu Lys Leu Ile Pro Asp Pro Lys Asn Glu Ser
           195                 200                 205
   Lys Gln Lys Thr Lys Thr Ile Arg Ser Thr Leu Asn Pro Gln Trp Asn
       210                 215                 220
   Glu Ser Phe Thr Phe Lys Leu Lys Pro Ser Asp Lys Asp Arg Arg Leu
   225                 230                 235                 240
   Ser Val Glu Ile Trp Asp Trp Asp Arg Thr Thr Arg Asn Asp Phe Met
                   245                 250                 255
   Gly Ser Leu Ser Phe Gly Val Ser Glu Leu Met Lys Met Pro Ala Ser
               260                 265                 270
   Gly Trp Tyr Lys Leu Leu Asn Gln Glu Glu Gly Glu Tyr Tyr Asn Val
           275                 280                 285
   Pro Ile Pro Glu Gly Asp Glu Glu Gly Asn Met Glu Leu Arg Gln Lys
       290                 295                 300
   Phe Glu Lys Ala Lys Leu Gly Pro Ala Gly Asn Lys Val Ile Ser Pro
   305                 310                 315                 320
   Ser Glu Asp Arg Lys Gln Pro Ser Asn Asn Leu Asp Arg Val Lys Leu
                   325                 330                 335
   Thr Asp Phe Asn Phe Leu Met Val Leu Gly Lys Gly Ser Phe Gly Lys
               340                 345                 350
   Val Met Leu Ala Asp Arg Lys Gly Thr Glu Glu Leu Tyr Ala Ile Lys
           355                 360                 365
   Ile Leu Lys Lys Asp Val Val Ile Gln Asp Asp Asp Val Glu Cys Thr
       370                 375                 380
   Met Val Glu Lys Arg Val Leu Ala Leu Leu Asp Lys Pro Pro Phe Leu
   385                 390                 395                 400
   Thr Gln Leu His Ser Cys Phe Gln Thr Val Asp Arg Leu Tyr Phe Val
                   405                 410                 415
   Met Glu Tyr Val Asn Gly Gly Asp Leu Met Tyr His Ile Gln Gln Val
               420                 425                 430
   Gly Lys Phe Lys Glu Pro Gln Ala Val Phe Tyr Ala Ala Glu Ile Ser
           435                 440                 445
   Ile Gly Leu Phe Phe Leu His Lys Arg Gly Ile Ile Tyr Arg Asp Leu
       450                 455                 460
   Lys Leu Asp Asn Val Met Leu Asp Ser Glu Gly His Ile Lys Ile Ala
   465                 470                 475                 480
   Asp Phe Gly Met Cys Lys Glu His Met Met Asp Gly Val Thr Thr Arg
                   485                 490                 495
   Thr Phe Cys Gly Thr Pro Asp Tyr Ile Ala Pro Glu Ile Ile Ala Tyr
               500                 505                 510
   Gln Pro Tyr Gly Lys Ser Val Asp Trp Trp Ala Tyr Gly Val Leu Leu
           515                 520                 525
   Tyr Glu Met Leu Ala Gly Gln Pro Pro Phe Asp Gly Glu Asp Glu Asp
       530                 535                 540
   Glu Leu Phe Gln Ser Ile Met Glu His Asn Val Ser Tyr Pro Lys Ser
   545                 550                 555                 560
   Leu Ser Lys Glu Ala Val Ser Val Cys Lys Gly Leu Met Thr Lys His
                   565                 570                 575
   Pro Ala Lys Arg Leu Gly Cys Gly Pro Glu Gly Glu Arg Asp Val Arg
               580                 585                 590
   Glu His Ala Phe Phe Arg Arg Ile Asp Trp Glu Lys Leu Glu Asn Arg
           595                 600                 605
   Glu Ile Gln Pro Pro Phe Lys Pro Lys Val Thr Leu Cys Thr Lys Met
       610                 615                 620
   His Trp Leu Gln Trp Ala Ser Arg Ser Ser Cys Gly Lys Gly Ala Glu
   625                 630                 635                 640
   Asn Phe Asp Lys Phe Phe Thr Arg Gly Gln Pro Val Leu Thr Pro Pro
                   645                 650                 655
   Asp Gln Leu Val Ile Ala Asn Ile Asp Gln Ser Asp Phe Glu Gly Phe
               660                 665                 670
   Ser Tyr Val Asn Pro Gln Phe Val His Pro Ile Leu Gln Ser Ala Val
           675                 680                 685
<210> 11
   <211> 10974
   <212> DNA
   <213> Homo sapiens
<400> 11
   gattcacagg gcctctgagc attatccccc atactcctcc ccatcattct ccacccagct 60
   gttggagcca tctgtctgat caccttggac tccatagtac actggggcaa agcacagccc 120
   cagtttctgg aggcagatgg gtaaccagga aaaggcatga atgagggggc cccaggagac 180
   agtgacttag agactgaggc aagagtgccg tggtcaatca tgggtcattg tcttcgaact 240
   ggacaggcca gaatgtctgc cacacccaca cctgcaggtg aaggagccag aagctcttca 300
   acctgtagct ccctgagcag gctgttctgg tctcaacttg agcacataaa ctgggatgga 360
   gccacagcca agaactttat taatttaagg gagttcttct cttttctgct ccctgcattg 420
   agaaaagctc aaattgaaat tattccatgc aagatctgtg gagacaaatc atcaggaatc 480
   cattatggtg tcattacatg tgaaggctgc aagggctttt tcaggagaag tcagcaaagc 540
   aatgccacct actcctgtcc tcgtcagaag aactgtttga ttgatcgaac cagtagaaac 600
   cgctgccaac actgtcgatt acagaaatgc cttgccgtag ggatgtctcg agatgctgta 660
   aaatttggcc gaatgtcaaa aaagcagaga gacagcttgt atgcagaagt acagaaacac 720
   cggatgcagc agcagcagcg cgaccaccag cagcagcctg gagaggctga gccgctgacg 780
   cccacctaca acatctcggc caacgggctg acggaacttc acgacgacct cagtaactac 840
   attgacgggc acacccctga ggggagtaag gcagactccg ccgtcagcag cttctacctg 900
   gacatacagc cttccccaga ccagtcaggt cttgatatca atggaatcaa accagaacca 960
   atatgtgact acacaccagc atcaggcttc tttccctact gttcgttcac caacggcgag 1020
   acttccccaa ctgtgtccat ggcagaatta gaacaccttg cacagaatat atctaaatcg 1080
   catctggaaa cctgccaata cttgagagaa gagctccagc agataacgtg gcagaccttt 1140
   ttacaggaag aaattgagaa ctatcaaaac aagcagcggg aggtgatgtg gcaattgtgt 1200
   gccatcaaaa ttacagaagc tatacagtat gtggtggagt ttgccaaacg cattgatgga 1260
   tttatggaac tgtgtcaaaa tgatcaaatt gtgcttctaa aagcaggttc tctagaggtg 1320
   gtgtttatca gaatgtgccg tgcctttgac tctcagaaca acaccgtgta ctttgatggg 1380
   aagtatgcca gccccgacgt cttcaaatcc ttaggttgtg aagactttat tagctttgtg 1440
   tttgaatttg gaaagagttt atgttctatg cacctgactg aagatgaaat tgcattattt 1500
   tctgcatttg tactgatgtc agcagatcgc tcatggctgc aagaaaaggt aaaaattgaa 1560
   aaactgcaac agaaaattca gctagctctt caacacgtcc tacagaagaa tcaccgagaa 1620
   gatggaatac taacaaagtt aatatgcaag gtgtctacat taagagcctt atgtggacga 1680
   catacagaaa agctaatggc atttaaagca atatacccag acattgtgcg acttcatttt 1740
   cctccattat acaaggagtt gttcacttca gaatttgagc cagcaatgca aattgatggg 1800
   taaatgttat cacctaagca cttctagaat gtctgaagta caaacatgaa aaacaaacaa 1860
   aaaaattaac cgagacactt tatatggccc tgcacagacc tggagcgcca cacactgcac 1920
   atcttttggt gatcggggtc aggcaaagga ggggaaacaa tgaaaacaaa taaaagttga 1980
   acttgttttt ctcatgcata tgatttccat tatgcctaca gatatggacc ctttttctgt 2040
   cttgacttct tgatcattga cctctgttta caacaggagg agggtactaa agtcggagga 2100
   tttccttttc ttgtagctca ctgcccacag actttctaca gagtcaccaa tctgtcagta 2160
   acaacagaga gtccagcaat aatcggtgac tggtgtgcat agcggaggtt gcggcattac 2220
   tttgcacaac tagctctttg tttcatgaag gaagttttta ttttttcacc gattattgcc 2280
   agtccgcagg atggcatgaa aagggtccat agcagtagca acaatagcat tataatatat 2340
   tacagggtaa atgggcatga agactatata tagctaaaag agatattgtt tatatattgt 2400
   tttaagtaat ataaaatgta gttactggtg tagcttttcc tgttgaattg ataaggcact 2460
   ttcattttgc acctttttct ttaaattaaa tgctagcgtg ttcactgtcg tgtcgcatgt 2520
   gcaccagaaa cacaagttta actgagaagg cttggaaggt acgttgggag gtatttatgc 2580
   tgctgtttac aaaattattt ttaagagact ggctggtcat atctagaaat caccacgttg 2640
   gatttttttt ttaacatgtg aatttggaat tagaaacgga actctcccta aattatactt 2700
   tgctttttgg taagtttaat gatagatgtg tttatgcttc atacaaagtt gaatgattga 2760
   ttggcgtggt ggacatatac catcatgctc attttttttt tttaaagctt tttaaaatgc 2820
   cacctcatgg aggcgagggg gaggagaagc tcattttaca caattcagta gttaaatatg 2880
   gactcggtct caacttggaa ttcttatgct ttgagaacaa atcaacaacc agaatattta 2940
   ttggaatcta gcttttatta taagaaggac ccaaagatta tatcctgagc aaatgcacac 3000
   tccccatgtg aggacatgaa gtatttactt tgtgaatgtt tatgttcttg gtataatcta 3060
   ggaaccctat gagtttatct cagagtgaac taattctaga tttgttgtca atagatgcta 3120
   taattcaaga atgttgctct ccatatttga aaaacgatgg ataggagggt gagggaagca 3180
   tacaatgttg aaccagtttc tcttatttaa atattaaata ctttaagcct ttaaagtgaa 3240
   gttgatgcta gctgcaaaca tttactcttg tatttatctt cactaggaaa ctgtggactg 3300
   taatttattt tattaaatat ttagaagatt atttggcttg tgtgttcagg tgagaaatac 3360
   tgagttgttt ttgtttaatt tcatggtttt ttttttttta atgatcccta gtgggggaag 3420
   gggaaaggaa tagtctgata aacagatgtg catattttaa aaacaagtga cctttgggga 3480
   atgtaggcat ttagacgatg attttagtcg cactaggggt gggattcaaa ctactggtca 3540
   aagaccattt tgtacagaaa agggaacatc tccgatgggt gttaaggtag gagtttccat 3600
   gcagcccttt atgtctgaga aatagtctcc tctgccattg gggtccctgc ggaatcttct 3660
   acaggaattg cagctcttca cgtcatgcta ggttaccagc atgggcttcc cagagcactt 3720
   cactctgttt ctaactccac tgacttttct gacttgtttc ttgagggact tggaaaaggg 3780
   gaaggtatta ttcacacaga tgtgtgtatg aagcctaaat aacatccaac tttttttcca 3840
   aaaacatagt aagagtttaa ccataaatag aagagtaata ttcctttaaa tttcttacag 3900
   gcatgtaaaa ttttatgtgt ttatagagag atgctaactg tcagcatata gtatttatat 3960
   tggggcaaga agggttaaat caaatcttta atttaagtaa gcatagttcc tttaaagatc 4020
   agtagtattt atactctgaa aagagtacca agcttagttc agttatttat tcatccattt 4080
   tttttctatt gtttctcctc tggggggaaa tggtgttttg tttttgtttg agttttgtgt 4140
   tttagttttt tggttttggt tttgttttgt ttttttgtca ttcagattca ctaaatttgg 4200
   ggatggtttt attaaagtat attaacttct ttttaaccaa agctttctga atatgaccag 4260
   cctcaggtgc tagcgcatta aagagcaact aaacctaatt ccagtgtcga tttgtgaaat 4320
   aataaaagct aattgaattt ctctaagggt gagagagaac ataatgatga acttaaaaag 4380
   aaattccttt tcccagaagg gattctgcat gtacctacaa acaatcatgc tctaaccaca 4440
   gtaatagtca tgccatggtg acattgcttt tacggtaaac caagataggt aatatgatgc 4500
   ttcttcccag gtgtttctga aagaaaagca gcggtggagc ttaagagcca agtccactga 4560
   ttgacataat aggatggaat tgtagacaga gacatgctcc atgaaacaag gaaacaactg 4620
   actactattt ggatctaagt tgggatctga tgttaaacaa taaattcagt ttaaaaaaaa 4680
   tatggagctc agaaaaggat gtgaaaaact ttgcattttc ctttcttcat tattacaaaa 4740
   acacctatgt gatgacataa aatacttggg tgatatcaga acaattatcc ttaatagttt 4800
   ttataattaa agtttcctaa acttcagtct ccaatagtct tttaaggatt ggaaccacat 4860
   cactgtcagc cccgctgcta caatgccttt gtacaatttt tttacataag gcaaataagg 4920
   cttttgtaca aagcctgaat accttctgtt ccaatggtgt ccaaatggtt attatattgt 4980
   gaaaaacctg gccttggtca caatgcaaac aaaagtacag atgaaagtgc tttttggaca 5040
   gtttgcaaat tgtgttaaag ctacggattt tttttaaagt gttcagcatc ttaacgtgta 5100
   ttaaagctat ggattttttt ttaaagtctt cagcatccta attcaccctt tctaacttaa 5160
   gaaaaacatg acttaagaca ctgcttctaa gtttggttgt tctttatagt gtggataccc 5220
   agttatctgt gagcgtatgg gggtgggctg agggtcaggt gaggaaggag tgtgtgtgtg 5280
   tgtgtgtgtg tgtgtgtgtg tgtgtgtgat ttgcatgtgt atgatgtgtg tgcgtcggac 5340
   cgcttctagg ctactaagtg tcaatggaaa agaaaatgta ttcaaaatac ttaaatcaaa 5400
   actagaagat ggggaaaaaa agatttattc tatacaaagc cttgtctgga ccactttaga 5460
   gagacttcta tttttttaac ccttctataa atatttgatg gcacttgaaa tattcctgca 5520
   ataaaatgtg atttgtgtaa agaaaaaaag attttgtaat gtgaaacaaa gaaagaaagt 5580
   aatgtaattt tctaaaaaaa aaaatacaaa caaacaaact ttgtattatt ttcttgatgg 5640
   aatttgtcta tctgtctttg gaaaactttt tatttcattg aatgtgccat agtagaaatg 5700
   tgtgttttta gttttagact aaggaatagc tagttgttgt gttccgacat tccaaaatgc 5760
   aaaacaacct agtagtatct ttcatgaaaa ggtttaagta gtatgtaagc ttctctcatt 5820
   gttgcttttt tgcacatgtt cttcattcct ctctagtgca atatgtacat agagcacttg 5880
   cgggtgtacc ttgatccctc agggaaaaat acatatttgt acagtttttt ggggtttttt 5940
   tgttttttgg ttttttttgt tttgcctttt gtttttggct aaggaatgtc gatcgaatca 6000
   cttgttattg ttgaggggca gccagataat aatcctaaag ccactgtttc caacattgat 6060
   tgtttaaatc atatgtcctt ccaatgctat tattttaaga taataataaa aagttatttt 6120
   ctgacagttc tttgtgctga ctggtgaaaa acaagggtaa ataagcacct tataattgac 6180
   ttactgtgaa tgacaatcca tcttggtatc aacgatagaa gccctatcat tttggagttg 6240
   gggttaagag tcagaaacaa tgtgctcagg gatctcctaa aactcttaaa acagggtggc 6300
   cagtactact gggacaaatt gtgtttttta ttattaataa taatgataat aatactatcc 6360
   ctccaaggca caagtgaact atatagaact gcgtgtgtgt aaactcttta ctctcgtctc 6420
   attttgttga gtttagaact tgatgtgctc gtcagtcttg tgtttcaaaa cactgaataa 6480
   cttccaaagc aaagttatgc cagtgtgttc aaaagataaa ttaataatgt accagcaaag 6540
   agcatcattc aaagtatagt ccttgcatat tccagttacc atttctctaa taattaaaat 6600
   attcatgata aatatatata tagcatcata tgttaaaaac tatttcaaat tctacatatt 6660
   aaggatgaaa attttaaaat ccagtaataa gaggagagac ctgcctatca gtacagtgat 6720
   ataggtaaaa gatgaaatat gtttttaaaa taccagcaat taactattgt tttcatgggt 6780
   tctcctctag aagcaaacca aaaattcctt catggaaaac aattcttact tctacatgtg 6840
   tagtttatat ctgatgcatt acaagagcta atgtttaaag acaaaacaaa acctgcctgt 6900
   atgacagcag caactcgagc caacatttag tgttacatgt tatatttttg aaataccgtt 6960
   ttgttatcat attccacata ttactttcca taaagtcaga gaagttcaat gtaattgttg 7020
   gctctgattc ttccaccttg ggatacacat tcacaagaag atttatatat tttcttacta 7080
   tgatagagga acataatctg ggaaaacttc ccatgtctgt aagatgaaaa ggataccttt 7140
   accatgttgt ttttgataat aaagaatatg gaaaatggta gaaatctctc ctctctatgt 7200
   gtatttgtat atgtgtgtat acacacacac gttgacattt ttacataaca tgtgattgcc 7260
   acttcttata aagttatgat atagaacccc atgaaaacaa ctttatatta tagatcaaat 7320
   aatgtgccca gaaacgcaat tgcaacagta aatcttgatc tattggtaag agtctatgat 7380
   acaggtcttc attctatccc taaacataat gttagtaaag aggttccatc agattgtatt 7440
   atagagacct tcctattgct atttattttt aagagatgag aagactgact agcaatgtct 7500
   ccacagtgca attggtttca cttctgggtc tgtctgtctg tttgtgtagg tgagatcagt 7560
   gttgtcaggc tctcagaata atttttaaaa gaatccagaa gcctgacttc acaccaagta 7620
   gccatcctag atgggcgggg gggatctcca tgttcaacca aaccctcaga acttagagca 7680
   ataaatacat tcagtcattt atttataaat gaatgacaga atattcaatc caaatagaaa 7740
   caaccttttg tcagtgatgc aacataacat ggagtcttat attgagaaag tgaatatgaa 7800
   tattttaaat agatgcctag gaaatctgtg tttgctgttt acatttaatg tactttgcca 7860
   cattagcagt accacacttc ttttactttc atccttctaa gaactaatga aagatagctt 7920
   gctattagct ttgacatgtt gcacatgcca ttatgttgtt ctctaagaac aactaaattc 7980
   tctccagtgt tcatgtgtga ttcctttttc atcattatta ctttaatgtg gatgaatact 8040
   atttctagga acttgattat cactgaacag attgcatatg taaatgcaga taattcttga 8100
   gcaatatagt gaaaatgatt tcacaaaaaa aatccatatg tactgtatac atcttcctga 8160
   atccaaaatt ctcctgttat ccacacagtt tgaaatctca tattaaacag tggtaaattt 8220
   ttaaacatta ggacattagc tggctgcttc tataaatgta cctgtattgt ctgcctgctc 8280
   cttttgtgga gatgtgtttt tgtattggat gtttgggcca atgggaggct tcaagctaca 8340
   acatattttc ccactttaaa taatatttaa catatgacaa accccagaca aggcttttaa 8400
   tatgtataaa gaactgcagt gttaggtggt ttatttgcaa actgttttca gtgttgtcca 8460
   tttttatggc acctttaaca atattcttgt ttccaaagta caaaaaaaat aggttaaata 8520
   atctagccat aactgaatgt caagcaataa aacaaatgac ttttgtgcat agacttaaaa 8580
   aatacaaatt ttagacatct gcatgtaaat gcaatctctt gtttactgct ttcttcaact 8640
   tgagcaatcg attccttatt tactattaac ttaagaactt gtaacggcct gtaaacattt 8700
   tctctcttac tcttctttca aatttacctg tccctgcttt tgagtcataa tatttaatgt 8760
   tgttctgcac atctctatac agttaacttt ttggctttca ttctgtatag ataagaaaat 8820
   gttatattat aaacagccta ctcagtgcaa atatttatct gtttatcaaa tccacaatat 8880
   gctgtataat accggtttta ctatataatc tattttagac atagctgttt agaactagag 8940
   tgtgctattt ttgtgttttt ctgatgtgtg gtgctagaca agttactttt gtgaacaaca 9000
   aaaattatcc cttttattcc tagacaatac cacctttggg tcttgttaat ttcactgagt 9060
   ataactatat atttgtatat atatacatat atatatatat ctacctatgc ccaactggca 9120
   gctgtatcag agtgctggat ttgggacatg cttttctctt taaatacata atatcattat 9180
   ataaattatt ctagagtgta tttaattagg ataaaattac ttccttagta tggatatttg 9240
   acatctatag ggtgaatttg tttataaata tggctatatg gaaacttatt agcatttact 9300
   ttatgtttgc tacttggctt tacagcatat ctcctaagct gaaaaataat ttgccaggcc 9360
   ttcaagatcc taaagaaact tgtttaatgg agtaatatac ttttttttct tattaaggaa 9420
   ttgtattact ggcacctaac acagttgtat tcttagctcc tattatagat aatgggcatt 9480
   tacataaaat atcctagatg gcttgatggc agaataaacc tttcccctcc tacctgagtc 9540
   atgagaagga tggagacgtc ctctgccata acatgggcca taaagcaaat tcgacatggg 9600
   atgttctgtt tcagtatgac ctcaaccagt tccatgaact gagtgaagga ccttcatttt 9660
   caaagttatt taataagtag cttaattaag cctttctacc cattctccca agatctactg 9720
   gcattattga aaagcaaagt ttatcaaata tctaactaag gatgtagtta accttattaa 9780
   atattgatta gaattgttct gtaatattac tgaatttgta agatctttag caaagatttt 9840
   tgagcaattt ataaatgtag agcaaatgtt tctgtttact gcactttttg taactgaagg 9900
   tgataaattc tcaagccatg attattggct tccatgcact gcaatattta tccacaattc 9960
   tagacatttt ccatttttgt ggaagagttg ctgttacctt aattataaat gcaattgtgt 10020
   ggttaatgag agctaatgct agtagttaac cttttaaagt ggattggcta cagttgaggg 10080
   agaaatctct tttaatataa atcacatcat tccttaactg cctctcttgg aaagagattg 10140
   aaaccttttt tttaaagcac gatttagcat cctaagcttc ctgagggtag agattgtatc 10200
   tttttgcgtc tgcacaatgg ctagcacatg tcagcatttg acaattgtta aatgataaca 10260
   agtgtgcccc aattaaaacg tttttcctgg gttgttttgt taaatttaca aagtaagcca 10320
   agccttacgg ttaacattct cctctacaac caagtattaa agccacattt aaaaagacca 10380
   catgaaatgc tgattctaat tgtgtgtagg tcttgaggat taagcacaca aatttcacaa 10440
   acttctgttt gagtaaacaa actcagcctt ctgtaaatat acatgcaagt ttggaaacag 10500
   taatactgta cctataaata tatgctgtct gttttgtgta cagtatgtaa aaactccttt 10560
   tctgccacac taaaaatgca agccatttat gggaatccta aaactagtat tgaactaaaa 10620
   ctttgctaat gatctttatt agaggatcgt ccaacttttc acttaccttg ggttttcttt 10680
   tcaattcact cttacactag tctgcttatt tccagctgtt tattttattg agtcctgaat 10740
   ttaaaaaaaa aatattttga ttcattttgt aaatacaagc tgtacaaaaa agagagattt 10800
   aatgttgtct tttaaatact ccaattttca ttctaatatg aatgttgtta tattgtactt 10860
   agaaactgta cctttaatat tacattacct ttattaaaag tgcattgaac acatcaattt 10920
   tagatgtgct ttatgtactg ttatcctata ataaaacttc agcttctaat ggaa       10974
<210> 12
   <211> 548
   <212> PRT
   <213> Homo sapiens
<400> 12
   Met Asn Glu Gly Ala Pro Gly Asp Ser Asp Leu Glu Thr Glu Ala Arg
    1               5                  10                  15
   Val Pro Trp Ser Ile Met Gly His Cys Leu Arg Thr Gly Gln Ala Arg
               20                  25                  30
   Met Ser Ala Thr Pro Thr Pro Ala Gly Glu Gly Ala Arg Ser Ser Ser
           35                  40                  45
   Thr Cys Ser Ser Leu Ser Arg Leu Phe Trp Ser Gln Leu Glu His Ile
       50                  55                  60
   Asn Trp Asp Gly Ala Thr Ala Lys Asn Phe Ile Asn Leu Arg Glu Phe
   65                  70                  75                  80
   Phe Ser Phe Leu Leu Pro Ala Leu Arg Lys Ala Gln Ile Glu Ile Ile
                   85                  90                  95
   Pro Cys Lys Ile Cys Gly Asp Lys Ser Ser Gly Ile His Tyr Gly Val
               100                 105                 110
   Ile Thr Cys Glu Gly Cys Lys Gly Phe Phe Arg Arg Ser Gln Gln Ser
           115                 120                 125
   Asn Ala Thr Tyr Ser Cys Pro Arg Gln Lys Asn Cys Leu Ile Asp Arg
       130                 135                 140
   Thr Ser Arg Asn Arg Cys Gln His Cys Arg Leu Gln Lys Cys Leu Ala
   145                 150                 155                 160
   Val Gly Met Ser Arg Asp Ala Val Lys Phe Gly Arg Met Ser Lys Lys
                   165                 170                 175
   Gln Arg Asp Ser Leu Tyr Ala Glu Val Gln Lys His Arg Met Gln Gln
               180                 185                 190
   Gln Gln Arg Asp His Gln Gln Gln Pro Gly Glu Ala Glu Pro Leu Thr
           195                 200                 205
   Pro Thr Tyr Asn Ile Ser Ala Asn Gly Leu Thr Glu Leu His Asp Asp
       210                 215                 220
   Leu Ser Asn Tyr Ile Asp Gly His Thr Pro Glu Gly Ser Lys Ala Asp
   225                 230                 235                 240
   Ser Ala Val Ser Ser Phe Tyr Leu Asp Ile Gln Pro Ser Pro Asp Gln
                   245                 250                 255
   Ser Gly Leu Asp Ile Asn Gly Ile Lys Pro Glu Pro Ile Cys Asp Tyr
               260                 265                 270
   Thr Pro Ala Ser Gly Phe Phe Pro Tyr Cys Ser Phe Thr Asn Gly Glu
           275                 280                 285
   Thr Ser Pro Thr Val Ser Met Ala Glu Leu Glu His Leu Ala Gln Asn
       290                 295                 300
   Ile Ser Lys Ser His Leu Glu Thr Cys Gln Tyr Leu Arg Glu Glu Leu
   305                 310                 315                 320
   Gln Gln Ile Thr Trp Gln Thr Phe Leu Gln Glu Glu Ile Glu Asn Tyr
                   325                 330                 335
   Gln Asn Lys Gln Arg Glu Val Met Trp Gln Leu Cys Ala Ile Lys Ile
               340                 345                 350
   Thr Glu Ala Ile Gln Tyr Val Val Glu Phe Ala Lys Arg Ile Asp Gly
           355                 360                 365
   Phe Met Glu Leu Cys Gln Asn Asp Gln Ile Val Leu Leu Lys Ala Gly
       370                 375                 380
   Ser Leu Glu Val Val Phe Ile Arg Met Cys Arg Ala Phe Asp Ser Gln
   385                 390                 395                 400
   Asn Asn Thr Val Tyr Phe Asp Gly Lys Tyr Ala Ser Pro Asp Val Phe
                   405                 410                 415
   Lys Ser Leu Gly Cys Glu Asp Phe Ile Ser Phe Val Phe Glu Phe Gly
               420                 425                 430
   Lys Ser Leu Cys Ser Met His Leu Thr Glu Asp Glu Ile Ala Leu Phe
           435                 440                 445
   Ser Ala Phe Val Leu Met Ser Ala Asp Arg Ser Trp Leu Gln Glu Lys
       450                 455                 460
   Val Lys Ile Glu Lys Leu Gln Gln Lys Ile Gln Leu Ala Leu Gln His
   465                 470                 475                 480
   Val Leu Gln Lys Asn His Arg Glu Asp Gly Ile Leu Thr Lys Leu Ile
                   485                 490                 495
   Cys Lys Val Ser Thr Leu Arg Ala Leu Cys Gly Arg His Thr Glu Lys
               500                 505                 510
   Leu Met Ala Phe Lys Ala Ile Tyr Pro Asp Ile Val Arg Leu His Phe
           515                 520                 525
   Pro Pro Leu Tyr Lys Glu Leu Phe Thr Ser Glu Phe Glu Pro Ala Met
       530                 535                 540
   Gln Ile Asp Gly
   545
<210> 13
   <211> 3818
   <212> DNA
   <213> Homo sapiens
<400> 13
   ctccccggta aagtctcgcg gtgctgccgg gctcagcccc gtctcctcct cttgctccct 60
   cggccgggcg gcggtgactg tgcaccgacg tcggcgcggg ctgcaccgcc gcgtccgccc 120
   gcccgccagc atggccacca ccgccacctg cacccgtttc accgacgact accagctctt 180
   cgaggagctt ggcaagggtg ctttctctgt ggtccgcagg tgtgtgaaga aaacctccac 240
   gcaggagtac gcagcaaaaa tcatcaatac caagaagttg tctgcccggg atcaccagaa 300
   actagaacgt gaggctcgga tatgtcgact tctgaaacat ccaaacatcg tgcgcctcca 360
   tgacagtatt tctgaagaag ggtttcacta cctcgtgttt gaccttgtta ccggcgggga 420
   gctgtttgaa gacattgtgg ccagagagta ctacagtgaa gcagatgcca gccactgtat 480
   acatcagatt ctggagagtg ttaaccacat ccaccagcat gacatcgtcc acagggacct 540
   gaagcctgag aacctgctgc tggcgagtaa atgcaagggt gccgccgtca agctggctga 600
   ttttggccta gccatcgaag tacagggaga gcagcaggct tggtttggtt ttgctggcac 660
   cccaggttac ttgtcccctg aggtcttgag gaaagatccc tatggaaaac ctgtggatat 720
   ctgggcctgc ggggtcatcc tgtatatcct cctggtgggc tatcctccct tctgggatga 780
   ggatcagcac aagctgtatc agcagatcaa ggctggagcc tatgatttcc catcaccaga 840
   atgggacacg gtaactcctg aagccaagaa cttgatcaac cagatgctga ccataaaccc 900
   agcaaagcgc atcacggctg accaggctct caagcacccg tgggtctgtc aacgatccac 960
   ggtggcatcc atgatgcatc gtcaggagac tgtggagtgt ttgcgcaagt tcaatgcccg 1020
   gagaaaactg aagggtgcca tcctcacgac catgcttgtc tccaggaact tctcagctgc 1080
   caaaagccta ttgaacaaga agtcggatgg cggtgtcaag ccacagagca acaacaaaaa 1140
   cagtctcgta agcccagccc aagagcccgc gcccttgcag acggccatgg agccacaaac 1200
   cactgtggta cacaacgcta cagatgggat caagggctcc acagagagct gcaacaccac 1260
   cacagaagat gaggacctca aagctgcccc gctccgcact gggaatggca gctcggtgcc 1320
   tgaaggacgg agctcccggg acagaacagc cccctctgca ggcatgcagc cccagccttc 1380
   tctctgctcc tcagccatgc gaaaacagga gatcattaag attacagaac agctgattga 1440
   agccatcaac aatggggact ttgaggccta cacgaagatt tgtgatccag gcctcacttc 1500
   ctttgagcct gaggcccttg gtaacctcgt ggaggggatg gatttccata agttttactt 1560
   tgagaatctc ctgtccaaga acagcaagcc tatccatacc accatcctaa acccacacgt 1620
   ccacgtgatt ggggaggacg cagcgtgcat cgcctacatc cgcctcaccc agtacatcga 1680
   cgggcagggt cggcctcgca ccagccagtc agaagagacc cgggtctggc accgtcggga 1740
   tggcaagtgg ctcaatgtcc actatcactg ctcaggggcc cctgccgcac cgctgcagtg 1800
   agctcagcca caggggcttt aggagattcc agccggaggt ccaaccttcg cagccagtgg 1860
   ctctggaggg cctgagtgac agcggcagtc ctgtttgttt gaggtttaaa acaattcaat 1920
   tacaaaagcg gcagcagcca atgcacgccc ctgcatgcag ccctcccgcc cgcccttcgt 1980
   gtctgtctct gctgtaccga ggtgtttttt acatttaaga aaaaaaaaaa agaaaaaaag 2040
   attgtttaaa aaaaaaagga atccatacca tgatgcgttt taaaaccacc gacagccctt 2100
   gggttggcaa gaaggcagga gtatgtatga ggtccatcct ggcatgagca gtggctcacc 2160
   caccggcctt gaagaggtga gcttggcctc tctggtcccc atggacttag ggggaccagg 2220
   caagaactct gacagagctt tgggggccgt gatgtgattg cagctcctga ggtggcctgc 2280
   ttaccccagg tctaggaatg aacttctttg gaacttgcat aggcgcctag aatggggctg 2340
   atgagaacat cgtgaccatc agacctactt gggagagaac gcagagctcc cagcctgctg 2400
   tggaggcagc tgagaagtgg tggcctcagg actgagagcc cggacgttgc tgtactgtct 2460
   tgtttagtgt agaagggaag agaattggtg ctgcagaagt gtacccgcca tgaagccgat 2520
   gagaaacctc gtgttagtct gacatgcact cactcatcca tttctatagg atgcacaatg 2580
   catgtgggcc ctaatattga ggccttatcc ctgcagctag gagggggagg ggttgttgct 2640
   gctttgcttc gtgttttctt ctaacctggc aaggagagag ccaggccctg gtcagggctc 2700
   ccgtgccgcc tttggcggtt ctgtttctgt gctgatctgg accatctttg tcttgccttt 2760
   tcacggtagt ggtccccatg ctgaccctca tctgggcctg ggccctctgc caagtgcccc 2820
   tgtgggatgg gaggagtgag gcagtgggag aagaggtggt ggtcgtttct atgcattcag 2880
   gctgcctttg gggctgcctc ccttcttatt cttccttgct gcacgtccat ctcttttcct 2940
   gtctttgaga ttgacctgac tgctctggca agaagaagag gtgtccttac agaggcctct 3000
   ttactgacca actgaagtat agacttactg ctggacaatc tgcatgggca tcacccctcc 3060
   ccgcatgtaa cccaaaagag gtgtccagag ccaaggcttc taccttcatt gtccctctct 3120
   gtgctcaagg agttccattc caggaggaag agatctatac cctaagcaga tagcaaagaa 3180
   gataatggag gagcaattgg tcatggcctt ggtttccctc aaaacaacgc tgcagattta 3240
   tctgcacaaa catctccact tttgggggaa aggtgggtag attccagttc cctggactac 3300
   cttcaggagg cacgagagct gggagaagag gcaaagctac aggtttactt gggagccagc 3360
   tgagaagaga gcagactcac aggtgctggt gcttggattt agccaggctc ctccgagcac 3420
   ctcatgcatg tcccagcccc tgggccctag ccctttcctg ccctgcagtc tgcagtgcca 3480
   gcacgcaaat cccttcacca cagggtttcg ttttgctggc ttgaagacaa atggtcttag 3540
   aattcattga gacccatagc ttcatatggc tgctccagcc ccacttctta gcattcttac 3600
   tcctcttctg gggctaatgt cagcatctat agacaataga ctattaaaaa atcacctttt 3660
   aaacaagaaa cggaaggcat ttgatgcaga atttttgcat gacaacatag aaataattta 3720
   aaaatagtgt ttgttctgaa tgttggtaga cccttcatag ctttgttaca atgaaacctt 3780
   gaactgaaaa tatttaataa aataaccttt aaacagtc                        3818
<210> 14
   <211> 556
   <212> PRT
   <213> Homo sapiens
<400> 14
   Met Ala Thr Thr Ala Thr Cys Thr Arg Phe Thr Asp Asp Tyr Gln Leu
    1               5                  10                  15
   Phe Glu Glu Leu Gly Lys Gly Ala Phe Ser Val Val Arg Arg Cys Val
               20                  25                  30
   Lys Lys Thr Ser Thr Gln Glu Tyr Ala Ala Lys Ile Ile Asn Thr Lys
           35                  40                  45
   Lys Leu Ser Ala Arg Asp His Gln Lys Leu Glu Arg Glu Ala Arg Ile
       50                  55                  60
   Cys Arg Leu Leu Lys His Pro Asn Ile Val Arg Leu His Asp Ser Ile
   65                  70                  75                  80
   Ser Glu Glu Gly Phe His Tyr Leu Val Phe Asp Leu Val Thr Gly Gly
                   85                  90                  95
   Glu Leu Phe Glu Asp Ile Val Ala Arg Glu Tyr Tyr Ser Glu Ala Asp
               100                 105                 110
   Ala Ser His Cys Ile His Gln Ile Leu Glu Ser Val Asn His Ile His
           115                 120                 125
   Gln His Asp Ile Val His Arg Asp Leu Lys Pro Glu Asn Leu Leu Leu
       130                 135                 140
   Ala Ser Lys Cys Lys Gly Ala Ala Val Lys Leu Ala Asp Phe Gly Leu
   145                 150                 155                 160
   Ala Ile Glu Val Gln Gly Glu Gln Gln Ala Trp Phe Gly Phe Ala Gly
                   165                 170                 175
   Thr Pro Gly Tyr Leu Ser Pro Glu Val Leu Arg Lys Asp Pro Tyr Gly
                   180                 185                 190
   Lys Pro Val Asp Ile Trp Ala Cys Gly Val Ile Leu Tyr Ile Leu Leu
           195                 200                 205
   Val Gly Tyr Pro Pro Phe Trp Asp Glu Asp Gln His Lys Leu Tyr Gln
       210                 215                 220
   Gln Ile Lys Ala Gly Ala Tyr Asp Phe Pro Ser Pro Glu Trp Asp Thr
   225                 230                 235                 240
   Val Thr Pro Glu Ala Lys Asn Leu Ile Asn Gln Met Leu Thr Ile Asn
                   245                 250                 255
   Pro Ala Lys Arg Ile Thr Ala Asp Gln Ala Leu Lys His Pro Trp Val
               260                 265                 270
   Cys Gln Arg Ser Thr Val Ala Ser Met Met His Arg Gln Glu Thr Val
           275                 280                 285
   Glu Cys Leu Arg Lys Phe Asn Ala Arg Arg Lys Leu Lys Gly Ala Ile
       290                 295                 300
   Leu Thr Thr Met Leu Val Ser Arg Asn Phe Ser Ala Ala Lys Ser Leu
   305                 310                 315                 320
   Leu Asn Lys Lys Ser Asp Gly Gly Val Lys Pro Gln Ser Asn Asn Lys
                   325                 330                 335
   Asn Ser Leu Val Ser Pro Ala Gln Glu Pro Ala Pro Leu Gln Thr Ala
               340                 345                 350
   Met Glu Pro Gln Thr Thr Val Val His Asn Ala Thr Asp Gly Ile Lys
           355                 360                 365
   Gly Ser Thr Glu Ser Cys Asn Thr Thr Thr Glu Asp Glu Asp Leu Lys
       370                 375                 380
   Ala Ala Pro Leu Arg Thr Gly Asn Gly Ser Ser Val Pro Glu Gly Arg
   385                 390                 395                 400
   Ser Ser Arg Asp Arg Thr Ala Pro Ser Ala Gly Met Gln Pro Gln Pro
                   405                 410                 415
   Ser Leu Cys Ser Ser Ala Met Arg Lys Gln Glu Ile Ile Lys Ile Thr
               420                 425                 430
   Glu Gln Leu Ile Glu Ala Ile Asn Asn Gly Asp Phe Glu Ala Tyr Thr
           435                 440                 445
   Lys Ile Cys Asp Pro Gly Leu Thr Ser Phe Glu Pro Glu Ala Leu Gly
       450                 455                 460
   Asn Leu Val Glu Gly Met Asp Phe His Lys Phe Tyr Phe Glu Asn Leu
   465                 470                 475                 480
   Leu Ser Lys Asn Ser Lys Pro Ile His Thr Thr Ile Leu Asn Pro His
                   485                 490                 495
   Val His Val Ile Gly Glu Asp Ala Ala Cys Ile Ala Tyr Ile Arg Leu
               500                 505                 510
   Thr Gln Tyr Ile Asp Gly Gln Gly Arg Pro Arg Thr Ser Gln Ser Glu
           515                 520                 525
   Glu Thr Arg Val Trp His Arg Arg Asp Gly Lys Trp Leu Asn Val His
       530                 535                 540
   Tyr His Cys Ser Gly Ala Pro Ala Ala Pro Leu Gln
   545                 550                 555
<210> 15
   <211> 3338
   <212> DNA
   <213> Homo sapiens
<400> 15
   gacatcatgg gctattttta ggggttgact ggtagcagat aagtgttgag ctcgggctgg 60
   ataagggctc agagttgcac tgagtgtggc tgaagcagcg aggcgggagt ggaggtgcgc 120
   ggagtcaggc agacagacag acacagccag ccagccaggt cggcagtata gtccgaactg 180
   caaatcttat tttcttttca ccttctctct aactgcccag agctagcgcc tgtggctccc 240
   gggctggtgt ttcgggagtg tccagagagc ctggtctcca gccgcccccg ggaggagagc 300
   cctgctgccc aggcgctgtt gacagcggcg gaaagcagcg gtacccacgc gcccgccggg 360
   ggaagtcggc gagcggctgc agcagcaaag aactttcccg gctgggagga ccggagacaa 420
   gtggcagagt cccggagcga acttttgcaa gcctttcctg cgtcttaggc ttctccacgg 480
   cggtaaagac cagaaggcgg cggagagcca cgcaagagaa gaaggacgtg cgctcagctt 540
   cgctcgcacc ggttgttgaa cttgggcgag cgcgagccgc ggctgccggg cgccccctcc 600
   ccctagcagc ggaggagggg acaagtcgtc ggagtccggg cggccaagac ccgccgccgg 660
   ccggccactg cagggtccgc actgatccgc tccgcgggga gagccgctgc tctgggaagt 720
   gagttcgcct gcggactccg aggaaccgct gcgcccgaag agcgctcagt gagtgaccgc 780
   gacttttcaa agccgggtag cgcgcgcgag tcgacaagta agagtgcggg aggcatctta 840
   attaaccctg cgctccctgg agcgagctgg tgaggagggc gcagcgggga cgacagccag 900
   cgggtgcgtg cgctcttaga gaaactttcc ctgtcaaagg ctccgggggg cgcgggtgtc 960
   ccccgcttgc cagagccctg ttgcggcccc gaaacttgtg cgcgcagccc aaactaacct 1020
   cacgtgaagt gacggactgt tctatgactg caaagatgga aacgaccttc tatgacgatg 1080
   ccctcaacgc ctcgttcctc ccgtccgaga gcggacctta tggctacagt aaccccaaga 1140
   tcctgaaaca gagcatgacc ctgaacctgg ccgacccagt ggggagcctg aagccgcacc 1200
   tccgcgccaa gaactcggac ctcctcacct cgcccgacgt ggggctgctc aagctggcgt 1260
   cgcccgagct ggagcgcctg ataatccagt ccagcaacgg gcacatcacc accacgccga 1320
   cccccaccca gttcctgtgc cccaagaacg tgacagatga gcaggagggc ttcgccgagg 1380
   gcttcgtgcg cgccctggcc gaactgcaca gccagaacac gctgcccagc gtcacgtcgg 1440
   cggcgcagcc ggtcaacggg gcaggcatgg tggctcccgc ggtagcctcg gtggcagggg 1500
   gcagcggcag cggcggcttc agcgccagcc tgcacagcga gccgccggtc tacgcaaacc 1560
   tcagcaactt caacccaggc gcgctgagca gcggcggcgg ggcgccctcc tacggcgcgg 1620
   ccggcctggc ctttcccgcg caaccccagc agcagcagca gccgccgcac cacctgcccc 1680
   agcagatgcc cgtgcagcac ccgcggctgc aggccctgaa ggaggagcct cagacagtgc 1740
   ccgagatgcc cggcgagaca ccgcccctgt cccccatcga catggagtcc caggagcgga 1800
   tcaaggcgga gaggaagcgc atgaggaacc gcatcgctgc ctccaagtgc cgaaaaagga 1860
   agctggagag aatcgcccgg ctggaggaaa aagtgaaaac cttgaaagct cagaactcgg 1920
   agctggcgtc cacggccaac atgctcaggg aacaggtggc acagcttaaa cagaaagtca 1980
   tgaaccacgt taacagtggg tgccaactca tgctaacgca gcagttgcaa acattttgaa 2040
   gagagaccgt cgggggctga ggggcaacga agaaaaaaaa taacacagag agacagactt 2100
   gagaacttga caagttgcga cggagagaaa aaagaagtgt ccgagaacta aagccaaggg 2160
   tatccaagtt ggactgggtt gcgtcctgac ggcgccccca gtgtgcacga gtgggaagga 2220
   cttggcgcgc cctcccttgg cgtggagcca gggagcggcc gcctgcgggc tgccccgctt 2280
   tgcggacggg ctgtccccgc gcgaacggaa cgttggactt ttcgttaaca ttgaccaaga 2340
   actgcatgga cctaacattc gatctcattc agtattaaag gggggagggg gagggggtta 2400
   caaactgcaa tagagactgt agattgcttc tgtagtactc cttaagaaca caaagcgggg 2460
   ggagggttgg ggaggggcgg caggagggag gtttgtgaga gcgaggctga gcctacagat 2520
   gaactctttc tggcctgcct tcgttaactg tgtatgtaca tatatatatt ttttaatttg 2580
   atgaaagctg attactgtca ataaacagct tcatgccttt gtaagttatt tcttgtttgt 2640
   ttgtttgggt atcctgccca gtgttgtttg taaataagag atttggagca ctctgagttt 2700
   accatttgta ataaagtata taattttttt atgttttgtt tctgaaaatt ccagaaagga 2760
   tatttaagaa aatacaataa actattggaa agtactcccc taacctcttt tctgcatcat 2820
   ctgtagatac tagctatcta ggtggagttg aaagagttaa gaatgtcgat taaaatcact 2880
   ctcagtgctt cttactatta agcagtaaaa actgttctct attagacttt agaaataaat 2940
   gtacctgatg tacctgatgc tatggtcagg ttatactcct cctcccccag ctatctatat 3000
   ggaattgctt accaaaggat agtgcgatgt ttcaggaggc tggaggaagg ggggttgcag 3060
   tggagaggga cagcccactg agaagtcaaa catttcaaag tttggattgt atcaagtggc 3120
   atgtgctgtg accatttata atgttagtag aaattttaca ataggtgctt attctcaaag 3180
   caggaattgg tggcagattt tacaaaagat gtatccttcc aatttggaat cttctctttg 3240
   acaattccta gataaaaaga tggcctttgc ttatgaatat ttataacagc attcttgtca 3300
   caataaatgt attcaaatac caaaaaaaaa aaaaaaaa 3338
<210> 16
   <211> 331
   <212> PRT
   <213> Homo sapiens
<400> 16
   Met Thr Ala Lys Met Glu Thr Thr Phe Tyr Asp Asp Ala Leu Asn Ala
    1               5                  10                  15
   Ser Phe Leu Pro Ser Glu Ser Gly Pro Tyr Gly Tyr Ser Asn Pro Lys
               20                  25                  30
   Ile Leu Lys Gln Ser Met Thr Leu Asn Leu Ala Asp Pro Val Gly Ser
           35                  40                  45
   Leu Lys Pro His Leu Arg Ala Lys Asn Ser Asp Leu Leu Thr Ser Pro
       50                  55                  60
   Asp Val Gly Leu Leu Lys Leu Ala Ser Pro Glu Leu Glu Arg Leu Ile
   65                  70                  75                  80
   Ile Gln Ser Ser Asn Gly His Ile Thr Thr Thr Pro Thr Pro Thr Gln
                   85                  90                  95
   Phe Leu Cys Pro Lys Asn Val Thr Asp Glu Gln Glu Gly Phe Ala Glu
               100                 105                 110
   Gly Phe Val Arg Ala Leu Ala Glu Leu His Ser Gln Asn Thr Leu Pro
           115                 120                 125
   Ser Val Thr Ser Ala Ala Gln Pro Val Asn Gly Ala Gly Met Val Ala
       130                 135                 140
   Pro Ala Val Ala Ser Val Ala Gly Gly Ser Gly Ser Gly Gly Phe Ser
   145                 150                 155                 160
   Ala Ser Leu His Ser Glu Pro Pro Val Tyr Ala Asn Leu Ser Asn Phe
                   165                 170                 175
   Asn Pro Gly Ala Leu Ser Ser Gly Gly Gly Ala Pro Ser Tyr Gly Ala
               180                 185                 190
   Ala Gly Leu Ala Phe Pro Ala Gln Pro Gln Gln Gln Gln Gln Pro Pro
           195                 200                 205
   His His Leu Pro Gln Gln Met Pro Val Gln His Pro Arg Leu Gln Ala
       210                 215                 220
   Leu Lys Glu Glu Pro Gln Thr Val Pro Glu Met Pro Gly Glu Thr Pro
   225                 230                 235                 240
   Pro Leu Ser Pro Ile Asp Met Glu Ser Gln Glu Arg Ile Lys Ala Glu
                   245                 250                 255
   Arg Lys Arg Met Arg Asn Arg Ile Ala Ala Ser Lys Cys Arg Lys Arg
               260                 265                 270
   Lys Leu Glu Arg Ile Ala Arg Leu Glu Glu Lys Val Lys Thr Leu Lys
           275                 280                 285
   Ala Gln Asn Ser Glu Leu Ala Ser Thr Ala Asn Met Leu Arg Glu Gln
       290                 295                 300
   Val Ala Gln Leu Lys Gln Lys Val Met Asn His Val Asn Ser Gly Cys
   305                 310                 315                 320
   Gln Leu Met Leu Thr Gln Gln Leu Gln Thr Phe
                   325                 330
<210> 17
   <211> 4830
   <212> DNA
   <213> Homo sapiens
<400> 17
   caaccggttc cgagtttgag gcactaggag gagggggaga agcggctgca gcggccgcgg 60
   caggagcagc gggagctaca gcatcagcaa gagcaacagt agctacagcc ccggcggcgg 120
   tgcctgttcc agtctttgct gctgcagtcc gtgcaaccac ccagaggggg aggggggaac 180
   caccagtcgc tgaggaacaa gagaaggggg gaaagtttag gcgagccttg gggggggggg 240
   ggccagcgcc ggagccgcgt gagagaggga gccgtgtttt ggtagggggg agtcggactg 300
   caactggcag cagagcgtct ccccggccgt gtggactcta caccccctac tcctgccgct 360
   tctgctgctg cctgtggctg gagggtcccc ctggggctga atctttggga cttgaccccg 420
   ttccctcccc cttccctcac tccccagccg ggcgggagca tttattcccc agattaattc 480
   cccttttggg ggggggcggg tgtgtgtgtg tgtgtgtgtg tgtgtgtgtg ttgggggaag 540
   cgtccctgaa atagtaaata ttattgagct ctttttgccc ttttcctgtc cgttttttta 600
   atttcctttt ttgaggtggg aaaactgaaa cccaccttga ttcgtcccct ctcccccctc 660
   cccaccttcc ctcgccctaa tcccccaacg aggaaggaag gagcagttgg ttcaatctct 720
   ggtaatctat gccagcaatt atgacaatgt tagcagacca tgcagctcgt cagctgcttg 780
   atttcagcca aaaactggat atcaacttat tagataatgt ggtgaattgc ttataccatg 840
   gagaaggagc ccagcaaaga atggctcaag aagtactgac acatttaaag gagcatcctg 900
   atgcttggac aagagtcgac acaattttgg aattttctca gaatatgaat acgaaatact 960
   atggactaca aattttggaa aatgtgataa aaacaaggtg gaagattctt ccaaggaacc 1020
   agtgcgaagg aataaaaaaa tacgttgttg gcctcattat caagacgtca tctgacccaa 1080
   cttgtgtaga gaaagaaaag gtgtatatcg gaaaattaaa tatgatcctt gttcagatac 1140
   tgaaacaaga atggcccaaa cattggccaa cttttatcag tgatattgtt ggagcaagta 1200
   ggaccagcga aagtctctgt caaaataata tggtgattct taaactcttg agtgaagaag 1260
   tatttgattt ctctagtgga cagataaccc aagtcaaatc taagcattta aaagacagca 1320
   tgtgcaatga attctcacag atatttcaac tgtgtcagtt tgtaatggaa aattctcaaa 1380
   atgctccact tgtacatgca accttggaaa cattgctcag atttctgaac tggattcccc 1440
   tgggatatat ttttgagacc aaattaatca gcacattgat ttataagttc ctgaatgttc 1500
   caatgtttcg aaatgtctct ctgaagtgcc tcactgagat tgctggtgtg agtgtaagcc 1560
   aatatgaaga acaatttgta acactattta ctctgacaat gatgcaacta aagcagatgc 1620
   ttcctttaaa taccaatatt cgacttgcgt actcaaatgg aaaagatgat gaacagaact 1680
   tcattcaaaa tctcagtttg tttctctgca cctttcttaa ggaacatgat caacttatag 1740
   aaaaaagatt aaatctcagg gaaactctta tggaggccct tcattatatg ttgttggtat 1800
   ctgaagtaga agaaactgaa atctttaaaa tttgtcttga atactggaat catttggctg 1860
   ctgaactcta tagagagagt ccattctcta catctgcctc tccgttgctt tctggaagtc 1920
   aacattttga tgttcctccc aggagacagc tatatttgcc catgttattc aaggtccgtt 1980
   tattaatggt tagtcgaatg gctaaaccag aggaagtatt ggttgtagag aatgatcaag 2040
   gagaagttgt gagagaattc atgaaggata cagattccat aaatttgtat aagaatatga 2100
   gggaaacatt ggtttatctt actcatctgg attatgtaga tacagaaaga ataatgacag 2160
   agaagcttca caatcaagtg aatggtacag agtggtcatg gaaaaatttg aatacattgt 2220
   gttgggcaat aggctccatt agtggagcaa tgcatgaaga ggacgaaaaa cgatttcttg 2280
   ttactgttat aaaggatcta ttaggattat gtgaacagaa aagaggcaaa gataataaag 2340
   ctattattgc atcaaatatc atgtacatag taggtcaata cccacgtttt ttgagagctc 2400
   actggaaatt tctgaagact gtagttaaca agctgttcga attcatgcat gagacccatg 2460
   atggagtcca ggatatggct tgtgatactt tcattaaaat agcccaaaaa tgccgcaggc 2520
   atttcgttca ggttcaggtt ggagaagtga tgccatttat tgatgaaatt ttgaacaaca 2580
   ttaacactat tatttgtgat cttcagcctc aacaggttca tacgttttat gaagctgtgg 2640
   ggtacatgat tggtgcacaa acagatcaaa cagtacaaga acacttgata gaaaagtaca 2700
   tgttactccc taatcaagtg tgggatagta taatccagca ggcaaccaaa aatgtggata 2760
   tactgaaaga tcctgaaaca gtcaagcagc ttggtagcat tttgaaaaca aatgtgagag 2820
   cctgcaaagc tgttggacac ccctttgtaa ttcagcttgg aagaatttat ttagatatgc 2880
   ttaatgtata caagtgcctc agtgaaaata tttctgcagc tatccaagct aatggtgaaa 2940
   tggttacaaa gcaaccattg attagaagta tgcgaactgt aaaaagggaa actttaaagt 3000
   taatatctgg ttgggtgagc cgatccaatg atccacagat ggtcgctgaa aattttgttc 3060
   cccctctgtt ggatgcagtt ctcattgatt atcagagaaa tgtcccagct gctagagaac 3120
   cagaagtgct tagtactatg gccataattg tcaacaagtt agggggacat ataacagctg 3180
   aaatacctca aatatttgat gctgtttttg aatgcacatt gaatatgata aataaggact 3240
   ttgaagaata tcctgaacat agaacgaact ttttcttact acttcaggct gtcaattctc 3300
   attgtttccc agcattcctt gctattccac ctacacagtt taaacttgtt ttggattcca 3360
   tcatttgggc tttcaaacat actatgagga atgtcgcaga tacgggctta cagatacttt 3420
   ttacactctt acaaaatgtt gcacaagaag aagctgcagc tcagagtttt tatcaaactt 3480
   atttttgtga tattctccag catatctttt ctgttgtgac agacacttca catactgctg 3540
   gtttaacaat gcatgcatca attcttgcat atatgtttaa tttggttgaa gaaggaaaaa 3600
   taagtacatc attaaatcct ggaaatccag ttaacaacca aatctttctt caggaatatg 3660
   tggctaatct ccttaagtcg gccttccctc acctacaaga tgctcaagta aagctctttg 3720
   tgacagggct tttcagctta aatcaagata ttcctgcttt caaggaacat ttaagagatt 3780
   tcctagttca aataaaggaa tttgcaggtg aagacacttc tgatttgttt ttggaagaga 3840
   gagaaatagc cctacggcag gctgatgaag agaaacataa acgtcaaatg tctgtccctg 3900
   gcatctttaa tccacatgag attccagaag aaatgtgtga ttaaaatcca aattcatgct 3960
   gttttttttc tctgcaactc gttagcagag gaaaacagca tgtgggtatt tgtcgaccaa 4020
   aatgatgcca atttgtaaat taaaatgtca cctagtggcc ctttttctta tgtgtttttt 4080
   tgtataagaa attttctgtg aaatatcctt ccattgttta agcttttgtt ttggtcatct 4140
   ttatttagtt tgcatgaagt tgaaaattaa ggcattttta aaaattttac ttcatgccca 4200
   tttttgtggc tgggctgggg ggaggaggca aattcgattt gaacatatac ttgtaattct 4260
   aatgcaaaat tatacaattt ttcctgtaaa caataccaat ttttaattag ggagcatttt 4320
   ccttctagtc tatttcagcc tagaagaaaa gataatgagt aaaacaaatt gcgttgttta 4380
   aaggattata gtgctgcatt gtctgaagtt agcacctctt ggactgaatc gtttgtctag 4440
   actacatgta ttacaaagtc tctttggcaa gattgcagca agatcatgtg catatcatcc 4500
   cattgtaaag cgacttcaaa aatatgggaa cacagttagt tatttttaca cagttctttt 4560
   tgtttttgtg tgtgtgtgct gtcgcttgtc gacaacagct ttttgttttc ctcaatgagg 4620
   agtgttgctc atttgtgagc cttcattaac tcgaagtgaa atggttaaaa atatttatcc 4680
   tgttagaata ggctgcatct ttttaacaac tcattaaaaa acaaaacaac tctggctttt 4740
   gagatgactt atactaattt acattgttta ccaagctgta gtgctttaag aacactactt 4800
   aaaaagcaaa ataaacttgg tttacattta 4830
<210> 18
   <211> 1071
   <212> PRT
   <213> Homo sapiens
<400> 18
   Met Pro Ala Ile Met Thr Met Leu Ala Asp His Ala Ala Arg Gln Leu
    1               5                  10                  15
   Leu Asp Phe Ser Gln Lys Leu Asp Ile Asn Leu Leu Asp Asn Val Val
               20                  25                  30
   Asn Cys Leu Tyr His Gly Glu Gly Ala Gln Gln Arg Met Ala Gln Glu
           35                  40                  45
   Val Leu Thr His Leu Lys Glu His Pro Asp Ala Trp Thr Arg Val Asp
       50                  55                  60
   Thr Ile Leu Glu Phe Ser Gln Asn Met Asn Thr Lys Tyr Tyr Gly Leu
   65                  70                  75                  80
   Gln Ile Leu Glu Asn Val Ile Lys Thr Arg Trp Lys Ile Leu Pro Arg
                   85                  90                  95
   Asn Gln Cys Glu Gly Ile Lys Lys Tyr Val Val Gly Leu Ile Ile Lys
               100                 105                 110
   Thr Ser Ser Asp Pro Thr Cys Val Glu Lys Glu Lys Val Tyr Ile Gly
           115                 120                 125
   Lys Leu Asn Met Ile Leu Val Gln Ile Leu Lys Gln Glu Trp Pro Lys
       130                 135                 140
   His Trp Pro Thr Phe Ile Ser Asp Ile Val Gly Ala Ser Arg Thr Ser
   145                 150                 155                 160
   Glu Ser Leu Cys Gln Asn Asn Met Val Ile Leu Lys Leu Leu Ser Glu
                   165                 170                 175
   Glu Val Phe Asp Phe Ser Ser Gly Gln Ile Thr Gln Val Lys Ser Lys
               180                 185                 190
   His Leu Lys Asp Ser Met Cys Asn Glu Phe Ser Gln Ile Phe Gln Leu
           195                 200                 205
   Cys Gln Phe Val Met Glu Asn Ser Gln Asn Ala Pro Leu Val His Ala
       210                 215                 220
   Thr Leu Glu Thr Leu Leu Arg Phe Leu Asn Trp Ile Pro Leu Gly Tyr
   225                 230                 235                 240
   Ile Phe Glu Thr Lys Leu Ile Ser Thr Leu Ile Tyr Lys Phe Leu Asn
                   245                 250                 255
   Val Pro Met Phe Arg Asn Val Ser Leu Lys Cys Leu Thr Glu Ile Ala
               260                 265                 270
   Gly Val Ser Val Ser Gln Tyr Glu Glu Gln Phe Val Thr Leu Phe Thr
           275                 280                 285
   Leu Thr Met Met Gln Leu Lys Gln Met Leu Pro Leu Asn Thr Asn Ile
       290                 295                 300
   Arg Leu Ala Tyr Ser Asn Gly Lys Asp Asp Glu Gln Asn Phe Ile Gln
   305                 310                 315                 320
   Asn Leu Ser Leu Phe Leu Cys Thr Phe Leu Lys Glu His Asp Gln Leu
                   325                 330                 335
   Ile Glu Lys Arg Leu Asn Leu Arg Glu Thr Leu Met Glu Ala Leu His
               340                 345                 350
   Tyr Met Leu Leu Val Ser Glu Val Glu Glu Thr Glu Ile Phe Lys Ile
           355                 360                 365
   Cys Leu Glu Tyr Trp Asn His Leu Ala Ala Glu Leu Tyr Arg Glu Ser
       370                 375                 380
   Pro Phe Ser Thr Ser Ala Ser Pro Leu Leu Ser Gly Ser Gln His Phe
   385                 390                 395                 400
   Asp Val Pro Pro Arg Arg Gln Leu Tyr Leu Pro Met Leu Phe Lys Val
                   405                 410                 415
   Arg Leu Leu Met Val Ser Arg Met Ala Lys Pro Glu Glu Val Leu Val
               420                 425                 430
   Val Glu Asn Asp Gln Gly Glu Val Val Arg Glu Phe Met Lys Asp Thr
           435                 440                 445
   Asp Ser Ile Asn Leu Tyr Lys Asn Met Arg Glu Thr Leu Val Tyr Leu
       450                 455                 460
   Thr His Leu Asp Tyr Val Asp Thr Glu Arg Ile Met Thr Glu Lys Leu
   465                 470                 475                 480
   His Asn Gln Val Asn Gly Thr Glu Trp Ser Trp Lys Asn Leu Asn Thr
                       485                 490                 495
   Leu Cys Trp Ala Ile Gly Ser Ile Ser Gly Ala Met His Glu Glu Asp
                   500                 505                 510
   Glu Lys Arg Phe Leu Val Thr Val Ile Lys Asp Leu Leu Gly Leu Cys
               515                 520                 525
   Glu Gln Lys Arg Gly Lys Asp Asn Lys Ala Ile Ile Ala Ser Asn Ile
       530                 535                 540
   Met Tyr Ile Val Gly Gln Tyr Pro Arg Phe Leu Arg Ala His Trp Lys
   545                 550                 555                 560
   Phe Leu Lys Thr Val Val Asn Lys Leu Phe Glu Phe Met His Glu Thr
                       565                 570                 575
   His Asp Gly Val Gln Asp Met Ala Cys Asp Thr Phe Ile Lys Ile Ala
                   580                 585                 590
   Gln Lys Cys Arg Arg His Phe Val Gln Val Gln Val Gly Glu Val Met
               595                 600                 605
   Pro Phe Ile Asp Glu Ile Leu Asn Asn Ile Asn Thr Ile Ile Cys Asp
       610                 615                 620
   Leu Gln Pro Gln Gln Val His Thr Phe Tyr Glu Ala Val Gly Tyr Met
   625                 630                 635                 640
   Ile Gly Ala Gln Thr Asp Gln Thr Val Gln Glu His Leu Ile Glu Lys
                       645                 650                 655
   Tyr Met Leu Leu Pro Asn Gln Val Trp Asp Ser Ile Ile Gln Gln Ala
                   660                 665                 670
   Thr Lys Asn Val Asp Ile Leu Lys Asp Pro Glu Thr Val Lys Gln Leu
               675                 680                 685
   Gly Ser Ile Leu Lys Thr Asn Val Arg Ala Cys Lys Ala Val Gly His
       690                 695                 700
   Pro Phe Val Ile Gln Leu Gly Arg Ile Tyr Leu Asp Met Leu Asn Val
   705                 710                 715                 720
   Tyr Lys Cys Leu Ser Glu Asn Ile Ser Ala Ala Ile Gln Ala Asn Gly
                       725                 730                 735
   Glu Met Val Thr Lys Gln Pro Leu Ile Arg Ser Met Arg Thr Val Lys
                   740                 745                 750
   Arg Glu Thr Leu Lys Leu Ile Ser Gly Trp Val Ser Arg Ser Asn Asp
               755                 760                 765
   Pro Gln Met Val Ala Glu Asn Phe Val Pro Pro Leu Leu Asp Ala Val
       770                 775                 780
   Leu Ile Asp Tyr Gln Arg Asn Val Pro Ala Ala Arg Glu Pro Glu Val
   785                 790                 795                 800
   Leu Ser Thr Met Ala Ile Ile Val Asn Lys Leu Gly Gly His Ile Thr
                       805                 810                 815
   Ala Glu Ile Pro Gln Ile Phe Asp Ala Val Phe Glu Cys Thr Leu Asn
                   820                 825                 830
   Met Ile Asn Lys Asp Phe Glu Glu Tyr Pro Glu His Arg Thr Asn Phe
               835                 840                 845
   Phe Leu Leu Leu Gln Ala Val Asn Ser His Cys Phe Pro Ala Phe Leu
       850                 855                 860
   Ala Ile Pro Pro Thr Gln Phe Lys Leu Val Leu Asp Ser Ile Ile Trp
   865                 870                 875                 880
   Ala Phe Lys His Thr Met Arg Asn Val Ala Asp Thr Gly Leu Gln Ile
                       885                 890                 895
   Leu Phe Thr Leu Leu Gln Asn Val Ala Gln Glu Glu Ala Ala Ala Gln
                   900                 905                 910
   Ser Phe Tyr Gln Thr Tyr Phe Cys Asp Ile Leu Gln His Ile Phe Ser
               915                 920                 925
   Val Val Thr Asp Thr Ser His Thr Ala Gly Leu Thr Met His Ala Ser
       930                 935                 940
   Ile Leu Ala Tyr Met Phe Asn Leu Val Glu Glu Gly Lys Ile Ser Thr
   945                 950                 955                 960
   Ser Leu Asn Pro Gly Asn Pro Val Asn Asn Gln Ile Phe Leu Gln Glu
                       965                 970                 975
   Tyr Val Ala Asn Leu Leu Lys Ser Ala Phe Pro His Leu Gln Asp Ala
                   980                 985                 990
   Gln Val Lys Leu Phe Val Thr Gly Leu Phe Ser Leu Asn Gln Asp Ile
           995                 1000                1005
   Pro Ala Phe Lys Glu His Leu Arg Asp Phe Leu Val Gln Ile Lys Glu
       1010                1015                1020
   Phe Ala Gly Glu Asp Thr Ser Asp Leu Phe Leu Glu Glu Arg Glu Ile
   1025                1030                1035                1040
   Ala Leu Arg Gln Ala Asp Glu Glu Lys His Lys Arg Gln Met Ser Val
                   1045                1050                1055
   Pro Gly Ile Phe Asn Pro His Glu Ile Pro Glu Glu Met Cys Asp
               1060                1065                1070
<210> 19
   <211> 4099
   <212> DNA
   <213> Homo sapiens
<400> 19
   agccagccct tgccgtggcc ggagccgagc ggcgcatccg ggccggagaa gaggacgacg 60
   acgaggtcct cgaagtggac ccgtttgcga agcgccaggg agaaggagga gcggacgcat 120
   cgtagaaagg ggtggtggcg cccgaccccg cgccccggcc cgaagctctg agggcttccc 180
   ggcccccact gcctgcggca tggcccgggg ctcggcgctc ccgcggcggc cgctgctgtg 240
   catcccggcc gtctgggcgg ccgccgcgct tctgctctca gtgtcccgga cttcaggtga 300
   agtggaggtt ctggatccga acgacccttt aggacccctt gatgggcagg acggcccgat 360
   tccaactctg aaaggttact ttctgaattt tctggagcca gtaaacaata tcaccattgt 420
   ccaaggccag acggcaattc tgcactgcaa ggtggcagga aacccacccc ctaacgtgcg 480
   gtggctaaag aatgatgccc cggtggtgca ggagccgcgg cggatcatca tccggaagac 540
   agaatatggt tcacgactgc gaatccagga cctggacacg acagacactg gctactacca 600
   gtgcgtggcc accaacggga tgaagaccat taccgccact ggcgtcctgt ttgtgcggct 660
   gggtccaacg cacagcccaa atcataactt tcaggatgat taccacgagg atgggttctg 720
   ccagccttac cggggaattg cctgtgcacg cttcattggc aaccggacca tttatgtgga 780
   ctcgcttcag atgcaggggg agattgaaaa ccgaatcaca gcggccttca ccatgatcgg 840
   cacgtctacg cacctgtcgg accagtgctc acagttcgcc atcccatcct tctgccactt 900
   cgtgtttcct ctgtgcgacg cgcgctcccg gacacccaag ccgcgtgagc tgtgccgcga 960
   cgagtgcgag gtgctggaga gcgacctgtg ccgccaggag tacaccatcg cccgctccaa 1020
   cccgctcatc ctcatgcggc ttcagctgcc caagtgtgag gcgctgccca tgcctgagag 1080
   ccccgacgct gccaactgca tgcgcattgg catcccagcc gagaggctgg gccgctacca 1140
   tcagtgctat aacggctcag gcatggatta cagaggaacg gcaagcacca ccaagtcagg 1200
   ccaccagtgc cagccgtggg ccctgcagca cccccacagc caccacctgt ccagcacaga 1260
   cttccctgag cttggagggg ggcacgccta ctgccggaac cccggaggcc agatggaggg 1320
   cccctggtgc tttacgcaga ataaaaacgt acgcatggaa ctgtgtgacg taccctcgtg 1380
   tagtccccga gacagcagca agatggggat tctgtacatc ttggtcccca gcatcgcaat 1440
   tccactggtc atcgcttgcc ttttcttctt ggtttgcatg tgccggaata agcagaaggc 1500
   atctgcgtcc acaccgcagc ggcgacagct gatggcctcg cccagccaag acatggaaat 1560
   gcccctcatt aaccagcaca aacaggccaa actcaaagag atcagcctgt ctgcggtgag 1620
   gttcatggag gagctgggag aggaccggtt tgggaaagtc tacaaaggtc acctgttcgg 1680
   ccctgccccg ggggagcaga cccaggctgt ggccatcaaa acgctgaagg acaaagcgga 1740
   ggggcccctg cgggaggagt tccggcatga ggctatgctg cgagcacggc tgcaacaccc 1800
   caacgtcgtc tgcctgctgg gcgtggtgac caaggaccag cccctgagca tgatcttcag 1860
   ctactgttcg cacggcgacc tccacgaatt cctggtcatg cgctcgccgc actcggacgt 1920
   gggcagcacc gatgatgacc gcacggtgaa gtccgccctg gagccccccg acttcgtgca 1980
   ccttgtggca cagatcgcgg cggggatgga gtacctatcc agccaccacg tggttcacaa 2040
   ggacctggcc acccgcaatg tgctagtgta cgacaagctg aacgtgaaga tctcagactt 2100
   gggcctcttc cgagaggtgt atgccgccga ttactacaag ctgctgggga actcgctgct 2160
   gcctatccgc tggatggccc cagaggccat catgtacggc aagttctcca tcgactcaga 2220
   catctggtcc tacggtgtgg tcctgtggga ggtcttcagc tacggcctgc agccctactg 2280
   cgggtactcc aaccaggatg tggtggagat gatccggaac cggcaggtgc tgccttgccc 2340
   cgatgactgt cccgcctggg tgtatgccct catgatcgag tgctggaacg agttccccag 2400
   ccggcggccc cgcttcaagg acatccacag ccggctccga gcctggggca acctttccaa 2460
   ctacaacagc tcggcgcaga cctcgggggc cagcaacacc acgcagacca gctccctgag 2520
   caccagccca gtgagcaatg tgagcaacgc ccgctacgtg gggcccaagc agaaggcccc 2580
   gcccttccca cagccccagt tcatccccat gaagggccag atcagaccca tggtgccccc 2640
   gccgcagctc tacgtccccg tcaacggcta ccagccggtg ccggcctatg ggcctacctt 2700
   gcccaacttc tacccggtgc agatcccaat gcagatggcc ccgcagcagg tgcctcctca 2760
   gatggtcccc aagcccagct cacaccacag tggcagtggc tccaccagca caggctacgt 2820
   caccacggcc ccctccaaca catccatggc agacagggca gccctgctct cagagggcgc 2880
   tgatgacaca cagaacgccc cagaagatgg ggcccagagc accgtgcagg aagcagagga 2940
   ggaggaggaa ggctctgtcc cagagactga gctgctgggg gactgtgaca ctctgcaggt 3000
   ggacgaggcc caagtccagc tggaagcttg agtggcacca gggcccgggg ttcggggata 3060
   gaagccccgc cgagacccca cagggacctc agtcaccttt gagaagacac catactcagc 3120
   aatcacaaga gcccgccggc cagtgggctt gtttgcagac tgggtgaggt ggagccctgc 3180
   tcctctctgt cctctgacac agagagctgc cctgcctagg agcacccaag ccaggcaggg 3240
   ggtctggcag cacggcgtcc tggggagcag gacacatggt catccccagg gctgtataca 3300
   ttgattctgg tggtagactg gtagtgagca gcaaatgcct ttcaagaaaa taggtggcag 3360
   cttcactcca tgtcatatat ggagtgaata tttcaaaacg ttgggaataa gggcctgcaa 3420
   aaggcagcga ggaggcacct cgggtcttga ggttcctgac aaccgatctg gtctgttggt 3480
   ttgaggatga aggggctcca tttctgctgc ctccctgctg agaatattct ccctttagca 3540
   gccaaagatt cgctggaacg gaggctgccc tctgctgcct gttggggtcg gaagacaagg 3600
   ggcttctgaa atgggagttc ctgagataca acaaaatgtg tgccttcaaa gaaactgaca 3660
   gctttgtatt tggtgaaatg gttttaatta tactccatgt gtattttgcc cacttttttt 3720
   gggaattcaa gggaaagtgt ttcttgggtt tggaatgttc agaggaagca gtattgtaca 3780
   gaacacggta ttgttatttt tgttaagaat catgtacaga gcttaaatgt aatttatatg 3840
   tttttaatat gccattttca ttgaagtatt ttggtcttaa gatgacttta gtaatttaac 3900
   tgtttatgtt acccacgttg ggatccagtt ggtcttggtt tgcttctctc tgtaccacgt 3960
   gcacatgagg tccattcatt ttacagcccc tgttacacac agacccacag gcagccgtct 4020
   gtgccccgca cacattgttg gtcctatttg taaatcccac acccggtgta tccaataaag 4080
   tgaaacaaag catgtgaaa 4099
<210> 20
   <211> 943
   <212> PRT
   <213> Homo sapiens
<400> 20
<210> 21
   <211> 4304
   <212> DNA
   <213> Homo sapiens
<400> 21
   cacacggact acaggggagt tttgttgaag ttgcaaagtc ctggagcctc cagagggctg 60
   tcggcgcagt agcagcgagc agcagagtcc gcacgctccg gcgaggggca gaagagcgcg 120
   agggagcgcg gggcagcaga agcgagagcc gagcgcggac ccagccagga cccacagccc 180
   tccccagctg cccaggaaga gccccagcca tggaacacca gctcctgtgc tgcgaagtgg 240
   aaaccatccg ccgcgcgtac cccgatgcca acctcctcaa cgaccgggtg ctgcgggcca 300
   tgctgaaggc ggaggagacc tgcgcgccct cggtgtccta cttcaaatgt gtgcagaagg 360
   aggtcctgcc gtccatgcgg aagatcgtcg ccacctggat gctggaggtc tgcgaggaac 420
   agaagtgcga ggaggaggtc ttcccgctgg ccatgaacta cctggaccgc ttcctgtcgc 480
   tggagcccgt gaaaaagagc cgcctgcagc tgctgggggc cacttgcatg ttcgtggcct 540
   ctaagatgaa ggagaccatc cccctgacgg ccgagaagct gtgcatctac accgacaact 600
   ccatccggcc cgaggagctg ctgcaaatgg agctgctcct ggtgaacaag ctcaagtgga 660
   acctggccgc aatgaccccg cacgatttca ttgaacactt cctctccaaa atgccagagg 720
   cggaggagaa caaacagatc atccgcaaac acgcgcagac cttcgttgcc ctctgtgcca 780
   cagatgtgaa gttcatttcc aatccgccct ccatggtggc agcggggagc gtggtggccg 840
   cagtgcaagg cctgaacctg aggagcccca acaacttcct gtcctactac cgcctcacac 900
   gcttcctctc cagagtgatc aagtgtgacc cggactgcct ccgggcctgc caggagcaga 960
   tcgaagccct gctggagtca agcctgcgcc aggcccagca gaacatggac cccaaggccg 1020
   ccgaggagga ggaagaggag gaggaggagg tggacctggc ttgcacaccc accgacgtgc 1080
   gggacgtgga catctgaggg cgccaggcag gcgggcgcca ccgccacccg cagcgagggc 1140
   ggagccggcc ccaggtgctc ccctgacagt ccctcctctc cggagcattt tgataccaga 1200
   agggaaagct tcattctcct tgttgttggt tgttttttcc tttgctcttt cccccttcca 1260
   tctctgactt aagcaaaaga aaaagattac ccaaaaactg tctttaaaag agagagagag 1320
   aaaaaaaaaa tagtatttgc ataaccctga gcggtggggg aggagggttg tgctacagat 1380
   gatagaggat tttatacccc aataatcaac tcgtttttat attaatgtac ttgtttctct 1440
   gttgtaagaa taggcattaa cacaaaggag gcgtctcggg agaggattag gttccatcct 1500
   ttacgtgttt aaaaaaaagc ataaaaacat tttaaaaaca tagaaaaatt cagcaaacca 1560
   tttttaaagt agaagagggt tttaggtaga aaaacatatt cttgtgcttt tcctgataaa 1620
   gcacagctgt agtggggttc taggcatctc tgtactttgc ttgctcatat gcatgtagtc 1680
   actttataag tcattgtatg ttattatatt ccgtaggtag atgtgtaacc tcttcacctt 1740
   attcatggct gaagtcacct cttggttaca gtagcgtagc gtgcccgtgt gcatgtcctt 1800
   tgcgcctgtg accaccaccc caacaaacca tccagtgaca aaccatccag tggaggtttg 1860
   tcgggcacca gccagcgtag cagggtcggg aaaggccacc tgtcccactc ctacgatacg 1920
   ctactataaa gagaagacga aatagtgaca taatatattc tatttttata ctcttcctat 1980
   ttttgtagtg acctgtttat gagatgctgg ttttctaccc aacggccctg cagccagctc 2040
   acgtccaggt tcaacccaca gctacttggt ttgtgttctt cttcatattc taaaaccatt 2100
   ccatttccaa gcactttcag tccaataggt gtaggaaata gcgctgtttt tgttgtgtgt 2160
   gcagggaggg cagttttcta atggaatggt ttgggaatat ccatgtactt gtttgcaagc 2220
   aggactttga ggcaagtgtg ggccactgtg gtggcagtgg aggtggggtg tttgggaggc 2280
   tgcgtgccag tcaagaagaa aaaggtttgc attctcacat tgccaggatg ataagttcct 2340
   ttccttttct ttaaagaagt tgaagtttag gaatcctttg gtgccaactg gtgtttgaaa 2400
   gtagggacct cagaggttta cctagagaac aggtggtttt taagggttat cttagatgtt 2460
   tcacaccgga aggtttttaa acactaaaat atataattta tagttaaggc taaaaagtat 2520
   atttattgca gaggatgttc ataaggccag tatgatttat aaatgcaatc tccccttgat 2580
   ttaaacacac agatacacac acacacacac acacacacaa accttctgcc tttgatgtta 2640
   cagatttaat acagtttatt tttaaagata gatcctttta taggtgagaa aaaaacaatc 2700
   tggaagaaaa aaaccacaca aagacattga ttcagcctgt ttggcgtttc ccagagtcat 2760
   ctgattggac aggcatgggt gcaaggaaaa ttagggtact caacctaagt tcggttccga 2820
   tgaattctta tcccctgccc cttcctttaa aaaacttagt gacaaaatag acaatttgca 2880
   catcttggct atgtaattct tgtaattttt atttaggaag tgttgaaggg aggtggcaag 2940
   agtgtggagg ctgacgtgtg agggaggaca ggcgggagga ggtgtgagga ggaggctccc 3000
   gaggggaagg ggcggtgccc acaccgggga caggccgcag ctccattttc ttattgcgct 3060
   gctaccgttg acttccaggc acggtttgga aatattcaca tcgcttctgt gtatctcttt 3120
   cacattgttt gctgctattg gaggatcagt tttttgtttt acaatgtcat atactgccat 3180
   gtactagttt tagttttctc ttagaacatt gtattacaga tgcctttttt gtagtttttt 3240
   ttttttttat gtgatcaatt ttgacttaat gtgattactg ctctattcca aaaaggttgc 3300
   tgtttcacaa tacctcatgc ttcacttagc catggtggac ccagcgggca ggttctgcct 3360
   gctttggcgg gcagacacgc gggcgcgatc ccacacaggc tggcgggggc cggccccgag 3420
   gccgcgtgcg tgagaaccgc gccggtgtcc ccagagacca ggctgtgtcc ctcttctctt 3480
   ccctgcgcct gtgatgctgg gcacttcatc tgatcggggg cgtagcatca tagtagtttt 3540
   tacagctgtg ttattctttg cgtgtagcta tggaagttgc ataattatta ttattattat 3600
   tataacaagt gtgtcttacg tgccaccacg gcgttgtacc tgtaggactc tcattcggga 3660
   tgattggaat agcttctgga atttgttcaa gttttgggta tgtttaatct gttatgtact 3720
   agtgttctgt ttgttattgt tttgttaatt acaccataat gctaatttaa agagactcca 3780
   aatctcaatg aagccagctc acagtgctgt gtgccccggt cacctagcaa gctgccgaac 3840
   caaaagaatt tgcaccccgc tgcgggccca cgtggttggg gccctgccct ggcagggtca 3900
   tcctgtgctc ggaggccatc tcgggcacag gcccaccccg ccccacccct ccagaacacg 3960
   gctcacgctt acctcaacca tcctggctgc ggcgtctgtc tgaaccacgc gggggccttg 4020
   agggacgctt tgtctgtcgt gatggggcaa gggcacaagt cctggatgtt gtgtgtatcg 4080
   agaggccaaa ggctggtggc aagtgcacgg ggcacagcgg agtctgtcct gtgacgcgca 4140
   agtctgaggg tctgggcggc gggcggctgg gtctgtgcat ttctggttgc accgcggcgc 4200
   ttcccagcac caacatgtaa ccggcatgtt tccagcagaa gacaaaaaga caaacatgaa 4260
   agtctagaaa taaaactggt aaaaccccaa aaaaaaaaaa aaaa 4304
<210> 22
   <211> 295
   <212> PRT
   <213> Homo sapiens
<400> 22
   Met Glu His Gln Leu Leu Cys Cys Glu Val Glu Thr Ile Arg Arg Ala
    1               5                  10                  15
   Tyr Pro Asp Ala Asn Leu Leu Asn Asp Arg Val Leu Arg Ala Met Leu
               20                  25                  30
   Lys Ala Glu Glu Thr Cys Ala Pro Ser Val Ser Tyr Phe Lys Cys Val
           35                  40                  45
   Gln Lys Glu Val Leu Pro Ser Met Arg Lys Ile Val Ala Thr Trp Met
       50                  55                  60
   Leu Glu Val Cys Glu Glu Gln Lys Cys Glu Glu Glu Val Phe Pro Leu
   65                  70                  75                  80
   Ala Met Asn Tyr Leu Asp Arg Phe Leu Ser Leu Glu Pro Val Lys Lys
                   85                  90                  95
   Ser Arg Leu Gln Leu Leu Gly Ala Thr Cys Met Phe Val Ala Ser Lys
               100                 105                 110
   Met Lys Glu Thr Ile Pro Leu Thr Ala Glu Lys Leu Cys Ile Tyr Thr
           115                 120                 125
   Asp Asn Ser Ile Arg Pro Glu Glu Leu Leu Gln Met Glu Leu Leu Leu
       130                 135                 140
   Val Asn Lys Leu Lys Trp Asn Leu Ala Ala Met Thr Pro His Asp Phe
   145                 150                 155                 160
   Ile Glu His Phe Leu Ser Lys Met Pro Glu Ala Glu Glu Asn Lys Gln
                   165                 170                 175
   Ile Ile Arg Lys His Ala Gln Thr Phe Val Ala Leu Cys Ala Thr Asp
               180                 185                 190
   Val Lys Phe Ile Ser Asn Pro Pro Ser Met Val Ala Ala Gly Ser Val
           195                 200                 205
   Val Ala Ala Val Gln Gly Leu Asn Leu Arg Ser Pro Asn Asn Phe Leu
       210                 215                 220
   Ser Tyr Tyr Arg Leu Thr Arg Phe Leu Ser Arg Val Ile Lys Cys Asp
   225                 230                 235                 240
   Pro Asp Cys Leu Arg Ala Cys Gln Glu Gln Ile Glu Ala Leu Leu Glu
                   245                 250                 255
   Ser Ser Leu Arg Gln Ala Gln Gln Asn Met Asp Pro Lys Ala Ala Glu
               260                 265                 270
   Glu Glu Glu Glu Glu Glu Glu Glu Val Asp Leu Ala Cys Thr Pro Thr
           275                 280                 285
   Asp Val Arg Asp Val Asp Ile
       290                 295
<210> 23
   <211> 3415
   <212> DNA
   <213> Homo sapiens
<400> 23
   aggatacagc ggcttctgcg cgacttataa gagctccttg tgcggcgcca ttttaagcct 60
   ctcggtctgt ggcagcagcg ttggcccggc cccgggagcg gagagcgagg ggaggcggag 120
   acggaggaag gtctgaggag cagcttcagt ccccgccgag ccgccaccgc aggtcgagga 180
   cggtcggact cccgcggcgg gaggagcctg ttcccctgag ggtatttgaa gtataccata 240
   caactgtttt gaaaatccag cgtggacaat ggctactcaa gctgatttga tggagttgga 300
   catggccatg gaaccagaca gaaaagcggc tgttagtcac tggcagcaac agtcttacct 360
   ggactctgga atccattctg gtgccactac cacagctcct tctctgagtg gtaaaggcaa 420
   tcctgaggaa gaggatgtgg atacctccca agtcctgtat gagtgggaac agggattttc 480
   tcagtccttc actcaagaac aagtagctga tattgatgga cagtatgcaa tgactcgagc 540
   tcagagggta cgagctgcta tgttccctga gacattagat gagggcatgc agatcccatc 600
   tacacagttt gatgctgctc atcccactaa tgtccagcgt ttggctgaac catcacagat 660
   gctgaaacat gcagttgtaa acttgattaa ctatcaagat gatgcagaac ttgccacacg 720
   tgcaatccct gaactgacaa aactgctaaa tgacgaggac caggtggtgg ttaataaggc 780
   tgcagttatg gtccatcagc tttctaaaaa ggaagcttcc agacacgcta tcatgcgttc 840
   tcctcagatg gtgtctgcta ttgtacgtac catgcagaat acaaatgatg tagaaacagc 900
   tcgttgtacc gctgggacct tgcataacct ttcccatcat cgtgagggct tactggccat 960
   ctttaagtct ggaggcattc ctgccctggt gaaaatgctt ggttcaccag tggattctgt 1020
   gttgttttat gccattacaa ctctccacaa ccttttatta catcaagaag gagctaaaat 1080
   ggcagtgcgt ttagctggtg ggctgcagaa aatggttgcc ttgctcaaca aaacaaatgt 1140
   taaattcttg gctattacga cagactgcct tcaaatttta gcttatggca accaagaaag 1200
   caagctcatc atactggcta gtggtggacc ccaagcttta gtaaatataa tgaggaccta 1260
   tacttacgaa aaactactgt ggaccacaag cagagtgctg aaggtgctat ctgtctgctc 1320
   tagtaataag ccggctattg tagaagctgg tggaatgcaa gctttaggac ttcacctgac 1380
   agatccaagt caacgtcttg ttcagaactg tctttggact ctcaggaatc tttcagatgc 1440
   tgcaactaaa caggaaggga tggaaggtct ccttgggact cttgttcagc ttctgggttc 1500
   agatgatata aatgtggtca cctgtgcagc tggaattctt tctaacctca cttgcaataa 1560
   ttataagaac aagatgatgg tctgccaagt gggtggtata gaggctcttg tgcgtactgt 1620
   ccttcgggct ggtgacaggg aagacatcac tgagcctgcc atctgtgctc ttcgtcatct 1680
   gaccagccga caccaagaag cagagatggc ccagaatgca gttcgccttc actatggact 1740
   accagttgtg gttaagctct tacacccacc atcccactgg cctctgataa aggctactgt 1800
   tggattgatt cgaaatcttg ccctttgtcc cgcaaatcat gcacctttgc gtgagcaggg 1860
   tgccattcca cgactagttc agttgcttgt tcgtgcacat caggataccc agcgccgtac 1920
   gtccatgggt gggacacagc agcaatttgt ggagggggtc cgcatggaag aaatagttga 1980
   aggttgtacc ggagcccttc acatcctagc tcgggatgtt cacaaccgaa ttgttatcag 2040
   aggactaaat accattccat tgtttgtgca gctgctttat tctcccattg aaaacatcca 2100
   aagagtagct gcaggggtcc tctgtgaact tgctcaggac aaggaagctg cagaagctat 2160
   tgaagctgag ggagccacag ctcctctgac agagttactt cactctagga atgaaggtgt 2220
   ggcgacatat gcagctgctg ttttgttccg aatgtctgag gacaagccac aagattacaa 2280
   gaaacggctt tcagttgagc tgaccagctc tctcttcaga acagagccaa tggcttggaa 2340
   tgagactgct gatcttggac ttgatattgg tgcccaggga gaaccccttg gatatcgcca 2400
   ggatgatcct agctatcgtt cttttcactc tggtggatat ggccaggatg ccttgggtat 2460
   ggaccccatg atggaacatg agatgggtgg ccaccaccct ggtgctgact atccagttga 2520
   tgggctgcca gatctggggc atgcccagga cctcatggat gggctgcctc caggtgacag 2580
   caatcagctg gcctggtttg atactgacct gtaaatcatc ctttagctgt attgtctgaa 2640
   cttgcattgt gattggcctg tagagttgct gagagggctc gaggggtggg ctggtatctc 2700
   agaaagtgcc tgacacacta accaagctga gtttcctatg ggaacaattg aagtaaactt 2760
   tttgttctgg tcctttttgg tcgaggagta acaatacaaa tggattttgg gagtgactca 2820
   agaagtgaag aatgcacaag aatggatcac aagatggaat ttatcaaacc ctagccttgc 2880
   ttgttaaatt tttttttttt tttttttaag aatatctgta atggtactga ctttgcttgc 2940
   tttgaagtag ctcttttttt tttttttttt ttttttttgc agtaactgtt ttttaagtct 3000
   ctcgtagtgt taagttatag tgaatactgc tacagcaatt tctaattttt aagaattgag 3060
   taatggtgta gaacactaat tcataatcac tctaattaat tgtaatctga ataaagtgta 3120
   acaattgtgt agcctttttg tataaaatag acaaatagaa aatggtccaa ttagtttcct 3180
   ttttaatatg cttaaaataa gcaggtggat ctatttcatg tttttgatca aaaactattt 3240
   gggatatgta tgggtagggt aaatcagtaa gaggtgttat ttggaacctt gttttggaca 3300
   gtttaccagt tgccttttat cccaaagttg ttgtaacctg ctgtgatacg atgcttcaag 3360
   agaaaatgcg gttataaaaa atggttcaga attaaacttt taattcattc gattg      3415
<210> 24
   <211> 781
   <212> PRT
   <213> Homo sapiens
<400> 24
<210> 25
   <211> 6945
   <212> DNA
   <213> Homo sapiens
<400> 25
   atgagaaatt ctgaagaaca gccaagtgga gggaccacgg tattgcagcg tttgctacaa 60
   gagcagcttc gctatggcaa tcctagtgag aatcgcagcc tgcttgccat acaccagcaa 120
   gccacaggga atggccctcc tttccccagt ggcagtggga acccgggccc tcagagtgat 180
   gtgttgagtc cccaagacca ccaccaacag cttgtggctc atgctgctcg acaagaaccc 240
   caggggcagg aaatccagtc agaaaacctc atcatggaga agcagctgtc tcctcgaatg 300
   caaaataatg aagaactccc gacctatgaa gaagccaagg tccagtccca gtactttcgg 360
   ggccaacagc atgccagtgt tggagctgcc ttctatgtca ctggagtcac caaccagaag 420
   atgaggactg agggacgccc atcagttcag cggctcaatc ctggaaagat gcaccaagat 480
   gagggactca gagaccttaa gcaagggcat gtccgttcct tgagtgaacg actaatgcag 540
   atgtcactgg ccaccagtgg agttaaggcc catccacctg ttaccagtgc tcccctctcc 600
   ccaccacaac ccaatgacct ctacaagaat cccacaagtt ccagtgaatt ctacaaggcc 660
   caagggccac ttcctaacca gcatagcctg aagggcatgg aacaccgagg ccccccacca 720
   gaatatccct tcaagggcat gccaccccaa tctgtagtgt gcaagcccca agagccaggg 780
   cacttctata gtgagcatcg cctgaaccag ccagggagaa cagaggggca actgatgagg 840
   tatcagcatc cccctgagta tggagcagcc aggccagcgc aggacatctc attgccattg 900
   tcagccagga actcgcagcc tcacagccct acttcttctc tgacctctgg ggggtccttg 960
   cccttgctac aatctccacc atccactaga ttgtctcctg cccgacaccc cttggtccca 1020
   aaccagggag accattcagc tcacctgcct aggccgcagc agcatttcct tcctaatcag 1080
   gctcaccagg gggatcatta ccgtctctcc caacctggcc tgagtcagca gcagcagcaa 1140
   cagcagcagc agcaccatca tcaccatcac caccaacaac agcagcagca gcagccacag 1200
   cagcagccag gagaagccta ttcagctatg cctcgggctc agccatcctc tgcttcttat 1260
   cagccagtgc cagcagaccc ttttgccatt gtttccagag cccagcagat ggttgagatc 1320
   ctctcagacg agaaccggaa cttgaggcaa gagttggaag gatgctatga gaaggtggca 1380
   agactgcaga aggtggagac agaaatccag cgcgtctcgg aggcatatga gaacctcgtg 1440
   aagtcatcct ccaaaagaga ggccctagag aaagccatga gaaacaagct agagggcgag 1500
   attcggagga tgcatgattt caacagggat ctgagagagc gtctagagac tgccaacaag 1560
   cagcttgcag agaaggaata tgaggggtca gaggacacca gaaaaaccat ctcgcagctc 1620
   tttgcaaaaa ataaagaaag ccagcgtgag aaggagaagc tggaagcgga gctggccact 1680
   gcccgttcta ccaatgagga ccaaagacga cacatcgaaa tccgagatca ggccctgagt 1740
   aatgcccagg ccaaggtggt aaagctggaa gaagagctga aaaagaagca agtgtacgtt 1800
   gacaaggtgg agaagatgca gcaggccctt gtacagctcc aggcagcatg tgaaaaacgt 1860
   gagcagctag agcaccgtct ccggacacga ctggagaggg aactggaatc cctgagaatc 1920
   cagcagcgtc agggcaactg tcagcccacc aacgtttcag aatacaatgc tgctgcactg 1980
   atggagctcc ttcgggagaa agaggagagg attctggctc tggaagctga tatgacaaag 2040
   tgggagcaga aatatttgga ggagaatgtg atgagacatt ttgctctgga tgctgctgca 2100
   actgtggctg ctcagaggga cacaacagtc atcagtcact ctcctaacac cagctatgac 2160
   acagctctag aagctcgcat ccagaaagag gaggaagaaa tcttgatggc caataagcgt 2220
   tgccttgaca tggagggcag gattaagacc ctccatgccc agattattga gaaggatgcc 2280
   atgatcaaag tactccagca gcgttcccgg aaggagccga gcaagacaga gcagctgtcg 2340
   tgcatgcggc cagcgaagtc tctgatgtcc atttccaatg ctggatcagg cttgctctcc 2400
   cactcatcca ccctgactgg ctcccccatc atggaagaaa agcgagacga caagagctgg 2460
   aaggggagcc taggcattct cctgggtgga gactaccgtg ctgaatatgt cccttccaca 2520
   ccctcgcctg tgccaccctc gactcccctg ctctcggctc actccaagac aggcagccga 2580
   gactgcagta cccaaactga acgtgggacg gaatcgaaca aaactgcagc tgttgctccc 2640
   atctctgttc ctgctccagt tgctgctgcc gccactgctg ccgccatcac tgccactgct 2700
   gccaccatca ccaccaccat ggtagctgct gctccagttg ctgttgctgc tgctgctgct 2760
   ccagctgctg ctgctgcccc gtctccagcc actgccgctg ctactgctgc tgctgtttct 2820
   ccagctgctg ctggtcagat tccagctgct gcctctgttg cctcagctgc tgccgttgct 2880
   ccttctgctg ctgctgctgc tgctgttcag gttgctccag ctgctccggc tccagttcca 2940
   gctccggctc tggttccggt tccagctcca gcagcggctc aggcttctgc tcctgctcag 3000
   actcaggcac caacttcagc tccggctgtg gctccaactc cagctccaac tccaactcca 3060
   gctgtggctc aggctgaggt tcctgcaagt ccagctaccg gtcctggacc acatcgtttg 3120
   tctataccaa gtttgacctg caatccagac aaaacagatg ggcctgtgtt ccactccaat 3180
   actctggaaa gaaaaactcc cattcagatc ctgggacaag agcctgatgc agagatggtg 3240
   gaatatctca tctaaacggc caaatcaaga gctgcagatt atcagcaaaa atgcttttaa 3300
   tcattttccc ccttttattg gttcttgttt tgagggtgag gacaagggtt gtggggaggg 3360
   gatgtttttt aacaggactt tttattggaa caatgtacta cttgagtaat accatgtgaa 3420
   caccagtcta ttttggtatg cttagggagt acctctaaag acagattaat cagaatgtgc 3480
   tctaaagctt attgtttgaa tttatacgaa tactgggact gttaacaggt ggctatacat 3540
   cgacgttttc aatgtgctta aatttgttta aattttccat attctagatc attttttatt 3600
   gaagagcaca gtatgtgtgg aagacagtgt ataacacgta gtttggaagt gggaagctag 3660
   agagaattga gtgtgtgctg ttttgtatag ttactatcct gtgcagcagc tggagaaagc 3720
   actcacctca ggcttacaaa agggaatagt ttcaggagct atgtaagctg gaaaaaaggt 3780
   agggagtttt ggggtgcaga agggtactgg agctaatttt ttcttccagt ttcccagcta 3840
   ccctgcccca gggaattgtg tttgtcttca tttcagtggt gctttggaaa tggattcttt 3900
   tggttccctc ctggaggttc atacattcat atatatgctc tggagtaatt tatgcatttg 3960
   gataattaat atattgcttt cagatgctgg gagagtacat taactgagtg atgcgcaact 4020
   tcctctctct tagggaatta gaccatcaga ggccttgatg gagagttgca tggggtgcta 4080
   tatgcagact tccatggttt gtgtgtagcc atgaacacag cttgcttgca tttagtaaga 4140
   ccaatcagct tagtgtttat ttcttctaca gcacagattc actggctggg tctccagtct 4200
   caaattgcca atcatttgca aagtgaggaa ggatctttgt tgacaggttg aatgctttga 4260
   atttctggtg actactttga aataacttgt tttgtttgtc aaattctaag catatgtctt 4320
   aaaaggcatt tttgactatc acctccaagg gaatagcttg agaaacccaa agtactatgc 4380
   tgcagtcggg ggagaggtgg attgcagcag tatcctcaac tacctcttct cactgtcagt 4440
   gacaccatct tggaatacct ttgggaagca gcaggaaatg tgcatgtggg tagagatcaa 4500
   aggaggcaat ggctccaagc cttgccatag ggctgcctcc aaggacacag aaggatgcca 4560
   gttgccacag gtccctgccc tgtgtcacct gtctgccctt cattaaggtg agaaatctgc 4620
   agatagcatc attaagatca gttttaaggg gtatagggag ggtgagggaa gtggggggtg 4680
   ttaggtaagg gttgggggta gaggttttgg gatgtcttag ttagaaacca gattaataga 4740
   agagtaggcc tgatatatta catcatgagc catagtggtg ggaaagaact ttagcaatat 4800
   agccctacct cctcatttta gtgatgagga atctgagaac tggagaggtt cagtgacttt 4860
   ttgaaagtca tacaacacag ctaaccatta tgccaatcac catgcttatt ttgggaaact 4920
   ctttatcttt tttaaattcc attttatgaa aaggcatctt catggtccag ggaatatgta 4980
   tcttgtaaaa tgtacctggt tggagtagct tgtccagtct tgacaaacta ctgaatttct 5040
   gtcttgcctc tccttcagtg ccttttaaaa ggttttccct tttctgatct gcatttcaac 5100
   atagagtcac ataaatgtcc ccctgagaaa ccaatcccac ttctttctag gagattgggt 5160
   atcttagata atcttttggg gttcctctgt gagtatagga atggtatcct tcctaattat 5220
   cttccaaagg aattattttg tgtgtgtgcc tgtgtgtgtg tagagacata aaggagggtg 5280
   atgtgatttt cagctagtcc tttcacattt tcaataatga ggtaatcatg ttacatacac 5340
   attagtcctc agttataaag tgaatctcag atagaaatta aaagtgcagt tgtgttaaga 5400
   ctctttcata ctacccttta gtcataagga gaaaaaaaca ctcaaatagt agaagcagca 5460
   agtagcaaac ttcaggagag ctactttcta tccaaataat ttaaaaaaca cttttcacct 5520
   actcctttca tggttataac acattggcag actttttgct ggctctggga gccatgattt 5580
   taatcacatt ctgcaaggtg acaaatgtca tacattccac attgtgtggt agccatctct 5640
   ttagactcat gtgttttggg gaaaggaaga agttcttggc tgagtactat tttgaacttt 5700
   ccagaaccct ctcacaccag agacagttct tctctgttca gtttccaatc cccgataatt 5760
   tgctaaaata acattgtaca tccaagagag ggaagaagag tatgtcagta tattatgcag 5820
   aagatagata cagccttttc agaagatctc cactagtttt tgttccaaaa attcaagttt 5880
   atgggagaaa tctcaattag ccaccttttc acagttgtgt ggatataaca tttgggggat 5940
   ctttctggac tcctacctat ctgtgcattt taccggcacc tcaggaaagg agggtgacca 6000
   ggttgtctta gcttgtactg cttggtgatc tctgaggacc ttctaattca gttgtacccc 6060
   agtgttccat gtatagaaaa acttcattag aacaaacttt acttgatatg aaactcctat 6120
   taacagtctt tttttgaaat aaaaagtagc ttgagctttc ttttaaaatc atgtatcttg 6180
   attgttgatt taatgaagga tttcctttta atgctgcttt tgagcttcaa ggtaatagga 6240
   cagcaggaac ctaaaatatc tgccatcatc tgccatagga aagataccca gagacccatc 6300
   atgttctctt tttgttgtta cactgttggg tgggtataac aattggaaaa tgaacaaact 6360
   gattgattgt gcaaactact ttttatgaca agcctaaacc ctcataatgc ggcagcttaa 6420
   agtgtataca tatgcactaa ctttgatcaa ttatattctc atatctgtta gctacacagt 6480
   ctcctattat ctcaattgct tatgtgcata tggaatatgt tacttaaaac gtgtgcattc 6540
   ttactgaaaa tgttttcaaa ggaaggtatc agctgtgggc taattgccac caatttcagc 6600
   ctgccacgat tcttggaaat atgtcttcca agtgccatcc atcatcagta ggacaagtgt 6660
   cgggagtttg tttatttttt tccagtagca acgatgggtt acatggagcc atgaaacctc 6720
   cttctggcct cccttgtgat taatggcatg tgtttgtaaa atggatagct ggggttggca 6780
   gatggctaga gaagaatcgc ctttggttta aaatgtatgt ggtcccctaa tgattgtgac 6840
   cccattctgt aatcaactga gctagttcca ataaagttaa gcaggtttaa atccactttg 6900
   tgcctatctt ttcactgaca ataaagttag ctattttaaa atgca 6945
<210> 26
   <211> 1084
   <212> PRT
   <213> Homo sapiens
<400> 26
<210> 27
   <211> 3046
   <212> DNA
   <213> Homo sapiens
<400> 27
   agtcacgtga catgaggaga ggtgggcggg tacctggagg aagctcgcgg cgtcggtggc 60
   ggtggcgcgc ggcggccgct gagaccgggg ctttgagtcg caccccgcgg cccgcccccc 120
   gccgccaccc tcgcagatcc gtgctttttc ccctttgctt ctctcccgta ctgggtcagt 180
   cctgtccgcg ctcgcgcgtc ggtttgcggg tgtgcgcagg cgcggcaggg gccattagcc 240
   ctttgggtgg gcggtggagc ccgggagcgc gcgggcgaga ccatggcggg tagcagcact 300
   gggggcggtg gggttgggga gacgaaggtg atttaccacc tggatgagga agagactccc 360
   tacctggtga agatccctgt ccccgccgag cgcatcaccc tcggcgattt caagagcgtc 420
   ctgcagcggc ccgcgggcgc caagtacttt ttcaagtcta tggatcagga tttcggggtg 480
   gtgaaggaag aaatttcaga tgacaacgcc cgcctcccct gcttcaacgg aagggtggta 540
   tcctggctgg tgtcctcaga taatccccaa cccgagatgg cccctccagt ccatgagcct 600
   cgggcagaac tggcgcctcc agccccacct ttacctcctt tgccacccga gaggaccagc 660
   ggcattgggg actcaaggcc tccatccttc caccctaatg tgtccagcag ccatgagaat 720
   ctggagcctg agacagaaac cgagtcagta gtgtcactga ggcgggagcg gcctcgcagg 780
   agagacagca gtgagcatgg cgctgggggc cacaggactg gtggcccctc aaggctggag 840
   cgccacctgg ccggatacga gagctcctct accctcatga ccagcgagct ggagagtacc 900
   agcctggggg actcggacga ggaggacacc atgagcaggt tcagcagctc cacggagcag 960
   agcagtgcct cccgcctcct taagcgccac cggcggcgaa ggaagcagag gccaccccgc 1020
   ctggagagga cgtcatcctt cagcagcgtc acagattcca caatgtctct caatatcatc 1080
   acagtcacgc taaacatgga gaagtacaac ttcctgggta tctccattgt tggccagagc 1140
   aatgagcggg gagacggagg catctacatt ggctccatca tgaagggtgg ggctgtggcg 1200
   gccgacgggc gcattgagcc aggggacatg cttttgcagg tgaatgacat gaactttgag 1260
   aacatgagca acgatgacgc tgtgcgggtg ctgagggaca ttgtgcacaa gcctggcccc 1320
   attgtgctga ctgtggccaa gtgctgggat ccctctcctc aggcctattt cactctcccc 1380
   cgaaatgagc ccatccagcc aattgaccct gctgcctggg tgtcccattc cgcggctctg 1440
   actggcacct tcccagccta tccaggttcc tcctccatga gcaccattac atctggatcg 1500
   tctttgcctg atggctgtga aggccggggt ctctccgtcc atacggacat ggcatcggtg 1560
   accaaggcca tggcagctcc agagtctgga ctggaagtcc gggaccgcat gtggctcaag 1620
   atcaccatcc ctaatgcctt tctgggctcg gatgtggttg actggctcta ccatcacgtg 1680
   gagggctttc ctgagcggcg ggaggcccgc aagtatgcca gcgggctgct caaagcaggc 1740
   ctgatccgac acaccgtcaa caagatcacc ttctctgagc agtgctatta cgtcttcgga 1800
   gacctcagtg gtggctgtga gagctaccta gtcaacctgt ctctcaatga caacgatggc 1860
   tccagtgggg cttcagacca ggataccctg gctcctctgc ctggggccac cccctggccc 1920
   ctgctgccca ctttctccta ccaataccct gccccacacc cctacagccc gcagcctcca 1980
   ccctaccatg agctttcatc ttacacctat ggtgggggca gtgccagcag ccagcatagt 2040
   gagggcagcc ggagcagtgg gtcgacacgg agtgatgggg gggcagggcg cacggggagg 2100
   cccgaggagc gggcccccga gtccaagtcc ggcagtggca gtgagtctga gccctccagc 2160
   cgagggggca gccttcggcg gggtggggaa gcaagtggga ctagcgatgg gggccctcct 2220
   ccatccagag gctcaactgg gggtgcccct aatctccgag cccacccagg gctccatccc 2280
   tatggaccgc cccctggcat ggccctcccc tacaacccca tgatggtggt catgatgccc 2340
   ccacctccac ctccagtccc tccagcagtg cagcctccgg gggcccctcc agtcagagac 2400
   ctgggctctg tgcccccaga actgacagcc agccgccaaa gcttccacat ggccatgggc 2460
   aatcccagcg agttctttgt ggatgttatg tagcccactg tggggccagg ctgggccggg 2520
   cgctcctggt gtgtgactgg gtgtcctggc cgtcatgtgc ttgctcttac agtgcctggg 2580
   ctcagcctac cagctgctgc catacaggag attgtggcca ctgtgactct caccagcagt 2640
   gcctggttcc tcccccttcc ctcaggggta gacaagggac ctttgattat ttttagcttt 2700
   gtttttttat aagccttttt gggggttaaa atagagtttc ttacattttt gggacttttt 2760
   taataggcat ttcctctttt atatgaagaa ttcccatcca ttgggcccct tctaacccca 2820
   gaatgtgacc tcctcctcca gttacccaca gccctgccct ttgcagggtt gggggtggtc 2880
   agcggtaccc cggggttagg catcctagac agcagcctga ggaagctggg agatttgggc 2940
   catgtagctg cctttgttac tctatttatt ttagtcactt gtataaaaca ccaaataaag 3000
   caatagaggc aaactcaaaa aaaaaaaaaa aaaaaaaaaa aaaaaa                3046
<210> 28
   <211> 736
   <212> PRT
   <213> Hono sapiens
<400> 28
   Met Ala Gly Ser Ser Thr Gly Gly Gly Gly Val Gly Glu Thr Lys Val
   1               5                   10                  15
   Ile Tyr His Leu Asp Glu Glu Glu Thr Pro Tyr Leu Val Lys Ile Pro
               20                  25                  30
   Val Pro Ala Glu Arg Ile Thr Leu Gly Asp Phe Lys Ser Val Leu Gln
           35                  40                  45
   Arg Pro Ala Gly Ala Lys Tyr Phe Phe Lys Ser Met Asp Gln Asp Phe
       50                  55                  60
   Gly Val Val Lys Glu Glu Ile Ser Asp Asp Asn Ala Arg Leu Pro Cys
   65                  70                  75                  80
   Phe Asn Gly Arg Val Val Ser Trp Leu Val Ser Ser Asp Asn Pro Gln
                   85                  90                  95
   Pro Glu Met Ala Pro Pro Val His Glu Pro Arg Ala Glu Leu Ala Pro
               100                 105                 110
   Pro Ala Pro Pro Leu Pro Pro Leu Pro Pro Glu Arg Thr Ser Gly Ile
           115                 120                 125
   Gly Asp Ser Arg Pro Pro Ser Phe His Pro Asn Val Ser Ser Ser His
       130                 135                 140
   Glu Asn Leu Glu Pro Glu Thr Glu Thr Glu Ser Val Val Ser Leu Arg
   145                 150                 155                 160
   Arg Glu Arg Pro Arg Arg Arg Asp Ser Ser Glu His Gly Ala Gly Gly
                   165                 170                 175
   His Arg Thr Gly Gly Pro Ser Arg Leu Glu Arg His Leu Ala Gly Tyr
               180                 185                 190
   Glu Ser Ser Ser Thr Leu Met Thr Ser Glu Leu Glu Ser Thr Ser Leu
           195                 200                 205
   Gly Asp Ser Asp Glu Glu Asp Thr Met Ser Arg Phe Ser Ser Ser Thr
       210                 215                 220
   Glu Gln Ser Ser Ala Ser Arg Leu Leu Lys Arg His Arg Arg Arg Arg
   225                 230                 235                 240
   Lys Gln Arg Pro Pro Arg Leu Glu Arg Thr Ser Ser Phe Ser Ser Val
                   245                 250                 255
   Thr Asp Ser Thr Met Ser Leu Asn Ile Ile Thr Val Thr Leu Asn Met
               260                 265                 270
   Glu Lys Tyr Asn Phe Leu Gly Ile Ser Ile Val Gly Gln Ser Asn Glu
           275                 280                 285
   Arg Gly Asp Gly Gly Ile Tyr Ile Gly Ser Ile Met Lys Gly Gly Ala
       290                 295                 300
   Val Ala Ala Asp Gly Arg Ile Glu Pro Gly Asp Met Leu Leu Gln Val
   305                 310                 315                 320
   Asn Asp Met Asn Phe Glu Asn Met Ser Asn Asp Asp Ala Val Arg Val
                   325                 330                 335
   Leu Arg Asp Ile Val His Lys Pro Gly Pro Ile Val Leu Thr Val Ala
               340                 345                 350
   Lys Cys Trp Asp Pro Ser Pro Gln Ala Tyr Phe Thr Leu Pro Arg Asn
           355                 360                 365
   Glu Pro Ile Gln Pro Ile Asp Pro Ala Ala Trp Val Ser His Ser Ala
       370                 375                 380
   Ala Leu Thr Gly Thr Phe Pro Ala Tyr Pro Gly Ser Ser Ser Met Ser
   385                 390                 395                 400
   Thr Ile Thr Ser Gly Ser Ser Leu Pro Asp Gly Cys Glu Gly Arg Gly
                   405                 410                 415
   Leu Ser Val His Thr Asp Met Ala Ser Val Thr Lys Ala Met Ala Ala
               420                 425                 430
   Pro Glu Ser Gly Leu Glu Val Arg Asp Arg Met Trp Leu Lys Ile Thr
           435                 440                 445
   Ile Pro Asn Ala Phe Leu Gly Ser Asp Val Val Asp Trp Leu Tyr His
       450                 455                 460
   His Val Glu Gly Phe Pro Glu Arg Arg Glu Ala Arg Lys Tyr Ala Ser
   465                 470                 475                 480
   Gly Leu Leu Lys Ala Gly Leu Ile Arg His Thr Val Asn Lys Ile Thr
                   485                 490                 495
   Phe Ser Glu Gln Cys Tyr Tyr Val Phe Gly Asp Leu Ser Gly Gly Cys
               500                 505                 510
   Glu Ser Tyr Leu Val Asn Leu Ser Leu Asn Asp Asn Asp Gly Ser Ser
           515                 520                 525
   Gly Ala Ser Asp Gln Asp Thr Leu Ala Pro Leu Pro Gly Ala Thr Pro
       530                 535                 540
   Trp Pro Leu Leu Pro Thr Phe Ser Tyr Gln Tyr Pro Ala Pro His Pro
   545                 550                 555                 560
   Tyr Ser Pro Gln Pro Pro Pro Tyr His Glu Leu Ser Ser Tyr Thr Tyr
                   565                 570                 575
   Gly Gly Gly Ser Ala Ser Ser Gln His Ser Glu Gly Ser Arg Ser Ser
               580                 585                 590
   Gly Ser Thr Arg Ser Asp Gly Gly Ala Gly Arg Thr Gly Arg Pro Glu
           595                 600                 605
   Glu Arg Ala Pro Glu Ser Lys Ser Gly Ser Gly Ser Glu Ser Glu Pro
       610                 615                 620
   Ser Ser Arg Gly Gly Ser Leu Arg Arg Gly Gly Glu Ala Ser Gly Thr
   625                 630                 635                 640
   Ser Asp Gly Gly Pro Pro Pro Ser Arg Gly Ser Thr Gly Gly Ala Pro
                   645                 650                 655
   Asn Leu Arg Ala His Pro Gly Leu His Pro Tyr Gly Pro Pro Pro Gly
               660                 665                 670
   Met Ala Leu Pro Tyr Asn Pro Met Met Val Val Met Met Pro Pro Pro
           675                 680                 685
   Pro Pro Pro Val Pro Pro Ala Val Gln Pro Pro Gly Ala Pro Pro Val
       690                 695                 700
   Arg Asp Leu Gly Ser Val Pro Pro Glu Leu Thr Ala Ser Arg Gln Ser
   705                 710                 715                 720
   Phe His Met Ala Met Gly Asn Pro Ser Glu Phe Phe Val Asp Val Met
                   725                 730                 735
<210> 29
   <211> 7533
   <212> DNA
   <213> Homo sapiens
<400> 29
   gcgacgctca cgaacgatca gagctgcggg cgacgcaacg aagcccggag gccgcaggct 60
   gcgcgctccc tcgcagcagc cgggcgggca aaagccccca gtcctcggcc cccgcgcaag 120
   cgacgccggg aaatgcccac atccgggaaa cctgcagcgg agtgcggcgg cggcgacact 180
   gagtggaagg caaaatggcg gcggcggcgg cggtggcctg gtgttaaggg gagagccagg 240
   tcctcacgac ccctgggacg ggccgcgctg gcccgcggca gcccccccgt tcgtctcccc 300
   gctctgcccc accagggata cttggggttg ctgggacgga ctctggccgc ctcagcgtcc 360
   gccctcaggc ccgtggccgc tgtccaggag ctctgctctc ccctccagag ttaattattt 420
   atattgtaaa gaattttaac agtcctgggg acttccttga aggatcattt tcacttttgc 480
   tcagaagaaa gctctggatc tatcaaataa agaagtcctt cgtgtgggct acatatatag 540
   atgttttcat gaagaggagt gaaaagccag aaggatatag acaaatgagg cctaagacct 600
   ttcctgccag taactatact gtcagtagcc ggcaaatgtt acaagaaatt cgggaatccc 660
   ttaggaattt atctaaacca tctgatgctg ctaaggctga gcataacatg agtaaaatgt 720
   caaccgaaga tcctcgacaa gtcagaaatc cacccaaatt tgggacgcat cataaagcct 780
   tgcaggaaat tcgaaactct ctgcttccat ttgcaaatga aacaaattct tctcggagta 840
   cttcagaagt taatccacaa atgcttcaag acttgcaagc tgctggattt gatgaggata 900
   tggttataca agctcttcag aaaactaaca acagaagtat agaagcagca attgaattca 960
   ttagtaaaat gagttaccaa gatcctcgac gagagcagat ggctgcagca gctgccagac 1020
   ctattaatgc cagcatgaaa ccagggaatg tgcagcaatc agttaaccgc aaacagagct 1080
   ggaaaggttc taaagaatcc ttagttcctc agaggcatgg cccgccacta ggagaaagtg 1140
   tggcctatca ttctgagagt cccaactcac agacagatgt aggaagacct ttgtctggat 1200
   ctggtatatc agcatttgtt caagctcacc ctagcaacgg acagagagtg aaccccccac 1260
   caccacctca agtaaggagt gttactcctc caccacctcc aagaggccag actccccctc 1320
   caagaggtac aactccacct cccccttcat gggaaccaaa ctctcaaaca aagcgctatt 1380
   ctggaaacat ggaatacgta atctcccgaa tctctcctgt cccacctggg gcatggcaag 1440
   agggctatcc tccaccacct ctcaacactt cccccatgaa tcctcctaat caaggacaga 1500
   gaggcattag ttctgttcct gttggcagac aaccaatcat catgcagagt tctagcaaat 1560
   ttaactttcc atcagggaga cctggaatgc agaatggtac tggacaaact gatttcatga 1620
   tacaccaaaa tgttgtccct gctggcactg tgaatcggca gccaccacct ccatatcctc 1680
   tgacagcagc taatggacaa agcccttctg ctttacaaac agggggatct gctgctcctt 1740
   cgtcatatac aaatggaagt attcctcagt ctatgatggt gccaaacaga aatagtcata 1800
   acatggaact atataacatt agtgtacctg gactgcaaac aaattggcct cagtcatctt 1860
   ctgctccagc ccagtcatcc ccgagcagtg ggcatgaaat ccctacatgg caacctaaca 1920
   taccagtgag gtcaaattct tttaataacc cattaggaaa tagagcaagt cactctgcta 1980
   attctcagcc ttctgctaca acagtcactg caattacacc agctcctatt caacagcctg 2040
   tgaaaagtat gcgtgtatta aaaccagagc tacagactgc tttagcacct acacaccctt 2100
   cttggatacc acagccaatt caaactgttc aacccagtcc ttttcctgag ggaaccgctt 2160
   caaatgtgac tgtgatgcca cctgttgctg aagctccaaa ctatcaagga ccaccaccac 2220
   cctacccaaa acatctgctg caccaaaacc catctgttcc tccatacgag tcaatcagta 2280
   agcctagcaa agaggatcag ccaagcttgc ccaaggaaga tgagagtgaa aagagttatg 2340
   aaaatgttga tagtggggat aaagaaaaga aacagattac aacttcacct attactgtta 2400
   ggaaaaacaa gaaagatgaa gagcgaaggg aatctcgtat tcaaagttat tctcctcaag 2460
   catttaaatt ctttatggag caacatgtag aaaatgtact caaatctcat cagcagcgtc 2520
   tacatcgtaa aaaacaatta gagaatgaaa tgatgcgggt tggattatct caagatgccc 2580
   aggatcaaat gagaaagatg ctttgccaaa aagaatctaa ttacatccgt cttaaaaggg 2640
   ctaaaatgga caagtctatg tttgtgaaga taaagacact aggaatagga gcatttggtg 2700
   aagtctgtct agcaagaaaa gtagatacta aggctttgta tgcaacaaaa actcttcgaa 2760
   agaaagatgt tcttcttcga aatcaagtcg ctcatgttaa ggctgagaga gatatcctgg 2820
   ctgaagctga caatgaatgg gtagttcgtc tatattattc attccaagat aaggacaatt 2880
   tatactttgt aatggactac attcctgggg gtgatatgat gagcctatta attagaatgg 2940
   gcatctttcc agaaagtctg gcacgattct acatagcaga acttacctgt gcagttgaaa 3000
   gtgttcataa aatgggtttt attcatagag atattaaacc tgataatatt ttgattgatc 3060
   gtgatggtca tattaaattg actgactttg gcctctgcac tggcttcaga tggacacacg 3120
   attctaagta ctatcagagt ggtgaccatc cacggcaaga tagcatggat ttcagtaatg 3180
   aatgggggga tccctcaagc tgtcgatgtg gagacagact gaagccatta gagcggagag 3240
   ctgcacgcca gcaccagcga tgtctagcac attctttggt tgggactccc aattatattg 3300
   cacctgaagt gttgctacga acaggataca cacagttgtg tgattggtgg agtgttggtg 3360
   ttattctttt tgaaatgttg gtgggacaac ctcctttctt ggcacaaaca ccattagaaa 3420
   cacaaatgaa ggttatcaac tggcaaacat ctcttcacat tccaccacaa gctaaactca 3480
   gtcctgaagc ttctgatctt attattaaac tttgccgagg acccgaagat cgcttaggca 3540
   agaatggtgc tgatgaaata aaagctcatc cattttttaa aacaattgac ttctccagtg 3600
   acctgagaca gcagtctgct tcatacattc ctaaaatcac acacccaaca gatacatcaa 3660
   attttgatcc tgttgatcct gataaattat ggagtgatga taacgaggaa gaaaatgtaa 3720
   atgacactct caatggatgg tataaaaatg gaaagcatcc tgaacatgca ttctatgaat 3780
   ttaccttccg aaggtttttt gatgacaatg gctacccata taattatccg aagcctattg 3840
   aatatgaata cattaattca caaggctcag agcagcagtc ggatgaagat gatcaaaaca 3900
   caggctcaga gattaaaaat cgcgatctag tatatgttta acacactagt aaataaatgt 3960
   aatgaggatt tgtaaaaggg cctgaaatgc gaggtgtttt gaggttctga gagtaaaatt 4020
   atgcaaatat gacagagcta tatatgtgtg ctctgtgtac aatattttat tttcctaaat 4080
   tatgggaaat ccttttaaaa tgttaattta ttccagccgt ttaaatcagt atttagaaaa 4140
   aaattgttat aaggaaagta aattatgaac tgaatattat agtcagttct tggtacttaa 4200
   agtacttaaa ataagtagtg ctttgtttaa aaggagaaac ctggtatcta tttgtatata 4260
   tgctaaataa ttttaaaata caagagtttt tgaaattttt ttgaaagaca gttttagttt 4320
   tatcttgctt taaccaaata tgaaacatac cccctatttt acagagctct tttttcccct 4380
   cataaccttg tttttggtag aaaataagct agagaaatta agccatcgtg ttggtgagtg 4440
   ttcctaggct aatgataatc tgtataattc acatcctgaa actaaggaat acagggttga 4500
   aaaaatatta atatgtttgt cagaaggaaa aataatgcat ttatcttccc ccccaccccc 4560
   cgccccatgg aatatttaat ctatttaatc ttcttgcatt tatttctcaa gaattactgg 4620
   ctttaaaaga agccaaagca ctactagctt tttttccata ttggtatttt tgatgctgct 4680
   tccaatttta aaagggaaca aagctgccat aaatcgaaat gttcaatact aaaagctaaa 4740
   atatttctca ccatcctaag cagataatta ttttaatttt catatacttt tcctgtatag 4800
   taactatttt gattatatca tcaatgttac ctgtttcctc tttcagaaca gtgctgcata 4860
   tacagattgt tattggcaaa ggaaaatctg gctatctggc aatattttac ctaagcgcag 4920
   attaattggt gaaaaaatta actcttaaga tggccattaa taattaggaa agtttacaga 4980
   gtggtcttag tagaaaattc aagtcctcct aatttattta aggttcaata atgcgttcaa 5040
   catgcctgtt atgtataacg cttaggttct aaggaagatt aaggtttcat accaaaatac 5100
   atgtagctta tcttttagga aggggaaaaa ggctccattt tgaccatagt aaaatttgtg 5160
   ttgtgtttta tttccttttc ttaagctcca ctgataaggg attgttttta tcaaaagtta 5220
   ctatttgtag attggaggca taattttagt gattttcata cttttagctt tcttcgcata 5280
   aaagctaatt gaaaccgtat atgtagtaaa attaaaggca gagctgttgc agttgaattg 5340
   gagagttagg gcaaagaaca cttattagcc cacacttccc acctttctac aggtggtcct 5400
   ttcagagctc agcctgaaaa cccactactg tgttatcgtg cgtcttttgg ggttagtggt 5460
   tcttttgaga atctgaagga agctgtggac tcttcctaga aaaaaaaacc acacatacac 5520
   atacaatgtt gcatgcagtt tcaagggatt ttggacatat tgaaacctat cacaggctgt 5580
   aggttatgga cctctgtgcc atgagaaaat tgatacatta aactaagaac tttgttttta 5640
   acttaccaat cactactcag cacatcttat ataagctgat aatttgtgat ggaaaaggtc 5700
   tgtagcatgt gatataaggt gaccttatga atgcctctct tgctggtaca ttaagttgtt 5760
   ttaatatatc atttggaggg gactgaaatg ttaggctcat tacaagcttg atacagaaat 5820
   atttctgaag gatttctaat cagaattgta aaacaatgtg ctatcatgaa atcgcagtct 5880
   tcacctcatg gttcatggaa catttggtta gtcccataaa atcctatgca aaacaaagta 5940
   gttcaagaat ttttaggtgg gtagtcacat ttataaggta ttcctcttac tctttgggct 6000
   ttttcagtct gatttattta aattttcatt tagttgtttt acttttggac taaggtgcaa 6060
   tacagtagaa gataactttg ttacatttat gttgtaggaa aactaaggtg ctgtctcctc 6120
   ccccttccct tcccacaaaa tctgtattcc ccctattgct gaaatgtaac agacactaca 6180
   aattttgtat tctttttttg ttttttgttt tgagacaggg tctcactctg tcacccaggc 6240
   tggagggcag tggcgcttca cagctcactg catcctcaac cttgggggct cacgcagtcc 6300
   tcccgcctca gcctcccaag tagctgggca tgcgccacca agcccagcta atttttgtat 6360
   ctttagtaga gatggggttt cgccatgttg cccaggttgg tgtggaattc ctgggctcca 6420
   gttatatgcc cacctcagcc tcccaaagtg ctgggattac agacgtgacc caccgcgcct 6480
   ggcgcaaata tgtattcttt taaaatttcc tctgatacta taagcttttt gcatttatct 6540
   gaagcagtat acatgccttt ggtatcagca attttaacag tttggatata cttatcagct 6600
   atcttattcc aaaactacat ctacttcttc cagtatagaa tctggtgctt cctgaccaaa 6660
   aagatgagaa aaacaatgtt aaaaatatag atgctttcca ttgaaatgga gtgaaaacat 6720
   tggttctata tgttttcttt taaaataatt ttcttattaa aaacttgctg tctttattat 6780
   acttaccctt tttatgcata tcaatagtat ttataagatg tgttctataa ttatgtaatt 6840
   gtagatactg ttatgcattg tccagtgaca tcataaggca ggccctactg ctgtatcttt 6900
   tctaccttct tatttgtaat agaaactata gaatgtatga ctaaaaagtc actttgagat 6960
   tgactttttt aaaaagttat taccttctgc tgttgcaaag tgcaaaactg tgagtggaat 7020
   tgttttattc tgacttaatg tgttagaaat tagagaatac agtgggagga tttttagaca 7080
   ttgctgctgc tgttacccaa ggtattttag ataaaaaatt tttaataaac atccctttgg 7140
   tatttaaagt ggaacattta gcctgttcat tttaatctaa agcaaaaagt aatttgggtc 7200
   aaaatattgg tatatttgta aagcgcctta atatatccct ttgtggaagg cactacacag 7260
   tttactttta tattgtattg tgtatataag tattttgtat taaaattgaa tcagtggcaa 7320
   cattaaagtt ttataaaatc atgctttgtt agaaaaagaa ttacagcttt gcaatataac 7380
   taattgtttc gcataattct gaatgtaata gatatgaata atcagcctgt gtttttaatg 7440
   aacttatttg tattttccca atcattttct ctagtgtaat gtttgctggg ataataaaaa 7500
   aaattcaaat ctttcaaaaa aaaaaaaaaa aaa                              7533
<210> 30
   <211> 1130
   <212> PRT
   <213> Homo sapiens
<400> 30
   Met Lys Arg Ser Glu Lys Pro Glu Gly Tyr Arg Gln Met Arg Pro Lys
    1              5                   10                  15
   Thr Phe Pro Ala Ser Asn Tyr Thr Val Ser Ser Arg Gln Met Leu Gln
               20                  25                  30
   Glu Ile Arg Glu Ser Leu Arg Asn Leu Ser Lys Pro Ser Asp Ala Ala
           35                  40                  45
   Lys Ala Glu His Asn Met Ser Lys Met Ser Thr Glu Asp Pro Arg Gln
       50                  55                  60
   Val Arg Asn Pro Pro Lys Phe Gly Thr His His Lys Ala Leu Gln Glu
   65                  70                  75                  80
   Ile Arg Asn Ser Leu Leu Pro Phe Ala Asn Glu Thr Asn Ser Ser Arg
                   85                  90                  95
   Ser Thr Ser Glu Val Asn Pro Gln Met Leu Gln Asp Leu Gln Ala Ala
               100                 105                 110
   Gly Phe Asp Glu Asp Met Val Ile Gln Ala Leu Gln Lys Thr Asn Asn
           115                 120                 125
   Arg Ser Ile Glu Ala Ala Ile Glu Phe Ile Ser Lys Met Ser Tyr Gln
       130                 135                 140
   Asp Pro Arg Arg Glu Gln Met Ala Ala Ala Ala Ala Arg Pro Ile Asn
   145                 150                 155                 160
   Ala Ser Met Lys Pro Gly Asn Val Gln Gln Ser Val Asn Arg Lys Gln
                   165                 170                 175
   Ser Trp Lys Gly Ser Lys Glu Ser Leu Val Pro Gln Arg His Gly Pro
               180                 185                 190
   Pro Leu Gly Glu Ser Val Ala Tyr His Ser Glu Ser Pro Asn Ser Gln
           195                 200                 205
   Thr Asp Val Gly Arg Pro Leu Ser Gly Ser Gly Ile Ser Ala Phe Val
       210                 215                 220
   Gln Ala His Pro Ser Asn Gly Gln Arg Val Asn Pro Pro Pro Pro Pro
   225                 230                 235                 240
   Gln Val Arg Ser Val Thr Pro Pro Pro Pro Pro Arg Gly Gln Thr Pro
                   245                 250                 255
   Pro Pro Arg Gly Thr Thr Pro Pro Pro Pro Ser Trp Glu Pro Asn Ser
               260                 265                 270
   Gln Thr Lys Arg Tyr Ser Gly Asn Met Glu Tyr Val Ile Ser Arg Ile
           275                 280                 285
   Ser Pro Val Pro Pro Gly Ala Trp Gln Glu Gly Tyr Pro Pro Pro Pro
       290                 295                 300
   Leu Asn Thr Ser Pro Met Asn Pro Pro Asn Gln Gly Gln Arg Gly Ile
   305                 310                 315                 320
   Ser Ser Val Pro Val Gly Arg Gln Pro Ile Ile Met Gln Ser Ser Ser
                   325                 330                 335
   Lys Phe Asn Phe Pro Ser Gly Arg Pro Gly Met Gln Asn Gly Thr Gly
               340                 345                 350
   Gln Thr Asp Phe Met Ile His Gln Asn Val Val Pro Ala Gly Thr Val
           355                 360                 365
   Asn Arg Gln Pro Pro Pro Pro Tyr Pro Leu Thr Ala Ala Asn Gly Gln
       370                 375                 380
   Ser Pro Ser Ala Leu Gln Thr Gly Gly Ser Ala Ala Pro Ser Ser Tyr
   385                 390                 395                 400
   Thr Asn Gly Ser Ile Pro Gln Ser Met Met Val Pro Asn Arg Asn Ser
                   405                 410                 415
   His Asn Met Glu Leu Tyr Asn Ile Ser Val Pro Gly Leu Gln Thr Asn
               420                 425                 430
   Trp Pro Gln Ser Ser Ser Ala Pro Ala Gln Ser Ser Pro Ser Ser Gly
           435                 440                 445
   His Glu Ile Pro Thr Trp Gln Pro Asn Ile Pro Val Arg Ser Asn Ser
       450                 455                 460
   Phe Asn Asn Pro Leu Gly Asn Arg Ala Ser His Ser Ala Asn Ser Gln
   465                 470                 475                 480
   Pro Ser Ala Thr Thr Val Thr Ala Ile Thr Pro Ala Pro Ile Gln Gln
                   485                 490                 495
   Pro Val Lys Ser Met Arg Val Leu Lys Pro Glu Leu Gln Thr Ala Leu
               500                 505                 510
   Ala Pro Thr His Pro Ser Trp Ile Pro Gln Pro Ile Gln Thr Val Gln
           515                 520                 525
   Pro Ser Pro Phe Pro Glu Gly Thr Ala Ser Asn Val Thr Val Met Pro
       530                 535                 540
   Pro Val Ala Glu Ala Pro Asn Tyr Gln Gly Pro Pro Pro Pro Tyr Pro
   545                 550                 555                 560
   Lys His Leu Leu His Gln Asn Pro Ser Val Pro Pro Tyr Glu Ser Ile
                   565                 570                 575
   Ser Lys Pro Ser Lys Glu Asp Gln Pro Ser Leu Pro Lys Glu Asp Glu
               580                 585                 590
   Ser Glu Lys Ser Tyr Glu Asn Val Asp Ser Gly Asp Lys Glu Lys Lys
           595                 600                 605
   Gln Ile Thr Thr Ser Pro Ile Thr Val Arg Lys Asn Lys Lys Asp Glu
       610                 615                 620
   Glu Arg Arg Glu Ser Arg Ile Gln Ser Tyr Ser Pro Gln Ala Phe Lys
   625                 630                 635                 640
   Phe Phe Met Glu Gln His Val Glu Asn Val Leu Lys Ser His Gln Gln
                   645                 650                 655
   Arg Leu His Arg Lys Lys Gln Leu Glu Asn Glu Met Met Arg Val Gly
               660                 665                 670
   Leu Ser Gln Asp Ala Gln Asp Gln Met Arg Lys Met Leu Cys Gln Lys
           675                 680                 685
   Glu Ser Asn Tyr Ile Arg Leu Lys Arg Ala Lys Met Asp Lys Ser Met
       690                 695                 700
   Phe Val Lys Ile Lys Thr Leu Gly Ile Gly Ala Phe Gly Glu Val Cys
   705                 710                 715                 720
   Leu Ala Arg Lys Val Asp Thr Lys Ala Leu Tyr Ala Thr Lys Thr Leu
                   725                 730                 735
   Arg Lys Lys Asp Val Leu Leu Arg Asn Gln Val Ala His Val Lys Ala
               740                 745                 750
   Glu Arg Asp Ile Leu Ala Glu Ala Asp Asn Glu Trp Val Val Arg Leu
           755                 760                 765
   Tyr Tyr Ser Phe Gln Asp Lys Asp Asn Leu Tyr Phe Val Met Asp Tyr
       770                 775                 780
   Ile Pro Gly Gly Asp Met Met Ser Leu Leu Ile Arg Met Gly Ile Phe
   785                 790                 795                 800
   Pro Glu Ser Leu Ala Arg Phe Tyr Ile Ala Glu Leu Thr Cys Ala Val
                   805                 810                 815
   Glu Ser Val His Lys Met Gly Phe Ile His Arg Asp Ile Lys Pro Asp
               820                 825                 830
   Asn Ile Leu Ile Asp Arg Asp Gly His Ile Lys Leu Thr Asp Phe Gly
           835                 840                 845
   Leu Cys Thr Gly Phe Arg Trp Thr His Asp Ser Lys Tyr Tyr Gln Ser
       850                 855                 860
   Gly Asp His Pro Arg Gln Asp Ser Met Asp Phe Ser Asn Glu Trp Gly
   865                 870                 875                 880
   Asp Pro Ser Ser Cys Arg Cys Gly Asp Arg Leu Lys Pro Leu Glu Arg
                   885                 890                 895
   Arg Ala Ala Arg Gln His Gln Arg Cys Leu Ala His Ser Leu Val Gly
               900                 905                 910
   Thr Pro Asn Tyr Ile Ala Pro Glu Val Leu Leu Arg Thr Gly Tyr Thr
           915                 920                 925
   Gln Leu Cys Asp Trp Trp Ser Val Gly Val Ile Leu Phe Glu Met Leu
       930                 935                 940
   Val Gly Gln Pro Pro Phe Leu Ala Gln Thr Pro Leu Glu Thr Gln Met
   945                 950                 955                 960
   Lys Val Ile Asn Trp Gln Thr Ser Leu His Ile Pro Pro Gln Ala Lys
                   965                 970                 975
   Leu Ser Pro Glu Ala Ser Asp Leu Ile Ile Lys Leu Cys Arg Gly Pro
               980                 985                 990
   Glu Asp Arg Leu Gly Lys Asn Gly Ala Asp Glu Ile Lys Ala His Pro
           995                 1000                1005
   Phe Phe Lys Thr Ile Asp Phe Ser Ser Asp Leu Arg Gln Gln Ser Ala
       1010                1015                1020
   Ser Tyr Ile Pro Lys Ile Thr His Pro Thr Asp Thr Ser Asn Phe Asp
   1025                1030                1035                1040
   Pro Val Asp Pro Asp Lys Leu Trp Ser Asp Asp Asn Glu Glu Glu Asn
                   1045                1050                1055
   Val Asn Asp Thr Leu Asn Gly Trp Tyr Lys Asn Gly Lys His Pro Glu
               1060                1065                1070
   His Ala Phe Tyr Glu Phe Thr Phe Arg Arg Phe Phe Asp Asp Asn Gly
           1075                1080                1085
   Tyr Pro Tyr Asn Tyr Pro Lys Pro Ile Glu Tyr Glu Tyr Ile Asn Ser
       1090                1095                1100
   Gln Gly Ser Glu Gln Gln Ser Asp Glu Asp Asp Gln Asn Thr Gly Ser
   1105                1110                1115                1120
   Glu Ile Lys Asn Arg Asp Leu Val Tyr Val
                   1125                1130
<210> 31
   <211> 5581
   <212> DNA
   <213> Homo sapiens
<400> 31
   attcccgtgc cctagtttag cgacgtgtgg ggggtttggc tttctcttct gatgtaagct 60
   cacctgcgct tagctcccca gccggactct ggagatgggg attctgacat cattcacatc 120
   ttgccatcac atccttcagc ccaactccag aacttctggg gagctttccc tcggggatgg 180
   agtgcggcct ggagcaaacc cgtttgtgaa atgcaagtct ccagtgtccc gagggttggg 240
   aacggggggc gaggcgagga atggaccctg aagaagagag tgataatctt caaaatgaag 300
   actttggaaa attttaggtt ctctatagga actacaaaaa tggaaggaaa gaacattttc 360
   aaaaggaaat tattttgaaa gtatgtttac aacaaactga tactattgac agtttttttt 420
   tttaaataat aaaacacttt aagaagattg tatttatggt aaaaggaaac tggactaaca 480
   atgaggccaa agacttttcc tgccacgact tattctggaa atagccggca gcgactgcaa 540
   gagattcgtg aggggttaaa acagccatcc aagtcttcgg ttcaggggct acccgcagga 600
   ccaaacagtg acacttccct ggatgccaaa gtcctgggga gcaaagatgc caccaggcag 660
   cagcagcaga tgagagccac cccaaagttc ggaccttatc agaaagcctt gagggaaatc 720
   agatattcct tgttgccttt tgctaatgaa tcgggcacct ctgcagctgc agaagtgaac 780
   cggcaaatgc tgcaggaact ggtgaacgca ggatgcgacc aggagatggc tggccgagct 840
   ctcaagcaga ctggcagcag gagcatcgag gccgccctgg agtacatcag caagatgggc 900
   tacctggacc cgaggaatga gcagattgtg cgggtcatta agcagacctc cccaggaaag 960
   gggctcatgc caaccccagt gacgcggagg cccagcttcg aaggaaccgg cgattcgttt 1020
   gcgtcctacc accagctgag cggtaccccc tacgagggcc caagcttcgg cgctgacggc 1080
   cccacggcgc tggaggagat gccgcggccg tacgtggact acctttcccc cggagtcggc 1140
   ccccacgggc ccggccacca gcaccagcac ccacccaagg gctacggtgc cagcgtagag 1200
   gcagcagggg cacacttccc gctgcagggc gcgcactacg ggcggccgca cctgctggtg 1260
   cctggggaac ccctgggcta cggagtgcag cgcagcccct ccttccagag caagacgccg 1320
   ccggagaccg ggggttacgc cagcctgccc acgaagggcc agggaggacc gccaggcgcc 1380
   ggcctcgctt tcccaccccc tgccgccggg ctctacgtgc cgcacccaca ccacaagcag 1440
   gccggtcccg cggcccacca gctgcatgtg ctgggctccc gcagccaggt gttcgccagc 1500
   gacagccccc cgcagagcct gctcactccc tcgcggaaca gcctcaacgt ggacctgtat 1560
   gaattgggca gcacctccgt ccagcagtgg ccggctgcca ccctggcccg ccgggactcc 1620
   ctgcagaagc cgggcctgga ggcgccgccg cgcgcgcacg tggccttccg gcctgactgc 1680
   ccagtgccca gcaggaccaa ctccttcaac agccaccagc cgcggcccgg tccgcctggc 1740
   aaggccgagc cctccctgcc cgcccccaac accgtgacgg ctgtcacggc cgcgcacatc 1800
   ttgcacccgg tgaagagcgt gcgtgtgctg aggccggagc cgcagacggc tgtggggccc 1860
   tcgcaccccg cctgggtgcc cgcgcctgcc ccggcccccg cccccgcccc cgccccggct 1920
   gcggagggct tggacgccaa ggaggagcat gccctggcgc tgggcggcgc aggcgccttc 1980
   ccgctggacg tggagtacgg aggcccagac cggaggtgcc cgcctccgcc ctacccgaag 2040
   cacctgctgc tgcgcagcaa gtcggagcag tacgacctgg acagcctgtg cgcaggcatg 2100
   gagcagagcc tccgtgcggg ccccaacgag cccgagggcg gcgacaagag ccgcaaaagc 2160
   gccaaggggg acaaaggcgg aaaggataaa aagcagattc agacctctcc cgttcccgtc 2220
   cgcaaaaaca gcagagacga agagaagaga gagtcacgca tcaagagcta ctcgccatac 2280
   gcctttaagt tcttcatgga gcagcacgtg gagaatgtca tcaaaaccta ccagcagaag 2340
   gttaaccgga ggctgcagct ggagcaagaa atggccaaag ctggactctg tgaagctgag 2400
   caggagcaga tgcggaagat cctctaccag aaagagtcta attacaacag gttaaagagg 2460
   gccaagatgg acaagtctat gtttgtcaag atcaaaaccc tggggatcgg tgcctttgga 2520
   gaagtgtgcc ttgcttgtaa ggtggacact cacgccctgt acgccatgaa gaccctaagg 2580
   aaaaaggatg tcctgaaccg gaatcaggtg gcccacgtca aggccgagag ggacatcctg 2640
   gccgaggcag acaatgagtg ggtggtcaaa ctctactact ccttccaaga caaagacagc 2700
   ctgtactttg tgatggacta catccctggt ggggacatga tgagcctgct gatccggatg 2760
   gaggtcttcc ctgagcacct ggcccggttc tacatcgcag agctgacttt ggccattgag 2820
   agtgtccaca agatgggctt catccaccga gacatcaagc ctgataacat tttgatagat 2880
   ctggatggtc acattaaact cacagatttc ggcctctgca ctgggttcag gtggactcac 2940
   aattccaaat attaccagaa agggagccat gtcagacagg acagcatgga gcccagcgac 3000
   ctctgggatg atgtgtctaa ctgtcggtgt ggggacaggc tgaagaccct agagcagagg 3060
   gcgcggaagc agcaccagag gtgcctggca cattcactgg tggggactcc aaactacatc 3120
   gcacccgagg tgctcctccg caaagggtac actcaactct gtgactggtg gagtgttgga 3180
   gtgattctct tcgagatgct ggtggggcag ccgccctttt tggcacctac tcccacagaa 3240
   acccagctga aggtgatcaa ctgggagaac acgctccaca ttccagccca ggtgaagctg 3300
   agccctgagg ccagggacct catcaccaag ctgtgctgct ccgcagacca ccgcctgggg 3360
   cggaatgggg ccgatgacct gaaggcccac cccttcttca gcgccattga cttctccagt 3420
   gacatccgga agcagccagc cccctacgtt cccaccatca gccaccccat ggacacctcg 3480
   aatttcgacc ccgtagatga agaaagccct tggaacgatg ccagcgaagg tagcaccaag 3540
   gcctgggaca cactcacctc gcccaataac aagcatcctg agcacgcatt ttacgaattc 3600
   accttccgaa ggttctttga tgacaatggc tacccctttc gatgcccaaa gccttcagga 3660
   gcagaagctt cacaggctga gagctcagat ttagaaagct ctgatctggt ggatcagact 3720
   gaaggctgcc agcctgtgta cgtgtagatg ggggccaggc acccccacca ctcgctgcct 3780
   cccaggtcag ggtcccggag ccggtgccct cacaggccaa tagggaagcc gagggctgtt 3840
   ttgttttaaa ttagtccgtc gattacttca cttgaaattc tgctcttcac caagaaaacc 3900
   caaacaggac acttttgaaa acaggactca gcatcgcttt caataggctt ttcaggacct 3960
   tcactgcatt aaaacaatat ttttgaaaat ttagtacagt ttagaaagag cacttatttt 4020
   gtttatatcc attttttctt actaaattat agggattaac tttgacaaat catgctgctg 4080
   ttattttcta catttgtatt ttatccatag cacttattca catttaggaa aagacataaa 4140
   aactgaagaa cattgatgag aaatctctgt gcaataatgt aaaaaaaaaa aaagataaca 4200
   ctctgctcaa tgtcacggag accattttat ccacacaatg gtttttgttt tttatttttt 4260
   cccatgtttc aaaattgtga tataatgata taatgttaaa agctgctttt tttggctttt 4320
   tgcatatcta gtataatagg aagtgtgagc aaggtgatga tgtggctgtg atttccgacg 4380
   tctggtgtgt ggagagtact gcatgagcag agttcttcta ttataaaatt accatatctt 4440
   gccattcaca gcaggtcctg tgaatacgtt tttactgagt gtctttaaat gaggtgttct 4500
   agacagtgtg ctgataatgt attgtgcggg tgacctcttc gctatgattg tatctcttac 4560
   tgttttgtta aagaaatgca gatgtgtaac tgagaagtga tttgtgtgtg tgtcttggtt 4620
   gtgattggat tctttggggg gggggaactg aaacatttgt catatactga acttatatac 4680
   atcaaaaggg attaatacag cgatgccaaa aagtttaatc acggacacat gtccgtttct 4740
   gtagtccgta tgctctttca ttcttggtag agctggtatg tggaatgcca tacctctgac 4800
   cctactactt acctttttac tgacagactg cccacactga aagcttcagt gaatgttctt 4860
   agtcctgttt tcttctgtta ctgtcaggaa actgagtgat ctaatggttc tctcactttt 4920
   tttttgttct tttagtgtac tttgaagtat caaatcttaa cttggtttaa acaatacata 4980
   ttcctaacct ttgtaaaaaa gcaaagattc ttcaaaatga cattgaaata aaaagtaagc 5040
   catacgtatt ttcttagaag tatagatgta tgtgcgtgta tacacacaca cacacacaca 5100
   cagagataaa cacaatattc cttatttcaa attagtatga ttcctattta aagtgattta 5160
   tatttgagta aaaagttcaa ttcttttttg ctttttaaaa aatctgatgc ttcataattt 5220
   tcattatatt attccacata tttttccttg aagttcttag cataatgtat ccattactta 5280
   gtatatatct aggcaacaac acttagaagt ttatcagtgt ttaaactaaa aaaataaaga 5340
   ttcctgtgta ctggtttaca tttgtgtgag tggcatactc aagtctgctg tgcctgtcgt 5400
   cgtgactgtc agtattctcg ctattttata gtcgtgccat gttgttactc acagcgctct 5460
   gacatacttt catgtggtag gttctttctc aggaactcag tttaactatt atttattgat 5520
   atatcattac ctttgaaaag cttctactgg cacaatttat tattaaaatt ttgaatccaa 5580
   a                                                                 5581
<210> 32
   <211> 1088
   <212> PRT
   <213> Homo sapiens
<400> 32
   Met Arg Pro Lys Thr Phe Pro Ala Thr Thr Tyr Ser Gly Asn Ser Arg
    1               5                  10                  15
   Gln Arg Leu Gln Glu Ile Arg Glu Gly Leu Lys Gln Pro Ser Lys Ser
               20                  25                  30
   Ser Val Gln Gly Leu Pro Ala Gly Pro Asn Ser Asp Thr Ser Leu Asp
           35                  40                  45
   Ala Lys Val Leu Gly Ser Lys Asp Ala Thr Arg Gln Gln Gln Gln Met
   50 55 60
   Arg Ala Thr Pro Lys Phe Gly Pro Tyr Gln Lys Ala Leu Arg Glu Ile
   65 70 75 80
   Arg Tyr Ser Leu Leu Pro Phe Ala Asn Glu Ser Gly Thr Ser Ala Ala
   85 90 95
   Ala Glu Val Asn Arg Gln Met Leu Gln Glu Leu Val Asn Ala Gly Cys
   100 105 110
   Asp Gln Glu Met Ala Gly Arg Ala Leu Lys Gln Thr Gly Ser Arg Ser
   115 120 125
   Ile Glu Ala Ala Leu Glu Tyr Ile Ser Lys Met Gly Tyr Leu Asp Pro
   130 135 140
   Arg Asn Glu Gln Ile Val Arg Val Ile Lys Gln Thr Ser Pro Gly Lys
   145 150 155 160
   Gly Leu Met Pro Thr Pro Val Thr Arg Arg Pro Ser Phe Glu Gly Thr
   165 170 175
   Gly Asp Ser Phe Ala Ser Tyr His Gln Leu Ser Gly Thr Pro Tyr Glu
   180 185 190
   Gly Pro Ser Phe Gly Ala Asp Gly Pro Thr Ala Leu Glu Glu Met Pro
   195 200 205
   Arg Pro Tyr Val Asp Tyr Leu Phe Pro Gly Val Gly Pro His Gly Pro
   210 215 220
   Gly His Gln His Gln His Pro Pro Lys Gly Tyr Gly Ala Ser Val Glu
   225 230 235 240
   Ala Ala Gly Ala His Phe Pro Leu Gln Gly Ala His Tyr Gly Arg Pro
   245 250 255
   His Leu Leu Val Pro Gly Glu Pro Leu Gly Tyr Gly Val Gln Arg Ser
   260 265 270
   Pro Ser Phe Gln Ser Lys Thr Pro Pro Glu Thr Gly Gly Tyr Ala Ser
   275 280 285
   Leu Pro Thr Lys Gly Gln Gly Gly Pro Pro Gly Ala Gly Leu Ala Phe
   290 295 300
   Pro Pro Pro Ala Ala Gly Leu Tyr Val Pro His Pro His His Lys Gln
   305 310 315 320
   Ala Gly Pro Ala Ala His Gln Leu His Val Leu Gly Ser Arg Ser Gln
   325 330 335
   Val Phe Ala Ser Asp Ser Pro Pro Gln Ser Leu Leu Thr Pro Ser Arg
   340 345 350
   Asn Ser Leu Asn Val Asp Leu Tyr Glu Leu Gly Ser Thr Ser Val Gln
   355 360 365
   Gln Trp Pro Ala Ala Thr Leu Ala Arg Arg Asp Ser Leu Gln Lys Pro
   370 375 380
   Gly Leu Glu Ala Pro Pro Arg Ala His Val Ala Phe Arg Pro Asp Cys
   385 390 395 400
   Pro Val Pro Ser Arg Thr Asn Ser Phe Asn Ser His Gln Pro Arg Pro
   405 410 415
   Gly Pro Pro Gly Lys Ala Glu Pro Ser Leu Pro Ala Pro Asn Thr Val
   420 425 430
   Thr Ala Val Thr Ala Ala His Ile Leu His Pro Val Lys Ser Val Arg
   435 440 445
   Val Leu Arg Pro Glu Pro Gln Thr Ala Val Gly Pro Ser His Pro Ala
   450 455 460
   Trp Val Pro Ala Pro Ala Pro Ala Pro Ala Pro Ala Pro Ala Pro Ala
   465 470 475 480
   Ala Glu Gly Leu Asp Ala Lys Glu Glu His Ala Leu Ala Leu Gly Gly
   485 490 495
   Ala Gly Ala Phe Pro Leu Asp Val Glu Tyr Gly Gly Pro Asp Arg Arg
   500 505 510
   Cys Pro Pro Pro Pro Tyr Pro Lys His Leu Leu Leu Arg Ser Lys Ser
   515 520 525
   Glu Gln Tyr Asp Leu Asp Ser Leu Cys Ala Gly Met Glu Gln Ser Leu
   530 535 540
   Arg Ala Gly Pro Asn Glu Pro Glu Gly Gly Asp Lys Ser Arg Lys Ser
   545                 550                 555                 560
   Ala Lys Gly Asp Lys Gly Gly Lys Asp Lys Lys Gln Ile Gln Thr Ser
                   565                 570                 575
   Pro Val Pro Val Arg Lys Asn Ser Arg Asp Glu Glu Lys Arg Glu Ser
               580                 585                 590
   Arg Ile Lys Ser Tyr Ser Pro Tyr Ala Phe Lys Phe Phe Met Glu Gln
           595                 600                 605
   His Val Glu Asn Val Ile Lys Thr Tyr Gln Gln Lys Val Asn Arg Arg
       610                 615                 620
   Leu Gln Leu Glu Gln Glu Met Ala Lys Ala Gly Leu Cys Glu Ala Glu
   625                 630                 635                 640
   Gln Glu Gln Met Arg Lys Ile Leu Tyr Gln Lys Glu Ser Asn Tyr Asn
                   645                 650                 655
   Arg Leu Lys Arg Ala Lys Met Asp Lys Ser Met Phe Val Lys Ile Lys
               660                 665                 670
   Thr Leu Gly Ile Gly Ala Phe Gly Glu Val Cys Leu Ala Cys Lys Val
           675                 680                 685
   Asp Thr His Ala Leu Tyr Ala Met Lys Thr Leu Arg Lys Lys Asp Val
       690                 695                 700
   Leu Asn Arg Asn Gln Val Ala His Val Lys Ala Glu Arg Asp Ile Leu
   705                 710                 715                 720
   Ala Glu Ala Asp Asn Glu Trp Val Val Lys Leu Tyr Tyr Ser Phe Gln
                   725                 730                 735
   Asp Lys Asp Ser Leu Tyr Phe Val Met Asp Tyr Ile Pro Gly Gly Asp
               740                 745                 750
   Met Met Ser Leu Leu Ile Arg Met Glu Val Phe Pro Glu His Leu Ala
           755                 760                 765
   Arg Phe Tyr Ile Ala Glu Leu Thr Leu Ala Ile Glu Ser Val His Lys
       770                 775                 780
   Met Gly Phe Ile His Arg Asp Ile Lys Pro Asp Asn Ile Leu Ile Asp
   785                 790                 795                 800
   Leu Asp Gly His Ile Lys Leu Thr Asp Phe Gly Leu Cys Thr Gly Phe
                   805                 810                 815
   Arg Trp Thr His Asn Ser Lys Tyr Tyr Gln Lys Gly Ser His Val Arg
               820                 825                 830
   Gln Asp Ser Met Glu Pro Ser Asp Leu Trp Asp Asp Val Ser Asn Cys
           835                 840                 845
   Arg Cys Gly Asp Arg Leu Lys Thr Leu Glu Gln Arg Ala Arg Lys Gln
       850                 855                 860
   His Gln Arg Cys Leu Ala His Ser Leu Val Gly Thr Pro Asn Tyr Ile
   865                 870                 875                 880
   Ala Pro Glu Val Leu Leu Arg Lys Gly Tyr Thr Gln Leu Cys Asp Trp
                   885                 890                 895
   Trp Ser Val Gly Val Ile Leu Phe Glu Met Leu Val Gly Gln Pro Pro
               900                 905                 910
   Phe Leu Ala Pro Thr Pro Thr Glu Thr Gln Leu Lys Val Ile Asn Trp
           915                 920                 925
   Glu Asn Thr Leu His Ile Pro Ala Gln Val Lys Leu Ser Pro Glu Ala
       930                 935                 940
   Arg Asp Leu Ile Thr Lys Leu Cys Cys Ser Ala Asp His Arg Leu Gly
   945                 950                 955                 960
   Arg Asn Gly Ala Asp Asp Leu Lys Ala His Pro Phe Phe Ser Ala Ile
                   965                 970                 975
   Asp Phe Ser Ser Asp Ile Arg Lys Gln Pro Ala Pro Tyr Val Pro Thr
               980                 985                 990
   Ile Ser His Pro Met Asp Thr Ser Asn Phe Asp Pro Val Asp Glu Glu
           995                 1000                1005
   Ser Pro Trp Asn Asp Ala Ser Glu Gly Ser Thr Lys Ala Trp Asp Thr
       1010                1015                1020
   Leu Thr Ser Pro Asn Asn Lys His Pro Glu His Ala Phe Tyr Glu Phe
   1025                1030                1035                1040
   Thr Phe Arg Arg Phe Phe Asp Asp Asn Gly Tyr Pro Phe Arg Cys Pro
                   1045                1050                1055
   Lys Pro Ser Gly Ala Glu Ala Ser Gln Ala Glu Ser Ser Asp Leu Glu
               1060                1065                1070
   Ser Ser Asp Leu Val Asp Gln Thr Glu Gly Cys Gln Pro Val Tyr Val
           1075                1080                1085
<210> 33
   <211> 7091
   <212> DNA
   <213> Homo sapiens
<400> 33
   gctacccact tccgccccct ccccctgcca ttggaactag ctgagccgaa ctagttgcgg 60
   ccaccgagca gccggctctc ggcacctcct cctccgcctc cctgtctcct gttccattcg 120
   cctttcctct tctttcctgg cccacgccgc tccgaggcct cgcgaccgcc gagcctgcag 180
   cctgccccgc ggccaacatg agcttcttgt tgagttctca gcctgaagtt gactggaact 240
   ttcagttaac aagtatttat cgaatacctg atctgtagtg ttggacttag acctatggaa 300
   ggagctactg atgtgaatga aagtggtagt cgctcttcta aaacttttaa accaaagaag 360
   aacattccag agggttctca ccagtatgag ctcttaaaac acgcagaagc cacacttggc 420
   agtggcaacc ttcggatggc tgtcatgctt cctgaagggg aagatctcaa tgaatgggtt 480
   gcagttaaca ctgtggattt cttcaatcag atcaacatgc tttatggaac tatcacagac 540
   ttctgtacag aagagagttg tccagtgatg tcagctggcc caaaatatga gtatcattgg 600
   gcagatggaa cgaacataaa gaaacctatt aagtgctctg caccaaagta tattgattac 660
   ttgatgactt gggttcagga ccagttggat gatgagacgt tatttccatc aaaaattggt 720
   gtcccgttcc caaagaattt catgtctgtg gcaaaaacta tactcaaacg cctctttagg 780
   gtttatgctc acatttatca tcagcatttt gaccctgtga tccagcttca ggaggaagca 840
   catctaaata catctttcaa gcactttatt ttttttgtcc aggaattcaa ccttattgat 900
   agaagagaac ttgcaccact ccaagaactg attgaaaaac tcacctcaaa agacagataa 960
   aaggatgcag agctgtgcaa attgttcctc aaatgaagca gtgtggagtg tattggggat 1020
   tttgttatat tttgttttta tctggattgt ttttgtccta ggtttggggg cgggggcttg 1080
   tttgggttcc tttttcttta ttctgattat gtgaaaccat attctattgc taggggaagc 1140
   caagaaccat tctctataca cttgataagg gtaaatttat cttagtgttt ttaaacttgg 1200
   ttttggttac ttgaggagtt ttttaataat attgtgtgct gcaagaaagt gcttgttgat 1260
   tgaactgccg atggattggt ttctgtgtgg tataaattgt ggcccattta tgaagtcccc 1320
   aaaagagtta tgtttttaag tgccttggca ggctcacttc tgaggtgcaa aacatagata 1380
   tagaactgaa cagggcttga aacaatatta ggattactac ccagggcact tactggtgca 1440
   tgttgtaaca tatctatgat aaaagccata gtttacctaa aatggtgatt tccagccttt 1500
   actgctttga agaaacagaa tttgtaaagg tatgcatgta gaacataaaa aatatttctt 1560
   aattattttt tatattgatg gtaatatatt acgttcaaca atgcttaaag ctctacaagc 1620
   aggtcttttc ccacctcttg atatctgtga tactgaaact tgaggatgtt gaaatgtatt 1680
   acattttggc ttctttctac atgttaactg cactgtagat gtaaaaattc aggttatata 1740
   taggattgcc atcttcagag gtgatgctga actgtgaggt ttcctagtaa ttgccaaatg 1800
   agccgtaagt ctgcagaatt cccttctact ttgaagagaa ggggatagga atgtatattt 1860
   ggctgggggc atggagatgt tcgtatgtat gaggagttag ggatggggag tcaagttcta 1920
   gaaagttttg tctgaaaacc tttgaataga atggcatgaa gattttaatc aattacttat 1980
   aaacaaagtc ttagagactt ccttttagga atcaacttcc atgagaagtt aaaaataaat 2040
   tattaatttt aggtacagac attaaacatg gaatttaagg actgttgggg gaaattgatc 2100
   acttcttagc atttccattc agtgaatgga gctgatgttt gcctgtcatt ttaagatgat 2160
   accatacctt ctttggttat tataggtcca gtttgaagca ttctgacttc tggtttttcc 2220
   accctgaaag gaaatgtttt tctttgcagc agtattagat aatgaaaaat gctaattcag 2280
   tagttattaa cttctaaatt ttattcgcca tgactttcta gtgaattatt accataaata 2340
   acaatttcag aaacttagtt tttagaataa atattaattt ttccacttca gttttattct 2400
   agaaaatacc ctttttagaa atccagtttt agttttgtca ttttcgataa atctttcttc 2460
   agttagaaat atatatcctt ccttcagttg aaacatatac ctttttcaca tctaggaaga 2520
   aatgcttgct ctgaaatagt atagattaaa aacactcagt agaaaagaat ctaaaattaa 2580
   atgaatttgt tttgccatta aagtagagca gtgatacaat ttaatgccat tacaattatg 2640
   ttgactagaa actgcctttt tctccacttc atttctagca attatttacc aagtaccaac 2700
   agtagaagta acaggaaagc ctggcagagt taaatatctt ggacatttat tggtaaagct 2760
   tatttataaa ctgcagtcag agctagttaa tttccttaaa tctttttgta ttcagataga 2820
   taatatgaat cattatgggt tgattcagaa ataaaatttg tgaggtgatt ttgaatcttg 2880
   tccatatagg aaaatgaagc acagaattac tcagtcttcc atattgtatt tgacttcata 2940
   tcaatctagt aaaaaaggag ttgcaatagc caagtataga gagaatagtg aaaaattaat 3000
   cttgcccttt caagccttat acagtagtac actgtacttg tttttagtag taagacctac 3060
   tttcccacta tatgtagata gtttgttttc actgtgccag aatctcaggt gcctgcttag 3120
   agtatttctt taatcacagt cactgggaag taaggagatg tatatatgtg tatatatggt 3180
   aacaaagcat agcagttctc taggggagag gcctggcatt gcacatggtg ttacatggct 3240
   acaagtaagg aaaaaatcag aaagtgaaag aactgatgta ataaaaggtt gatttggttg 3300
   gttcccatga aagttagtaa gatgcccttt taaatataag gatcagtgct ttgttctgca 3360
   gcagagtttg ctgataaatg tctgttggat tctttttgga tttctttaat taatttgtaa 3420
   gtaaccaaga taattatttt cccccttgct ctctatatta atacgtagct ataaagcaac 3480
   agttggtttt cttatccttt gataaaagca tcccataaaa tataaagtag taagttaaca 3540
   tagtattatt gtcacacaca atgctttttt tggttaaatg ttgatacgaa gcaatgtttt 3600
   ggaattactt taattgatgg agtagtggtg gtagagagaa attaataaca aaaagagtga 3660
   aaatatttta attagcagta gatggtgcta ctggctttca tttgctgact tgattattcc 3720
   ctttctctta aaaaccatgg cattagactg cactaaatta acaagcatgt tagttgctgg 3780
   tagaggtttt ggaggttaat ttaccttaaa ttggaagact tttaattgca gtctctttct 3840
   accttccctc tgttagtcat ttgtaaattc taaatggtca ccataaaatg tattaggtag 3900
   gagaagatac gttttatgta taatatatct cagactgagt tactgcctgt cttatcagga 3960
   tggataaaac actacagtct cttatcagga aatagagatg atgtggatat ttatatatta 4020
   catatataac caccagactc cattttatat attagcattt tccttgctta tgggaaaata 4080
   gcaaaacaac atttcattta tacttttgtt tacccctctc tgagacaggt tttgataacc 4140
   actgaaatgg tagaatatgt gagatacaaa tattgagttg tagaactttc tttttaaggt 4200
   gaataagtca tgccttaaca tccaaataag agttcatctt cagagtggtt cttttgggag 4260
   cactgtttat tccagctata ctgcaaaagt ataatgtttt tggaactgtt ctagagcata 4320
   ccatgaaaag cagtttgtta ttatgcagga aaatcagttt catcatttta gttacactaa 4380
   acacttttgg cagcttaata tgaccttttt aaattttttt tatttttttt atttttattt 4440
   ctttaagatg gagtcttgct ctgttgcccg ggctggagta caatggcatg atctcagctc 4500
   actgcaacct ccacctcctg ggttcaagca tttctcctgc ctcagcctcc caagtagctg 4560
   ggattacagg cagcaccaca cctggctaat tttcatattt ttagtagaga tggggtttca 4620
   acatattggc caggctggtc tcaaactcct gacctcaagt gatccgccct ccccagcctc 4680
   ccaaagtgct gggattacag gtgtgagcca ccacagccag ccagtatgac ctatcttaat 4740
   catcagctca actgtaattt aaatttggct gttctctgga gctaaaccat tagggaagtt 4800
   caaaggaatg tgccatgatt tccgaatttg cacaagagaa tgttttaagc attggtagca 4860
   taattgaata aaagaatagt ttcctgatgt cactattttg aagtggaaat tatcacttgg 4920
   atgtggaggt tttacttttt aaaaacattc agcttaatta ccttacctta attaccttag 4980
   ttagatatac taatggaaaa aaaccaagtc ctttctctag aacttgtttt ctatttttgt 5040
   tccttttcat gaaaacttct caatttaatt ttaactactg taggatagta ttgattgaat 5100
   ggatactatg gaaaagtgga tccaatattt aagatagaag tagtttaagg agacaacagc 5160
   ctttactgcc attttttttt aaatgttttc actcagatga acaatttgac tttaataaaa 5220
   gactggagat ttttgtacaa agaaatagga ataagtttca tatactaatt atgctgagtt 5280
   ttaagcctac atatcacaaa atatttagaa ttgtataacc ttttcatata tttataactt 5340
   ttaatgtctt tttaaaagat gtgggaccaa aaatatattt ataatttgga aatgtgactg 5400
   cataccaata agaaaactta ccttattttg aaatttatct gggatattaa agaatctacc 5460
   aattcttaaa aacacagatt tatactttaa gcttatttta aaattaaaga atatatacca 5520
   attcttagaa acactttaag gactactctt aaataactta aatatcagag ttttgttgta 5580
   atattaaaat ttaccgtgga aatcactgtt gttcagctat caccttaatt gtgtatgata 5640
   tgataaatgt ttagcagtaa agctatctta agatttaatg gaaaagttta atttgaagat 5700
   gtaacaaaaa ttctgaccac agttgattct gaatttttaa ggctttccta ataggctgat 5760
   cacagagaat aatccatttt gaaggtataa aactgcactg tatgtctgtc acttgtagct 5820
   gaactgattc acattttgac aaaagagaga aaatacaaaa atgagttttg caaatgtaat 5880
   aactttttct gcatatagaa ctaaataatt gaaaaatatg ggctatagtt ctcaaaggta 5940
   gatagtaaaa tcactggctt tttccagctg tatgtttttc cactgtgcgt gtacacacac 6000
   actggaaaat aattaggctg attttgcagg tcttcattgt tagagattct gaagtattta 6060
   ctgtcaattc ataggtttca gtttatttag gaaattagtg tttgacagct ttttttaaat 6120
   tatttcactg aagctgagat tattagtgat acaaagttaa aatttcaata tttaatttct 6180
   ctatatatta ttaatattaa attgtttttt acttataaat tcatgttctc atctgattta 6240
   atattaaatt tgtataggtg ggcgtttctt accattttgc acaagttttt gtttttctga 6300
   aatacttaat tgtgcaggtt gtaaaaaaga ttagtgcatt ttcattttaa ggatgctttg 6360
   ctccttaaat tgttcgacag aaatgacttt ttagggaaag tagttttttt ggagctacta 6420
   acttgtattt attattgtac atgcataacc agggtggtga gggcactaat cttgtaggaa 6480
   acacttactt gatgttttat ttgaactttt cctataggtt taacttttac tgcatagaat 6540
   taacactagg aacagtgtca tgaaatctgg gttgaaggag aatacagtat atatgagaac 6600
   acttaaagtt caaatagaaa tcatttctga agacaaaagc agaggaatat tgtcagtgcc 6660
   aagtaatgga agaataaggg cggcatttac actgtgcaag tattgagaag agtgcataaa 6720
   gacagggaac tactctcatg gagacagttt ctctcttata atcaagtaac tagaagggga 6780
   aaaatcatct aagttatgaa atccaacata ggcgctatat tacaaactgt gccggattat 6840
   gcaaattgta gttgttactg atcaaagttt aattgcttca tttttgttta aaaagggata 6900
   ctgatgtcag aaaatctgta atatgtttta ttcaaaagat gtaaataatg tatacagact 6960
   tgtatgtgat gggatgggaa atatttaaat tctaggtgtt tttttttttt taaagaagaa 7020
   actcaatgtt tataagaaaa aaatgaataa atagttacgt ttggccatga atcctgaaaa 7080
   aaaaaaaaaa a                                                      7091
<210> 34
   <211> 221
   <212> PRT
   <213> Homo sapiens
<400> 34
   Met Glu Gly Ala Thr Asp Val Asn Glu Ser Gly Ser Arg Ser Ser Lys
    1              5                   10                  15
   Thr Phe Lys Pro Lys Lys Asn Ile Pro Glu Gly Ser His Gln Tyr Glu
               20                  25                  30
   Leu Leu Lys His Ala Glu Ala Thr Leu Gly Ser Gly Asn Leu Arg Met
           35                  40                  45
   Ala Val Met Leu Pro Glu Gly Glu Asp Leu Asn Glu Trp Val Ala Val
       50                  55                  60
   Asn Thr Val Asp Phe Phe Asn Gln Ile Asn Met Leu Tyr Gly Thr Ile
   65                  70                  75                  80
   Thr Asp Phe Cys Thr Glu Glu Ser Cys Pro Val Met Ser Ala Gly Pro
                   85                  90                  95
   Lys Tyr Glu Tyr His Trp Ala Asp Gly Thr Asn Ile Lys Lys Pro Ile
               100                 105                 110
   Lys Cys Ser Ala Pro Lys Tyr Ile Asp Tyr Leu Met Thr Trp Val Gln
           115                 120                 125
   Asp Gln Leu Asp Asp Glu Thr Leu Phe Pro Ser Lys Ile Gly Val Pro
       130                 135                 140
   Phe Pro Lys Asn Phe Met Ser Val Ala Lys Thr Ile Leu Lys Arg Leu
   145                 150                 155                 160
   Phe Arg Val Tyr Ala His Ile Tyr His Gln His Phe Asp Pro Val Ile
                   165                 170                 175
   Gln Leu Gln Glu Glu Ala His Leu Asn Thr Ser Phe Lys His Phe Ile
               180                 185                 190
   Phe Phe Val Gln Glu Phe Asn Leu Ile Asp Arg Arg Glu Leu Ala Pro
           195                 200                 205
   Leu Gln Glu Leu Ile Glu Lys Leu Thr Ser Lys Asp Arg
       210                 215                 220
<210> 35
   <211> 5020
   <212> DNA
   <213> Homo sapiens
<400> 35
   gtgacgcgag gcgggttctt ggactgagtg tgcggcgcgg tgcgccgcct tccgaggctc 60
   ctcccgcggg tggcagcgga cggggcgcgc ccctcggcca gtcctcggtc ctcaggcttg 120
   tggctccgtt gagcaccggc cgccgggcct ctgggtccgt cgagtggaga ctctctgaaa 180
   agcgtgggct ccgtggcctc cggcgcggcc gcggcgggtc ggtctcctag atcatccggg 240
   aagcccacgg gaccctcagg cgggcaggat gaacgactgg cacaggatct tcacccaaaa 300
   cgtgcttgtc cctccccacc cacagagagc gcgccagcct tggaaggaat ccacggcatt 360
   ccagtgtgtc ctcaagtggc tggacggacc ggtaattagg cagggcgtgc tggaggtact 420
   gtcagaggtt gaatgccatc tgcgagtgtc tttctttgat gtcacctacc ggcacttctt 480
   tgggaggacg tggaaaacca cagtgaagcc gacgaagaga ccgccgtcca ggatcgtctt 540
   taatgagccc ttgtattttc acacatccct aaaccaccct catatcgtgg ctgtggtgga 600
   agtggtcgct gagggcaaga aacgggatgg gagcctccag acattgtcct gtgggtttgg 660
   aattcttcgg atcttcagca accagccgga ctctcctatc tctgcttccc aggacaaaag 720
   gttgcggctg taccatggca cccccagagc cctcctgcac ccgcttctcc aggaccccgc 780
   agagcaaaac agacacatga ccctcattga gaactgcagc ctgcagtaca cgctgaagcc 840
   acacccggcc ctggagcctg cgttccacct tcttcctgag aaccttctgg tgtctggtct 900
   gcagcagata cctggcctgc ttccagctca tggagaatcc ggcgacgctc tccgaaagcc 960
   tcgcctccag aagcccatca cggggcactt ggatgactta ttcttcaccc tgtacccctc 1020
   cctggagaag tttgaggaag agctgctgga gctccacgtc caggaccact tccaggaggg 1080
   atgtggccca ctggacggtg gtgccctgga gatcctggag cggcgcctgc gtgtgggcgt 1140
   gcacaatggt ctgggcttcg tgcagaggcc gcaggtcgtt gtactggtgc ctgagatgga 1200
   tgtggccttg acgcgctcag ctagcttcag caggaaagtg gtctcctctt ccaagaccag 1260
   ctccgggagc caagctctgg ttttgagaag ccgcctccgc ctcccagaga tggtcggcca 1320
   ccctgcattt gcggtcatct tccagctgga gtacgtgttc agcagccctg caggagtgga 1380
   cggcaatgca gcttcggtca cctctctgtc caacctggca tgcatgcaca tggtccgctg 1440
   ggctgtttgg aaccccttgc tggaagctga ttctggaagg gtgaccctgc ctctgcaggg 1500
   tgggatccag cccaacccct cgcactgtct ggtctacaag gtaccctcag ccagcatgag 1560
   ctctgaagag gtgaagcagg tggagtcggg tacactccgg ttccagttct cgctgggctc 1620
   agaagaacac ctggatgcac ccacggagcc tgtcagtggc cccaaagtgg agcggcggcc 1680
   ttccaggaaa ccacccacgt ccccttcgag cccgccagcg ccagtacctc gagttctcgc 1740
   tgccccgcag aactcacctg tgggaccagg gttgtcaatt tcccagctgg cggcctcccc 1800
   gcggtccccg actcagcact gcttggccag gcctacttca cagctacccc atggctctca 1860
   ggcctccccg gcccaggcac aggagttccc gttggaggcc ggtatctccc acctggaagc 1920
   cgacctgagc cagacctccc tggtcctgga aacatccatt gccgaacagt tacaggagct 1980
   gccgttcacg cctttgcatg cccctattgt tgtgggaacc cagaccagga gctctgcagg 2040
   gcagccctcg agagcctcca tggtgctcct gcagtcctcc ggctttcccg agattctgga 2100
   tgccaataaa cagccagccg aggctgtcag cgctacagaa cctgtgacgt ttaaccctca 2160
   gaaggaagaa tcagattgtc tacaaagcaa cgagatggtg ctacagtttc ttgcctttag 2220
   cagagtggcc caggactgcc gaggaacatc atggccaaag actgtgtatt tcaccttcca 2280
   gttctaccgc ttcccacccg caacgacgcc acgactgcag ctggtccagc tggatgaggc 2340
   cggccagccc agctctggcg ccctgaccca catcctcgtg cctgtgagca gagatggcac 2400
   ctttgatgct gggtctcctg gcttccagct gaggtacatg gtgggccctg ggttcctgaa 2460
   gccaggtgag cggcgctgct ttgcccgcta cctggccgtg cagaccctgc agattgacgt 2520
   ctgggacgga gactccctgc tgctcatcgg atctgctgcc gtccagatga agcatctcct 2580
   ccgccaaggc cggccggctg tgcaggcctc ccacgagctt gaggtcgtgg caactgaata 2640
   cgagcaggac aacatggtgg tgagtggaga catgctgggg tttggccgcg tcaagcccat 2700
   cggcgtccac tcggtggtga agggccggct gcacctgact ttggccaacg tgggtcaccc 2760
   gtgtgaacag aaagtgagag gttgtagcac attgccaccg tccagatctc gggtcatctc 2820
   aaacgatgga gccagccgct tctctggagg cagcctcctc acgactggaa gctcaaggcg 2880
   aaaacacgtg gtgcaagcac agaagctggc ggacgtggac agtgagctgg ctgccatgct 2940
   actgacccat gcccggcagg gcaaggggcc ccaggacgtc agccgcgagt cggatgccac 3000
   ccgcaggcgt aagctggagc ggatgaggtc tgtgcgcctg caggaggccg ggggagactt 3060
   gggccggcgc gggacgagcg tgttggcgca gcagagcgtc cgcacacagc acttgcggga 3120
   cctacaggtc atcgccgcct accgggaacg cacgaaggcc gagagcatcg ccagcctgct 3180
   gagcctggcc atcaccacgg agcacacgct ccacgccacg ctgggggtcg ccgagttctt 3240
   tgagtttgtg cttaagaacc cccacaacac acagcacacg gtgactgtgg agatcgacaa 3300
   ccccgagctc agcgtcatcg tggacagtca ggagtggagg gacttcaagg gtgctgctgg 3360
   cctgcacaca ccggtggagg aggacatgtt ccacctgcgt ggcagcctgg ccccccagct 3420
   ctacctgcgc ccccacgaga ccgcccacgt ccccttcaag ttccagagct tctctgcagg 3480
   gcagctggcc atggtgcagg cctctcctgg gttgagcaac gagaagggca tggacgccgt 3540
   gtcaccttgg aagtccagcg cagtgcccac taaacacgcc aaggtcttgt tccgagcgag 3600
   tggtggcaag cccatcgccg tgctctgcct gactgtggag ctgcagcccc acgtggtgga 3660
   ccaggtcttc cgcttctatc acccggagct ctccttcctg aagaaggcca tccgcctgcc 3720
   gccctggcac acatttccag gtgctccggt gggaatgctt ggtgaggacc ccccagtcca 3780
   tgttcgctgc agcgacccga acgtcatctg tgagacccag aatgtgggcc ccggggaacc 3840
   acgggacata tttctgaagg tggccagtgg tccaagcccg gagatcaaag acttctttgt 3900
   catcatttac tcggatcgct ggctggcgac acccacacag acgtggcagg tctacctcca 3960
   ctccctgcag cgcgtggatg tctcctgcgt cgcaggccag ctgacccgcc tgtcccttgt 4020
   ccttcggggg acacagacag tgaggaaagt gagagctttc acctctcatc cccaggagct 4080
   gaagacagac cccaaaggtg tcttcgtgct gccgcctcgt ggggtgcagg acctgcatgt 4140
   tggcgtgagg ccccttaggg ccggcagccg ctttgtccat ctcaacctgg tggacgtgga 4200
   ttgccaccag ctggtggcct cctggctcgt gtgcctctgc tgccgccagc cgctcatctc 4260
   caaggccttt gagatcatgt tggctgcggg cgaagggaag ggtgtcaaca agaggatcac 4320
   ctacaccaac ccctacccct cccggaggac attccacctg cacagcgacc acccggagct 4380
   gctgcggttc agagaggact ccttccaggt cgggggtgga gagacctaca ccatcggctt 4440
   gcagtttgcg cctagtcaga gagtgggtga ggaggagatc ctgatctaca tcaatgacca 4500
   tgaggacaaa aacgaagagg cattttgcgt gaaggtcatc taccagtgag ggcttgaggg 4560
   tgacgtcctt cctgcggcac ccagctgggg cctgtctgtg cccctcctgc cctgcaggct 4620
   gtcctccccg cctctctgca gcctttcact tcagtgccca cctggctgac ctgtgcactt 4680
   ggctgaggaa gcagagaccg agcgctggtc attttgtagt acctgcatcc agcttagctg 4740
   ctgctgacac ccagcaggcc tgggttccgt gagcgcgaac tccgtggtgg tgggtctggc 4800
   tctggtgctg ccatctacgc atgtgggacc ctcgttatcg ctgttgctca aaatgtattt 4860
   tatgaatcat cctaaatgag aaaattatgt ttttcttact ggattttgta caaacataat 4920
   ctattatttg ctatgcaata ttttatgctg gtattatatc tgttttttaa attgttgaac 4980
   aaaatactaa acttttacac gtcttcaaaa aaaaaaaaaa                       5020
<210> 36
   <211> 1426
   <212> PRT
   <213> Homo sapiens
<400> 36
<210> 37
   <211> 7971
   <212> DNA
   <213> Homo sapiens
<400> 37
   cgggcatgct cagtgggccg ggccggcagg tttgcgtggc cgctgagttg ccggcgccgg 60
   ctgagccagc ggacgccgcg ttccttggcg gccgccggtt cccgggaagt tacgtggcga 120
   agccggcttc cgaggagacg ccgggaggcc acgggtgctg ctgacgggcg ggcgaccggg 180
   cgaggccgac gtggccgggc tgcgaaagct gcgggaggcc gagtgggtgg ccgcgctcgg 240
   agggaggtgc cggtcgggcg cgccccgtgg agaagacccg ggcggggcgg gcgcttcccg 300
   gacttttgtc cgagttgaat tccctccccc tgggccgggc ccttccggcc gcccccgccc 360
   gtgccccgct cgctctcggg agatgtttat ttgggctgtg gcgtgaggag cgggcgggcc 420
   agcgccgcgg agtttcgggt ccgaggagcc tcgcgcggcg ctggagagag acaagatgtc 480
   cgccagagct gcggccgcca agagcacagc aatggaggaa acagctatat gggaacaaca 540
   tacagtgacg cttcacaggg ctcctggatt tggatttgga attgcaatat ctggtggacg 600
   agataatcct cattttcaga gtggggaaac gtcaatagtg atttcagatg tgctgaaagg 660
   aggaccagct gaaggacagc tacaggaaaa tgaccgagtt gcaatggtta acggagtttc 720
   aatggataat gttgaacatg cttttgctgt tcagcaacta aggaaaagtg ggaaaaatgc 780
   aaaaattaca attagaagga agaagaaagt tcaaatacca gtaagtcgtc ctgatcctga 840
   accagtatct gataatgaag aagatagtta tgatgaggaa atacatgatc caagaagtgg 900
   ccggagtggt gtggttaaca gaaggagtga gaagatttgg ccgagggata gaagtgcaag 960
   tagagagagg agcttgtccc cgcggtcaga caggcggtca gtggcttcca gccagcctgc 1020
   taaacctact aaagtcacac tggtgaaatc ccggaaaaat gaagaatatg gtcttcgatt 1080
   ggcaagccat atatttgtta aggaaatttc acaagatagt ttggcagcaa gagatggcaa 1140
   tattcaagaa ggtgatgttg tattgaagat aaatggtact gtgacagaaa atatgtcatt 1200
   gacagatgca aagacattga tagaaaggtc taaaggcaaa ttaaaaatgg tagttcaaag 1260
   agatgaacgg gctacgctat tgaatgtccc tgatctttct gacagcatcc actctgctaa 1320
   tgcctctgag agagacgaca tttcagaaat tcagtcactg gcatcagatc attctggtcg 1380
   atcacacgat aggcctcccc gccgcagccg gtcacgatct cctgaccagc ggtcagagcc 1440
   ttctgatcat tccaggcact cgccgcagca gccaagcaat ggcagtctcc ggagtagaga 1500
   tgaagagaga atttctaaac ctggggctgt ctcaactcct gtaaagcatg ctgatgatca 1560
   cacacctaaa acagtggaag aagttacagt tgaaagaaat gagaaacaaa caccttctct 1620
   tccagaacca aagcctgtgt atgcccaagt tgggcaacca gatgtggatt tacctgtcag 1680
   tccatctgat ggtgtcctac ctaattcaac tcatgaagat gggattcttc ggcccagcat 1740
   gaaattggta aaattcagaa aaggagatag tgtgggtttg cggctggctg gtggaaatga 1800
   tgttggaata tttgtagctg gcgttctaga agatagccct gcagccaagg aaggcttaga 1860
   ggaaggtgat caaattctca gggtaaacaa cgtagatttt acaaatatca taagagaaga 1920
   agccgtcctt ttcctgcttg acctccctaa aggagaagaa gtgaccatat tggctcagaa 1980
   gaagaaggat gtttatcgtc gcattgtaga atcagatgta ggagattctt tctatattag 2040
   aacccatttt gaatatgaaa aggaatctcc ctatggactt agttttaaca aaggagaggt 2100
   gttccgtgtt gtggatacct tgtacaatgg aaaactgggc tcttggcttg ctattcgaat 2160
   tggtaaaaat cataaggagg tagaacgagg catcatccct aataagaaca gagctgagca 2220
   gctagccagt gtacagtata cacttccaaa aacagcaggc ggagaccgtg ctgacttctg 2280
   gagattcaga ggtcttcgca gctccaagag aaatcttcga aaaagcagag aggatttgtc 2340
   cgctcagcct gttcaaacaa agtttccagc ttatgaaaga gtggttcttc gagaagctgg 2400
   atttctgagg cctgtaacca tttttggacc aatagctgat gttgccagag aaaagctggc 2460
   aagagaagaa ccagatattt atcaaattgc aaagagtgaa ccacgagacg ctggaactga 2520
   ccaacgtagc tctggcatta ttcgcctgca tacaataaag caaatcatag atcaagacaa 2580
   acatgcttta ttagatgtaa caccaaatgc agttgatcgt cttaactatg cccagtggta 2640
   tccaattgtt gtatttctta accctgattc taagcaagga gtaaaaacaa tgagaatgag 2700
   gttatgtcca gaatctcgga aaagtgccag gaagttatac gagcgatctc ataaacttcg 2760
   taaaaataat caccatcttt ttacaactac aattaactta aattcaatga atgatggttg 2820
   gtatggtgcg ctgaaagaag caattcaaca acagcaaaac cagctggtat gggtttccga 2880
   gggaaaggcg gatggtgcta caagtgatga ccttgatttg catgatgatc gtctgtccta 2940
   cctgtcagct ccaggtagtg aatactcaat gtatagcacg gacagtagac acacttctga 3000
   ctatgaagac acagacacag aaggcggggc ctacactgat caagaactag atgaaactct 3060
   taatgatgag gttgggactc caccggagtc tgccattaca cggtcctctg agcctgtaag 3120
   agaggactcc tctggaatgc atcatgaaaa ccaaacatat cctccttact caccacaagc 3180
   gcagccacaa ccaattcata gaatagactc ccctggattt aagccagcct ctcaacagaa 3240
   agcagaagct tcatctccag tcccttacct ttcgcctgaa acaaacccag catcatcaac 3300
   ctctgctgtt aatcataatg taaatttaac taatgtcaga ctggaggagc ccaccccagc 3360
   tccttccacc tcttactcac cacaagctga ttctttaaga acaccaagta ctgaggcagc 3420
   tcacataatg ctaagagatc aagaaccatc attgtcgtcg catgtagatc caacaaaggt 3480
   gtatagaaag gatccatatc ccgaggaaat gatgaggcag aaccatgttt tgaaacagcc 3540
   agccgttagt cacccagggc acaggccaga caaagagcct aatctgacct atgaacccca 3600
   actcccatac gtagagaaac aagccagcag agacctcgag cagcccacat acagatacga 3660
   gtcctcaagc tatacggacc agttttctcg aaactatgaa catcgtctgc gatacgaaga 3720
   tcgcgtcccc atgtatgaag aacagtggtc atattatgat gacaaacagc cctacccatc 3780
   tcggccacct tttgataatc agcactctca agaccttgac tccagacagc atcccgaaga 3840
   gtcctcagaa cgagggtact ttccacgttt tgaagagcca gcccctctgt cttacgacag 3900
   cagaccacgt tacgaacagg cacctagagc atccgccctg cggcacgaag agcagccagc 3960
   tcctgggtat gacacacatg gtagactcag accggaagcc cagccccacc cttcagcagg 4020
   gcccaagcct gcagagtcca agcagtattt tgagcaatat tcacgcagtt acgagcaagt 4080
   accaccccaa ggatttacct ctagagcagg tcattttgag cctctccatg gtgctgcagc 4140
   tgtccctccg ctgatacctt catctcagca taagccagaa gctctgcctt caaacaccaa 4200
   accactgcct ccacccccaa ctcaaaccga agaagaggaa gatccagcaa tgaagccaca 4260
   gtctgtactc accagagtta agatgtttga aaacaaaaga tctgcatcct tagagaccaa 4320
   gaaggatgta aatgacactg gcagttttaa gcctccagaa gtagcatcta aaccttcagg 4380
   tgctcccatc attggtccca aacccacttc tcagaatcaa ttcagtgaac atgacaaaac 4440
   tctgtacagg atcccagaac ctcaaaaacc tcaactgaag ccacctgaag atattgttcg 4500
   gtccaatcat tatgaccctg aagaagatga agaatattat cgaaaacagc tgtcatactt 4560
   tgaccgaaga agttttgaga ataagcctcc tgcacacatt gccgccagcc atctctccga 4620
   gcctgcaaag ccagcgcatt ctcagaatca atcaaatttt tctagttatt cttcaaaggg 4680
   aaagcctcct gaagctgatg gtgtggatag atcatttggc gagaaacgct atgaacccat 4740
   ccaggccact ccccctcctc ctccattgcc ctcgcagtat gcccagccat ctcagcctgt 4800
   caccagcgcg tctctccaca tacattctaa gggagcacat ggtgaaggta attcagtgtc 4860
   attggatttt cagaattcct tagtgtccaa accagaccca cctccatctc agaataagcc 4920
   agcaactttc agaccaccaa accgagaaga tactgctcag gcagctttct atccccagaa 4980
   aagttttcca gataaagccc cagttaatgg aactgaacag actcagaaaa cagtcactcc 5040
   agcatacaat cgattcacac caaaaccata tacaagttct gcccgaccat ttgaacgcaa 5100
   gtttgaaagt cctaaattca atcacaatct tctgccaagt gaaactgcac ataaacctga 5160
   cttgtcttca aaaactccca cttctccaaa aactcttgtg aaatcgcaca gtttggcaca 5220
   gcctcctgag tttgacagtg gagttgaaac tttctctatc catgcagaga agcctaaata 5280
   tcaaataaat aatatcagca cagtgcctaa agctattcct gtgagtcctt cagctgtgga 5340
   agaggatgaa gatgaagatg gtcatactgt ggtggccaca gcccgaggca tatttaacag 5400
   caatgggggc gtgctgagtt ccatagaaac tggtgttagt ataattatcc ctcaaggagc 5460
   cattcccgaa ggagttgagc aggaaatcta tttcaaggtc tgccgggaca acagcatcct 5520
   tccaccttta gataaagaga aaggtgaaac actgctgagt cctttggtga tgtgtggtcc 5580
   ccatggcctc aagttcctga agcctgtgga gctgcgctta ccacactgtg atcctaaaac 5640
   ctggcaaaac aagtgtcttc ccggagatcc aaattatctc gttggagcaa actgtgtttc 5700
   tgtccttatt gaccactttt aactcttgaa atataggaac ttaaataatg tgaaactgga 5760
   ttaaacttaa tctaaatgga accactctat caagtattat acctttttta gagttgatac 5820
   tacagtttgt tagtatgagg catttgtttg aactgataaa gatgagtgag catgcccctg 5880
   aaccatggtc ggaaaacatg ctacacactg catgtttgtg attgacggga ctgttggtat 5940
   tggctagagg ttcaaagata ttttgctttg tgatttttgt aattttttta tcgtcactgc 6000
   ttaacttcac atattgattt ccgttaaaat accagccagt aaatgggggt gcatttgagg 6060
   tctgttcttt ccaaagtaca ctgtttcaaa ctttactatg gccctggcct agcatacgta 6120
   cacattttat tttattatgc atgaagtaat atgcacacat tttttaaatg cacctggaat 6180
   atataaccag tgttgtggat ttaacagaaa tgtacagcaa ggagatttac aactggggga 6240
   gggtgaagtg aagacaatga cttactgtac atgaaaacac atttttctta gggaaggata 6300
   caaaagcatg tgagactggt tccatggcct cttcagatct ctaacttcac catattacca 6360
   cagacatact aaccagcaga aatgccttac cctcatgttc ttaattctta gctcattctc 6420
   cttgtgttac taagttttta tggcttttgt gcattatcta gatactgtat catgacaaag 6480
   actgagtacg ttgtgcattt ggtggtttca gaaatgtgtt atcacccaga agaaaatagt 6540
   ggtgtgattt ggggatattt ttttcttttc ttttcttttc tttttttttt tttttgacaa 6600
   ggggcagtgg tggttttctg ttctttctgg ctatgcattt gaaaattttg atgttttaag 6660
   gatgcttgta cataatgcgt gcataccact tttgttcttg gtttgtaaat taacttttat 6720
   aaactttacc ttttttatac ataaacaaga ccacgtttct aaaggctacc tttgtattct 6780
   ctcctgtacc tcttgagcct tgaactttga cctctgcagc aataaagcag cgtttctatg 6840
   acacatgcaa ggtcattttt tttaagaaaa aggatgcaca gagttgttac atttttaagt 6900
   gctgcattta aaagatacag ttactcagaa ttctctagtt tgattaaatt cttgcaaagt 6960
   atccctactg taatttgtga tacaatgctg tgccctaaag tgtatttttt tactaataga 7020
   caatttatta tggcacatca gcacgatttc tgtttagata atacaccact acattctgtt 7080
   aatcattagg tgtgactgaa tttcttttgc cgttattaaa aatctcaaat ttctaaatct 7140
   ccaaaataaa actttttaaa ataaagtgct ggcttggtct gtttgcccac tgttttctag 7200
   tttcatgcag ctttataatc ctgttttaaa atcctgcaca caaatcccta tcacccagcg 7260
   tcacctacca cctcgtcgtc tggtgttgca tgcagaattt ctccccttgg ccagcatgta 7320
   cagatgggtg ggcagtgctc atctgaaggg ctcagactga agtggggcag aaggacctgg 7380
   agacagagtg ggagaaggca gcaggccgac ttccccctgt gggtaaacac acacccctgc 7440
   gtggagaaac acccctgcat ggggacacac gtgcgtttgt gtgtgtgcgt gtaaccattt 7500
   gtatatggtt ttattcccca gataaatagc acgggcattg tttaatgtca cccacatttg 7560
   ggggaagaaa atgggtttag tgagcataaa tattccattg tggacatcct acttacttaa 7620
   ctgccacccc tgttgacatt tggattattg ctatagtgtt tcagtaaaca cctttgtgga 7680
   ctgaatccat ctatgtggat tttatttttt tcatctttgt taaggtataa tttacataga 7740
   gtaagttact gtttttaaaa tgtatagttc aaattttgac aaacacatac tgtttattta 7800
   ccacggttaa tacatacaac agtttcttta tcttctctaa tcatttgtac ccctttatac 7860
   tcaacccttc ttccagcccc tggcaggatg atctcttctg tttctatagt tgcctttttg 7920
   acagtgtcat ataaatggaa tcatacaata aaaaaaaaaa aaaaaaaaaa a          7971
<210> 38
   <211> 1748
   <212> PRT
   <213> Homo sapiens
<400> 38
   Met Ser Ala Arg Ala Ala Ala Ala Lys Ser Thr Ala Met Glu Glu Thr
   1               5                   10                  15
   Ala Ile Trp Glu Gln His Thr Val Thr Leu His Arg Ala Pro Gly Phe
               20                  25                  30
   Gly Phe Gly Ile Ala Ile Ser Gly Gly Arg Asp Asn Pro His Phe Gln
           35                  40                  45
   Ser Gly Glu Thr Ser Ile Val Ile Ser Asp Val Leu Lys Gly Gly Pro
       50                  55                  60
   Ala Glu Gly Gln Leu Gln Glu Asn Asp Arg Val Ala Met Val Asn Gly
   65                  70                  75                  80
   Val Ser Met Asp Asn Val Glu His Ala Phe Ala Val Gln Gln Leu Arg
                   85                  90                  95
   Lys Ser Gly Lys Asn Ala Lys Ile Thr Ile Arg Arg Lys Lys Lys Val
               100                 105                 110
   Gln Ile Pro Val Ser Arg Pro Asp Pro Glu Pro Val Ser Asp Asn Glu
           115                 120                 125
   Glu Asp Ser Tyr Asp Glu Glu Ile His Asp Pro Arg Ser Gly Arg Ser
       130                 135                 140
   Gly Val Val Asn Arg Arg Ser Glu Lys Ile Trp Pro Arg Asp Arg Ser
   145                 150                 155                 160
   Ala Ser Arg Glu Arg Ser Leu Ser Pro Arg Ser Asp Arg Arg Ser Val
                   165                 170                 175
   Ala Ser Ser Gln Pro Ala Lys Pro Thr Lys Val Thr Leu Val Lys Ser
               180                 185                 190
   Arg Lys Asn Glu Glu Tyr Gly Leu Arg Leu Ala Ser His Ile Phe Val
           195                 200                 205
   Lys Glu Ile Ser Gln Asp Ser Leu Ala Ala Arg Asp Gly Asn Ile Gln
       210                 215                 220
   Glu Gly Asp Val Val Leu Lys Ile Asn Gly Thr Val Thr Glu Asn Met
   225                 230                 235                 240
   Ser Leu Thr Asp Ala Lys Thr Leu Ile Glu Arg Ser Lys Gly Lys Leu
                   245                 250                 255
   Lys Met Val Val Gln Arg Asp Glu Arg Ala Thr Leu Leu Asn Val Pro
               260                 265                 270
   Asp Leu Ser Asp Ser Ile His Ser Ala Asn Ala Ser Glu Arg Asp Asp
           275                 280                 285
   Ile Ser Glu Ile Gln Ser Leu Ala Ser Asp His Ser Gly Arg Ser His
       290                 295                 300
   Asp Arg Pro Pro Arg Arg Ser Arg Ser Arg Ser Pro Asp Gln Arg Ser
   305                 310                 315                 320
   Glu Pro Ser Asp His Ser Arg His Ser Pro Gln Gln Pro Ser Asn Gly
   325 330 335
   Ser Leu Arg Ser Arg Asp Glu Glu Arg Ile Ser Lys Pro Gly Ala Val
   340 345 350
   Ser Thr Pro Val Lys His Ala Asp Asp His Thr Pro Lys Thr Val Glu
   355 360 365
   Glu Val Thr Val Glu Arg Asn Glu Lys Gln Thr Pro Ser Leu Pro Glu
   370 375 380
   Pro Lys Pro Val Tyr Ala Gln Val Gly Gln Pro Asp Val Asp Leu Pro
   385 390 395 400
   Val Ser Pro Ser Asp Gly Val Leu Pro Asn Ser Thr His Glu Asp Gly
   405 410 415
   Ile Leu Arg Pro Ser Met Lys Leu Val Lys Phe Arg Lys Gly Asp Ser
   420 425 430
   Val Gly Leu Arg Leu Ala Gly Gly Asn Asp Val Gly Ile Phe Val Ala
   435 440 445
   Gly Val Leu Glu Asp Ser Pro Ala Ala Lys Glu Gly Leu Glu Glu Gly
   450 455 460
   Asp Gln Ile Leu Arg Val Asn Asn Val Asp Phe Thr Asn Ile Ile Arg
   465 470 475 480
   Glu Glu Ala Val Leu Phe Leu Leu Asp Leu Pro Lys Gly Glu Glu Val
   485 490 495
   Thr Ile Leu Ala Gln Lys Lys Lys Asp Val Tyr Arg Arg Ile Val Glu
   500 505 510
   Ser Asp Val Gly Asp Ser Phe Tyr Ile Arg Thr His Phe Glu Tyr Glu
   515 520 525
   Lys Glu Ser Pro Tyr Gly Leu Ser Phe Asn Lys Gly Glu Val Phe Arg
   530 535 540
   Val Val Asp Thr Leu Tyr Asn Gly Lys Leu Gly Ser Trp Leu Ala Ile
   545 550 555 560
   Arg Ile Gly Lys Asn His Lys Glu Val Glu Arg Gly Ile Ile Pro Asn
   565 570 575
   Lys Asn Arg Ala Glu Gln Leu Ala Ser Val Gln Tyr Thr Leu Pro Lys
   580 585 590
   Thr Ala Gly Gly Asp Arg Ala Asp Phe Trp Arg Phe Arg Gly Leu Arg
   595 600 605
   Ser Ser Lys Arg Asn Leu Arg Lys Ser Arg Glu Asp Leu Ser Ala Gln
   610 615 620
   Pro Val Gln Thr Lys Phe Pro Ala Tyr Glu Arg Val Val Leu Arg Glu
   625 630 635 640
   Ala Gly Phe Leu Arg Pro Val Thr Ile Phe Gly Pro Ile Ala Asp Val
   645 650 655
   Ala Arg Glu Lys Leu Ala Arg Glu Glu Pro Asp Ile Tyr Gln Ile Ala
   660 665 670
   Lys Ser Glu Pro Arg Asp Ala Gly Thr Asp Gln Arg Ser Ser Gly Ile
   675 680 685
   Ile Arg Leu His Thr Ile Lys Gln Ile Ile Asp Gln Asp Lys His Ala
   690 695 700
   Leu Leu Asp Val Thr Pro Asn Ala Val Asp Arg Leu Asn Tyr Ala Gln
   705 710 715 720
   Trp Tyr Pro Ile Val Val Phe Leu Asn Pro Asp Ser Lys Gln Gly Val
   725 730 735
   Lys Thr Met Arg Met Arg Leu Cys Pro Glu Ser Arg Lys Ser Ala Arg
   740 745 750
   Lys Leu Tyr Glu Arg Ser His Lys Leu Arg Lys Asn Asn His His Leu
   755 760 765
   Phe Thr Thr Thr Ile Asn Leu Asn Ser Met Asn Asp Gly Trp Tyr Gly
   770 775 780
   Ala Leu Lys Glu Ala Ile Gln Gln Gln Gln Asn Gln Leu Val Trp Val
   785 790 795 800
   Ser Glu Gly Lys Ala Asp Gly Ala Thr Ser Asp Asp Leu Asp Leu His
   805 810 815
   Asp Asp Arg Leu Ser Tyr Leu Ser Ala Pro Gly Ser Glu Tyr Ser Met
               820                 825                 830
   Tyr Ser Thr Asp Ser Arg His Thr Ser Asp Tyr Glu Asp Thr Asp Thr
           835                 840                 845
   Glu Gly Gly Ala Tyr Thr Asp Gln Glu Leu Asp Glu Thr Leu Asn Asp
       850                 855                 860
   Glu Val Gly Thr Pro Pro Glu Ser Ala Ile Thr Arg Ser Ser Glu Pro
   865                 870                 875                 880
   Val Arg Glu Asp Ser Ser Gly Met His His Glu Asn Gln Thr Tyr Pro
                   885                 890                 895
   Pro Tyr Ser Pro Gln Ala Gln Pro Gln Pro Ile His Arg Ile Asp Ser
               900                 905                 910
   Pro Gly Phe Lys Pro Ala Ser Gln Gln Lys Ala Glu Ala Ser Ser Pro
           915                 920                 925
   Val Pro Tyr Leu Ser Pro Glu Thr Asn Pro Ala Ser Ser Thr Ser Ala
       930                 935                 940
   Val Asn His Asn Val Asn Leu Thr Asn Val Arg Leu Glu Glu Pro Thr
   945                 950                 955                 960
   Pro Ala Pro Ser Thr Ser Tyr Ser Pro Gln Ala Asp Ser Leu Arg Thr
                   965                 970                 975
   Pro Ser Thr Glu Ala Ala His Ile Met Leu Arg Asp Gln Glu Pro Ser
               980                 985                 990
   Leu Ser Ser His Val Asp Pro Thr Lys Val Tyr Arg Lys Asp Pro Tyr
           995                 1000                1005
   Pro Glu Glu Met Met Arg Gln Asn His Val Leu Lys Gln Pro Ala Val
       1010                1015                1020
   Ser His Pro Gly His Arg Pro Asp Lys Glu Pro Asn Leu Thr Tyr Glu
   1025                1030                1035                1040
   Pro Gln Leu Pro Tyr Val Glu Lys Gln Ala Ser Arg Asp Leu Glu Gln
                   1045                1050                1055
   Pro Thr Tyr Arg Tyr Glu Ser Ser Ser Tyr Thr Asp Gln Phe Ser Arg
               1060                1065                1070
   Asn Tyr Glu His Arg Leu Arg Tyr Glu Asp Arg Val Pro Met Tyr Glu
           1075                1080                1085
   Glu Gln Trp Ser Tyr Tyr Asp Asp Lys Gln Pro Tyr Pro Ser Arg Pro
       1090                1095                1100
   Pro Phe Asp Asn Gln His Ser Gln Asp Leu Asp Ser Arg Gln His Pro
   1105                1110                1115                1120
   Glu Glu Ser Ser Glu Arg Gly Tyr Phe Pro Arg Phe Glu Glu Pro Ala
                   1125                1130                1135
   Pro Leu Ser Tyr Asp Ser Arg Pro Arg Tyr Glu Gln Ala Pro Arg Ala
               1140                1145                1150
   Ser Ala Leu Arg His Glu Glu Gln Pro Ala Pro Gly Tyr Asp Thr His
           1155                1160                1165
   Gly Arg Leu Arg Pro Glu Ala Gln Pro His Pro Ser Ala Gly Pro Lys
       1170                1175                1180
   Pro Ala Glu Ser Lys Gln Tyr Phe Glu Gln Tyr Ser Arg Ser Tyr Glu
   1185                1190                1195                1200
   Gln Val Pro Pro Gln Gly Phe Thr Ser Arg Ala Gly His Phe Glu Pro
                   1205                1210                1215
   Leu His Gly Ala Ala Ala Val Pro Pro Leu Ile Pro Ser Ser Gln His
               1220                1225                1230
   Lys Pro Glu Ala Leu Pro Ser Asn Thr Lys Pro Leu Pro Pro Pro Pro
           1235                1240                1245
   Thr Gln Thr Glu Glu Glu Glu Asp Pro Ala Met Lys Pro Gln Ser Val
       1250                1255                1260
   Leu Thr Arg Val Lys Met Phe Glu Asn Lys Arg Ser Ala Ser Leu Glu
   1265                1270                1275                1280
   Thr Lys Lys Asp Val Asn Asp Thr Gly Ser Phe Lys Pro Pro Glu Val
                   1285                1290                1295
   Ala Ser Lys Pro Ser Gly Ala Pro Ile Ile Gly Pro Lys Pro Thr Ser
               1300                1305                1310
   Gln Asn Gln Phe Ser Glu His Asp Lys Thr Leu Tyr Arg Ile Pro Glu
           1315                1320                1325
   Pro Gln Lys Pro Gln Leu Lys Pro Pro Glu Asp Ile Val Arg Ser Asn
       1330                1335                1340
   His Tyr Asp Pro Glu Glu Asp Glu Glu Tyr Tyr Arg Lys Gln Leu Ser
   1345                1350                1355                1360
   Tyr Phe Asp Arg Arg Ser Phe Glu Asn Lys Pro Pro Ala His Ile Ala
                   1365                1370                1375
   Ala Ser His Leu Ser Glu Pro Ala Lys Pro Ala His Ser Gln Asn Gln
               1380                1385                1390
   Ser Asn Phe Ser Ser Tyr Ser Ser Lys Gly Lys Pro Pro Glu Ala Asp
           1395                1400                1405
   Gly Val Asp Arg Ser Phe Gly Glu Lys Arg Tyr Glu Pro Ile Gln Ala
       1410                1415                1420
   Thr Pro Pro Pro Pro Pro Leu Pro Ser Gln Tyr Ala Gln Pro Ser Gln
   1425                1430                1435                1440
   Pro Val Thr Ser Ala Ser Leu His Ile His Ser Lys Gly Ala His Gly
                   1445                1450                1455
   Glu Gly Asn Ser Val Ser Leu Asp Phe Gln Asn Ser Leu Val Ser Lys
               1460                1465                1470
   Pro Asp Pro Pro Pro Ser Gln Asn Lys Pro Ala Thr Phe Arg Pro Pro
           1475                1480                1485
   Asn Arg Glu Asp Thr Ala Gln Ala Ala Phe Tyr Pro Gln Lys Ser Phe
       1490                1495                1500
   Pro Asp Lys Ala Pro Val Asn Gly Thr Glu Gln Thr Gln Lys Thr Val
   1505                1510                1515                1520
   Thr Pro Ala Tyr Asn Arg Phe Thr Pro Lys Pro Tyr Thr Ser Ser Ala
                   1525                1530                1535
   Arg Pro Phe Glu Arg Lys Phe Glu Ser Pro Lys Phe Asn His Asn Leu
               1540                1545                1550
   Leu Pro Ser Glu Thr Ala His Lys Pro Asp Leu Ser Ser Lys Thr Pro
           1555                1560                1565
   Thr Ser Pro Lys Thr Leu Val Lys Ser His Ser Leu Ala Gln Pro Pro
       1570                1575                1580
   Glu Phe Asp Ser Gly Val Glu Thr Phe Ser Ile His Ala Glu Lys Pro
   1585                1590                1595                1600
   Lys Tyr Gln Ile Asn Asn Ile Ser Thr Val Pro Lys Ala Ile Pro Val
                   1605                1610                1615
   Ser Pro Ser Ala Val Glu Glu Asp Glu Asp Glu Asp Gly His Thr Val
               1620                1625                1630
   Val Ala Thr Ala Arg Gly Ile Phe Asn Ser Asn Gly Gly Val Leu Ser
           1635                1640                1645
   Ser Ile Glu Thr Gly Val Ser Ile Ile Ile Pro Gln Gly Ala Ile Pro
       1650                1655                1660
   Glu Gly Val Glu Gln Glu Ile Tyr Phe Lys Val Cys Arg Asp Asn Ser
   1665                1670                1675                1680
   Ile Leu Pro Pro Leu Asp Lys Glu Lys Gly Glu Thr Leu Leu Ser Pro
                   1685                1690                1695
   Leu Val Met Cys Gly Pro His Gly Leu Lys Phe Leu Lys Pro Val Glu
               1700                1705                1710
   Leu Arg Leu Pro His Cys Asp Pro Lys Thr Trp Gln Asn Lys Cys Leu
           1715                1720                1725
   Pro Gly Asp Pro Asn Tyr Leu Val Gly Ala Asn Cys Val Ser Val Leu
       1730                1735                1740
   Ile Asp His Phe
   1745
<210> 39
   <211> 4725
   <212> DNA
   <213> Homo sapiens
<400> 39
   gacgcggttc gccgcaggag cctcgaaggc gcggcgccgg cgagcccttc cccggcaggc 60
   gcgtgggtgg tagcggccaa tttgacagtt tcccgggccg ggcggccagc gcggaggcgc 120
   cacgctcggg tcgggggcgg gctgacgccg ccgccgccgc gggaggaggg acaaaggggt 180
   gggtccccgc gggtcggcac cccggcggtt gggctgcggg tcagagcact gtccggtggt 240
   gcccaggagg agtaggagca ggagcagaag cagaagcggg gtccggagct gcgcgcctac 300
   gcgggacctg tgtccgaaat gccggtgcga ggagaccgcg ggtttccacc ccggcgggag 360
   ctgtcaggtt ggctccgcgc cccaggcatg gaagagctga tatgggaaca gtacactgtg 420
   accctacaaa aggattccaa aagaggattt ggaattgcag tgtccggagg cagagacaac 480
   ccccactttg aaaatggaga aacgtcaatt gtcatttctg atgtgctccc gggtgggcct 540
   gctgatgggc tgctccaaga aaatgacaga gtggtcatgg tcaatggcac ccccatggag 600
   gatgtgcttc attcgtttgc agttcagcag ctcagaaaaa gtgggaaggt cgctgctatt 660
   gtggtcaaga ggccccggaa ggtccaggtg gccgcacttc aggccagccc tcccctggat 720
   caggatgacc gggcttttga ggtgatggac gagtttgatg gcagaagttt ccggagtggc 780
   tacagcgaga ggagccggct gaacagccat ggggggcgca gccgcagctg ggaggacagc 840
   ccggaaaggg ggcgtcccca tgagcgggcc cggagccggg agcgggacct cagccgggac 900
   cggagccgtg gccggagcct ggagcggggc ctggaccaag accatgcgcg cacccgagac 960
   cgcagccgtg gccggagcct ggagcggggc ctggaccacg actttgggcc atcccgggac 1020
   cgggaccgtg accgcagccg cggccggagc attgaccagg actacgagcg agcctatcac 1080
   cgggcctacg acccagacta cgagcgggcc tacagcccgg agtacaggcg cggggcccgc 1140
   cacgatgccc gctctcgggg accccgaagc cgcagccgcg agcacccgca ctcacggagc 1200
   cccagccccg agcctagggg gcggccgggg cccatcgggg tcctcctgat gaaaagcaga 1260
   gcgaacgaag agtatggtct ccggcttggg agtcagatct tcgtaaagga aatgacccga 1320
   acgggtctgg caactaaaga tggcaacctt cacgaaggag acataattct caagatcaat 1380
   gggactgtaa ctgagaacat gtctttaacg gatgctcgaa aattgataga aaagtcaaga 1440
   ggaaaactac agctagtggt gttgagagac agccagcaga ccctcatcaa catcccgtca 1500
   ttaaatgaca gtgactcaga aatagaagat atttcagaaa tagagtcaaa ccgatcattt 1560
   tctccagagg agagacgtca tcagtattct gattatgatt atcattcctc aagtgagaag 1620
   ctgaaggaaa ggccaagttc cagagaggac acgccgagca gattgtccag gatgggtgcg 1680
   acacccactc cctttaagtc cacaggggat attgcaggca cagttgtccc agagaccaac 1740
   aaggaaccca gataccaaga ggacccccca gctcctcaac caaaagcagc cccgagaact 1800
   tttcttcgtc ctagtcctga agatgaagca atatatggcc ctaataccaa aatggtaagg 1860
   ttcaagaagg gagacagcgt gggcctccgg ttggctggtg gcaatgatgt cgggatattt 1920
   gttgctggca ttcaagaagg gacctcggcg gagcaggagg gccttcaaga aggagaccag 1980
   attctgaagg tgaacacaca ggatttcaga ggattagtgc gggaggatgc cgttctctac 2040
   ctgttagaaa tccctaaagg tgaaatggtg accattttag ctcagagccg agccgatgtg 2100
   tatagagaca tcctggcttg tggcagaggg gattcgtttt ttataagaag ccactttgaa 2160
   tgtgagaagg aaactccaca gagcctggcc ttcaccagag gggaggtctt ccgagtggta 2220
   gacacactgt atgacggcaa gctgggcaac tggctggctg tgaggattgg gaacgagttg 2280
   gagaaaggct taatccccaa caagagcaga gctgaacaaa tggccagtgt tcaaaatgcc 2340
   cagagagaca acgctgggga ccgggcagat ttctggagaa tgcgtggcca gaggtctggg 2400
   gtgaagaaga acctgaggaa aagtcgggaa gacctcacag ctgttgtgtc tgtcagcacc 2460
   aagttcccag cttatgagag ggttttgctg cgagaagctg gtttcaagag acctgtggtc 2520
   ttattcggcc ccatagctga tatagcaatg gaaaaattgg ctaatgagtt acctgactgg 2580
   tttcaaactg ctaaaacgga accaaaagat gcaggatctg agaaatccac tggagtggtc 2640
   cggttaaata ccgtgaggca aattattgaa caggataagc atgcactact ggatgtgact 2700
   ccgaaagctg tggacctgtt gaattacacc cagtggttcc caattgtgat ttttttcaac 2760
   ccagactcca gacaaggtgt caaaaccatg agacaaaggt taaatccaac gtccaacaaa 2820
   agttctcgaa agttatttga tcaagccaac aagcttaaaa aaacgtgtgc acaccttttt 2880
   acagctacaa tcaacctaaa ttcagccaat gatagctggt ttggcagctt aaaggacact 2940
   attcagcatc agcaaggaga agcggtttgg gtctctgaag gaaagatgga agggatggat 3000
   gatgaccccg aagaccgcat gtcctactta accgccatgg gcgcggacta tctgagttgc 3060
   gacagccgcc tcatcagtga ctttgaagac acggacggtg aaggaggcgc ctacactgac 3120
   aatgagctgg atgagccagc cgaggagccg ctggtgtcgt ccatcacccg ctcctcggag 3180
   ccggtgcagc acgaggagag cataaggaaa cccagcccag agccacgagc tcagatgagg 3240
   agggctgcta gcagcgatca acttagggac aatagcccgc ccccagcatt caagccagag 3300
   ccgcccaagg ccaaaaccca gaacaaagaa gaatcctatg acttctccaa atcctatgaa 3360
   tataagtcaa acccctctgc cgttgctggt aatgaaactc ctggggcatc taccaaaggt 3420
   tatcctcctc ctgttgcagc aaaacctacc tttgggcggt ctatactgaa gccctccact 3480
   cccatccctc ctcaagaggg tgaggaggtg ggagagagca gtgaggagca agataatgct 3540
   cccaaatcag tcctgggcaa agtcaaaata tttgagaaga tggatcacaa ggccaggtta 3600
   cagagaatgc aggagctcca ggaagcacag aatgcaagga tcgaaattgc ccagaagcat 3660
   cctgatatct atgcagttcc aatcaaaacg cacaagccag accctggcac gccccagcac 3720
   acgagttcca gaccccctga gccacagaaa gctccttcca gaccttatca ggataccaga 3780
   ggaagttatg gcagtgatgc cgaggaggag gagtaccgcc agcagctgtc agaacactcc 3840
   aagcgcggtt actatggcca gtctgcccga taccgggaca cagaattata gatgtctgag 3900
   cacggactct cccaggcctg cctgcatggc atcagactag ccactcctgc caggccgccg 3960
   ggatggttct tctccagtta gaatgcacca tggagacgtg gtgggactcc agctcgtgtg 4020
   tcctcatgga gaacccaggg gacagctggt gcaaattcag aactgagggc tctgtttgtg 4080
   ggactgggtt agaggagtct gtggcttttt gttcagaatt aagcagaaca ctgcagtcag 4140
   atcctgttac ttgcttcagt ggaccgaaat ctgtattctg tttgcgtact tgtaatatgt 4200
   atattaagaa gcaataacta tttttcctca ttaatagctg ccttcaagga ctgtttcagt 4260
   gtgagtcaga atgtgaaaaa ggaataaaaa atactgttgg gctcaaacta aattcaaaga 4320
   agtactttat tgcaactctt ttaagtgcct tggatgagaa gtgtcttaaa ttttcttcct 4380
   ttgaagcttt aggcagagcc ataatggact aaaacatttt gactaagttt ttataccagc 4440
   ttaatagctg tagttttccc tgcactgtgt catcttttca aggcatttgt ctttgtaata 4500
   ttttccataa atttggactg tctatatcat aactatactt gatagtttgg ctataagtgc 4560
   tcaatagctt gaagcccaag aagttggtat cgaaatttgt tgtttgttta aacccaagtg 4620
   ctgcacaaaa gcagatactt gaggaaaaca ctatttccaa aagcacatgt attgacaaca 4680
   gttttataat ttaataaaaa ggaatacatt gcaatccgta atttt                4725
<210> 40
   <211> 1190
   <212> PRT
   <213> Homo sapiens
<400> 40
<210> 41
   <211> 6753
   <212> DNA
   <213> Homo sapiens
<400> 41
   gcggccgccc gggctaagag cggccggctg gagccgctga gcccccgctg cggccgggag 60
   ctgcatgggg gagcgccggc agcgcttggg aagatgcccc ggccggagct gcccctgccg 120
   gagggctggg aggaggcgcg cgacttcgac ggcaaggtct actacataga ccacacgaac 180
   cgcaccacca gctggatcga cccgcgggac aggtacacca aaccgctcac ctttgctgac 240
   tgcattagtg atgagttgcc gctaggatgg gaagaggcat atgacccaca ggttggagat 300
   tacttcatag accacaacac caaaaccact cagattgagg atcctcgagt acaatggcgg 360
   cgggagcagg aacatatgct gaaggattac ctggtggtgg cccaggaggc tctgagtgca 420
   caaaaggaga tctaccaggt gaagcagcag cgcctggagc ttgcacagca ggagtaccag 480
   caactgcatg ccgtctggga gcataagctg ggctcccagg tcagcttggt ctctggttca 540
   tcatccagct ccaagtatga ccctgagatc ctgaaagctg aaattgccac tgcaaaatcc 600
   cgggtcaaca agctgaagag agagatggtt cacctccagc acgagctgca gttcaaagag 660
   cgtggctttc agaccctgaa gaaaatcgat aagaaaatgt ctgatgctca gggcagctac 720
   aaactggatg aagctcaggc tgtcttgaga gaaacaaaag ccatcaaaaa ggctattacc 780
   tgtggggaaa aggaaaagca agatctcatt aagagccttg ccatgttgaa ggacggcttc 840
   cgcactgaca gggggtctca ctcagacctg tggtccagca gcagctctct ggagagttcg 900
   agtttcccgc taccgaaaca gtacctggat gtgagctccc agacagacat ctcgggaagc 960
   ttcggcatca acagcaacaa tcagttggca gagaaggtca gattgcgcct tcgatatgaa 1020
   gaggctaaga gaaggatcgc caacctgaag atccagctgg ccaagcttga cagtgaggcc 1080
   tggcctgggg tgctggactc agagagggac cggctgatcc ttatcaacga gaaggaggag 1140
   ctgctgaagg agatgcgctt catcagcccc cgcaagtgga cccaggggga ggtggagcag 1200
   ctggagatgg cccggaagcg gctggaaaag gacctgcagg cagcccggga cacccagagc 1260
   aaggcgctga cggagaggtt aaagttaaac agtaagagga accagcttgt gagagaactg 1320
   gaggaagcca cccggcaggt ggcaactctg cactcccagc tgaaaagtct ctcaagcagc 1380
   atgcagtccc tgtcctcagg cagcagcccc ggatccctca cgtccagccg gggctccctg 1440
   gttgcatcca gcctggactc ctccacttca gccagcttca ctgacctcta ctatgacccc 1500
   tttgagcagc tggactcaga gctgcagagc aaggtggagt tcctgctcct ggagggggcc 1560
   accggcttcc ggccctcagg ctgcatcacc accatccacg aggatgaggt ggccaagacc 1620
   cagaaggcag agggaggtgg ccgcctgcag gctctgcgtt ccctgtctgg caccccaaag 1680
   tccatgacct ccctatcccc acgttcctct ctctcctccc cctccccacc ctgttcccct 1740
   ctcatggctg accccctcct ggctggtgat gccttcctca actccttgga gtttgaagac 1800
   ccggagctga gtgccactct ttgtgaactg agccttggta acagcgccca ggaaagatac 1860
   cggctggagg aaccaggaac ggagggcaag cagctgggcc aagctgtgaa tacggcccag 1920
   gggtgtggcc tgaaagtggc ctgtgtctca gccgccgtat cggacgagtc agtggctgga 1980
   gacagtggtg tgtacgaggc ttccgtgcag agactgggtg cttcagaagc tgctgcattt 2040
   gacagtgacg aatcggaagc agtgggtgcg acccgaattc agattgccct gaagtatgat 2100
   gagaagaata agcaatttgc aatattaatc atccagctga gtaacctttc tgctctgttg 2160
   cagcaacaag accagaaagt gaatatccgc gtggctgtcc ttccttgctc tgaaagcaca 2220
   acctgcctgt tccggacccg gcctctggac gcctcagaca ctctagtgtt caatgaggtg 2280
   ttctgggtat ccatgtccta tccagccctt caccagaaga ccttaagagt cgatgtctgt 2340
   accaccgaca ggagccatct ggaagagtgc ctgggaggcg cccagatcag cctggcggag 2400
   gtctgccggt ctggggagag gtcgactcgc tggtacaacc ttctcagcta caaatacttg 2460
   aagaaacaga gcagggagct caagccagtg ggagtcatgg cccctgcctc agggcctgcc 2520
   agcacggacg ctgtgtctgc tctgttggaa cagacagcag tggagctgga gaagaggcag 2580
   gagggcagga gcagcacaca gacactggaa gacagctgga ggtatgagga gaccagtgag 2640
   aatgaggcag tagccgagga agaggaggag gaggtggagg aggaggaggg agaagaggat 2700
   gttttcaccg agaaagcctc acctgatatg gatgggtacc cagcattaaa ggtggacaaa 2760
   gagaccaaca cggagacccc ggccccatcc cccacagtgg tgcgacctaa ggaccggaga 2820
   gtgggcaccc cgtcccaggg gccatttctt cgagggagca ccatcatccg ctctaagacc 2880
   ttctccccag gaccccagag ccagtacgtg tgccggctga atcggagtga tagtgacagc 2940
   tccactctgt ccaaaaagcc accttttgtt cgaaactccc tggagcgacg cagcgtccgg 3000
   atgaagcggc cgtccccacc cccacagcct tcctcggtca agtcgctgcg ctccgagcgt 3060
   ctgatccgta cctcgctgga cctggagtta gacctgcagg cgacaagaac ctggcacagc 3120
   caattgaccc aggagatctc ggtgctgaag gagctcaagg agcagctgga acaagccaag 3180
   agccacgggg agaaggagct gccacagtgg ttgcgtgagg acgagcgttt ccgcctgctg 3240
   ctgaggatgc tggagaagcg gcagatggac cgagcggagc acaagggtga gcttcagaca 3300
   gacaagatga tgagggcagc tgccaaggat gtgcacaggc tccgaggcca gagctgtaag 3360
   gaacccccag aagttcagtc tttcagggag aagatggcat ttttcacccg gcctcggatg 3420
   aatatcccag ctctctctgc agatgacgtc taatcgccag aaaagtattt cctttgttcc 3480
   actgaccagg ctgtgaacat tgactgtggc taaagttatt tatgtggtgt tatatgaagg 3540
   tactgagtca caagtcctct agtgctcttg ttggtttgaa gatgaaccga ctttttagtt 3600
   tgggtcctac tgttgttatt aaaaacagaa caaaaacaaa acacacacac acacaaaaac 3660
   agaaacaaaa aaaaccagca ttaaaataat aagattgtat agtttgtata tttaggagtg 3720
   tatttttggg aaagaaaatt taaatgaact aaagcagtat tgagttgctg ctcttcttaa 3780
   aatcgtttag attttttttg gtttgtacag ctccaccttt tagaggtctt actgcaataa 3840
   gaagtaatgc ctgggggacg gtaatcctaa taggacgtcc cgcacttgtc acagtacagc 3900
   taatttttcc tagttaacat attttgtaca atattaaaaa aatgcacaga aaccattggg 3960
   ggggattcag aggtgcatcc acggatcttc ttgagctgtg acgtgttttt atgtggctgc 4020
   ccaacgtgga gcgggcagtg tgataggctg ggtgggctaa gcagcctagt ctatgtgggt 4080
   gacaggccac gctggtctca gatgcccagt gaagccacta acatgagtga ggggagggct 4140
   gtggggaact ccattcagtt ttatctccat caataaagtg gcctttcaaa aagaatcttc 4200
   ctcttgctct ctttttcttt cctacccctc acttcatctg tttccctgat ttttgactct 4260
   cccctttcca gtcatttctt tcccacccat ccgcagtcct ggaaacattt attttttctt 4320
   ttgcccactg ttttcatttg ctcattaaat taaaatgact gctcggctca ttgggaatcc 4380
   acatccccaa gttagactgg ggatatgctc tctgtactgt ctccttctat ggaattccac 4440
   cccacccaga gagagatgac ttcacagttt gttcatatga gcatcatccc atgtcgtccc 4500
   caacccaggg gctggtaggt gctacagctt gtgcttcatg ctgctcctgt ggcccctatt 4560
   cctacccagc tcagagcttt gcagggtcta ctgcagacaa tcagaagtca gtttctaaca 4620
   aatagcaaca gccacaaatc tcttcctcct tcctctctga cattaccctg tgcaactttt 4680
   ctcaaagtct gttgcaccac tcagcaaagc aagttgcacc agctatatca gaatcacctg 4740
   gggcagccta ttaaaaatgc agactgttga gaccatcctg gctaacacgg tgaaaccctg 4800
   tctctactaa aaatacaaaa aattagccgg ccgtggtggc gggcagctac tcgggaggct 4860
   gaggcaggag aatgacctga acctgggagg cagagcttgc agtgagctga gattgtgcca 4920
   ctgcactcca gcctgggtga cagagcaaga ctccgtctca aaaaaaaaaa aaaaagaaaa 4980
   atgcagactg gctggcgcgg tggctcatgc ttgtaatccc agcactttgg gaggctgagg 5040
   cgggcagatc aactgagatc gggggttcga gaccagcctg atcaacatgg agaaacctgg 5100
   tctctactaa aaatacataa ttagctgggc gtagtggcgc atacctgtaa tcccagctac 5160
   ttgggtggcc aaggcttgaa cccaggaggc ggaggttgcg gtgagccgag atggcgccat 5220
   tgactccagc ctgggcaaca agagcgtaac tccatctcaa aaaaataata ataataaaaa 5280
   taaaatgcag actgtcaggt cctctctaga cccactgaac tatcccgccc accctgggac 5340
   ggtccccagt gccagtgttt tacccatgtt ccctctgtaa gtcttatgca caccgcaatt 5400
   tgagagcccc actggtataa agtgagtgaa acccaggcag agacaggcaa aatggtgagg 5460
   cccgcatcct attggtgagt cacacaggga acgctgaaat tcatggcctt ctccaggcta 5520
   ctgggaaccc atgggcggtg atggagggag tggtgccacc ttcacttgct gctgaggctc 5580
   ttccccctct ccccagcata ctccttacct ggctgtctct cctcatgtat aaggaaccta 5640
   tagtggtgta gaatctactc ttcttccccc tgtggcttcc aggcccccct gtatagctgc 5700
   tatccagccc gatttctcac cactggccca gggcttcctc ctgccccgtg gcttcacgtc 5760
   tctgaacaca tcaatctctg atgttctctc tccttccatt gaattccacc agacacattc 5820
   agggtttact tcgtaatgtc ttcatatgag tatcaatcaa caccttcccc aactcaattg 5880
   tactaggttg tagagcacaa ggatggtctc gtgctgctct gtggcacctg tgcctacact 5940
   cctctgagct ttgaggaggc tgctctcttt gctgacccca tgatcttttc tgcccttctg 6000
   ttaagggcat tggccacagc aacggggcaa atgccccaag ctggctgtaa gtgacccatc 6060
   cctttggctc ccatgattag accaaggaga ggcatggggt ccagctgagc cattcagaac 6120
   cattccttag cattttccac tcaaaggtta gagatgagat tttctcttcc caaggctacc 6180
   tctggccatg gttccagctt catgggggca atgggattag gaaaatgagg tcaacctgca 6240
   aaggaaagca gatgcaagag atggagacag aatgggggtg tcctggggat cttggagcct 6300
   gaattcattg gcacaaaagg cagcagcatc ctcactgtat ctgcagtcca tttggactca 6360
   ataaaaactt tgaaagtcac atgtgttatg gaattccttc tcagtgacac attcatctgt 6420
   gctcagttgt cccagcaagg gtcagcccct catacccctg cagcatccgc tgctatgaag 6480
   cagagctgta aacgccctcc ctgtgtatag gaaaagctac atggagcaaa tcctcctgcc 6540
   tgaagaagtg catctcagca tcacttcagc tgtcggggca tttgtgggga gaaccagacc 6600
   acctctgcgg aaggcagcag accctcttcc agccatggat ggagttgaat tctctataaa 6660
   cggttcacca gcaaaccacc aatacattcc attgtttgcc tagagagaaa tttaaaaata 6720
   aataaatgtt cacttataaa aaaaaaaaaa aaa                              6753
<210> 42
   <211> 1119
   <212> PRT
   <213> Homo sapiens
<400> 42
   Met Pro Arg Pro Glu Leu Pro Leu Pro Glu Gly Trp Glu Glu Ala Arg
    1               5                  10                  15
   Asp Phe Asp Gly Lys Val Tyr Tyr Ile Asp His Thr Asn Arg Thr Thr
               20                  25                  30
   Ser Trp Ile Asp Pro Arg Asp Arg Tyr Thr Lys Pro Leu Thr Phe Ala
           35                  40                  45
   Asp Cys Ile Ser Asp Glu Leu Pro Leu Gly Trp Glu Glu Ala Tyr Asp
       50                  55                  60
   Pro Gln Val Gly Asp Tyr Phe Ile Asp His Asn Thr Lys Thr Thr Gln
   65                  70                  75                  80
   Ile Glu Asp Pro Arg Val Gln Trp Arg Arg Glu Gln Glu His Met Leu
                   85                  90                  95
   Lys Asp Tyr Leu Val Val Ala Gln Glu Ala Leu Ser Ala Gln Lys Glu
               100                 105                 110
   Ile Tyr Gln Val Lys Gln Gln Arg Leu Glu Leu Ala Gln Gln Glu Tyr
           115                 120                 125
   Gln Gln Leu His Ala Val Trp Glu His Lys Leu Gly Ser Gln Val Ser
       130                 135                 140
   Leu Val Ser Gly Ser Ser Ser Ser Ser Lys Tyr Asp Pro Glu Ile Leu
   145                 150                 155                 160
   Lys Ala Glu Ile Ala Thr Ala Lys Ser Arg Val Asn Lys Leu Lys Arg
                   165                 170                 175
   Glu Met Val His Leu Gln His Glu Leu Gln Phe Lys Glu Arg Gly Phe
               180                 185                 190
   Gln Thr Leu Lys Lys Ile Asp Lys Lys Met Ser Asp Ala Gln Gly Ser
           195                 200                 205
   Tyr Lys Leu Asp Glu Ala Gln Ala Val Leu Arg Glu Thr Lys Ala Ile
       210                 215                 220
   Lys Lys Ala Ile Thr Cys Gly Glu Lys Glu Lys Gln Asp Leu Ile Lys
   225                 230                 235                 240
   Ser Leu Ala Met Leu Lys Asp Gly Phe Arg Thr Asp Arg Gly Ser His
                   245                 250                 255
   Ser Asp Leu Trp Ser Ser Ser Ser Ser Leu Glu Ser Ser Ser Phe Pro
               260                 265                 270
   Leu Pro Lys Gln Tyr Leu Asp Val Ser Ser Gln Thr Asp Ile Ser Gly
           275                 280                 285
   Ser Phe Gly Ile Asn Ser Asn Asn Gln Leu Ala Glu Lys Val Arg Leu
       290                 295                 300
   Arg Leu Arg Tyr Glu Glu Ala Lys Arg Arg Ile Ala Asn Leu Lys Ile
   305                 310                 315                 320
   Gln Leu Ala Lys Leu Asp Ser Glu Ala Trp Pro Gly Val Leu Asp Ser
                   325                 330                 335
   Glu Arg Asp Arg Leu Ile Leu Ile Asn Glu Lys Glu Glu Leu Leu Lys
               340                 345                 350
   Glu Met Arg Phe Ile Ser Pro Arg Lys Trp Thr Gln Gly Glu Val Glu
           355                 360                 365
   Gln Leu Glu Met Ala Arg Lys Arg Leu Glu Lys Asp Leu Gln Ala Ala
       370                 375                 380
   Arg Asp Thr Gln Ser Lys Ala Leu Thr Glu Arg Leu Lys Leu Asn Ser
   385                 390                 395                 400
   Lys Arg Asn Gln Leu Val Arg Glu Leu Glu Glu Ala Thr Arg Gln Val
                   405                 410                 415
   Ala Thr Leu His Ser Gln Leu Lys Ser Leu Ser Ser Ser Met Gln Ser
               420                 425                 430
   Leu Ser Ser Gly Ser Ser Pro Gly Ser Leu Thr Ser Ser Arg Gly Ser
           435                 440                 445
   Leu Val Ala Ser Ser Leu Asp Ser Ser Thr Ser Ala Ser Phe Thr Asp
       450                 455                 460
   Leu Tyr Tyr Asp Pro Phe Glu Gln Leu Asp Ser Glu Leu Gln Ser Lys
   465                 470                 475                 480
   Val Glu Phe Leu Leu Leu Glu Gly Ala Thr Gly Phe Arg Pro Ser Gly
                   485                 490                 495
   Cys Ile Thr Thr Ile His Glu Asp Glu Val Ala Lys Thr Gln Lys Ala
               500                 505                 510
   Glu Gly Gly Gly Arg Leu Gln Ala Leu Arg Ser Leu Ser Gly Thr Pro
           515                 520                 525
   Lys Ser Met Thr Ser Leu Ser Pro Arg Ser Ser Leu Ser Ser Pro Ser
       530                 535                 540
   Pro Pro Cys Ser Pro Leu Met Ala Asp Pro Leu Leu Ala Gly Asp Ala
   545                 550                 555                 560
   Phe Leu Asn Ser Leu Glu Phe Glu Asp Pro Glu Leu Ser Ala Thr Leu
                   565                 570                 575
   Cys Glu Leu Ser Leu Gly Asn Ser Ala Gln Glu Arg Tyr Arg Leu Glu
               580                 585                 590
   Glu Pro Gly Thr Glu Gly Lys Gln Leu Gly Gln Ala Val Asn Thr Ala
           595                 600                 605
   Gln Gly Cys Gly Leu Lys Val Ala Cys Val Ser Ala Ala Val Ser Asp
       610                 615                 620
   Glu Ser Val Ala Gly Asp Ser Gly Val Tyr Glu Ala Ser Val Gln Arg
   625                 630                 635                 640
   Leu Gly Ala Ser Glu Ala Ala Ala Phe Asp Ser Asp Glu Ser Glu Ala
   645 650 655
   Val Gly Ala Thr Arg Ile Gln Ile Ala Leu Lys Tyr Asp Glu Lys Asn
   660 665 670
   Lys Gln Phe Ala Ile Leu Ile Ile Gln Leu Ser Asn Leu Ser Ala Leu
   675 680 685
   Leu Gln Gln Gln Asp Gln Lys Val Asn Ile Arg Val Ala Val Leu Pro
   690 695 700
   Cys Ser Glu Ser Thr Thr Cys Leu Phe Arg Thr Arg Pro Leu Asp Ala
   705 710 715 720
   Ser Asp Thr Leu Val Phe Asn Glu Val Phe Trp Val Ser Met Ser Tyr
   725 730 735
   Pro Ala Leu His Gln Lys Thr Leu Arg Val Asp Val Cys Thr Thr Asp
   740 745 750
   Arg Ser His Leu Glu Glu Cys Leu Gly Gly Ala Gln Ile Ser Leu Ala
   755 760 765
   Glu Val Cys Arg Ser Gly Glu Arg Ser Thr Arg Trp Tyr Asn Leu Leu
   770 775 780
   Ser Tyr Lys Tyr Leu Lys Lys Gln Ser Arg Glu Leu Lys Pro Val Gly
   785 790 795 800
   Val Met Ala Pro Ala Ser Gly Pro Ala Ser Thr Asp Ala Val Ser Ala
   805 810 815
   Leu Leu Glu Gln Thr Ala Val Glu Leu Glu Lys Arg Gln Glu Gly Arg
   820 825 830
   Ser Ser Thr Gln Thr Leu Glu Asp Ser Trp Arg Tyr Glu Glu Thr Ser
   835 840 845
   Glu Asn Glu Ala Val Ala Glu Glu Glu Glu Glu Glu Val Glu Glu Glu
   850 855 860
   Glu Gly Glu Glu Asp Val Phe Thr Glu Lys Ala Ser Pro Asp Met Asp
   865 870 875 880
   Gly Tyr Pro Ala Leu Lys Val Asp Lys Glu Thr Asn Thr Glu Thr Pro
   885 890 895
   Ala Pro Ser Pro Thr Val Val Arg Pro Lys Asp Arg Arg Val Gly Thr
   900 905 910
   Pro Ser Gln Gly Pro Phe Leu Arg Gly Ser Thr Ile Ile Arg Ser Lys
   915 920 925
   Thr Phe Ser Pro Gly Pro Gln Ser Gln Tyr Val Cys Arg Leu Asn Arg
   930 935 940
   Ser Asp Ser Asp Ser Ser Thr Leu Ser Lys Lys Pro Pro Phe Val Arg
   945 950 955 960
   Asn Ser Leu Glu Arg Arg Ser Val Arg Met Lys Arg Pro Ser Pro Pro
   965 970 975
   Pro Gln Pro Ser Ser Val Lys Ser Leu Arg Ser Glu Arg Leu Ile Arg
   980 985 990
   Thr Ser Leu Asp Leu Glu Leu Asp Leu Gln Ala Thr Arg Thr Trp His
   995 1000 1005
   Ser Gln Leu Thr Gln Glu Ile Ser Val Leu Lys Glu Leu Lys Glu Gln
   1010 1015 1020
   Leu Glu Gln Ala Lys Ser His Gly Glu Lys Glu Leu Pro Gln Trp Leu
   1025 1030 1035 1040
   Arg Glu Asp Glu Arg Phe Arg Leu Leu Leu Arg Met Leu Glu Lys Arg
   1045 1050 1055
   Gln Met Asp Arg Ala Glu His Lys Gly Glu Leu Gln Thr Asp Lys Met
   1060 1065 1070
   Met Arg Ala Ala Ala Lys Asp Val His Arg Leu Arg Gly Gln Ser Cys
   1075 1080 1085
   Lys Glu Pro Pro Glu Val Gln Ser Phe Arg Glu Lys Met Ala Phe Phe
   1090 1095 1100
   Thr Arg Pro Arg Met Asn Ile Pro Ala Leu Ser Ala Asp Asp Val
   1105 1110 1115
<210> 43
   <211> 5052
   <212> DNA
   <213> Homo sapiens
<400> 43
   gacacactcc tctacaacac cagagactcc caaacacaag gccttatatt gactcatttc 60
   agctcacatc ctggcgactc tcaagagaga aacctcagag tgactaaaat ctccataatg 120
   agaagacatg tacattcagt atctattttg gcattttccc caatacatct ctgctcatct 180
   gactcttatc ttggcatctg cttcctggtg gatctgaact gacccataag ccacgcttac 240
   tagtgatttt ccagaagatg aatccggcct cggcgccccc tccgctcccg ccgcctgggc 300
   agcaagtgat ccacgtcacg caggacctag acacagacct cgaagccctc ttcaactctg 360
   tcatgaatcc gaagcctagc tcgtggcgga agaagatcct gccggagtct ttctttaagg 420
   agcctgattc gggctcgcac tcgcgccagt ccagcaccga ctcgtcgggc ggccacccgg 480
   ggcctcgact ggctgggggt gcccagcatg tccgctcgca ctcgtcgccc gcgtccctgc 540
   agctgggcac cggcgcgggt gctgcgggta gccccgcgca gcagcacgcg cacctccgcc 600
   agcagtccta cgacgtgacc gacgagctgc cactgccccc gggctgggag atgaccttca 660
   cggccactgg ccagaggtac ttcctcaatc acatagaaaa aatcaccaca tggcaagacc 720
   ctaggaaggc gatgaatcag cctctgaatc atatgaacct ccaccctgcc gtcagttcca 780
   caccagtgcc tcagaggtcc atggcagtat cccagccaaa tctcgtgatg aatcaccaac 840
   accagcagca gatggccccc agtaccctga gccagcagaa ccaccccact cagaacccac 900
   ccgcagggct catgagtatg cccaatgcgc tgaccactca gcagcagcag cagcagaaac 960
   tgcggcttca gagaatccag atggagagag aaaggattcg aatgcgccaa gaggagctca 1020
   tgaggcagga agctgccctc tgtcgacagc tccccatgga agctgagact cttgccccag 1080
   ttcaggctgc tgtcaaccca cccacgatga ccccagacat gagatccatc actaataata 1140
   gctcagatcc tttcctcaat ggagggccat atcattcgag ggagcagagc actgacagtg 1200
   gcctggggtt agggtgctac agtgtcccca caactccgga ggacttcctc agcaatgtgg 1260
   atgagatgga tacaggagaa aacgcaggac aaacacccat gaacatcaat ccccaacaga 1320
   cccgtttccc tgatttcctt gactgtcttc caggaacaaa cgttgactta ggaactttgg 1380
   aatctgaaga cctgatcccc ctcttcaatg atgtagagtc tgctctgaac aaaagtgagc 1440
   cctttctaac ctggctgtaa tcactaccat tgtaacttgg atgtagccat gaccttacat 1500
   ttcctgggcc tcttggaaaa agtgatggag cagagcaagt ctgcaggtgc accacttccc 1560
   gcctccatga ctcgtgctcc ctccttttta tgttgccagt ttaatcattg cctggttttg 1620
   attgagagta acttaagtta aacataaata aatattctat tttcattttc tgcaagcctg 1680
   cgttcttgtg acagattata cagaattgtg tctgcaggat tgattatgca gaatactttt 1740
   ctctttcttc tctgctgccc catggctaag ctttatgggt gttaattgaa atttatacac 1800
   caattgattt taaaccataa aaagctgacc acaggcagtt acttctgagg gcatcttggt 1860
   ccaggaaatg tgcacaaaat tcgacctgat ttacagtttc aaaaactgta ttgatgacag 1920
   tagtaccaaa tgctttaaaa actatttaac ttgagcttta aaaatcattg tatggatagt 1980
   aaaattctac tgtatggaat acaatgtaat tttgaatcca tgctggctct gatggctctt 2040
   attagtctgt atttataaag gcacacagtc ctattgtagc ttatctttcg ttattttact 2100
   gcagagcatc tagacaactt agtccctcca gcgggaaagt agcagcagca gcattagtca 2160
   caggtcttac actacagatc ttgtgaaaga gaccagtttg gtactaatta tgagcatttt 2220
   attcaaacaa aagtttttga aatattacaa ctggggattt aaaaaattgc agcttagaat 2280
   ctgatggttt ttttttttct tgatgttgtt tgtttgtttt tgagatcgag ttttgctctt 2340
   gttgtccagg ctggaatgca atggcacaat ctcggctcac tgcaacctct gccttctggg 2400
   ttcaagcgat tctcctgcct tagcctcccg agtagctggg attacaggca cctgccacca 2460
   cgtccggcta attttttgta ttttgagtag agacggggtt tcaccataat ggtcaggctg 2520
   ttctcaaact cctgatctca ggtgatccac ccatctcggc ctcccaaagt gctgggatta 2580
   ctggcgtgag ccaccgcacc cggccttgat gtttatttta taaagcactg taattttgta 2640
   gctgatgaca aaaggcagcc aaatgttttt gataaatcag tggcaactgt atttttgtct 2700
   tttgaaataa ctctgaaaac atcaggacaa catagatttc aacctgatag cacaccacac 2760
   acagtgagct gttgcttttt aaattctgaa gccttgtcag gtttgcttcc tagatttcaa 2820
   gtgtttaaaa taattctatc tatgaaactg aaggatgaag cagatctctg actgacatgt 2880
   aaaaaaaaat gccctttgag ggtgtatggt ggagataaat gtttctgaat tcagtaaaat 2940
   tgattcctaa gtatattatc ctaatcctgt ttgctacagt tggtataaaa aggcatgaaa 3000
   tatgtattca atacctctta tgtaaccaaa accattttta attagctttt aaggactgag 3060
   agagcatcat gttcaactgg catgcagtct gcctgcattg ccaatgaagt cctcaactgt 3120
   ttaatatttt gaactaatat tatttataat ctatgaattt aatctttttt gaaagacttt 3180
   aataatttga gtctctgaga ggatactttc aatttccatg gggacttatt ttgttgggga 3240
   tcttaaataa gattcctttt gatctaccgg aatatacatg tacagagtac attggatcat 3300
   gttggaaaga aggcaagtga aaaggtcaga gatgaagtag caaagttatg gaatatcgtg 3360
   gaaaggatac tagttgtgaa atggaaagag acaagttata gtaccccaaa agcaaaacaa 3420
   gcaggagatg caagagatgc cccaaaagga caaagcaaca attttctgtt gccaccttta 3480
   taccggaaga ctctgttgta gaagaaaaga aggctttggt gcaccttatg tgggaggagg 3540
   aggggcaggg catgctgatg ctgagcgtac aggcagacaa gagcgtagcc tgctgttgcc 3600
   tccatcacta tgaaatgact tattttacct gaaggaccca tggtttatgt tcctctaatt 3660
   cctttcactc tccctaagcc ctctgagaga gatgaagata gatgatttta ttgctactaa 3720
   attgaaggga gcactatttc tttttgtctt ttgttagcaa aaaattgcaa aaagaattgt 3780
   acattcttgc taaaaataaa taaataaata aaaaattaaa aaaacaaggg acctaacaaa 3840
   actcagcagt gttactgtat ttttaaaaaa tatttttata gactcatttt caggttatta 3900
   aatgtaagag aaacagatac ccctcttttt taaagtaggt aaatcattga tgatttatat 3960
   taccaatttt tagaagtaat tttctagtaa gcttgtggca tcagaaaata ctagaagatt 4020
   tttttagtta aattagttag aacatttatg aatgaatata ataaatattt tttcagaata 4080
   aaatatggac cctttgtgtt tactaataga taaagccaga tataattttt tgtttttaag 4140
   gccacaaaat atggcctttg ttaaagaaca ctaaagttag aaatctaaag ttagagcaac 4200
   tttttaatgg ctatttccta ttattgtaag tgttaaaacc cctgcagaat tcttgataag 4260
   gtgctattta tactatattt cttattataa gataactgtc tttagtcttc ttagtactag 4320
   tctttttagt actaaatcaa tcagtaaaca tcatcatttc accccaaaat tttgtcacag 4380
   aaaaggcgta tcaaatgaaa aataatttca gagatctttc tttcaagata ttttttcctg 4440
   ataaaataca ttgtcttgaa gtaaatacat tgtcaaaacc taattgcaat tctgttaaat 4500
   ctaagtaatt tttagacagt gtttcaccgt attatttagg atgtgaaatg ccatttcttt 4560
   cactgattac accatataca ggaaacaggt aaaacagtga aaactttatt gtgctggttg 4620
   atgccaactt ggttgaaaag ctctctgcag aagaagtgat ctagactgac agaagtgttg 4680
   ctaattacaa gttgtgttct catgacgtaa ttagaaagta acttctcaaa gtacaacttt 4740
   tatgaaaaaa ataagctgtt aaaaaaagga aatcgtaggt taatttaatt gggaaaatgg 4800
   gcaattgaca gagaccattt tcctaacaca tatatgtgct agtactttaa ctttttaaaa 4860
   ttttacttct acgttttgta atataaaaat ttctatttta agtttagaat gttatacgta 4920
   ccgaaagtat gcagccaaat cgatcagatc aaaccatttt acctggagtt tggtactggt 4980
   ttttacttct ctgaatctgt ataagaaaaa taaagacaat tgaacttcca aagaaaaaaa 5040
   aaaaaaaaaa aa                                                    5052
<210> 44
   <211> 400
   <212> PRT
   <213> Homo sapiens
<400> 44
   Met Asn Pro Ala Ser Ala Pro Pro Pro Leu Pro Pro Pro Gly Gln Gln
   1               5                   10                  15
   Val Ile His Val Thr Gln Asp Leu Asp Thr Asp Leu Glu Ala Leu Phe
               20                  25                  30
   Asn Ser Val Met Asn Pro Lys Pro Ser Ser Trp Arg Lys Lys Ile Leu
           35                  40                  45
   Pro Glu Ser Phe Phe Lys Glu Pro Asp Ser Gly Ser His Ser Arg Gln
       50                  55                  60
   Ser Ser Thr Asp Ser Ser Gly Gly His Pro Gly Pro Arg Leu Ala Gly
   65                  70                  75                  80
   Gly Ala Gln His Val Arg Ser His Ser Ser Pro Ala Ser Leu Gln Leu
                   85                  90                  95
   Gly Thr Gly Ala Gly Ala Ala Gly Ser Pro Ala Gln Gln His Ala His
               100                 105                 110
   Leu Arg Gln Gln Ser Tyr Asp Val Thr Asp Glu Leu Pro Leu Pro Pro
           115                 120                 125
   Gly Trp Glu Met Thr Phe Thr Ala Thr Gly Gln Arg Tyr Phe Leu Asn
       130                 135                 140
   His Ile Glu Lys Ile Thr Thr Trp Gln Asp Pro Arg Lys Ala Met Asn
   145                 150                 155                 160
   Gln Pro Leu Asn His Met Asn Leu His Pro Ala Val Ser Ser Thr Pro
                   165                 170                 175
   Val Pro Gln Arg Ser Met Ala Val Ser Gln Pro Asn Leu Val Met Asn
               180                 185                 190
   His Gln His Gln Gln Gln Met Ala Pro Ser Thr Leu Ser Gln Gln Asn
           195                 200                 205
   His Pro Thr Gln Asn Pro Pro Ala Gly Leu Met Ser Met Pro Asn Ala
       210                 215                 220
   Leu Thr Thr Gln Gln Gln Gln Gln Gln Lys Leu Arg Leu Gln Arg Ile
   225                 230                 235                 240
   Gln Met Glu Arg Glu Arg Ile Arg Met Arg Gln Glu Glu Leu Met Arg
                   245                 250                 255
   Gln Glu Ala Ala Leu Cys Arg Gln Leu Pro Met Glu Ala Glu Thr Leu
               260                 265                 270
   Ala Pro Val Gln Ala Ala Val Asn Pro Pro Thr Met Thr Pro Asp Met
           275                 280                 285
   Arg Ser Ile Thr Asn Asn Ser Ser Asp Pro Phe Leu Asn Gly Gly Pro
       290                 295                 300
   Tyr His Ser Arg Glu Gln Ser Thr Asp Ser Gly Leu Gly Leu Gly Cys
   305                 310                 315                 320
   Tyr Ser Val Pro Thr Thr Pro Glu Asp Phe Leu Ser Asn Val Asp Glu
                   325                 330                 335
   Met Asp Thr Gly Glu Asn Ala Gly Gln Thr Pro Met Asn Ile Asn Pro
               340                 345                 350
   Gln Gln Thr Arg Phe Pro Asp Phe Leu Asp Cys Leu Pro Gly Thr Asn
           355                 360                 365
   Val Asp Leu Gly Thr Leu Glu Ser Glu Asp Leu Ile Pro Leu Phe Asn
       370                 375                 380
   Asp Val Glu Ser Ala Leu Asn Lys Ser Glu Pro Phe Leu Thr Trp Leu
   385                 390                 395                 400
<210> 45
   <211> 5408
   <212> DNA
   <213> Homo sapiens
<400> 45
   gccgccgcca gggaaaagaa agggaggaag gaaggaacaa gaaaaggaaa taaagagaaa 60
   ggggaggcgg ggaaaggcaa cgagctgtcc ggcctccgtc aagggagttg gagggaaaaa 120
   gttctcaggc gccgcaggtc cgagtgcctc gcagcccctc ccgaggcgca gccgccagac 180
   cagtggagcc ggggcgcagg gcgggggcgg aggcgccggg gcgggggatg cggggccgcg 240
   gcgcagcccc ccggccctga gagcgaggac agcgccgccc ggcccgcagc cgtcgccgct 300
   tctccacctc ggcccgtgga gccggggcgt ccgggcgtag ccctcgctcg cctgggtcag 360
   ggggtgcgcg tcgggggagg cagaagccat ggatcccggg cagcagccgc cgcctcaacc 420
   ggccccccag ggccaagggc agccgccttc gcagcccccg caggggcagg gcccgccgtc 480
   cggacccggg caaccggcac ccgcggcgac ccaggcggcg ccgcaggcac cccccgccgg 540
   gcatcagatc gtgcacgtcc gcggggactc ggagaccgac ctggaggcgc tcttcaacgc 600
   cgtcatgaac cccaagacgg ccaacgtgcc ccagaccgtg cccatgaggc tccggaagct 660
   gcccgactcc ttcttcaagc cgccggagcc caaatcccac tcccgacagg ccagtactga 720
   tgcaggcact gcaggagccc tgactccaca gcatgttcga gctcattcct ctccagcttc 780
   tctgcagttg ggagctgttt ctcctgggac actgaccccc actggagtag tctctggccc 840
   agcagctaca cccacagctc agcatcttcg acagtcttct tttgagatac ctgatgatgt 900
   acctctgcca gcaggttggg agatggcaaa gacatcttct ggtcagagat acttcttaaa 960
   tcacatcgat cagacaacaa catggcagga ccccaggaag gccatgctgt cccagatgaa 1020
   cgtcacagcc cccaccagtc caccagtgca gcagaatatg atgaactcgg cttcaggtcc 1080
   tcttcctgat ggatgggaac aagccatgac tcaggatgga gaaatttact atataaacca 1140
   taagaacaag accacctctt ggctagaccc aaggcttgac cctcgttttg ccatgaacca 1200
   gagaatcagt cagagtgctc cagtgaaaca gccaccaccc ctggctcccc agagcccaca 1260
   gggaggcgtc atgggtggca gcaactccaa ccagcagcaa cagatgcgac tgcagcaact 1320
   gcagatggag aaggagaggc tgcggctgaa acagcaagaa ctgcttcggc aggtgaggcc 1380
   acaggcaatg cggaatatca atcccagcac agcaaattct ccaaaatgtc aggagttagc 1440
   cctgcgtagc cagttaccaa cactggagca ggatggtggg actcaaaatc cagtgtcttc 1500
   tcccgggatg tctcaggaat tgagaacaat gacgaccaat agctcagatc ctttccttaa 1560
   cagtggcacc tatcactctc gagatgagag tacagacagt ggactaagca tgagcagcta 1620
   cagtgtccct cgaaccccag atgacttcct gaacagtgtg gatgagatgg atacaggtga 1680
   tactatcaac caaagcaccc tgccctcaca gcagaaccgt ttcccagact accttgaagc 1740
   cattcctggg acaaatgtgg accttggaac actggaagga gatggaatga acatagaagg 1800
   agaggagctg atgccaagtc tgcaggaagc tttgagttct gacatcctta atgacatgga 1860
   gtctgttttg gctgccacca agctagataa agaaagcttt cttacatggt tatagagccc 1920
   tcaggcagac tgaattctaa atctgtgaag gatctaagga gacacatgca ccggaaattt 1980
   ccataagcca gttgcagttt tcaggctaat acagaaaaag atgaacaaac gtccagcaag 2040
   atactttaat cctctatttt gctcttcctt gtccattgct gctgttaatg tattgctgac 2100
   ctctttcaca gttggctcta aagaatcaaa agaaaaaaac tttttatttc ttttgctatt 2160
   aaaactactg ttcattttgg gggctggggg aagtgagcct gtttggatga tggatgccat 2220
   tccttttgcc cagttaaatg ttcaccaatc attttaacta aatactcaga cttagaagtc 2280
   agatgcttca tgtcacagca tttagtttgt tcaacagttg tttcttcagc ttcctttgtc 2340
   cagtggaaaa acatgattta ctggtctgac aagccaaaaa tgttatatct gatattaaat 2400
   acttaatgct gatttgaaga gatagctgaa accaaggctg aagactgttt tactttcagt 2460
   atttcttttt cctcctagtg ctatcattag tcacataatg accttgattt tattttagga 2520
   gcttataagg catgagacaa tttccatata aatatattaa ttattgccac atactctaat 2580
   atagattttg gtggataatt ttgtgggtgt gcattttgtt ctgttttgtt gggttttttg 2640
   ttttttttgt ttttggcagg gtcggtgggg gggttggttg gttggttggt tttgtcggaa 2700
   cctaggcaaa tgaccatatt agtgaatctg ttaatagttg tagcttggga tggttattgt 2760
   agttgttttg gtaaaatctt catttcctgg ttttttttac caccttattt aaatctcgat 2820
   tatctgctct ctcttttata tacatacaca cacccaaaca taacatttat aatagtgtgg 2880
   tagtggaatg tatccttttt taggtttccc tgctttccag ttaattttta aaatggtagc 2940
   gctttgtatg catttagaat acatgactag tagtttatat ttcactggta gtttaaatct 3000
   ggttggggca gtctgcagat gtttgaagta gtttagtgtt ctagaaagag ctattactgt 3060
   ggatagtgcc taggggagtg ctccacgccc tctgggcata cggtagatat tatctgatga 3120
   attggaaagg agcaaaccag aaatggcttt attttctccc ttggactaat ttttaagtct 3180
   cgattggaat tcagtgagta ggttcataat gtgcatgaca gaaataagct ttatagtggt 3240
   ttaccttcat ttagctttgg aagttttctt tgccttagtt ttggaagtaa attctagttt 3300
   gtagttctca tttgtaatga acacattaac gactagatta aaatattgcc ttcaagattg 3360
   ttcttactta caagacttgc tcctacttct atgctgaaaa ttgaccctgg atagaatact 3420
   ataaggtttt gagttagctg gaaaagtgat cagattaata aatgtatatt ggtagttgaa 3480
   tttagcaaag aaatagagat aatcatgatt atacctttat ttttacagga agagatgatg 3540
   taactagagt atgtgtctac aggagtaata atggtttcca aagagtattt tttaaaggaa 3600
   caaaacgagc atgaattaac tcttcaatat aagctatgaa gtaatagttg gttgtgaatt 3660
   aaagtggcac cagctagcac ctctgtgttt taagggtctt tcaatgtttc tagaataagc 3720
   ccttattttc aagggttcat aacaggcata aaatctcttc tcctggcaaa agctgctatg 3780
   aaaagcctca gcttgggaag atagattttt ttccccccaa ttacaaaatc taagtatttt 3840
   ggcccttcaa tttggaggag ggcaaaagtt ggaagtaaga agttttattt taagtacttt 3900
   cagtgctcaa aaaaatgcaa tcactgtgtt gtatataata gttcataggt tgatcactca 3960
   taataattga ctctaaggct tttattaaga aaacagcaga aagattaaat cttgaattaa 4020
   gtctgggggg aaatggccac tgcagatgga gttttagagt agtaatgaaa ttctacctag 4080
   aatgcaaaat tgggtatatg aattacatag catgttgttg ggattttttt taatgtgcag 4140
   aagatcaaag ctacttggaa ggagtgccta taatttgcca gtagccacag attaagatta 4200
   tatcttatat atcagcagat tagctttagc ttagggggag ggtgggaaag tttggggggg 4260
   gggttgtgaa gatttagggg gaccttgata gagaacttta taaacttctt tctctttaat 4320
   aaagacttgt cttacaccgt gctgccatta aaggcagctg ttctagagtt tcagtcacct 4380
   aagtacaccc acaaaacaat atgaatatgg agatcttcct ttacccctca actttaattt 4440
   gcccagttat acctcagtgt tgtagcagta ctgtgatacc tggcacagtg ctttgatctt 4500
   acgatgccct ctgtactgac ctgaaggaga cctaagagtc ctttcccttt ttgagtttga 4560
   atcatagcct tgatgtggtc tcttgtttta tgtccttgtt cctaatgtaa aagtgcttaa 4620
   ctgcttcttg gttgtattgg gtagcattgg gataagattt taactgggta ttcttgaatt 4680
   gcttttacaa taaaccaatt ttataatctt taaatttatc aactttttac atttgtgtta 4740
   ttttcagtca gggcttctta gatctactta tggttgatgg agcacattga tttggagttt 4800
   cagatcttcc aaagcactat ttgttgtaat aacttttcta aatgtagtgc ctttaaagga 4860
   aaaatgaaca cagggaagtg actttgctac aaataatgtt gctgtgttaa gtattcatat 4920
   taaatacatg ccttctatat ggaacatggc agaaagactg aaaaataaca gtaattaatt 4980
   gtgtaattca gaattcatac caatcagtgt tgaaactcaa acattgcaaa agtgggtggc 5040
   aatattcagt gcttaacact tttctagcgt tggtacatct gagaaatgag tgctcaggtg 5100
   gattttatcc tcgcaagcat gttgttataa gaattgtggg tgtgcctatc ataacaattg 5160
   ttttctgtat cttgaaaaag tattctccac attttaaatg ttttatatta gagaattctt 5220
   taatgcacac ttgtcaaata tatatatata gtaccaatgt taccttttta ttttttgttt 5280
   tagatgtaag agcatgctca tatgttaggt acttacataa attgttacat tattttttct 5340
   tatgtaatac ctttttgttt gtttatgtgg ttcaaatata ttctttcctt aaactcttaa 5400
   aaaaaaaa                                                          5408
<210> 46
   <211> 508
   <212> PRT
   <213> Homo sapiens
<400> 46
   Met Asp Pro Gly Gln Gln Pro Pro Pro Gln Pro Ala Pro Gln Gly Gln
   1               5                   10                  15
   Gly Gln Pro Pro Ser Gln Pro Pro Gln Gly Gln Gly Pro Pro Ser Gly
               20                  25                  30
   Pro Gly Gln Pro Ala Pro Ala Ala Thr Gln Ala Ala Pro Gln Ala Pro
           35                  40                  45
   Pro Ala Gly His Gln Ile Val His Val Arg Gly Asp Ser Glu Thr Asp
       50                  55                  60
   Leu Glu Ala Leu Phe Asn Ala Val Met Asn Pro Lys Thr Ala Asn Val
   65                  70                  75                  80
   Pro Gln Thr Val Pro Met Arg Leu Arg Lys Leu Pro Asp Ser Phe Phe
                   85                  90                  95
   Lys Pro Pro Glu Pro Lys Ser His Ser Arg Gln Ala Ser Thr Asp Ala
               100                 105                 110
   Gly Thr Ala Gly Ala Leu Thr Pro Gln His Val Arg Ala His Ser Ser
           115                 120                 125
   Pro Ala Ser Leu Gln Leu Gly Ala Val Ser Pro Gly Thr Leu Thr Pro
       130                 135                 140
   Thr Gly Val Val Ser Gly Pro Ala Ala Thr Pro Thr Ala Gln His Leu
   145                 150                 155                 160
   Arg Gln Ser Ser Phe Glu Ile Pro Asp Asp Val Pro Leu Pro Ala Gly
                   165                 170                 175
   Trp Glu Met Ala Lys Thr Ser Ser Gly Gln Arg Tyr Phe Leu Asn His
               180                 185                 190
   Ile Asp Gln Thr Thr Thr Trp Gln Asp Pro Arg Lys Ala Met Leu Ser
           195                 200                 205
   Gln Met Asn Val Thr Ala Pro Thr Ser Pro Pro Val Gln Gln Asn Met
       210                 215                 220
   Met Asn Ser Ala Ser Gly Pro Leu Pro Asp Gly Trp Glu Gln Ala Met
   225                 230                 235                 240
   Thr Gln Asp Gly Glu Ile Tyr Tyr Ile Asn His Lys Asn Lys Thr Thr
                   245                 250                 255
   Ser Trp Leu Asp Pro Arg Leu Asp Pro Arg Phe Ala Met Asn Gln Arg
               260                 265                 270
   Ile Ser Gln Ser Ala Pro Val Lys Gln Pro Pro Pro Leu Ala Pro Gln
           275                 280                 285
   Ser Pro Gln Gly Gly Val Met Gly Gly Ser Asn Ser Asn Gln Gln Gln
       290                 295                 300
   Gln Met Arg Leu Gln Gln Leu Gln Met Glu Lys Glu Arg Leu Arg Leu
   305                 310                 315                 320
   Lys Gln Gln Glu Leu Leu Arg Gln Val Arg Pro Gln Ala Met Arg Asn
                   325                 330                 335
   Ile Asn Pro Ser Thr Ala Asn Ser Pro Lys Cys Gln Glu Leu Ala Leu
               340                 345                 350
   Arg Ser Gln Leu Pro Thr Leu Glu Gln Asp Gly Gly Thr Gln Asn Pro
           355                 360                 365
   Val Ser Ser Pro Gly Met Ser Gln Glu Leu Arg Thr Met Thr Thr Asn
       370                 375                 380
   Ser Ser Asp Pro Phe Leu Asn Ser Gly Thr Tyr His Ser Arg Asp Glu
   385                 390                 395                 400
   Ser Thr Asp Ser Gly Leu Ser Met Ser Ser Tyr Ser Val Pro Arg Thr
                   405                 410                 415
   Pro Asp Asp Phe Leu Asn Ser Val Asp Glu Met Asp Thr Gly Asp Thr
               420                 425                 430
   Ile Asn Gln Ser Thr Leu Pro Ser Gln Gln Asn Arg Phe Pro Asp Tyr
           435                 440                 445
   Leu Glu Ala Ile Pro Gly Thr Asn Val Asp Leu Gly Thr Leu Glu Gly
       450                 455                 460
   Asp Gly Met Asn Ile Glu Gly Glu Glu Leu Met Pro Ser Leu Gln Glu
   465                 470                 475                 480
   Ala Leu Ser Ser Asp Ile Leu Asn Asp Met Glu Ser Val Leu Ala Ala
                   485                 490                 495
   Thr Lys Leu Asp Lys Glu Ser Phe Leu Thr Trp Leu
               500                 505

## Description

### SEQUENCE LISTING

This application contains a Sequence Listing which is submitted herewith in electronically readable format. The Sequence Listing file was created on June 23, 2016, is named "sequencelisting.txt," and its size is 320kb (328,378 bytes). The entire contents of the Sequence Listing in the sequencelisting.txt file are incorporated herein by this reference.

### FIELD OF THE DISCLOSURE

This disclosure relates to methods for the treatment of various cancers. In particular, the disclosure provides methods for treatment of various cancers by administering to a patient a selective inhibitor of Aurora A kinase if said patient is identified as a likely responder to the treatment by assessing the patient's genetic profile.

### BACKGROUND OF THE DISCLOSURE

Cells become cancerous when their genotype or phenotype alters in a way that there is uncontrolled growth that is not subject to the confines of the normal tissue environment. One or more genes is/are mutated, amplified, deleted, overexpressed or underexpressed. Chromosome portions can be lost or moved from one location to another. Some cancers have characteristic patterns by which genotypes or phenotypes are altered.

Many genes have mutations which are associated with cancer. Some genes have multiple sites where mutations can occur. Many cancers have mutations in and/or mis-expression of more than one gene. Gene mutations can facilitate tumor progression, tumor growth rate or whether a tumor will metastasize. Some mutations can affect whether a tumor cell will respond to therapy.

Regulation of the cell cycle checkpoints is a critical determinant of the manner in which tumor cells respond to many chemotherapies and radiation. Many effective cancer therapies work by causing DNA damage; however, resistance to these agents remains a significant limitation in the treatment of cancer. One important mechanism leading to drug resistance is the activation of a checkpoint pathway that arrests the cell cycle to provide time for repair. Through this mechanism cell cycle progression is prevented, and immediate cell death of the damaged cell may be avoided.

The cell division cycle also involves various protein kinases that are frequently overexpressed in cancer cells. Aurora A kinase, for example, is a key mitotic regulator that is implicated in the pathogenesis of several tumor types. The Aurora kinases, first identified in yeast (Ipl1), Xenopus (Eg2) and Drosophila (Aurora), are critical regulators of mitosis. (Embo J (1998) 17, 5627-5637; Genetics (1993) 135, 677-691; Cell (1995) 81, 95-105; J Cell Sci (1998) 111(Pt 5), 557-572). In humans, three isoforms of Aurora kinase exist, including Aurora A, Aurora B and Aurora C. Aurora A and Aurora B play critical roles in the normal progression of cells through mitosis, whereas Aurora C activity is largely restricted to meiotic cells. Aurora A and Aurora B are structurally closely related. Their catalytic domains lie in the C-terminus, where they differ in only a few amino acids. Greater diversity exists in their non-catalytic N-terminal domains. It is the sequence diversity in this region of Aurora A and Aurora B that dictates their interactions with distinct protein partners, allowing these kinases to have unique subcellular localizations and functions within mitotic cells.

The Aurora A gene (AURKA) localizes to chromosome 20q13.2 which is commonly amplified or overexpressed at a high incidence in a diverse array of tumor types. (Embo J (1998) 17, 3052-3065; Int J Cancer (2006) 118, 357-363; J Cell Biol (2003) 161, 267-280; Mol Cancer Ther (2007) 6, 1851-1857; J Natl Cancer Inst (2002) 94, 1320-1329). Increased Aurora A gene expression has been correlated to the etiology of cancer and to a worsened prognosis. (IntJ Oncol (2004) 25, 1631-1639; Cancer Res (2007) 67, 10436-10444; Clin Cancer Res (2004) 10, 2065-2071; Clin Cancer Res (2007) 13, 4098-4104; Int J Cancer (2001) 92, 370-373; Br J Cancer (2001) 84, 824-831; J Natl Cancer Inst (2002) 94, 1320-1329). This concept has been supported in experimental models, demonstrating that Aurora A overexpression leads to oncogenic transformation. (Cancer Res (2002) 62, 4115-4122; Mol Cancer Res (2009) 7, 678-688; Oncogene (2006) 25, 7148-7158; Cell Res (2006) 16, 356-366; Oncogene (2008) 27, 4305-4314; Nat Genet (1998) 20, 189-193). Overexpression of Aurora A kinase is suspected to result in a stoichiometric imbalance between Aurora A and its regulatory partners, leading to chromosomal instability and subsequent transforming events. The potential oncogenic role of Aurora A has led to considerable interest in targeting this kinase for the treatment of cancer.

As a key regulator of mitosis, Aurora A plays an essential role in mitotic entry and normal progression of cells through mitosis. (Nat Rev Mol Cell Biol (2003) 4, 842-854; Curr Top Dev Biol (2000) 49, 331-42; Nat Rev Mol Cell Biol (2001) 2(1), 21-32). During a normal cell cycle, Aurora A kinase is first expressed in the G2 stage where it localizes to centrosomes and functions in centrosome maturation and separation as well as in the entry of cells into mitosis. In mitotic cells Aurora A kinase predominantly localizes to centrosomes and the proximal portion of incipient mitotic spindles. There it interacts with and phosphorylates a diverse set of proteins that collectively function in the formation of mitotic spindle poles and spindles, the attachment of spindles to sister chromatid at the kinetochores, the subsequent alignment and separation of chromosome, the spindle assembly checkpoint and cytokinesis. (J Cell Sci (2007) 120, 2987-2996; Trends Cell Biol (1999) 9, 454-459; Nat Rev Mol Cell Biol (2003) 4, 842-854; Trends Cell Biol (2005) 15, 241-250).

Although selective inhibition of Aurora A kinase results in a delayed mitotic entry (The Journal of biological chemistry (2003) 278, 51786-51795), cells commonly enter mitosis despite having inactive Aurora A kinase. Cells in which Aurora A kinase has been selectively inhibited demonstrate a variety of mitotic defects including abnormal mitotic spindles (monopolar or multipolar spindles) and defects in the process of chromosome alignment. With time, monopolar and multipolar spindles may resolve to form two opposing spindle poles, although some of these defects may lead immediately to cell death via defective mitoses. While spindle defects resulting from Aurora A kinase inhibition induce mitotic delays, presumably through activation of the spindle assembly checkpoint, cells ultimately divide at a frequency near that of untreated cells. (Mol Cell Biol (2007) 27(12), 4513-25; Cell Cycle (2008) 7(17), 2691-704.; Mol Cancer Ther (2009) 8(7), 2046-56.). This inappropriate cell division occurs following a slow-acting suppression of the spindle assembly checkpoint due to loss of Aurora A kinase function. (Cell Cycle (2009) 8(6), 876-88). Bipolar spindles that are formed in the absence of Aurora A kinase function frequently show chromosome alignment and segregation defects, including chromosome congression defects at metaphase, lagging chromosomes at anaphase, and telophase bridges.

Some patients respond to one therapy better than another, presenting the potential for a patient to follow multiple therapeutic routes to effective therapy. Valuable time early in a patient's treatment program can be lost pursuing a therapy which eventually is proven ineffective for that patient. Many patients cannot afford the time for trial-and-error choices of therapeutic regimens. Expedient and accurate treatment decisions lead to effective management of the disease.

### SUMMARY OF THE DISCLOSURE

The present invention is defined by the appended claims. The present disclosure relates to prognosis, planning for treatment and treatment of tumors by measurement of at least one characteristic of a marker provided herein. Markers were identified in tumor biopsies and blood samples from 47 patients enrolled in a phase 1/2 clinical trial of single agent alisertib by associating their characteristics, e.g., size, sequence, composition, activity or amount, with outcome of subsequent treatment of the patient with alisertib therapy. The markers are predictive of whether there will be a favorable outcome (*e.g*., good response and/or long time-to-progression) after treatment of patients with an Aurora A Kinase inhibitor, such as alisertib. Testing patient samples comprising tumor cells to determine the presence, amounts or changes of genetic markers identifies particular patients who are expected to have a favorable outcome with treatment, *e.g*., with an Aurora A Kinase inhibitor, e.g., alisertib, and whose disease may be managed by standard or less aggressive treatment, as well as those patients who are expected to have an unfavorable outcome with the treatment and may require an alternative treatment to, a combination of treatments and/or more aggressive treatment with an Aurora A Kinase inhibitor to ensure a favorable outcome and/or successful management of the disease.

In one aspect, the disclosure provides kits useful in determination of characteristics, e.g., amounts, presence or changes, of the markers. In another aspect, the disclosure provides methods for determining prognosis and treatment or disease management strategies. In these aspects, the characteristic, e.g., size, sequence, composition, activity or amount of markers in a sample comprising tumor cells is measured. In one embodiment, the tumor is a liquid, e.g., hematological tumor, e.g., a leukemia, a lymphoma or a myeloma. In another embodiment, the tumor is a solid tumor, e.g., non-hematological tumor, e.g., breast cancer, ovarian cancer, prostate cancer, head and neck cancer, small cell lung cancer, non-small cell lung cancer, gastric cancer, renal cancer, pancreatic cancer, bladder cancer or melanoma.

In various embodiments, the characteristic, e.g., size, sequence, composition, activity or amount of marker DNA, the size, sequence, composition or amount of marker RNA and/or the size, sequence, composition, activity or amount of marker protein corresponding to a marker gene with one or more mutation, e.g., somatic mutation, described herein is measured. Useful information leading to the prognosis or treatment or disease management strategies is obtained when assays reveal information about a marker gene, e.g., whether the gene is mutated, or not, the identity of the mutation, and/or whether the RNA or protein amount of a mutated gene or genes indicates overexpression or underexpression. In one embodiment, the strategy is determined for therapy with Aurora A Kinase inhibitors, e.g., alisertib, a pharmaceutically acceptable salt or a pharmaceutical composition thereof (MLN8237).

A marker gene useful to test for determination of prognosis or treatment or disease management strategy is selected from the group consisting of the marker genes listed below in Table 8. In one embodiment a marker gene useful to test for determination of prognosis or treatment or disease management strategy is selected from the group consisting of genes within the Wnt signaling pathway or the Hippo signaling pathway. In yet a further embodiment, a marker gene useful to test for determination of prognosis or treatment or disease management strategy is selected from the group consisting of LEF1, MAP2K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWC1, WWTR1 and YAP1. Each marker gene includes mutations or alterations whose presence in DNA or whose effects, *e.g.,* on marker RNA and/or protein characteristics, e.g., amounts, size, sequence or composition, can provide information for determination of prognosis or treatment or disease management. In some embodiments, a gene or a mutant or modified form thereof useful as a marker, has a DNA, an RNA and/or protein characteristic, e.g., size, sequence, composition or amount, *e.g*., in a sample comprising tumor cells, which is different than a normal DNA, RNA and/or protein. Described herein are examples of modifications of these genes, referred to as "marker genes" whose mutation or amounts can provide such information.

The mutation of a marker gene of the present disclosure provides information about outcome after treatment, *e.g*., with an Aurora A Kinase inhibitor, *e.g*., alisertib. By examining a characteristic, e.g., size, sequence, composition, activity or amount of one or more of identified markers in a tumor, it is possible to determine which therapeutic agent, combination of agents, dosing and/or administration regimen is expected to provide a favorable outcome upon treatment. By examining the characteristic, e.g., size, sequence, composition, activity or amount of one or more of the identified markers or marker sets in a cancer, it is also possible to determine which therapeutic agent, combination of agents, dosing and/or administration regimen is less likely to provide a favorable outcome upon treatment. By examining the characteristic, e.g., size, sequence, composition, activity or amount of one or more of the identified markers, it is therefore possible to eliminate ineffective or inappropriate therapeutic agents or regimens. Importantly, these determinations can be made on a patient-by-patient basis. Thus, one can determine whether or not a particular therapeutic regimen is likely to benefit a particular patient or type of patient, and/or whether a particular regimen should be started or avoided, continued, discontinued or altered.

The present disclosure is directed to methods of identifying and/or selecting a cancer patient for treatment with a therapeutic regimen, *e.g.,* a therapeutic regimen comprising an Aurora A Kinase inhibitor, such as alisertib treatment, if the patient is expected to demonstrate a favorable outcome upon administration of the therapeutic regimen. The method may further comprise treating with the therapeutic regimen a cancer patient who is identified for a favorable outcome. Additionally provided are methods of identifying a patient for alternative therapy or supplemental therapy if the patient is expected to have an unfavorable outcome upon administration of such a therapeutic regimen. These methods typically include measuring, determining, receiving, storing or transmitting information about the characteristic, e.g., size, sequence, composition, activity or amount of one or more markers or mutation of marker genes in a patient's tumor *(e.g.,* in a patient's cancer cells, *e.g.,* non-hematological cancer cells, e.g., solid tumor cells), optionally comparing that to the characteristic, e.g., size, sequence, composition, activity or amount of a reference marker, and in a further embodiment, identifying or advising whether the result from the sample corresponds to a favorable outcome of a treatment regimen, *e.g*., an Aurora A Kinase inhibitor, e.g., alisertib regimen.

Additionally provided methods include therapeutic methods which further include the step of beginning, continuing, or commencing a therapy accordingly where the presence of a mutation in a marker gene or the characteristic, e.g., size, sequence, composition, activity or amount of a patient's marker or markers indicates that the patient is expected to demonstrate a favorable outcome with the therapy, *e.g*., an Aurora A Kinase inhibitor, such as alisertib therapeutic regimen. In addition, the methods include therapeutic methods which further include the step of stopping, discontinuing, altering or halting a therapy accordingly where the presence of a mutation in a marker gene or the characteristic, e.g., size, sequence, composition, activity or amount of a patient's marker indicates that the patient is expected to demonstrate an unfavorable outcome with the treatment, *e.g*., with an Aurora A Kinase inhibitor, such as alisertib treatment regimen, *e.g*., as compared to a patient identified as having a favorable outcome receiving the same therapeutic regimen. In another aspect, methods are provided for analysis of a patient not yet being treated with a therapy, *e.g*., an Aurora A Kinase inhibitor, such as alisertib, and identification and prediction of treatment outcome based upon the presence of a mutation in a marker gene or characteristic, e.g., size, sequence, composition, activity or amount of one or more of a patient's markers described herein. Such methods can include not being treated with the therapy, *e.g*., an Aurora A Kinase inhibitor, such as alisertib therapy; being treated with therapy, *e.g*., an Aurora A Kinase inhibitor, such as alisertib therapy in combination with one more additional therapies; being treated with an alternative therapy to an Aurora A Kinase inhibitor, such as alisertib therapy; or being treated with a more aggressive dosing and/or administration regimen of a therapy, *e.g.,* an Aurora A Kinase inhibitor, such as alisertib, *e.g.,* as compared to the dosing and/or administration regimen of a patient identified as having a favorable outcome to a standard Aurora A Kinase inhibitor, such as alisertib therapy. Thus, the provided methods of the disclosure can eliminate ineffective or inappropriate use of therapy, *e.g*., an Aurora A Kinase inhibitor, such as alisertib therapy regimens.

Additional methods include methods to determine the activity of an agent, the efficacy of an agent, or identify new therapeutic agents or combinations. Such methods include methods to identify an agent as useful, *e.g*., as an Aurora A Kinase inhibitor, such as alisertib, for treating a cancer, *e.g*., a non-hematological cancer, i.e., a solid tumor cancer (*e.g*., breast cancer, ovarian cancer, prostate cancer, head and neck cancer, small cell lung cancer, non-small cell lung cancer, gastric cancer, renal cancer, pancreatic cancer, bladder cancer or melanoma), based on its ability to affect the presence of a mutation in a marker gene or characteristic, e.g., size, sequence, composition, activity or amount of a marker or markers of the disclosure. In some embodiments, an inhibitor which decreases or increases the presence of a mutation in a marker gene or characteristic, *e.g*., size, sequence, composition, activity or amount of a marker or markers provided in a manner that indicates favorable outcome of a patient having cancer would be a candidate agent for the cancer. In another embodiment, an agent which is able to decrease the viability of a tumor cell comprising a marker indicative of an unfavorable outcome would be a candidate agent for the cancer.

The present disclosure is also directed to methods of treating a cancer patient with a therapeutic regimen, *e.g.,* an Aurora A Kinase inhibitor, such as alisertib therapeutic regimen *(e.g.,* alone, or in combination with an additional agent such as a chemotherapeutic agent), which includes the step of selecting for treatment a patient whose marker characteristic, e.g., size, sequence, composition, activity or amount indicates that the patient is expected to have a favorable outcome with the therapeutic regimen, and treating the patient with the therapy, *e.g*., an Aurora A Kinase inhibitor, such as alisertib therapy. In some embodiments, the method can include the step of selecting a patient whose marker characteristic, e.g., size, sequence, composition, activity or amount or amounts indicates that the patient is expected to have a favorable outcome and administering a therapy other than an Aurora A Kinase inhibitor therapy that demonstrates similar expected progression-free survival times as the Aurora A Kinase inhibitor, such as alisertib therapy.

Additional methods of treating a cancer patient include selecting patients that are unlikely to experience a favorable outcome upon treatment with a cancer therapy (*e.g*., an Aurora A Kinase inhibitor, such as alisertib therapy). Such methods can further include one or more of: administering a higher dose or increased dosing schedule of a therapy, *e.g*., an Aurora A Kinase inhibitor, such as alisertib as compared to the dose or dosing schedule of a patient identified as having a favorable outcome with standard therapy; administering a cancer therapy other than an Aurora A Kinase inhibitor, such as alisertib therapy; administering an Aurora A Kinase inhibitor, such as alisertib in combination with an additional agent. Further provided are methods for selection of a patient having aggressive disease which is expected to demonstrate more rapid time to progression.

Additional methods include a method to evaluate whether to treat or pay for the treatment of cancer, *e.g.,* non-hematological cancer, i.e., solid tumor cancer *(e.g.,* breast cancer, ovarian cancer, prostate cancer, head and neck cancer, small cell lung cancer, non-small cell lung cancer, gastric cancer, renal cancer, pancreatic cancer, bladder cancer or melanoma) by reviewing the amount of a patient's marker or markers for indication of outcome to a cancer therapy, *e.g*., an Aurora A Kinase inhibitor, such as alisertib therapy regimen, and making a decision or advising on whether payment should be made.

Other features and advantages of the disclosure will be apparent from the following detailed description, drawings and from the claims.

In one aspect, the disclosure provides a method for determining whether to treat a patient having cancer with an Aurora A kinase inhibitor, the method comprising the steps of: (a) obtaining a cancer cell sample from the patient; (b) determining whether any of the WNT pathway genes listed in Table 9 and/or any of the Hippo pathway genes listed in Table 10 contain mutations in comparison to each of the genes' respective wild type sequence; and determining whether to treat the patient with the Aurora A kinase inhibitor based on the mutation analysis in step b), wherein if at least one gene from Table 9 and/or 10 is found to be mutated, the patient may favorably respond to the drug.

In one aspect, the disclosure provides a method for identifying a patient having cancer as a candidate for treatment with an Aurora A kinase inhibitor, the method comprising the steps of: (a) obtaining a cancer cell sample from the patient; (b) determining whether any of the WNT pathway genes listed in Table 9 and/or any of the Hippo pathway genes listed in Table 10 contain mutations in comparison to each of the genes' respective wild type sequence; and identifying the patient as a candidate for treatment with the Aurora A kinase inhibitor if the mutation analysis in step b) indicates the presence of a mutation or the presence of several mutations in at least one gene from Table 9 and/or 10.

In one aspect, the disclosure provides a method for treating a patient having cancer, the method comprising the steps of: (a) obtaining a cancer cell sample from the patient; (b) determining whether any of the WNT pathway genes listed in Table 9 and/or any of the Hippo pathway genes listed in Table 10 contain mutations in comparison to each of the genes' respective wild type sequence; and treating the subject with an Aurora A Kinase inhibitor if the mutation analysis in b) indicates the presence of a mutation or the presence of several mutations in at least one gene from Table 9 and/or 10.

### BRIEF DESCRIPTION OF THE DRAWINGS

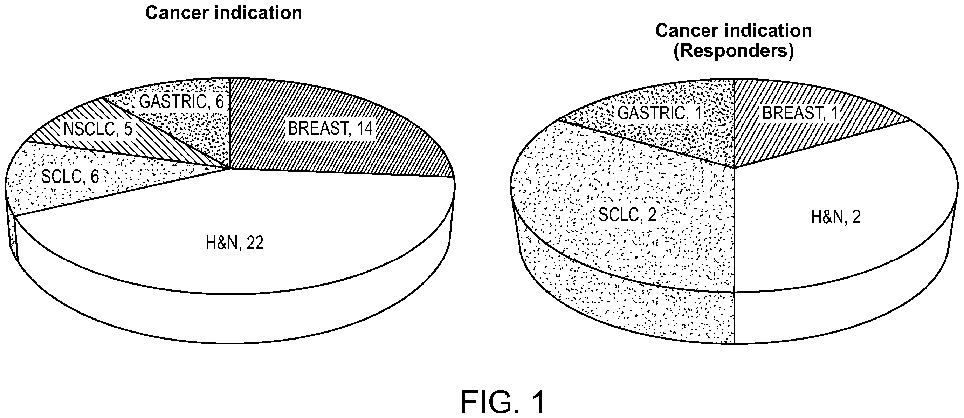

FIGURE 1. The left pie chart represents the tumor types of the 47 patients in the clinical trial. A total or five tumor types were treated among the patients, namely breast cancer, head and neck cancer (H&N), small cell lung cancer (SCLC), non-small cell lung cancer (NSCLC) and gastric cancer. The right pie chart indicates the tumour types of responders to treatment with alisertib.

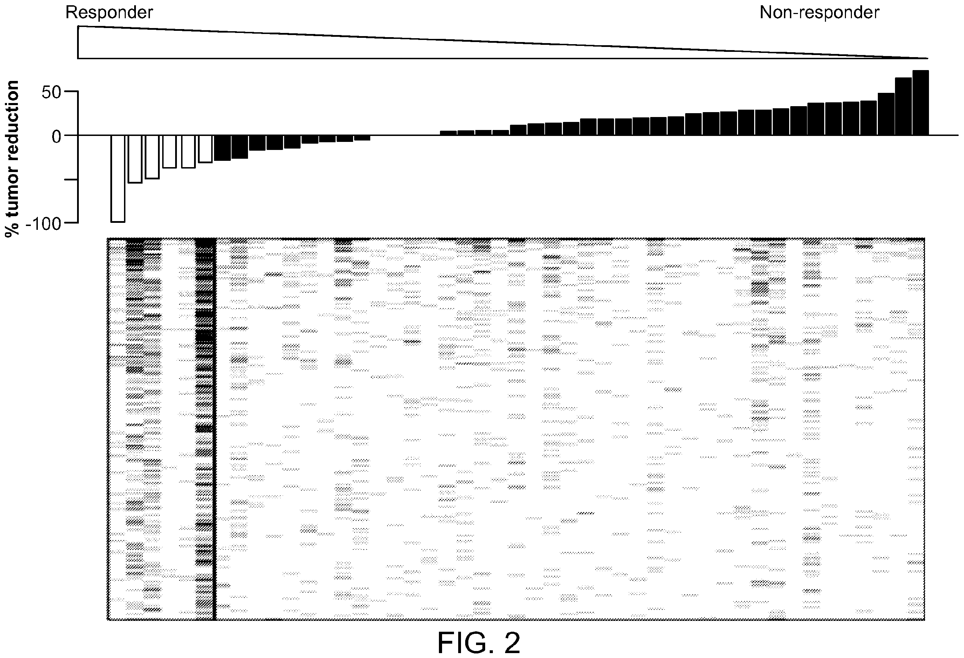

FIGURE 2. The mutational landscape of the 47 patients in the clinical trial. The patients (represented by the columns) are sorted by best tumor size change from responders to non-responders. Line segments in the heatmap indicate mutated genes.

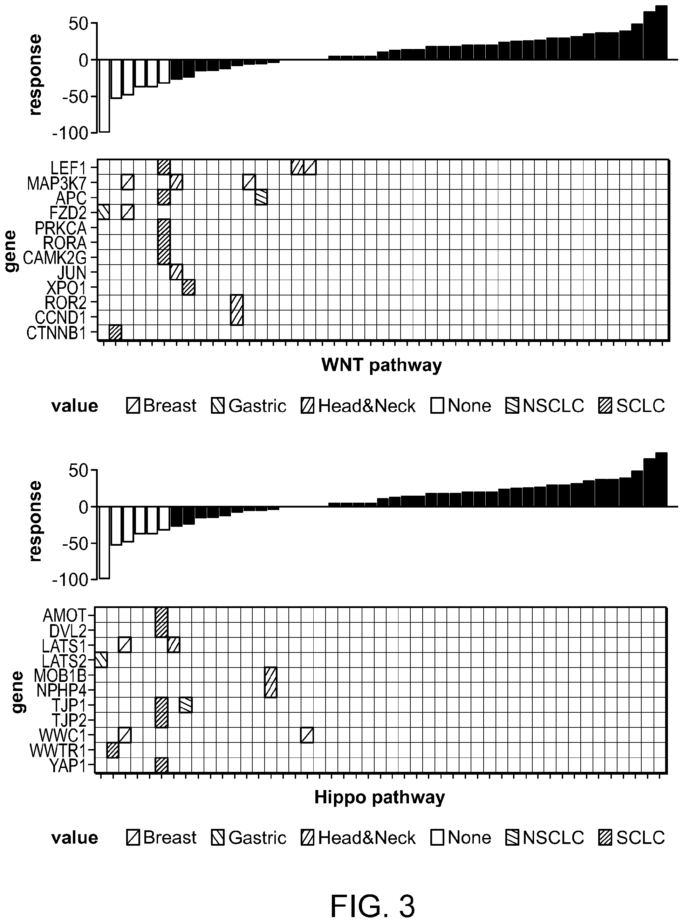

FIGURE 3. The heatmaps of mutated genes of the WNT and Hippo signaling pathways. In this figure, each column represents a patient, each row represents a mutated gene and each type of tumor is represented by the markings in the cells. The top waterfall plots indicate the distribution of the best tumor size change and each bar corresponds to a column (patient) of the heatmap. Surprisingly, it was determined that both of the pathways were associated with sensitivity to alisertib treatment. The WNT / β-catenin markers and Hippo markers identified herein are listed in Tables 9 and 10, respectively. In this figure we see that breast cancer patients harboring mutations in their LEF1, MAP3K7, FZD2, LATS1 and WWC1 genes, alone or in combination with other markers, are more likely to be responsive to an alisertib therapy regimen. We see that gastric cancer patients harboring mutations in their FZD2 and LATS2 genes, alone or in combination with other markers, are more likely to be responsive to an alisertib therapy regimen. We see that head and neck cancer patients harboring mutations in their LEF1, MAP3K7, JUN, ROR2, CCND1, LATS1, MOB1B and NPHP4 genes, alone or in combination with other markers, are more likely to be responsive to an alisertib therapy regimen. We see that non-small cell lung cancer patients harboring mutations in their XPO1 and TJP1 genes, alone or in combination with other markers, are more likely to be responsive to an alisertib therapy regimen. We see that small cell lung cancer patients harboring mutations in their LEF1, APC, PRKCA, RORA, CAMK2G, CTNNB1, AMOT, DVL2, TJP1, TJP2, WWTR1 and YAP1 genes, alone or in combination with other markers, are more likely to be responsive to an alisertib therapy regimen.

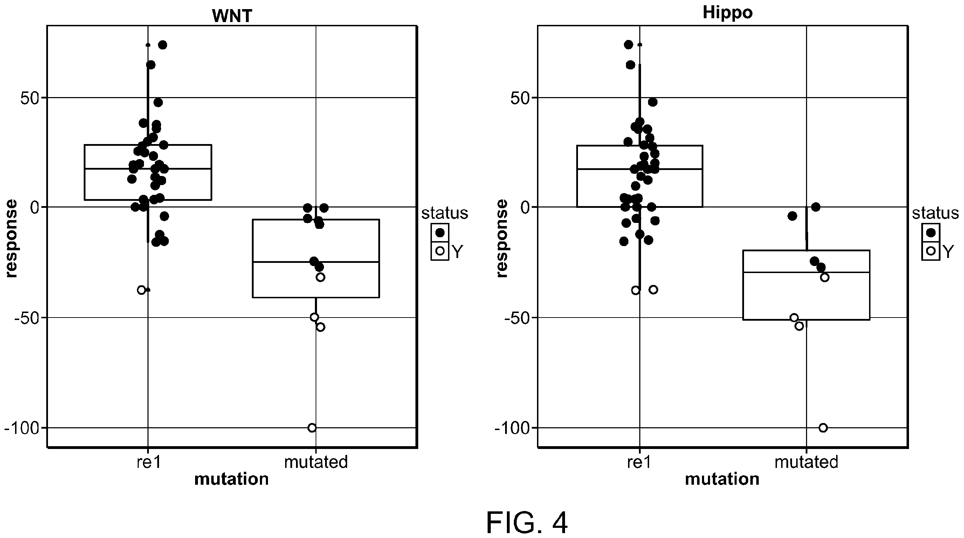

FIGURE 4. In each plot, the left box and right box represent wild type (WT) patients and mutant patients, respectively. The y-axis indicates the best tumor size change (%). Faint grey dots represent patients that responded to treatment with alisertib. As can be seen from these plots, patients with mutations in their WNT or Hippo signaling pathways respond more favorably to treatment with alisertib.

### DETAILED DESCRIPTION OF THE DISCLOSURE

One of the continued problems with therapy in cancer patients is individual differences in response to therapies. While advances in development of successful cancer therapies progress, only a subset of patients respond to any particular therapy. With the narrow therapeutic index and the toxic potential of many available cancer therapies, such differential responses potentially contribute to patients undergoing unnecessary, ineffective and even potentially harmful therapy regimens. If a designed therapy could be optimized to treat individual patients, such situations could be reduced or even eliminated. Furthermore, targeted designed therapy may provide more focused, successful patient therapy overall. Accordingly, there is a need to identify particular cancer patients who are expected to have a favorable outcome when administered particular cancer therapies as well as particular cancer patients who may have a favorable outcome using more aggressive and/or alternative cancer therapies, *e.g*., alternative to previous cancer therapies administered to the patient. It would therefore be beneficial to provide for the diagnosis, staging, prognosis, and monitoring of cancer patients, including, *e.g.,* non-hematological cancer patients, e.g., patients with solid tumors (*e.g.,* breast cancer, ovarian cancer, prostate cancer, head and neck cancer, small cell lung cancer, non-small cell lung cancer, gastric cancer, renal cancer, pancreatic cancer, bladder cancer or melanoma) who would benefit from particular cancer inhibition therapies as well as those who would benefit from a more aggressive and/or alternative cancer inhibition therapy, *e.g*., alternative to a cancer therapy or therapies the patient has received, thus resulting in appropriate preventative measures.

The present disclosure is based, in part, on the recognition that mutation of a marker gene can be associated with sensitivity of a cell comprising the mutated gene to an Aurora A Kinase inhibitor, e.g., alisertib. In some embodiments, the marker gene is involved in the WNT/ β -catenin signaling pathway, e.g., a gene whose mutation enables activation of the pathway. The WNT/ β -catenin signaling pathway is a well described oncogenic pathway. In another embodiment, the marker gene is involved in the Hippo signaling pathway. Hippo pathway components are largely known as tumor suppressors. Both the WNT/ β-catenin and Hippo signaling pathways have functional interactions with Aurora A. Aurora A inhibition with, for example, alisertib, induces cell mitotic defects and results in tetraploidy-induced cell cycle arrest. Interestingly, Hippo pathway genetically and functionally suppresses the WNT/ β -catenin signaling pathway. Silencing of the Aurora A gene also results in down-regulation of WNT/ β -catenin dependent signaling.

A marker gene can exhibit one or more mutations, e.g., somatic mutations, whose presence can affect expression or activity of the encoded gene product. In some embodiments, there can be more than one mutation in a marker gene in a tumor cell or tumor. In additional embodiments, there can be marker gene mutations in cells which have mutations in one or more additional genes, including mutations that can lead to tumorigenesis, but the additional mutated gene(s) may not be a marker gene as considered herein. In some embodiments, the mutation is an activating mutation. In other embodiments, the mutation affects the expression of the marker gene. In other embodiments, a mutation can result in an altered interaction of the encoded gene product with a cellular binding partner.

The identification and/or measurement of the mutation in the marker gene can be used to determine whether a favorable outcome can be expected by treatment of a tumor, *e.g*., with an Aurora Kinase inhibitor, e.g., alisertib therapy or whether an alternative therapy to and/or a more aggressive therapy with, *e.g*., an Aurora Kinase inhibitor, e.g., alisertib may enhance the response. For example, the compositions and methods provided herein can be used to determine whether a patient is expected to have a favorable outcome to an Aurora Kinase inhibitor, e.g., alisertib therapeutic agent dosing or administration regimen. Based on these identifications, the present disclosure provides, without limitation: 1) methods and compositions for determining whether an Aurora Kinase inhibitor, e.g., alisertib therapy regimen will or will not be effective to achieve a favorable outcome and/or manage the cancer; 2) methods and compositions for monitoring the effectiveness of an Aurora Kinase inhibitor, e.g., alisertib therapy (alone or in a combination of agents) and dosing and administrations used for the treatment of tumors; 3) methods and compositions for treatments of tumors comprising, *e.g.,* an Aurora Kinase inhibitor, e.g., alisertib therapy regimen; 4) methods and compositions for identifying specific therapeutic agents and combinations of therapeutic agents as well as dosing and administration regimens that are effective for the treatment of tumors in specific patients; and 5) methods and compositions for identifying disease management strategies.

There has been interest in public cataloging mutations associated with cancers. Examples of public databases which include information about mutations associated with cancers are the Database of Genotypes and Phenotypes (dbGaP) maintained by the National Center for Biotechnology Information (Bethesda, MD) and Catalogue of Somatic Mutations in Cancer (COSMIC) database maintained by the Wellcome Trust Sanger Institute (Cambridge, UK).

Compositions and methods are provided to identify mutations in marker genes in hematological *(e.g.,* multiple myeloma, leukemias, lymphoma, *etc.*) or solid (*e.g.,* breast cancer, ovarian cancer, prostate cancer, head and neck cancer, small cell lung cancer, non-small cell lung cancer, gastric cancer, renal cancer, pancreatic cancer, bladder cancer or melanoma) tumors to predict response to treatment, time-to-progression and survival upon treatment.

Markers were identified based on genetic profiles of tumor cells which exhibit sensitivity to treatment to alisertib. Observed sensitivity generally is consistent among tumor cells tested by more than one method.

Unless otherwise defined, all technical and scientific terms used herein have the meanings which are commonly understood by one of ordinary skill in the art to which this disclosure belongs. Generally, nomenclature utilized in connection with, and techniques of cell and tissue culture, molecular biology and protein and oligo- or polynucleotide chemistry and hybridization described herein are those known in the art. GenBank or GenPept accession numbers and useful nucleic acid and peptide sequences can be found at the website maintained by the National Center for Biotechnology Information, Bethesda, MD. Standard techniques are used for recombinant DNA, oligonucleotide synthesis, protein purification, tissue culture and transformation and transfection (e.g., electroporation, lipofection, etc). Enzymatic reactions are performed according to manufacturer's specifications or as commonly accomplished in the art or as described herein. The foregoing techniques and procedures generally are performed according to methods known in the art, e.g., as described in various general and more specific references that are cited and discussed throughout the present specification. See e.g., Sambrook et al. (2000) Molecular Cloning: A Laboratory Manual (3rd ed., Cold Spring Harbor Laboratory Press, Cold Spring Harbor, NY) or Harlow, E. and Lane, D. (1988) Antibodies: A Laboratory Manual (Cold Spring Harbor Laboratory Press, Cold Spring Harbor, NY). The nomenclatures utilized in connection with, and the laboratory procedures and techniques of, analytical chemistry, synthetic organic chemistry, and medicinal and pharmaceutical chemistry described herein are known in the art. Standard techniques are used for chemical syntheses, chemical analyses, pharmaceutical preparation, formulation and delivery, and treatment of patients. Furthermore, unless otherwise required by context, singular terms shall include pluralities and plural terms shall include the singular. In the case of conflict, the present specification, including definitions, will control.

### Definitions:

Terms used herein shall be accorded the following defined meanings, unless otherwise indicated.

As used herein, a "favorable" outcome or prognosis refers to long term survival, long time-to-progression (TTP), and/or good response. Conversely, an "unfavorable" outcome or prognosis refers to short term survival, short time-to-progression (TTP) and/or poor response.

A "marker" as used herein, includes a material associated with a marker gene which has been identified as having a mutation in tumor cells of a patient and furthermore that mutation is characteristic of a patient whose outcome is favorable or unfavorable with treatment e.g., by an Aurora A Kinase inhibitor, such as alisertib. Examples of a marker include a material, e.g., a chromosome locus, DNA for a gene, RNA for a gene or protein for a gene. For example, a marker includes a marker gene material, e.g., a chromosome locus, DNA, RNA or protein which demonstrates a characteristic, e.g., size, sequence, composition or amount indicative of a short term survival patient; alternatively a marker includes a marker gene material, e.g., a chromosome locus, DNA, RNA or protein which demonstrates a mutation or characteristic, e.g., size, sequence, composition or amount indicative of a long term survival patient. In another example, a marker includes a marker gene material, e.g., a chromosome locus, DNA, RNA or protein whose mutation or characteristic, e.g., size, sequence, composition or amount is indicative of a patient with a poor response to treatment; alternatively a marker includes a marker gene material, e.g., a chromosome locus, DNA, RNA or protein whose mutation or characteristic, e.g., size, sequence, composition or amount is indicative of a patient with a good response to treatment. In a further example, a marker includes a marker gene material, e.g., a chromosome locus, DNA, RNA or protein whose mutation or characteristic, e.g., size, sequence, composition or amount is indicative of a patient whose disease has a short time-to-progression (TTP) upon treatment; alternatively a marker includes a marker gene material, e.g., a chromosome locus, DNA, RNA or protein whose mutation or characteristic, e.g., size, sequence, composition or amount is indicative of a patient whose disease has a long TTP upon treatment. In a yet a further example, a marker includes a marker gene material, *e.g*., a chromosome locus, DNA, RNA or protein whose mutation or characteristic, e.g., size, sequence, composition or amount is indicative of a patient whose disease has a short term survival upon treatment; alternatively a marker includes a marker gene material, e.g., a chromosome locus, DNA, RNA or protein whose mutation or characteristic, e.g., size, sequence, composition or amount is indicative of a patient whose disease has a long term survival upon treatment. Thus, as used herein, marker is intended to include each and every one of these possibilities, and further can include each single marker individually as a marker; or alternatively can include one or more, or all of the characteristics collectively when reference is made to "markers" or "marker sets."

A "marker nucleic acid" is a nucleic acid (*e.g*., genomic DNA, mRNA, cDNA) encoded by or corresponding to a marker gene of the disclosure. Such marker nucleic acids include DNA, e.g., sense and anti-sense strands of genomic DNA (*e.g*., including any introns occurring therein), comprising the entire or a partial sequence, e.g., one or more of the exons of the genomic DNA, up to and including the open reading frame of any of the marker genes or the complement of such a sequence. The marker nucleic acids also include RNA comprising the entire or a partial sequence of any marker or the complement of such a sequence, wherein all thymidine residues are replaced with uridine residues, RNA generated by transcription of genomic DNA (*i.e.* prior to splicing), RNA generated by splicing of RNA transcribed from genomic DNA, and proteins generated by translation of spliced RNA (*i.e.* including proteins both before and after cleavage of normally cleaved regions such as transmembrane signal sequences). As used herein, a "marker nucleic acid" may also include a cDNA made by reverse transcription of an RNA generated by transcription of genomic DNA (including spliced RNA). A marker nucleic acid also includes sequences which differ, due to degeneracy of the genetic code, from the nucleotide sequence of nucleic acids encoding a protein which corresponds to a marker, e.g., a mutated marker, of the disclosure, and thus encode the same protein, e.g., mutated protein. As used herein, the phrase "allelic variant" refers to a nucleotide sequence which occurs at a given locus or to a polypeptide encoded by the nucleotide sequence. Such naturally occuring allelic variations can typically result in 1-5% variance in the nucleotide sequence of a given gene. Alternative alleles can be identified by sequencing the gene of interest in a number of different individuals, e.g., in cells, e.g., germline cells, of individuals without cancer. This can be readily carried out by using hybridization probes to identify the same genetic locus in a variety of individuals. Detection of any and all such nucleotide variations and resulting amino acid polymorphisms or variations that are the result of naturally occurring allelic variation and that do not alter the functional activity of a wild type marker gene is intended to be within the scope of the wild type version of a marker described herein. A "marker protein" is a protein encoded by or corresponding to a marker, e.g., a mutant nucleic acid, of the disclosure. The terms "protein" and "polypeptide' are used interchangeably. A protein of a marker specifically can be referred to by its name or amino acid sequence, but it is understood by those skilled in the art, that mutations, deletions and/or post-translational modifications can affect protein structure, appearance, cellular location and/or behavior. Unless indicated otherwise, such differences are not distinguished herein, and a marker described herein is intended to include any or all such varieties.

As used herein, a "marker gene" refers to a gene which can have a mutation such that its DNA, RNA and/or protein has a characteristic, e.g., size, sequence, composition or amount(s) which provide information about prognosis (*i.e.,* are "informative") upon treatment. Marker genes described herein as linked to outcome after treatment with an Aurora A Kinase inhibitor, such as alisertib (*e.g*., MLN8237) are examples of marker genes and are provided in Tables 8, 9 and 10. A marker gene listed in Tables 8, 9 and 10 can have isoforms which are either ubiquitous or have restricted expression. These sequences are not intended to limit the marker gene identity to that isoform or precursor. The additional isoforms and mature proteins are readily retrievable and understandable to one of skill in the art by reviewing the information provided under the Entrez Gene (database maintained by the National Center for Biotechnology Information, Bethesda, MD) identified by the ID number listed in Tables 9 and 10.

In the WNT pathway, "LEF1" refers to the gene associated with GenBank Accession No. NM_016269.4 (SEQ ID NO:1), encoding GenPept Accession No. NP_057353.1 (SEQ ID NO:2). Other names for LEF1 include TCF1-alpha and T cell-specific transcription factor 1-alpha. The protein encodes a transcription factor belonging to a family of proteins that share homology with the high mobility group protein-1. The protein encoded by this gene can bind to a functionally important site in the T-cell receptor-alpha enhancer, thereby conferring maximal enhancer activity. This transcription factor is involved in the WNT signaling pathway, and it may function in hair cell differentiation and follicle morphogenesis. Mutations in this gene have been found in somatic sebaceous tumors. This gene has also been linked to other cancers, including androgen-independent prostate cancer. Alternative splicing results in multiple transcript variants. Use of LEF1 as marker gene may be organ-specific, i.e., it can be a marker of breast neoplasms, colorectal neoplasms, insulin resistance, and other types of diseases particularly cancers.

In the WNT pathway, "MAP3K7" refers to the gene associated with GenBank Accession No. NM_145331.2 (SEQ ID NO:3), encoding GenPept Accession No. NP_663304.1 (SEQ ID NO:4). Other names for MAP3K7 include transforming growth factor-beta-activated kinase 1, TGF-beta activated kinase 1, and TGF-beta-activated kinase 1. The protein encoded by this gene is a member of the serine/threonine protein kinase family. This kinase mediates the signaling transduction induced by TGF beta and morphogenetic protein (BMP), and controls a variety of cell functions including transcription regulation and apoptosis. In response to IL-1, this protein forms a kinase complex including TRAF6, MAP3K7P1/TAB1 and MAP3K7P2/TAB2; this complex is required for the activation of nuclear factor kappa B. This kinase can also activate MAPK8/JNK, MAP2K4/MKK4, and thus plays a role in the cell response to environmental stresses. Four alternatively spliced transcript variants encoding distinct isoforms have been reported. Use of MAP3K7 as marker gene may be disease-specific, i.e., it can be a marker of arthritis, rheumatoid, endometrial neoplasms, and renal cancers.

In the WNT pathway, "APC" refers to the gene associated with GenBank Accession No. NM_000038.5 (SEQ ID NO:5), encoding GenPept Accession No. NP_000029.2 (SEQ ID NO:6). Other names for APC include protein phosphatase 1, regulatory subunit 46, adenomatosis polyposis coli tumor suppressor, adenomatous polyposis coli protein, deleted in polyposis 2.5, and adenomatosis polyposis coli. This gene encodes a tumor suppressor protein that acts as an antagonist of the Wnt signaling pathway. It is also involved in other processes including cell migration and adhesion, transcriptional activation, and apoptosis. Defects in this gene cause familial adenomatous polyposis (FAP), an autosomal dominant pre-malignant disease that usually progresses to malignancy. Disease-associated mutations tend to be clustered in a small region designated the mutation cluster region (MCR) and result in a truncated protein product. Use of APC as marker gene may cover many diseases including colorectal neoplasms and other cancer indications.

In the WNT pathway, "FZD2" refers to the gene associated with GenBank Accession No. NM_001466.3 (SEQ ID NO:7), encoding GenPept Accession No. NP_001457.1 (SEQ ID NO:8). Other names for FZD2 include frizzled homolog 2 (Drosophila), frizzled homolog 2, frizzled-2, frizzled (Drosophila) homolog 2, frizzled 2, seven transmembrane spanning receptor, and frizzled family receptor 2. This intronless gene is a member of the frizzled gene family. Members of this family encode seven-transmembrane domain proteins that are receptors for the wingless type MMTV integration site family of signaling proteins. This gene encodes a protein that is coupled to the beta-catenin canonical signaling pathway. Competition between the wingless-type MMTV integration site family, member 3A and wingless-type MMTV integration site family, member 5A gene products for binding of this protein is thought to regulate the beta-catenin-dependent and -independent pathways. Use of FZD2 as marker gene may be similar to MAP3K7, covering arthritis, rheumatoid, colorectal neoplasms, and endometrial neoplasms.

In the WNT pathway, "PRKCA" refers to the gene associated with GenBank Accession No. XM_011524990.1 (SEQ ID NO:9), encoding GenPept Accession No. XP_011523292.1 (SEQ ID NO:10). Other names for PRKCA include aging-associated gene 6, and protein kinase C alpha type, PKC-A. Protein kinase C (PKC) is a family of serine- and threonine-specific protein kinases that can be activated by calcium and the second messenger diacylglycerol. PKC family members phosphorylate a wide variety of protein targets and are known to be involved in diverse cellular signaling pathways. PKC family members also serve as major receptors for phorbol esters, a class of tumor promoters. Each member of the PKC family has a specific expression profile and is believed to play a distinct role in cells. The protein encoded by this gene is one of the PKC family members. This kinase has been reported to play roles in many different cellular processes, such as cell adhesion, cell transformation, cell cycle checkpoint, and cell volume control. Knockout studies in mice suggest that this kinase may be a fundamental regulator of cardiac contractility and Ca(2+) handling in myocytes.. Use of PRKCA as marker gene may be organ-specific; i.e. it can be a marker of Alzheimer disease and amyotrophic lateral.

In the WNT pathway, "RORA" refers to the gene associated with GenBank Accession No. NM_002943.3 (SEQ ID NO: 11), encoding GenPept Accession No. NP_002934.1 (SEQ ID NO: 12). Other names for RORA include retinoic acid receptor-related orphan receptor alpha, ROR-alpha, transcription factor RZR-alpha, thyroid hormone nuclear receptor alpha variant 4, nuclear receptor RZR-alpha, retinoid-related orphan receptor alpha, nuclear receptor ROR-alpha, and nuclear receptor subfamily 1 group F member 1. The protein encoded by this gene is a member of the NR1 subfamily of nuclear hormone receptors. It can bind as a monomer or as a homodimer to hormone response elements upstream of several genes to enhance the expression of those genes. The encoded protein has been shown to interact with NM23-2, a nucleoside diphosphate kinase involved in organogenesis and differentiation, as well as with NM23-1, the product of a tumor metastasis suppressor candidate gene. Also, it has been shown to aid in the transcriptional regulation of some genes involved in circadian rhythm. Four transcript variants encoding different isoforms have been described for this gene. Use of RORA as marker gene may various diseases; i.e. it can be a marker of anoxia, bipolar disorder, cancers, particularly non-small cell lung cancer.

In the WNT pathway, "CAMK2G" refers to the gene associated with GenBank Accession No. NM_172171.2 (SEQ ID NO:13), encoding GenPept Accession No. NP_751911.1 (SEQ ID NO:14). Other names for CAMK2G include calcium/calmodulin-dependent protein kinase (CaM kinase) II gamma, calcium/calmodulin-dependent protein kinase type II subunit gamma, and caMK-II subunit gamma. The product of this gene is one of the four subunits of an enzyme which belongs to the serine/threonine protein kinase family, and to the Ca(2+)/calmodulin-dependent protein kinase subfamily. Calcium signaling is crucial for several aspects of plasticity at glutamatergic synapses. In mammalian cells the enzyme is composed of four different chains: alpha, beta, gamma, and delta. The product of this gene is a gamma chain. Many alternatively spliced transcripts encoding different isoforms have been described but the full-length nature of all the variants has not been determined. Use of CAMK2G as marker gene may be neuro-degenerative diseases, cardiovascular diseases, and cancer; i.e. it can be a marker of Alzheimer disease, arrhythmias, colorectal neoplasms, and glioblastoma.

In the WNT pathway, "JUN" refers to the gene associated with GenBank Accession No. NM_002228.3 (SEQ ID NO:15), encoding GenPept Accession No. NP_002219.1 (SEQ ID NO: 16). Other names for JUN include v-jun avian sarcoma virus 17 oncogene homolog, activator protein 1, Jun activation domain binding protein, v-jun sarcoma virus 17 oncogene homolog, enhancer-binding protein AP1, jun oncogene, p39, proto-oncogene c-Jun, transcription factor AP-1, and v-jun sarcoma virus 17 oncogene homolog (avian). This gene is the putative transforming gene of avian sarcoma virus 17. It encodes a protein which is highly similar to the viral protein, and which interacts directly with specific target DNA sequences to regulate gene expression. This gene is intronless and is mapped to 1p32-p31, a chromosomal region involved in both translocations and deletions in human malignancies. Use of JUN as marker gene may be cancer and auto-immune diseases; i.e. it can be a marker of breast neoplasms, colorectal neoplasms, Crohn disease, and asthma.

In the WNT pathway, "XPO1" refers to the gene associated with GenBank Accession No. NM_003400.3 (SEQ ID NO:17), encoding GenPept Accession No. NP_003391.1 (SEQ ID NO:18). Other names for XPO1 include exportin 1 (CRM1, yeast, homolog), exportin-1 (required for chromosome region maintenance), exportin 1 (CRM1 homolog, yeast), exportin-1, chromosome region maintenance 1 protein homolog, and chromosome region maintenance 1 homolog. This cell-cycle-regulated gene encodes a protein that mediates leucine-rich nuclear export signal (NES)-dependent protein transport. The protein specifically inhibits the nuclear export of Rev and U snRNAs. It is involved in the control of several cellular processes by controlling the localization of cyclin B, MPAK, and MAPKAP kinase 2. This protein also regulates NFAT and AP-1. Use of XPO1 as marker gene may be related to cancer; i.e. it can be a marker of breast neoplasms, endometrial neoplasms, and ovarian neoplasms.

In the WNT pathway, "ROR2" refers to the gene associated with GenBank Accession No. NM_004560.3 (SEQ ID NO: 19), encoding GenPept Accession No. NP_004551.2 (SEQ ID NO:20). Other names for ROR2 include neurotrophic tyrosine kinase receptor-related 2, and tyrosine-protein kinase transmembrane receptor ROR2. The protein encoded by this gene is a receptor protein tyrosine kinase and type I transmembrane protein that belongs to the ROR subfamily of cell surface receptors. The protein may be involved in the early formation of the chondrocytes and may be required for cartilage and growth plate development. Mutations in this gene can cause brachydactyly type B, a skeletal disorder characterized by hypoplasia/aplasia of distal phalanges and nails. In addition, mutations in this gene can cause the autosomal recessive form of Robinow syndrome, which is characterized by skeletal dysplasia with generalized limb bone shortening, segmental defects of the spine, brachydactyly, and a dysmorphic facial appearance. Use of ROR2 as marker gene may be related to cancer; i.e. it can be a marker of breast neoplasms, non-small cell lung cancer, and colorectal neoplasms.

In the WNT pathway, "CCND1" refers to the gene associated with GenBank Accession No. NM_053056.2 (SEQ ID NO:21), encoding GenPept Accession No. NP_444284.1 (SEQ ID NO:22). Other names for CCND1 include B-cell CLL/lymphoma 1, BCL-1 oncogene, PRAD1 oncogene, B-cell lymphoma 1 protein, and G1/S-specific cyclin-Dl, and cyclin D1 (PRAD1: parathyroid adenomatosis 1). The protein encoded by this gene belongs to the highly conserved cyclin family, whose members are characterized by a dramatic periodicity in protein abundance throughout the cell cycle. Cyclins function as regulators of CDK kinases. Different cyclins exhibit distinct expression and degradation patterns which contribute to the temporal coordination of each mitotic event. This cyclin forms a complex with and functions as a regulatory subunit of CDK4 or CDK6, whose activity is required for cell cycle G1/S transition. This protein has been shown to interact with tumor suppressor protein Rb and the expression of this gene is regulated positively by Rb. Mutations, amplification and overexpression of this gene, which alters cell cycle progression, are observed frequently in a variety of tumors and may contribute to tumorigenesis. Use of CCND1 as marker gene may be related to many diseases; i.e. it can be a marker of various types of cancer and neuro-degenerative diseases.

In the WNT pathway, "CTNNB1" refers to the gene associated with GenBank Accession No. NM_001098209.1 (SEQ ID NO:23), encoding GenPept Accession No. NP_001091679.1 (SEQ ID NO:24). Other names for CTNNB1 include catenin beta-1, catenin (cadherin-associated protein), and beta 1 (88kD). The protein encoded by this gene is part of a complex of proteins that constitute adherens junctions (AJs). AJs are necessary for the creation and maintenance of epithelial cell layers by regulating cell growth and adhesion between cells. The encoded protein also anchors the actin cytoskeleton and may be responsible for transmitting the contact inhibition signal that causes cells to stop dividing once the epithelial sheet is complete. Finally, this protein binds to the product of the APC gene, which is mutated in adenomatous polyposis of the colon. Mutations in this gene are a cause of colorectal cancer (CRC), pilomatrixoma (PTR), medulloblastoma (MDB), and ovarian cancer. Three transcript variants encoding the same protein have been found for this gene. Use of CTNNB1 as marker gene may be related to many diseases; i.e. it can be a marker of various types of cancer particularly colorectal neoplasms and immune diseases.

In the hippo pathway, "AMOT" refers to the gene associated with GenBank Accession No. NM_001113490.1 (SEQ ID NO:25), encoding GenPept Accession No. NP_001106962.1 (SEQ ID NO:26). Other names for AMOT include angiomotin p130 isoform, and angiomotin p80 isoform. This gene belongs to the motin family of angiostatin binding proteins characterized by conserved coiled-coil domains and C-terminal PDZ binding motifs. The encoded protein is expressed predominantly in endothelial cells of capillaries as well as larger vessels of the placenta where it may mediate the inhibitory effect of angiostatin on tube formation and the migration of endothelial cells toward growth factors during the formation of new blood vessels. Alternative splicing results in multiple transcript variants encoding different isoforms. Use of AMOT as marker gene may be related to cancer; i.e. it can be a marker of breast neoplasms, endometrial neoplasms, and leukemia.

In the hippo pathway, "DVL2" refers to the gene associated with GenBank Accession No. NM_004422.2 (SEQ ID NO:27), encoding GenPept Accession No. NP_004413.1 (SEQ ID NO:28). Other names for DVL2 include dishevelled 2 (homologous to Drosophila dsh), segment polarity protein dishevelled homolog DVL-2, dishevelled, dsh homolog 2, and dishevelled, dsh homolog 2 (Drosophila). This gene belongs to the motin family of angiostatin binding proteins characterized by conserved coiled-coil domains and C-terminal PDZ binding motifs. This gene encodes a member of the dishevelled (dsh) protein family. The vertebrate dsh proteins have approximately 40% amino acid sequence similarity with Drosophila dsh. This gene encodes a 90-kD protein that undergoes posttranslational phosphorylation to form a 95-kD cytoplasmic protein, which may play a role in the signal transduction pathway mediated by multiple Wnt proteins. The mechanisms of dishevelled function in Wnt signaling are likely to be conserved among metazoans. Use of DVL2 as marker gene may be related to cancer and psychiatry diseases; i.e. it can be a marker of breast neoplasms, non-small cell cancer, bipolar disorder, and tobacco use disorder.

In the hippo pathway, "LATS1" refers to the gene associated with GenBank Accession No. NM_004690.3 (SEQ ID NO:29), encoding GenPept Accession No. NP_004681.1 (SEQ ID NO:30). Other names for LATS1 include WARTS protein kinase, LATS (large tumor suppressor, Drosophila) homolog 1, large tumor suppressor homolog 1, serine/threonine-protein kinase LATS1, h-warts, LATS, large tumor suppressor, homolog 1, LATS, and large tumor suppressor, homolog 1 (Drosophila). This gene belongs to the motin family of angiostatin binding proteins characterized by conserved coiled-coil domains and C-terminal PDZ binding motifs. This gene encodes a member of the dishevelled (dsh) protein family. The vertebrate dsh proteins have approximately 40% amino acid sequence similarity with Drosophila dsh. This gene encodes a 90-kD protein that undergoes posttranslational phosphorylation to form a 95-kD cytoplasmic protein, which may play a role in the signal transduction pathway mediated by multiple Wnt proteins. The mechanisms of dishevelled function in Wnt signaling are likely to be conserved among metazoans. Use of LATS1 as marker gene may be related to cancer and psychiatry diseases; i.e. it can be a marker of breast neoplasms, non-small cell cancer, bipolar disorder, and tobacco use disorder.

In the hippo pathway, "LATS2" refers to the gene associated with GenBank Accession No. XM_005266342.1 (SEQ ID NO:31), encoding GenPept Accession No. XP_005266399.1 (SEQ ID NO:32). Other names for LATS2 include serine/threonine kinase KPM, warts-like kinase, LATS (large tumor suppressor, Drosophila) homolog 2, kinase phosphorylated during mitosis protein, large tumor suppressor homolog 2, serine/threonine-protein kinase kpm, serine/threonine-protein kinase LATS2, LATS, large tumor suppressor, homolog 2, LATS, and large tumor suppressor, homolog 2 (Drosophila). This gene encodes a serine/threonine protein kinase belonging to the LATS tumor suppressor family. The protein localizes to centrosomes during interphase, and early and late metaphase. It interacts with the centrosomal proteins aurora-A and ajuba and is required for accumulation of gamma-tubulin and spindle formation at the onset of mitosis. It also interacts with a negative regulator of p53 and may function in a positive feedback loop with p53 that responds to cytoskeleton damage. Additionally, it can function as a co-repressor of androgen-responsive gene expression. Use of LATS2 as marker gene may be related to cancer and immune diseases; i.e. it can be a marker of breast neoplasms and asthma.

In the hippo pathway, "MOB1B" refers to the gene associated with GenBank Accession No. NM_001244766.1 (SEQ ID NO:33), encoding GenPept Accession No. NP_001231695.1 (SEQ ID NO:34). Other names for MOB1B include MOB1 Mps One Binder homolog B, MOB1 Mps One Binder homolog B (yeast), MOB1, Mps One Binder kinase activator-like 1A (yeast), mob1A, mps one binder kinase activator-like 1A, mob1 homolog 1A, MOB1, and Mps One Binder kinase activator-like 1A. The protein encoded by this gene is similar to the yeast Mob1 protein. Yeast Mob1 binds Mps1p, a protein kinase essential for spindle pole body duplication and mitotic checkpoint regulation. Three transcript variants encoding different isoforms have been found for this gene. Use of MOB1B as marker gene may be related to cancer; i.e. it can be a marker of breast neoplasms, endometrial neoplasms, and lung neoplasms.

In the hippo pathway, "NPHP4" refers to the gene associated with GenBank Accession No. NM_015102.4 (SEQ ID NO:35), encoding GenPept Accession No. NP_055917.1 (SEQ ID NO:36). Other names for NPHP4 include nephroretinin, nephrocystin-4, and POC10 centriolar protein homolog. This gene encodes a protein involved in renal tubular development and function. This protein interacts with nephrocystin, and belongs to a multifunctional complex that is localized to actin- and microtubule-based structures. Mutations in this gene are associated with nephronophthisis type 4, a renal disease, and with Senior-Loken syndrome type 4, a combination of nephronophthisis and retinitis pigmentosa. Alternative splicing results in multiple transcript variants. Use of NPHP4 as marker gene may be related to cancer and eye diseases; i.e. it can be a marker of breast neoplasms, endometrial neoplasms, and retinitis.

In the hippo pathway, "TJP1" refers to the gene associated with GenBank Accession No. NM_003257.4 (SEQ ID NO:37), encoding GenPept Accession No. NP_003248.3 (SEQ ID NO:38). Other names for TJP1 include tight junction protein ZO-1, zona occludens 1, and zonula occludens 1 protein. This gene encodes a protein located on a cytoplasmic membrane surface of intercellular tight junctions. The encoded protein may be involved in signal transduction at cell-cell junctions. Alternative splicing of this gene results in multiple transcript variants. Use of TJP1 as marker gene may be related to cancer and immune diseases; i.e. it can be a marker of breast neoplasms, hepatocellular neoplasms, and asthma.

In the hippo pathway, "TJP2" refers to the gene associated with GenBank Accession No. NM_004817.3 (SEQ ID NO:39), encoding GenPept Accession No. NP_004808.2 (SEQ ID N0:40). Other names for TJP2 include Friedreich ataxia region gene X104 (tight junction protein ZO-2), deafness, autosomal dominant 51, zonula occludens protein 2, tight junction protein ZO-2, and zona occludens 2. This gene encodes a zonula occluden that is a member of the membrane-associated guanylate kinase homolog family. The encoded protein functions as a component of the tight junction barrier in epithelial and endothelial cells and is necessary for proper assembly of tight junctions. Mutations in this gene have been identified in patients with hypercholanemia, and genomic duplication of a 270 kb region including this gene causes autosomal dominant deafness-51. Alternatively spliced transcripts encoding multiple isoforms have been observed for this gene. Use of TJP2 as marker gene may be related to cancer; i.e. it can be a marker of breast neoplasms.

In the hippo pathway, "WWC1" refers to the gene associated with GenBank Accession No. NM_001161661.1 (SEQ ID NO:41), encoding GenPept Accession No. NP_001155133.1 (SEQ ID NO:42). Other names for WWC1 include kidney and brain protein, WW, C2 and coiled-coil domain containing 1, HBeAg-binding protein 3, protein WWC1, protein KTBRA, protein phosphatase 1, and regulatory subunit 168. The protein encoded by this gene is a cytoplasmic phosphoprotein that interacts with PRKC-zeta and dynein light chain-1. Alleles of this gene have been found that enhance memory in some individuals. Three transcript variants encoding different isoforms have been found for this gene. Use of WWC1 as marker gene may be related to cancer and neurodegenerative diseases; i.e. it can be a marker of breast neoplasms and Alzheimer disease.

In the hippo pathway, "WWTR1" refers to the gene associated with GenBank Accession No. NM_001168278.1 (SEQ ID NO:43), encoding GenPept Accession No. NP_001161750.1 (SEQ ID NO:44). Other names for WWTR1 include transcriptional coactivator with PDZ-binding motif, transcriptional co-activator with PDZ-binding motif, and WW domain-containing transcription regulator protein 1. The molecular function of this gene is relatively unknown. Use of WWTR1 as marker gene may be related to cancer; i.e. it can be a marker of breast neoplasms, endometrial neoplasms, lung neoplasms, and ovarian neoplasms.

In the hippo pathway, "YAP1" refers to the gene associated with GenBank Accession No. NM_001282101.1 (SEQ ID NO:45), encoding GenPept Accession No. NP_001269030.1 (SEQ ID NO:46). Other names for YAP1 include Yes-associated protein 1, 65kDa, yes-associated protein 2, yorkie homolog, 65 kDa Yes-associated protein, protein yorkie homolog, transcriptional coactivator YAP1, and yes-associated protein YAP65 homolog. This gene encodes a downstream nuclear effector of the Hippo signaling pathway which is involved in development, growth, repair, and homeostasis. This gene is known to play a role in the development and progression of multiple cancers as a transcriptional regulator of this signaling pathway and may function as a potential target for cancer treatment. Alternative splicing results in multiple transcript variants encoding different isoforms. Use of YAP1 as marker gene may be related to cancer; i.e. it can be a marker of breast neoplasms.

As used herein, an "informative" characteristic, e.g., size, sequence, composition or amount of a marker refers to a characteristic, e.g., size, sequence, composition or amount whose difference is correlated to prognosis or outcome. The informative characteristic, e.g., size, sequence, composition or amount of a marker can be obtained by analyzing either nucleic acid, *e.g*., DNA or RNA, or protein corresponding to the marker gene. The characteristic, e.g., size (*e.g.,* length or molecular weight), sequence (e.g., nucleic acid sequence or protein sequence), composition (*e.g*., base or amino acid composition or peptide digest or gene fragment pattern) or amount (*e.g*., copy number and/or expression level) of a marker, *e.g.,* a marker in a sample from a patient is "informative" if it is different than the wild type or allelic variant of the substance being analyzed. In an embodiment where the amount of a marker is being measured, an amount is "informative" if it is greater than or less than a reference amount by a degree greater than the standard error of the assay employed to assess expression. The informative expression level of a marker can be determined upon statistical correlation of the measured expression level and the outcome, *e.g*., good response, poor response, long time-to-progression, short time-to-progression, short term survival or long term survival. The result of the statistical analysis can establish a threshold for selecting markers to use in the methods described herein. Alternatively, a marker, *e.g.,* a chromosome locus marker, or a marker gene that has differential characteristic, e.g., size, sequence, composition or amounts will have typical ranges of amounts that are predictive of outcome. An informative characteristic, e.g., size, sequence, composition or amount is a characteristic, e.g., size, sequence, composition or amount that falls within the range of characteristic, e.g., size, sequence, composition or amounts determined for the outcome. Still further, a set of markers may together be "informative" if the combination of their characteristics, e.g., sizes, sequences, compositions or amounts either meets or is above or below a pre-determined score for the combination of markers, *e.g*., chromosome locus markers, or marker genes, in a set as determined by methods provided herein. Measurement of only one characteristic, e.g., marker, of a marker gene (i.e., DNA, RNA or protein) can provide a prognosis, i.e., indicate outcome. Measurement of more than one characteristic, e.g., marker, of a marker gene can provide a prognosis when the informative amounts of the two characteristics are consistent with each other, *i.e.,* the biologies of the results are not contradictory. Examples of consistent results from measurement of multiple characteristics of a marker gene can be identification of a nonsense mutation or deletion in a DNA or RNA and a low amount or low molecular weight of encoded protein, or a mutation in a region which encodes a binding pocket or active site of a protein and low activity of the encoded protein. A different example can occur when a protein is in a pathway with a feedback loop controlling its synthesis based on its activity level. In this example, a low amount or activity of protein can be associated with a high amount of its mutated mRNA as a tissue, due to the marker gene mutation, thus is starved for the protein activity and repeatedly signals the production of the protein.

As used herein, "gene deletion" refers to an amount of DNA copy number less than 2 and "amplification" refers to an amount of DNA copy number greater than 2. A "diploid" amount refers to a copy number equal to 2. The term "diploid or amplification" can be interpreted as "not deletion" of a gene copy. In a marker whose alternative informative amount is gene deletion, amplification generally would not be seen. Conversely, the term "diploid or deletion" can be interpreted as "not amplification" of copy number. In a marker whose alternative informative amount is amplification, gene deletion generally would not be seen. For the sake of clarity, sequence deletion can occur within a gene as a result of marker gene mutation and can result in absence of transcribed protein or a shortened mRNA or protein. Such a deletion may not affect copy number.

The terms "long term survival" and "short term survival" refer to the length of time after receiving a first dose of treatment that a cancer patient is predicted to live. A "long term survivor" refers to a patient expected have a slower rate of progression or later death from the tumor than those patients identified as short term survivors. "Enhanced survival" or "a slower rate of death" are estimated life span determinations based upon characteristic, e.g., size, sequence, composition or amount of one or more of markers described herein, e.g., as compared to a reference standard such that 70%, 80%, 90% or more of the population will be alive a sufficient time period after receiving a first dose of treatment. A "faster rate of death" or "shorter survival time" refer to estimated life span determinations based upon characteristic, e.g., size, sequence, composition or amount of one or more of markers described herein, e.g., as compared to a reference standard such that 50%, 40%, 30%, 20%, 10% or less of the population will not live a sufficient time period after receiving a first dose of treatment. In some embodiments, the sufficient time period is at least 6, 12, 18, 24 or 30 months measured from the first day of receiving a cancer therapy.

A cancer is "responsive" to a therapeutic agent or there is a "good response" to a treatment if its rate of growth is inhibited as a result of contact with the therapeutic agent, compared to its growth in the absence of contact with the therapeutic agent. Growth of a cancer can be measured in a variety of ways, for instance, the characteristic, e.g., size of a tumor or the expression of tumor markers appropriate for that tumor type may be measured. For solid tumors, the Response Evaluation Criteria in Solid Tumors (RECIST) guidelines (Eisenhauer et al. (2009) E. J. Canc. 45:228-247) can be used to support the identification of markers associated with solid tumors and response of solid tumors to an Aurora A Kinase inhibitor. International Working Groups convene periodically to set, update and publish response criteria for various types of cancers. Such published reports can be followed to support the identification of markers of the subject tumors and their response to Aurora A Kinase inhibitors. Examples are criteria for Acute Myelogenous Leukemia (AML, Cheson et al. (2003) J.Clin. Oncol. 21:4642-4649), lymphomas, e.g., non-Hodgkin's and Hodgkin's lymphoma (Cheson et al. (2007) J.Clin. Oncol. 25:579-596). Criteria take into account analysis methods such as Positron Emission Tomography (PET), e.g., for identifying sites with measurable altered metabolic activity (e.g., at tumor sites) or to trace specific markers into tumors *in vivo,* immunohistochemistry, e.g., to identify tumor cells by detecting binding of antibodies to specific tumor markers, and flow cytometry, e.g., to characterize cell types by differential markers and fluorescent stains, in addition to traditional methods such as histology to identify cell composition (e.g., blast counts in a blood smear or a bone marrow biopsy, presence and number of mitotic figures) or tissue structure (e.g., disordered tissue architecture or cell infiltration of basement membrane). The quality of being responsive to an Aurora A Kinase inhibitor, such as alisertib therapy can be a variable one, with different cancers exhibiting different levels of "responsiveness" to a given therapeutic agent, under different conditions. In some embodiments, a breast cancer is responsive to Aurora A Kinase inhibition, e.g., alisertib therapy, if the breast cancer patient has mutations in marker genes LEF1, MAP3K7, FZD2, LATS1 and/or WWC1. In some embodiments, a gastric cancer is responsive to Aurora A Kinase inhibition, e.g., alisertib therapy, if the gastric cancer patient has mutations in marker genes FZD2 and/or LATS2. In some embodiments, a head and neck cancer is responsive to Aurora A Kinase inhibition, e.g., alisertib therapy, if the head and neck cancer patient has mutations in marker genes MAP3K7, JUN, ROR2, CCND1, LATS1, MOB1B and/or NPHP4. In some embodiments, a non-small cell lung cancer is responsive to Aurora A Kinase inhibition, e.g., alisertib therapy, if the non-small cell lung cancer patient has mutations in marker genes XPO1 and/or TJP1. In some embodiments, a small cell lung cancer is responsive to Aurora A Kinase inhibition, e.g., alisertib therapy, if the small cell lung cancer patient has mutations in marker genes LEF1, APC, PRKCA, RORA, CAMK2G, CTNNB1, AMOT, DVL2, TJP1, TJP2, WWTR1 and/or YAP1. Still further, measures of responsiveness can be assessed using additional criteria beyond growth size of a tumor, including patient quality of life, degree of metastases, etc. In addition, clinical prognostic markers and variables can be assessed (e.g., M protein in myeloma, PSA levels in prostate cancer) in applicable situations.

A cancer is "non-responsive" or has a "poor response" to a therapeutic agent or there is a poor response to a treatment if its rate of growth is not inhibited, or inhibited to a very low degree, as a result of contact with the therapeutic agent when compared to its growth in the absence of contact with the therapeutic agent. As stated above, growth of a cancer can be measured in a variety of ways, for instance, the size of a tumor or the expression of tumor markers appropriate for that tumor type may be measured. For example, the response definitions used to support the identification of markers associated with non- response of tumors to therapeutic agents, guidelines such as those described above can be used. The quality of being non-responsive to a therapeutic agent can be a highly variable one, with different cancers exhibiting different levels of "non-responsiveness" to a given therapeutic agent, under different conditions. Still further, measures of non-responsiveness can be assessed using additional criteria beyond growth size of a tumor, including patient quality of life, degree of metastases, etc. In addition, clinical prognostic markers and variables can be assessed (e.g., M protein in myeloma, PSA levels in prostate cancer) in applicable situations.

As used herein, "long time-to-progression, "long TTP" and "short time-to-progression," "short TTP" refer to the amount of time until when the stable disease brought by treatment converts into an active disease. On occasion, a treatment results in stable disease which is neither a good nor a poor response, *e.g.,* MR, the disease merely does not get worse, *e.g.,* become a progressive disease, for a period of time. This period of time can be at least 4-8 weeks, at least 3-6 months or more than 6 months.

"Treatment" shall mean the use of a therapy to prevent or inhibit further tumor growth, as well as to cause shrinkage of a tumor, and to provide longer survival times. Treatment is also intended to include prevention of metastasis of tumor. A tumor is "inhibited" or "treated" if at least one symptom (as determined by responsiveness/non-responsiveness, time to progression, or indicators known in the art and described herein) of the cancer or tumor is alleviated, terminated, slowed, minimized, or prevented. Any amelioration of any symptom, physical or otherwise, of a tumor pursuant to treatment using a therapeutic regimen (*e.g*., Aurora A Kinase inhibitor, such as alisertib regimen) as further described herein, is within the scope of the disclosure.

As used herein, the term "agent" is defined broadly as anything that cancer cells, including tumor cells, may be exposed to in a therapeutic protocol. In the context of the present disclosure, such agents include, but are not limited to, an Aurora A Kinase inhibitor, such as alisertib agents, as well as chemotherapeutic agents as known in the art and described in further detail herein.

The term "probe" refers to any molecule which is capable of selectively binding to a specifically intended target molecule, for example a marker of the disclosure. Probes can be either synthesized by one skilled in the art, or derived from appropriate biological preparations. For purposes of detection of the target molecule, probes may be specifically designed to be labeled, as described herein. Examples of molecules that can be utilized as probes include, but are not limited to, RNA, DNA, proteins, antibodies, enzymatic reporter reagents and organic monomers.

A "normal" characteristic, e.g., size, sequence, composition or amount of a marker may refer to the characteristic, e.g., size, sequence, composition or amount in a "reference sample." A reference sample can be a matched normal, e.g., germline, sample from the same patient from whom the tumor, e.g., with a somatic mutation, is derived. A reference sample can be a sample from a healthy subject not having the marker-associated disease or a reference characteristic e.g., the average characteristic, e.g., size, sequence, composition or amount of the wild type marker in several healthy subjects. A reference sample characteristic, e.g., size, sequence, composition or amount may be comprised of a characteristic, e.g., size, sequence, composition or amount of one or more markers from a reference database. Alternatively, a "normal" characteristic, e.g., size, sequence, composition or level of expression of a marker is the characteristic, e.g., size, sequence, composition or amount of the marker, e.g., marker gene in non-tumor cells in a similar environment or response situation from the same patient from whom the tumor is derived. The normal amount of DNA copy number is 2 or diploid, with the exception of X-linked genes in males, where the normal DNA copy number is 1.

"Over-expression" and "under-expression" of a marker gene, refer to expression of the marker gene of a patient at a greater or lesser level *(e.g.* more than three-halves-fold, at least two-fold, at least three-fold, greater or lesser level etc.), respectively, than normal level of expression of the marker gene, e.g., as measured by mRNA or protein, in a test sample that is greater than the standard error of the assay employed to assess expression. A "significant" expression level may refer to a level which either meets or is above or below a pre-determined score for a marker gene set as determined by methods provided herein.

"Complementary" refers to the broad concept of sequence complementarity between regions of two nucleic acid strands or between two regions of the same nucleic acid strand. It is known that an adenine residue of a first nucleic acid region is capable of forming specific hydrogen bonds ("base pairing") with a residue of a second nucleic acid region which is antiparallel to the first region if the residue is thymine or uracil. Similarly, it is known that a cytosine residue of a first nucleic acid strand is capable of base pairing with a residue of a second nucleic acid strand which is antiparallel to the first strand if the residue is guanine. A first region of a nucleic acid is complementary to a second region of the same or a different nucleic acid if, when the two regions are arranged in an antiparallel fashion, at least one nucleotide residue of the first region is capable of base pairing with a residue of the second region. In an embodiment, the first region comprises a first portion and the second region comprises a second portion, whereby, when the first and second portions are arranged in an antiparallel fashion, at least about 50%, at least about 75%, at least about 90%, or at least about 95% or all of the nucleotide residues of the first portion are capable of base pairing with nucleotide residues in the second portion.

"Homologous" as used herein, refers to nucleotide sequence similarity between two regions of the same nucleic acid strand or between regions of two different nucleic acid strands. When a nucleotide residue position in both regions is occupied by the same nucleotide residue, then the regions are homologous at that position. A first region is homologous to a second region if at least one nucleotide residue position of each region is occupied by the same residue. Homology between two regions is expressed in terms of the proportion of nucleotide residue positions of the two regions that are occupied by the same nucleotide residue (i.e., by percent identity). By way of example, a region having the nucleotide sequence 5'-ATTGCC-3' and a region having the nucleotide sequence 5'-TATGGC-3' share homology with 50% identity. In one embodiment, the first region comprises a first portion and the second region comprises a second portion, whereby, at least about 50%, at least about 75%, at least about 90%, or at least about 95% of the nucleotide residue positions of each of the portions are occupied by the same nucleotide residue. In an embodiment of 100% identity, all nucleotide residue positions of each of the portions are occupied by the same nucleotide residue.

Unless otherwise specified herewithin, the terms "antibody" and "antibodies" broadly encompass naturally-occurring forms of antibodies, e.g., polyclonal antibodies (*e.g*., IgG, IgA, IgM, IgE) and monoclonal and recombinant antibodies such as single-chain antibodies, two-chain and multi-chain proteins, chimeric, CDR-grafted, human and humanized antibodies and multi-specific antibodies, as well as fragments and derivatives of all of the foregoing, which fragments (e.g., dAbs, scFv, Fab, F(ab)'₂, Fab') and derivatives have at least an antigenic binding site. Antibody derivatives may comprise a protein or chemical moiety conjugated to an antibody. The term "antibody" also includes synthetic and genetically engineered variants.

A "kit" is any article of manufacture (*e.g*., a package or container) comprising at least one reagent, *e.g.* a probe, for specifically detecting a marker or marker set of the disclosure. The article of manufacture may be promoted, distributed, sold or offered for sale as a unit for performing, e.g., *in vitro,* the methods of the present disclosure, e.g., on a sample having been obtained from a patient. The reagents included in such a kit can comprise probes/primers and/or antibodies for use in detecting short term and long term survival marker expression. In addition, a kit of the present disclosure can contain instructions which describe a suitable detection assay. Such a kit can be conveniently used, *e.g.,* in a clinical or a contract testing setting, to generate information, e.g., on expression levels, characteristic, e.g., size, sequence or composition of one or more marker, to be recorded, stored, transmitted or received to allow for diagnosis, evaluation or treatment of patients exhibiting symptoms of cancer, in particular patients exhibiting the possible presence of a cancer capable of treatment with Aurora A Kinase inhibition therapy, including, *e.g.,* hematological cancers *e.g.,* myelomas (*e.g.,* multiple myeloma), lymphomas (*e.g.,* non-hodgkins lymphoma), leukemias *(e.g.,* acute myelogenous leukemia), and solid tumors (*e.g*., breast cancer, ovarian cancer, prostate cancer, head and neck cancer, small cell lung cancer, non-small cell lung cancer, gastric cancer, renal cancer, pancreatic cancer, bladder cancer or melanoma, *etc.*)*.*

The present methods and compositions are designed for use in diagnostics and therapeutics for a patient suffering from cancer. A cancer or tumor is treated or diagnosed according to the present methods. "Cancer" or "tumor" is intended to include any neoplastic growth in a patient, including an initial tumor and any metastases. The cancer can be of the hematological or solid tumor type. Hematological tumors include tumors of hematological origin, including, *e.g.,* myelomas (*e.g.,* multiple myeloma), leukemias (*e.g*., Waldenstrom's syndrome, chronic lymphocytic leukemia, acute myelogenous leukemia, chronic myelogenous leukemia, other leukemias), lymphomas (*e.g*., B-cell lymphomas, non-Hodgkin's lymphoma) and myelodysplastic syndrome. Solid tumors can originate in organs, and include cancers such as in skin, lung, brain, breast, prostate, ovary, colon, kidney, pancreas, liver, esophagus, stomach, intestine, bladder, uterus, cervix, testis, adrenal gland, etc. The cancer can comprise a cell in which a marker gene has a mutation. As used herein, cancer cells, including tumor cells, refer to cells that divide at an abnormal (increased) rate or whose control of growth or survival is different than for cells in the same tissue where the cancer cell arises or lives. Cancer cells include, but are not limited to, cells in carcinomas, such as squamous cell carcinoma, basal cell carcinoma, sweat gland carcinoma, sebaceous gland carcinoma, adenocarcinoma, papillary carcinoma, papillary adenocarcinoma, cystadenocarcinoma, medullary carcinoma, undifferentiated carcinoma, bronchogenic carcinoma, melanoma, renal cell carcinoma, hepatoma-liver cell carcinoma, bile duct carcinoma, cholangiocarcinoma, papillary carcinoma, transitional cell carcinoma, choriocarcinoma, semonoma, embryonal carcinoma, mammary carcinomas, gastrointestinal carcinoma, colonic carcinomas, bladder carcinoma, prostate carcinoma, and squamous cell carcinoma of the neck and head region; sarcomas, such as fibrosarcoma, myxosarcoma, liposarcoma, chondrosarcoma, osteogenic sarcoma, chordosarcoma, angiosarcoma, endotheliosarcoma, lymphangiosarcoma, synoviosarcoma and mesotheliosarcoma; hematologic cancers, such as myelomas, leukemias (*e.g*., acute myelogenous leukemia, chronic lymphocytic leukemia, granulocytic leukemia, monocytic leukemia, lymphocytic leukemia), and lymphomas (*e.g*., follicular lymphoma, mantle cell lymphoma, diffuse large Bcell lymphoma, malignant lymphoma, plasmocytoma, reticulum cell sarcoma, or Hodgkins disease); and tumors of the nervous system including glioma, meningoma, medulloblastoma, schwannoma or epidymoma.

As used herein, the terms "proliferative disorders" or "proliferative diseases" includes, but is not limited to, cancerous hyperproliferative disorders (*e.g*., brain, lung, squamous cell, bladder, gastric, pancreatic, breast, head and neck, renal, liver, kidney, ovarian, prostate, colorectal, colon, epidermoid, esophageal, testicular, gynecological or thyroid cancer, acute myeloid leukemia, multiple myeloma, mesothelioma, Non-small cell lung carcinoma (NSCLC), Small cell lung carcinoma (SCLC), neuroblastoma, and acute lymphoblastic leukemia (ALL)); non-cancerous hyperproliferative disorders (*e.g*., benign hyperplasia of the skin (*e.g*., psoriasis), restenosis, and benign prostatic hypertrophy (BPH)); and diseases related to vasculogenesis or angiogenesis (*e.g*., tumor angiogenesis, hemangioma, glioma, melanoma, Kaposi's sarcoma and ovarian, breast, lung, pancreatic, prostate, colon and epidermoid cancer).

As used herein, the term "noninvasive" refers to a procedure which inflicts minimal harm to a subject. In the case of clinical applications, a noninvasive sampling procedure can be performed quickly, e.g., in a walk-in setting, typically without anaesthesia and/or without surgical implements or suturing. Examples of noninvasive samples include blood, serum, saliva, urine, buccal swabs, throat cultures, stool samples and cervical smears. Noninvasive diagnostic analyses include x-rays, magnetic resonance imaging, positron emission tomography, etc.

As used herein, the term "Aurora A kinase" refers to a serine/threonine kinases involved in mitotic progression. Aurora A kinase is also known as AIK, ARK1, AURA, BTAK, STK6, STK7, STK15, AURORA2, MGC34538, and AURKA. A variety of cellular proteins that play a role in cell division are substrates for phosphorylation by the Aurora A kinase enzyme, including, without limitation, p53, TPX-2, XIEg5 (in *Xenopus*)*,* and D-TACC (in *Drosophila*)*.* The Aurora A kinase enzyme is also itself a substrate for autophosphorylation, *e.g*., at Thr288. Preferably, the Aurora A kinase is a human Aurora A kinase.

The term "inhibitor of Aurora A kinase" or "Aurora A kinase inhibitor" is used to signify a compound that is capable of interacting with Aurora A kinase and inhibiting its enzymatic activity. Inhibiting Aurora A kinase enzymatic activity means reducing the ability of Aurora A kinase to phosphorylate a substrate peptide or protein. In various embodiments, such reduction of Aurora A kinase activity is at least about 75%, at least about 90%, at least about 95%, or at least about 99%. In various embodiments, the concentration, e.g., the IC₅₀, of Aurora A kinase inhibitor required to reduce an Aurora A kinase enzymatic activity is less than about 1 µM, less than about 500 nM, less than about 100 nM, or less than about 50 nM. In one embodiment, the concentration that is required to inhbit the enzymatic activity of Aurora A kinase is lower than the concentration of the inhibitor that is required to inhibit the enzymatic activity of Aurora B kinase. In an embodiment, the concentration of Aurora A kinase inhibitor required to reduce an Aurora A kinase enzymatic activity is 50 nM to 100 nM. In an embodiment, the concentration of Aurora A kinase inhibitor required to reduce an Aurora A kinase enzymatic activity is 100 nM to 500 nM. In an embodiment, the concentration of Aurora A kinase inhibitor required to reduce an Aurora A kinase enzymatic activity is 50 nM to 500 nM. In an embodiment, the concentration of Aurora A kinase inhibitor required to reduce an Aurora A kinase enzymatic activity is 50 nM to 1 µM. In an embodiment, the concentration of Aurora A kinase inhibitor required to reduce an Aurora A kinase enzymatic activity is 100 nM to 1 µM. In an embodiment, the concentration of Aurora A kinase inhibitor required to reduce an Aurora A kinase enzymatic activity is 500 nM to 1 µM. In various embodiments, the concentration of an Aurora A kinase inhibitor that is required to reduce Aurora A kinase enzymatic activity is at least about 2-fold, at least about 5-fold, at least about 10-fold, at least about 20-fold, at least about 50-fold, at least about 100-fold, at least about 500-fold, or at least about 1000-fold lower than the concentration of the inhibitor that is required to reduce Aurora B kinase enzymatic activity. In other embodiments, the concentration of an Aurora A kinase inhibitor that is required to reduce Aurora A kinase enzymatic activity is 2-fold to 10-fold 5-fold to 50-fold, 10-fold to 100-fold, 20-fold to 200-fold or 50-fold to 500-fold lower than the concentration of the inhibitor that is required to reduce Aurora B kinase enzymatic activity.

Inhibition of Aurora A and inhibition of Aurora B result in markedly different cellular phenotypes. (Proc. Natl. Acad. Sci. (2007) 104: 4106; Mol Cancer Ther (2009) 8(7), 2046-56; Chem Biol. (2008) 15(6) 552-62). For example, inhibition of Aurora A in the absence of Aurora B inhibition results in increased mitotic index as measured by quantifying phosphorylated histone H3 on serine 10 (pHisH3). pHisH3 is a unique substrate of Aurora B in physiological systems (e.g. intact cells). By contrast, inhibition of Aurora B or dual inhibition of Aurora A and Aurora B results in a decrease in pHisH3. Accordingly, as used herein, the term "selective inhibitor of Aurora A kinase" or "selective Aurora A kinase inhibitor" refers to an inhibitor that exhibits an Aurora A kinase inhibitor phenotype at effective antitumor concentrations. In some embodiments, the selective Aurora A kinase inhibitor causes a transient mitotic delay, as measured by quantification of pHisH3, when administered to mice at a dose where the free fraction adjusted concentration (Cₐᵥₑ) in plasma is equivalent to the free fraction adjusted concentration achieved in plasma in humans at the maximum tolerated dose (MTD). As used herein, "free fraction adjusted concentration" refers to the plasma concentration of free drug (not protein bound).

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

As used herein, the term "comprises" means "includes, but is not limited to."

The term "patient", as used herein, means an animal, preferably a mammal, more preferably a human. In some embodiments, the patient has been treated with an agent, e.g., an Aurora A kinase selective inhibitor, prior to initiation of treatment according to the method of the disclosure. In some embodiments, the patient is a patient at risk of developing or experiencing a recurrence of a proliferative disorder.

The term "aliphatic" or "aliphatic group", as used herein, means a substituted or unsubstituted straight-chain, branched or cyclic C₁₋₁₂ hydrocarbon, which is completely saturated or which contains one or more units of unsaturation, but which is not aromatic. For example, suitable aliphatic groups include substituted or unsubstituted linear, branched or cyclic alkyl, alkenyl, alkynyl groups and hybrids thereof, such as (cylcoalkyl)alkyl, (cycloalkenyl)alkyl or (cycloalkyl)alkenyl.

The terms "alkyl", "alkenyl", and "alkynyl", used alone or as part of a larger moiety, refer to a straight and branched chain aliphatic group having from 1 to 12 carbon atoms. For purposes of the present disclosure, the term "alkyl" will be used when the carbon atom attaching the aliphatic group to the rest of the molecule is a saturated carbon atom. However, an alkyl group may include unsaturation at other carbon atoms. Thus, alkyl groups include, without limitation, methyl, ethyl, propyl, allyl, propargyl, butyl, pentyl, and hexyl.

For purposes of the present disclosure, the term "alkenyl" will be used when the carbon atom attaching the aliphatic group to the rest of the molecule forms part of a carbon-carbon double bond. Alkenyl groups include, without limitation, vinyl, 1-propenyl, 1-butenyl, 1-pentenyl, and 1-hexenyl.

For purposes of the present disclosure, the term "alkynyl" will be used when the carbon atom attaching the aliphatic group to the rest of the molecule forms part of a carbon-carbon triple bond. Alkynyl groups include, without limitation, ethynyl, 1-propynyl, 1-butynyl, 1-pentynyl, and 1-hexynyl.

The term "cycloaliphatic", used alone or as part of a larger moiety, refers to a saturated or partially unsaturated cyclic aliphatic ring system having from 3 to about 14 members, wherein the aliphatic ring system is optionally substituted. In some embodiments, the cycloaliphatic is a monocyclic hydrocarbon having 3-8 or 3-6 ring carbon atoms. Nonlimiting examples include cyclopropyl, cyclobutyl, cyclopentyl, cyclopentenyl, cyclohexyl, cyclohexenyl, cycloheptyl, cycloheptenyl, cyclooctyl, cyclooctenyl, and cyclooctadienyl. In some embodiments, the cycloaliphatic is a bridged or fused bicyclic hydrocarbon having 6-12, 6-10, or 6-8 ring carbon atoms, wherein any individual ring in the bicyclic ring system has 3-8 members.

In some embodiments, two adjacent substituents on the cycloaliphatic ring, taken together with the intervening ring atoms, form an optionally substituted fused 5- to 6-membered aromatic or 3-to 8-membered non-aromatic ring having 0-3 ring heteroatoms selected from the group consisting of O, N, and S. Thus, the term "cycloaliphatic" includes aliphatic rings that are fused to one or more aryl, heteroaryl, or heterocyclyl rings. Nonlimiting examples include indanyl, 5,6,7,8-tetrahydro-quinoxalinyl, decahydronaphthyl, or tetrahydronaphthyl, where the radical or point of attachment is on the aliphatic ring. The term "cycloaliphatic" may be used interchangeably with the terms "carbocycle", "carbocyclyl", "carbocyclo", or "carbocyclic".

The terms "aryl" and "ar-", used alone or as part of a larger moiety, e.g., "aralkyl", "aralkoxy", or "aryloxyalkyl", refer to a C₆ to C₁₄ aromatic hydrocarbon, comprising one to three rings, each of which is optionally substituted. Preferably, the aryl group is a C₆-₁₀ aryl group. Aryl groups include, without limitation, phenyl, naphthyl, and anthracenyl. In some embodiments, two adjacent substituents on the aryl ring, taken together with the intervening ring atoms, form an optionally substituted fused 5- to 6-membered aromatic or 4- to 8-membered non-aromatic ring having 0-3 ring heteroatoms selected from the group consisting of O, N, and S. Thus, the term "aryl", as used herein, includes groups in which an aromatic ring is fused to one or more heteroaryl, cycloaliphatic, or heterocyclyl rings, where the radical or point of attachment is on the aromatic ring. Nonlimiting examples of such fused ring systems include indolyl, isoindolyl, benzothienyl, benzofuranyl, dibenzofuranyl, indazolyl, benzimidazolyl, benzthiazolyl, quinolyl, isoquinolyl, cinnolinyl, phthalazinyl, quinazolinyl, quinoxalinyl, carbazolyl, acridinyl, phenazinyl, phenothiazinyl, phenoxazinyl, tetrahydroquinolinyl, tetrahydroisoquinolinyl, fluorenyl, indanyl, phenanthridinyl, tetrahydronaphthyl, indolinyl, phenoxazinyl, benzodioxanyl, and benzodioxolyl. An aryl group may be mono-, bi-, tri-, or polycyclic, preferably mono-, bi-, or tricyclic, more preferably mono- or bicyclic. The term "aryl" may be used interchangeably with the terms "aryl group", "aryl moiety", and "aryl ring".

An "aralkyl" or "arylalkyl" group comprises an aryl group covalently attached to an alkyl group, either of which independently is optionally substituted. Preferably, the aralkyl group is C₆₋₁₀ aryl(C₁₋₆)alkyl, C₆₋₁₀ aryl(C₁₋₄)alkyl, or C₆₋₁₀ aryl(C₁₋₃)alkyl, including, without limitation, benzyl, phenethyl, and naphthylmethyl.

The terms "heteroaryl" and "heteroar-", used alone or as part of a larger moiety, e.g., heteroaralkyl, or "heteroaralkoxy", refer to groups having 5 to 14 ring atoms, preferably 5, 6, 9, or 10 ring atoms; having 6, 10, or 14 □ electrons shared in a cyclic array; and having, in addition to carbon atoms, from one to four heteroatoms. The term "heteroatom" refers to nitrogen, oxygen, or sulfur, and includes any oxidized form of nitrogen or sulfur, and any quaternized form of a basic nitrogen. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, and pteridinyl. In some embodiments, two adjacent substituents on the heteroaryl, taken together with the intervening ring atoms, form an optionally substituted fused 5- to 6-membered aromatic or 4- to 8-membered non-aromatic ring having 0-3 ring heteroatoms selected from the group consisting of O, N, and S. Thus, the terms "heteroaryl" and "heteroar-", as used herein, also include groups in which a heteroaromatic ring is fused to one or more aryl, cycloaliphatic, or heterocyclyl rings, where the radical or point of attachment is on the heteroaromatic ring. Nonlimiting examples include indolyl, isoindolyl, benzothienyl, benzofuranyl, dibenzofuranyl, indazolyl, benzimidazolyl, benzthiazolyl, quinolyl, isoquinolyl, cinnolinyl, phthalazinyl, quinazolinyl, quinoxalinyl, 4*H*-quinolizinyl, carbazolyl, acridinyl, phenazinyl, phenothiazinyl, phenoxazinyl, tetrahydroquinolinyl, tetrahydroisoquinolinyl, and pyrido[2,3-b]-1,4-oxazin-3(4H)-one. A heteroaryl group may be mono-, bi-, tri-, or polycyclic, preferably mono-, bi-, or tricyclic, more preferably mono- or bicyclic. The term "heteroaryl" may be used interchangeably with the terms "heteroaryl ring", "heteroaryl group", or "heteroaromatic", any of which terms include rings that are optionally substituted. The term "heteroaralkyl" refers to an alkyl group substituted by a heteroaryl, wherein the alkyl and heteroaryl portions independently are optionally substituted.

As used herein, the terms "heterocycle", "heterocyclyl", "heterocyclic radical", and "heterocyclic ring" are used interchangeably and refer to a stable 3- to 7-membered monocyclic, or to a fused 7- to 10-membered or bridged 6- to 10-membered bicyclic heterocyclic moiety that is either saturated or partially unsaturated, and having, in addition to carbon atoms, one or more, preferably one to four, heteroatoms, as defined above. When used in reference to a ring atom of a heterocycle, the term "nitrogen" includes a substituted nitrogen. As an example, in a heterocyclyl ring having 1-3 heteroatoms selected from oxygen, sulfur or nitrogen, the nitrogen may be N (as in 3,4-dihydro-2*H-*pyrrolyl), NH (as in pyrrolidinyl) or ⁺NR (as in *N*-substituted pyrrolidinyl). A heterocyclic ring can be attached to its pendant group at any heteroatom or carbon atom that results in a stable structure, and any of the ring atoms can be optionally substituted. Examples of such saturated or partially unsaturated heterocyclic radicals include, without limitation, tetrahydrofuranyl, tetrahydrothienyl, pyrrolidinyl, pyrrolidonyl, piperidinyl, pyrrolinyl, tetrahydroquinolinyl, tetrahydroisoquinolinyl, decahydroquinolinyl, oxazolidinyl, piperazinyl, dioxanyl, dioxolanyl, diazepinyl, oxazepinyl, thiazepinyl, morpholinyl, and quinuclidinyl.

In some embodiments, two adjacent substituents on a heterocyclic ring, taken together with the intervening ring atoms, for an optionally substituted fused 5- to 6-membered aromatic or 3- to 8-membered non-aromatic ring having 0-3 ring heteroatoms selected from the group consisting of O, N, and S. Thus, the terms "heterocycle", "heterocyclyl", "heterocyclyl ring", "heterocyclic group", "heterocyclic moiety", and "heterocyclic radical", are used interchangeably herein, and include groups in which a heterocyclyl ring is fused to one or more aryl, heteroaryl, or cycloaliphatic rings, such as indolinyl, 3*H*-indolyl, chromanyl, phenanthridinyl, or tetrahydroquinolinyl, where the radical or point of attachment is on the heterocyclyl ring. A heterocyclyl group may be mono-, bi-, tri-, or polycyclic, preferably mono-, bi-, or tricyclic, more preferably mono- or bicyclic. The term "heterocyclylalkyl" refers to an alkyl group substituted by a heterocyclyl, wherein the alkyl and heterocyclyl portions independently are optionally substituted.

As used herein, the term "partially unsaturated" refers to a ring moiety that includes at least one double or triple bond between ring atoms. The term "partially unsaturated" is intended to encompass rings having multiple sites of unsaturation, but is not intended to include aryl or heteroaryl moieties, as herein defined.

The terms "haloaliphatic", "haloalkyl", "haloalkenyl" and "haloalkoxy" refer to an aliphatic, alkyl, alkenyl or alkoxy group, as the case may be, which is substituted with one or more halogen atoms. As used herein, the term "halogen" or "halo" means F, Cl, Br, or I. The term "fluoroaliphatic" refers to a haloaliphatic wherein the halogen is fluoro.

The term "alkylene" refers to a bivalent alkyl group. An "alkylene chain" is a polymethylene group, i.e., -(CH₂)ₙ-, wherein n is a positive integer, preferably from 1 to 6, from 1 to 4, from 1 to 3, from 1 to 2, or from 2 to 3. A substituted alkylene chain is a polymethylene group in which one or more methylene hydrogen atoms is replaced with a substituent. Suitable substituents include those described below for a substituted aliphatic group. An alkylene chain also may be substituted at one or more positions with an aliphatic group or a substituted aliphatic group.

The term "substituted", as used herein, means that a hydrogen radical of the designated moiety is replaced with the radical of a specified substituent, provided that the substitution results in a stable or chemically feasible compound. The phrase "one or more substituents", as used herein, refers to a number of substituents that equals from one to the maximum number of substituents possible based on the number of available bonding sites, provided that the above conditions of stability and chemical feasibility are met. Unless otherwise indicated, an optionally substituted group may have a substituent at each substitutable position of the group, and the substituents may be either the same or different.

An aryl (including the aryl moiety in aralkyl, aralkoxy, aryloxyalkyl and the like) or heteroaryl (including the heteroaryl moiety in heteroaralkyl and heteroaralkoxy and the like) group may contain one or more substituents. Examples of suitable substituents on the unsaturated carbon atom of an aryl or heteroaryl group
include -halo, -NO₂, -CN, -R*, -C(R*)=C(R*)₂, -C=C-R*, -OR*, -SR°, -S(O)R°, -SO₂R°, -SO₃R°, -SO ₂N(R⁺)₂, -N(R⁺)₂, -NR⁺C(O)R*, -NR⁺C(O)N(R⁺)₂, -NR⁺CO₂R°, -O-CO₂R*, -OC(O)N(R)₂, -O-C(O)R *, -CO₂R*, -C(O)-C(O)R*, -C(O)R*, -C(O)N(R⁺)₂, -C(O)N(R⁺)C(=NR⁺)-N(R⁺)₂, -N(R⁺)C(=NR⁺)-N( R⁺)-C(O)R*, -C(=NR⁺)-N(R⁺)₂, -C(=NR⁺)-OR*. -N(R⁺)-N(R⁺)₂, -N(R⁺)C(=NR⁺)-N(R⁺)₂, -NR⁺SO₂R°, -NR⁺SO₂N(R⁺)₂, -P(O)(R*)₂, -P(O)(OR*)₂, -O-P(O)-OR*, and -P(O)(NR⁺)-N(R⁺)₂; or two adjacent substituents, taken together with their intervening atoms, form a 5-6 membered unsaturated or partially unsaturated ring having 0-3 ring atoms selected from the group consisting of N, O, and S.

An aryl (including the aryl moiety in aralkyl, aralkoxy, aryloxyalkyl and the like) or heteroaryl (including the heteroaryl moiety in heteroaralkyl and heteroaralkoxy and the like) group may contain one or more substituents. Examples of suitable substituents on the unsaturated carbon atom of an aryl or heteroaryl group
include -halo, -NO₂, -CN, -R*, -C(R*)=C(R*)₂, -C=C-R*, -OR*, -SR°, -S(O)R°, -SO₂R°, -SO₃R°, -SO ₂N(R⁺)₂, -N(R⁺)₂, -NR⁺C(O)R*, -NR⁺C(O)N(R⁺)₂, -NR⁺CO₂R°_{;} -O-CO₂R*, -OC(O)N(R⁺)₂, -O-C(O)R *, -CO₂R*, -C(O)-C(O)R*, -C(O)R*, -C(O)N(R⁺)₂, -C(O)N(R⁺)C(=NR)-N(R⁺)₂, -N(R⁺)C(=NR⁺)-N( R⁺)-C(O)R*, -C(=NR⁺)N(R⁺)₂, -C(=NR⁺)-OR*, -N(R⁺)-N(R⁺)₂, -N(R⁺)C(=NR⁺)-N(R⁺)₂, -NR⁺SO₂R°, -NR⁺SO₂N(R⁺)₂, -P(O)(R*)₂, -P(O)(OR*)₂, -O-P(O)-OR*, and -P(O)(NR⁺)-N(R⁺)₂; or two adjacent substituents, taken together with their intervening atoms, form a 5-6 membered unsaturated or partially unsaturated ring having 0-3 ring atoms selected from the group consisting of N, O, and S.

Each R⁺, independently, is hydrogen or an optionally substituted aliphatic, aryl, heteroaryl, or heterocyclyl group, or two R⁺ on the same nitrogen atom, taken together with the nitrogen atom, form a 5-8 membered aromatic or non-aromatic ring having, in addition to the nitrogen atom, 0-2 ring heteroatoms selected from N, O, and S. Each R^{*} independently is hydrogen or an optionally substituted aliphatic, aryl, heteroaryl, or heterocyclyl group. Each R° is an optionally substituted aliphatic or aryl group.

An aliphatic group or a non-aromatic heterocyclic ring may be substituted with one or more substituents. Examples of suitable substituents on the saturated carbon of an aliphatic group or of a non-aromatic heterocyclic ring include, without limitation, those listed above for the unsaturated carbon of an aryl or heteroaryl group and the following: =O, =S, =C(R*)₂, =N-N(R*)₂, =N-OR*, =N-NHC(O)R*, =N-NHCO₂R°, =N-NHSO₂R°, or =N-R*, where each R* and R° is as defined above.

Suitable substituents on the nitrogen atom of a non-aromatic heterocyclic ring include -R*, -N(R*)₂, -C(O)R*, -CO₂R*, -C(O)-C(O)R* -C(O)CH₂C(O)R*, -SO₂R*, -SO₂N(R*)₂, -C( =S)N(R*)₂, -C(=NH)-N(R*)₂, and -NR*SO₂R*; wherein each R* is as defined above.

Unless otherwise stated, structures depicted herein are meant to include compounds which differ only in the presence of one or more isotopically enriched atoms. For example, compounds having the present structure except for the replacement of a hydrogen atom by a deuterium or tritium, or the replacement of a carbon atom by a ¹³C- or ¹⁴C-enriched carbon are within the scope of the disclosure.

It will be apparent to one skilled in the art that certain compounds described herein may exist in tautomeric forms, all such tautomeric forms of the compounds being within the scope of the disclosure. Unless otherwise stated, structures depicted herein are also meant to include all stereochemical forms of the structure; i.e., the *R* and *S* configurations for each asymmetric center. Therefore, single stereochemical isomers as well as enantiomeric and diastereomeric mixtures of the present compounds are within the scope of the disclosure.

Any molecule capable of selectively inhibiting the enzymatic activity of Aurora A kinase may be used in the methods, pharmaceutical compositions, and kits of the present disclosure. In some embodiments the selective Aurora A kinase inhibitor is a small molecular weight compound. In particular, selective inhibitors of Aurora A kinase include the compounds described herein, as well as compounds disclosed in, for example, US Publication No. 2008/0045501, US Patent No. 7,572,784, WO 05/111039, WO 08/021038, US Patent No. 7,718,648, WO 08/063525, US Publication No. 2008/0167292, US Patent No. 8,026,246, WO 10/134965, US Publication No. 2010/0310651, WO 11/014248, US Publication No. 2011/0039826, and US Publication No. 201110245234, as well as the compounds sodium 4-{[9-chloro-7-(2-fluoro-6-methoxyphenyl)-5*H*-pyrimido[5,4-*d*][2]benzazepin-2-yl]amino}-2-methoxybenzoate, KW-2449 (Kyowa), ENMD-2076 (EntreMed), and MK-5108 (Vertex/Merck). Also suitable for use in the methods, pharmaceutical compositions, and kits of the disclosure are solvated and hydrated forms of any of these compounds. Also suitable for use in the methods, pharmaceutical compositions, and kits of the disclosure are pharmaceutically acceptable salts of any of the compounds, and solvated and hydrated forms of such salts. These selective Aurora A kinase inhibitors can be prepared in a number of ways well known to one skilled in the art of organic synthesis, including, but not limited to, the methods of synthesis described in detail in the references referred to herein.

Aurora A kinase inhibitors can be assayed *in vitro* or *in vivo* for their ability to selectively bind to and/or inhibit an Aurora A kinase. *In vitro* assays include assays to determine selective inhibition of the ability of an Aurora A kinase to phosphorylate a substrate protein or peptide. Alternate *in vitro* assays quantitate the ability of the compound to selectively bind to an Aurora A kinase. Selective inhibitor binding may be measured by radiolabelling the inhibitor prior to binding, isolating the inhibitor/Aurora A kinase complex and determining the amount of radiolabel bound. Alternatively, selective inhibitor binding may be determined by running a competition experiment in which new inhibitors are incubated with Aurora A kinase bound to a known radioligand. The compounds also can be assayed for their ability to affect cellular or physiological functions mediated by Aurora A kinase activity. In order to assess selectivity for Aurora A kinase over Aurora B kinase, inhibitors can also be assayed *in vitro* and *in vivo* for their ability to selectively bind to and/or inhibit an Aurora B kinase, using assays analogous to those described above for Aurora A kinase. Inhibitors can be assayed *in vitro* and *in vivo* for their ability to inhibit Aurora A kinase in the absence of Aurora B kinase inhibition, by immunofluorescent detection of pH is H3. (Proc. Natl. Acad. Sci. (2007) 104, 4106). Assays for each of these activities are known in the art.

In some embodiments, the selective Aurora A kinase inhibitor is a compound represented by formula (*I*) : 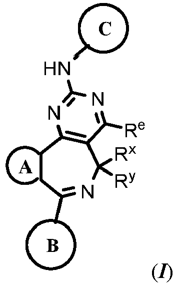 or a pharmaceutically acceptable salt thereof; wherein:
Ring A is a substituted or unsubstituted 5- or 6-membered aryl, heteroaryl, cycloaliphatic, or heterocyclyl ring;
Ring B is a substituted or unsubstituted aryl, heteroaryl, cycloaliphatic, or heterocyclyl ring;
Ring C is a substituted or unsubstituted aryl, heteroaryl, heterocyclyl, or cycloaliphatic ring;
R^{e} is hydrogen, -OR⁵, -N(R⁴)₂, -SR⁵, or a C₁₋₃ aliphatic optionally substituted with R³ or R⁷;
each of R^{x} and R^{y} independently is hydrogen, fluoro, or an optionally substituted C₁₋₆ aliphatic; or R^{x} and R^{y}, taken together with the carbon atom to which they are attached, form an optionally substituted 3- to 6-membered cycloaliphatic ring;
each R³ independently is selected from the group consisting of -halo, -OH, -O(C₁₋₃ alkyl), -CN, -N(R⁴)₂, -C(O)(C₁₋₃ alkyl), -CO₂H, -CO₂(C₁₋₃ alkyl), -C(O) NH₂, and -C(O)NH(C₁₋₃ alkyl);
each R⁴ independently is hydrogen or an optionally substituted aliphatic, aryl, heteroaryl, or heterocyclyl group; or two R⁴ on the same nitrogen atom, taken together with the nitrogen atom, form an optionally substituted 5- to 6-membered heteroaryl or 4- to 8-membered heterocyclyl ring having, in addition to the nitrogen atom, 0-2 ring heteroatoms selected from N, O, and S;
each R⁵ independently is hydrogen or an optionally substituted aliphatic, aryl, heteroaryl, or heterocyclyl group; and
each R⁷ independently is an optionally substituted aryl, heterocyclyl, or heteroaryl group.

Ring **A** is a substituted or unsubstituted 5- or 6-membered aryl, heteroaryl, cycloaliphatic, or heterocyclyl ring. Examples of Ring **A** include furano, dihydrofurano, thieno, dihydrothieno, cyclopenteno, cyclohexeno, 2H-pyrrolo, pyrrolo, pyrrolino, pyrrolidino, oxazolo, thiazolo, imidazolo, imidazolino, imidazolidino, pyrazolo, pyrazolino, pyrazolidino, isoxazolo, isothiazolo, oxadiazolo, triazolo, thiadiazolo, 2H-pyrano, 4H-pyrano, benzo, pyridino, piperidino, dioxano, morpholino, dithiano, thiomorpholino, pyridazino, pyrimidino, pyrazino, piperazino, and triazino, any of which groups may be substituted or unsubstituted. Preferred values for Ring **A** include, without limitation, substituted or unsubstituted rings selected from the group consisting of furano, thieno, pyrrolo, oxazolo, thiazolo, imidazolo, pyrazolo, isoxazolo, isothiazolo, triazolo, benzo, pyridino, pyridazino, pyrimidino, and pyrazino.

Ring **A** may be substituted or unsubstituted. In some embodiments, each substitutable saturated ring carbon atom in Ring **A** is unsubstituted or is substituted with =O, =S, =C(R⁵)₂, =N-N(R⁴)₂, =N-OR⁵, =N-NHC(O)R⁵, =N-NHCO₂R⁶, =N-NHSO₂R⁶, =N-R⁵ or -R^{b}, where R^{b}, R⁴, R⁵, and R⁶ are as defined below. Each substitutable unsaturated ring carbon atom in Ring **A** is unsubstituted or substituted with -R^{b}. Each substitutable ring nitrogen atom in Ring **A** is unsubstituted or is substituted with -R^{9b}, and one ring nitrogen atom in Ring **A** optionally is oxidized. Each R^{9b} independently is -C(O)R⁵, -C(O)N(R⁴)₂, -CO₂R⁶, -SO₂R⁶, -SO₂N(R⁴)₂, or a C₁₋₄ aliphatic optionally substituted with R³ or R⁷.

Each R^{b} independently is R^{2b}, an optionally substituted aliphatic, or an optionally substituted aryl, heterocyclyl, or heteroaryl group; or two adjacent R^{b}, taken together with the intervening ring atoms, form an optionally substituted fused 4- to 8-membered aromatic or non-aromatic ring having 0-3 ring heteroatoms selected from the group consisting of O, N, and S.

Each R^{2b} independently
is -halo, -NO₂, -CN, -C(R⁵)=C(R⁵)₂, -C(R⁵)=C(R⁵)(R¹⁰), -C=C-R⁵, -C≡C-R¹⁰, -OR⁵, -SR⁶, -S(O)R⁶, -S O₂R⁶, -SO₂N(R⁴)₂, -N(R⁴)₂, -NR⁴C(O)R⁵, -NR⁴C(O)N(R⁴)₂, -NR⁴CO₂R⁶, -O-CO₂R⁵, -OC(O)N(R⁴)₂, - O-C(O)R⁵, -CO₂R⁵, -C(O)-C(O)R⁵, -C(O)R⁵, -C(O)N(R⁴)₂, -C(=NR⁴)-N(R⁴)₂, -C(=NR⁴)-OR⁵, -N(R⁴)-N(R⁴)₂, N(R⁴)C(=NR⁴)-N(R⁴)₂, -N(R⁴)SO₂R⁶, -N(R⁴)SO₂N(R⁴)₂, -P(O)(R⁵)₂, or -P(O)(OR⁵)₂, where the variables R⁴, R⁵, and R⁷ have the values described above; each R⁶ independently is an optionally substituted aliphatic or aryl group; and each R¹⁰ independently is -CO₂R⁵ or -C(O)N(R⁴)₂.

In some embodiments, Ring **A** is substituted by 0-2 substituents R^{b}. In some such embodiments, each R^{b} independently is C₁₋₃ aliphatic or R^{2b}, and each R^{2b} independently is selected from the group consisting of -halo, -NO₂, -C(R⁵)=C(R⁵)₂, -C=C-R⁵, -OR⁵, and -N(R⁴)₂. In some embodiments, each R^{b} independently is selected from the group consisting of -halo, C₁₋₃ aliphatic, C₁₋₃ fluoroaliphatic, and -OR⁵, where R⁵ is hydrogen or C₁₋₃ aliphatic. In certain preferred embodiments, Ring **A** is substituted with 0, 1, or 2 substituents, preferably 0 or 1 substituents, independently selected from the group consisting of chloro, fluoro, bromo, methyl, trifluoromethyl, and methoxy.

In some embodiments, Ring **B** is a substituted or unsubstituted mono- or bicyclic aryl or heteroaryl ring selected from the group consisting of furanyl, thienyl, pyrrolyl, oxazolyl, thiazolyl, imidazolyl, pyrazolyl, isoxazolyl, isothiazolyl, oxadiazolyl, triazolyl, thiadiazolyl, phenyl, pyridyl, pyridazinyl, pyrimidinyl, pyrazinyl, triazinyl, indolizinyl, indolyl, isoindolyl, indazolyl, benzo[b]furanyl, benzo[b]thienyl, benzimidazolyl, benzthiazolyl, benzoxazolyl, purinyl, quinolyl, isoquinolyl, cinnolinyl, phthalazinyl, quinazolinyl, quinoxalinyl, naphthyridinyl, and pteridinyl.

Each substitutable saturated ring carbon atom in Ring **B** is unsubstituted or is substituted with =O, =S, =C(R⁵)₂, =N-N(R⁴)₂, =N-OR⁵, =N-NHC(O)R⁵, =N-NHCO₂R⁶, =N-NHSO₂R⁶, =N-R⁵ or -R^{c}. Each substitutable unsaturated ring carbon atom in Ring **B** is unsubstituted or substituted with -R^{c}. Each substitutable ring nitrogen atom in Ring **B** is unsubstituted or is substituted with -R^{9c}, and one ring nitrogen atom in Ring **B** optionally is oxidized. Each R^{9c} independently is -C(O)R⁵, -C(O)N(R⁴)₂, -CO₂R⁶, -SO₂R⁶, -SO₂N(R⁴)₂, or a C₁₋₄ aliphatic optionally substituted with R³ or R⁷. Ring **B** may be unsubstituted or may be substituted on any one or more of its component rings, wherein the substituents may be the same or different. In some embodiments, Ring **B** is substituted with 0-2 independently selected R^{c} and 0-3 independently selected R^{2c} or C₁₋₆ aliphatic groups. The variables R³, R⁴, R⁵, R⁶, and R⁷ are as defined above for Ring **A,** and R^{c} and R^{2c} are defined below.

Each R^{c} independently is R^{2c}, an optionally substituted C₁₋₆ aliphatic, or an optionally substituted aryl, heteroaryl, or heterocyclyl group.

Each R^{2c} independently is -halo, -NO₂, -CN, -
C(R⁵)=C(R⁵)₂, -C(R⁵)=C(R⁵)(R¹⁰), -C≡C-R⁵, -C≡C-R¹⁰, -OR⁵, -SR⁶, -S(O)R⁶, -SO₂R⁶, -SO₂N(R⁴)₂, -N( R⁴)₂, -NR⁴C(O)R⁵, -NR⁴C(O)N(R⁴)₂, -NR⁴CO₂R⁶, -O-CO₂R⁵, -OC(O)N(R⁴)₂, -O-C(O)R⁵, -CO₂R⁵, -C (O)-C(O)R⁵, -C(O)R⁵, -C(O)N(R⁴)₂, -C(=NR⁴)-N(R⁴)₂, -C(=NR⁴)-OR⁵, -N(R⁴)-N(R⁴)₂, -N(R⁴)C(=NR⁴ )-N(R⁴)₂, -N(R⁴)SO₂R⁶, -N(R⁴)SO₂N(R⁴)₂, -P(O)(R⁵)₂, or -P(O)(OR⁵)₂.

In some embodiments, Ring **B** is a monocyclic 5- or 6-membered aryl or heteroaryl ring, substituted with 0-2 independently selected R^{c} and 0-2 independently selected R^{2c} or C₁₋₆ aliphatic groups. In certain such embodiments, Ring **B** is a substituted or unsubstituted phenyl or pyridyl ring.

In some embodiments, Ring **B** is substituted with 0-2 substituents R^{c}. In some such embodiments, each R^{c} independently is C₁₋₃ aliphatic or R^{2c}, and each R^{2c} independently is selected from the group consisting of -halo, -NO₂, -C(R⁵)=C(R⁵)₂, -C=C-R⁵, -OR⁵, and -N(R⁴)₂. In some embodiments, each R^{c} independently is selected from the group consisting of -halo, C₁₋₃ aliphatic, C₁₋₃ haloaliphatic, and -OR⁵, where R⁵ is hydrogen or C₁₋₃ aliphatic. In certain preferred embodiments, Ring **B** is substituted with 0, 1, or 2 substituents, independently selected from the group consisting of chloro, fluoro, bromo, methyl, trifluoromethyl, and methoxy.

Each substitutable saturated ring carbon atom in Ring C is unsubstituted or is substituted with =O, =S, =C(R⁵)₂, =N-N(R⁴)₂, =N-OR⁵, =N-NHC(O)R⁵, =N-NHCO₂R⁶, =N-NHSO₂R⁶, =N-R⁵ or -R^{d}. Each substitutable unsaturated ring carbon atom in Ring **C** is unsubstituted or substituted with -R^{d}. Each substitutable ring nitrogen atom in Ring **C** is unsubstituted or is substituted with -R^{9d}, and one ring nitrogen atom in Ring **C** optionally is oxidized. Each R^{9d} independently is -C(O)R⁵, -C(O)N(R⁴)₂, -CO₂R⁶, -SO₂R⁶, -SO₂N(R⁴)₂, or a C₁₋₄ aliphatic optionally substituted with R³ or R⁷. Ring **C** may be unsubstituted or may be substituted on any one or more of its component rings, wherein the substituents may be the same or different. In some embodiments, Ring C is substituted with 0-2 independently selected R^{d} and 0-3 independently selected R^{2d} or C₁₋₆ aliphatic groups. The variables R³, R⁴, R⁵, R⁶, and R⁷ are as described above for Rings **A** and **B.** The variables R^{d} and R^{2d} are described below.

Each R^{d} independently is R^{2d}, an optionally substituted aliphatic, or an optionally substituted aryl, heteroaryl, or heterocyclyl group.

Each R^{2d} independently is -halo, -NO₂, -CN, -C(R⁵)=C(R⁵)₂, -
C(R⁵)=C(R⁵)₂(R¹⁰), -C=C-R⁵, -C≡C-R¹⁰, -OR⁵, -SR⁶, -S(O)R⁶, -SO₂R⁶, -SO₂N(R⁴)_{2,} -N(R⁴)₂, -NR⁴C(O )R⁵, -NR⁴C(O)N(R⁴)₂, -NR⁴CO₂R⁶, -O-CO₂R⁵, -OC(O)N(R⁴)₂, -O-C(O)R⁵, -CO₂R⁵, -C(O)-C(O)R⁵, - C(O)R⁵, -C(O)N(R⁴)₂, -C(=NR⁴)-N(R⁴)₂, -C(=NR⁴)-OR⁵, -N(R⁴)-N(R⁴)₂, -N(R⁴)C(=NR⁴)-N(R⁴)₂, -N( R⁴)SO₂R⁶, -N(R⁴)SO₂N(R⁴)₂, -P(O)(R⁵)₂, or -P(O)(OR⁵)₂. Additionally, R^{2d} can be -SO₃R⁵, -C(O)N(R⁴)C(=NR⁴)-N(R⁴)₂ or -N(R⁴)C(=NR⁴)-N(R⁴)-C(O)R⁵.

In some embodiments, Ring **C is** a monocyclic 5- or 6-membered aryl or heteroaryl ring, which is substituted with 0-2 independently selected substituents R^{d} and 0-2 independently selected R^{2d} or C₁₋₆ aliphatic groups. In some such embodiments, Ring **C** is an optionally substituted heteroaryl ring selected from the group consisting of pyridyl, pyrimidinyl, pyrazinyl, pyridazinyl, imidazolyl, pyrazolyl, and oxazolyl. In some other embodiments, Ring **C** is a substituted or unsubstituted phenyl ring. In some embodiments, Ring **C** is a monocyclic 5- or 6-membered aryl or heteroaryl ring, which is substituted with 0, 1, or 2 substituents R^{d}, as defined above.

In some other embodiments, Ring **C is** a monocyclic 5- or 6-membered heterocyclyl or cycloaliphatic ring, which is substituted with 0-2 independently selected substituents R^{d} and 0-2 independently selected R^{2d} or C₁₋₆ aliphatic groups.

In some embodiments, the selective Aurora A kinase inhibitor is a compound represented by formula (*II*): 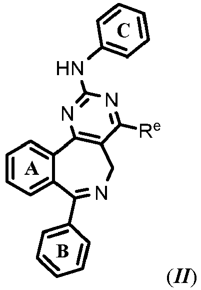 or a pharmaceutically acceptable salt thereof;
wherein:
R^{e} is hydrogen or a C₁₋₃ aliphatic optionally substituted with R³ or R⁷;
Ring **A** is substituted with 0-3 R^{b};
   each R^{b} independently is selected from the group consisting of C₁₋₆ aliphatic, R^{2b}, R^{7b}, -T¹-R^{2b}, and -T¹-R^{7b};
   each R^{2b} independently is -halo, -NO₂, -CN, -C(R⁵)=C(R⁵)₂, -C=C-R⁵, -OR⁵, -SR⁶, -S(O)R⁶, -SO₂R⁶, -SO₂N(R ⁴)₂, -N(R⁴)₂, -NR⁴C(O)R⁵, -NR⁴C(O)N(R⁴)₂, -NR⁴CO₂R⁶, -O-CO₂R⁵, -OC(O)N(R⁴)₂, -O-C(O)R⁵, -CO₂R⁵, -C(O)-C(O)R⁵, -C(O)R⁵, -C(O)N(R⁴)₂, -C(=NR⁴)-N(R⁴)₂, -C(=N R⁴)-OR⁵, -N(R⁴)-N(R⁴)₂, -N(R⁴)C(=NR⁴)-N(R⁴)₂, -N(R⁴)SO₂R⁶, -N(R⁴)SO₂N(R⁴)₂, -P( O)(R⁵)₂, or -P(O)(OR⁵)₂; each R^{7b} independently is an optionally substituted aryl, heterocyclyl, or heteroaryl group;
Ring **B** is substituted with 0-2 independently selected R^{c} and 0-2 independently selected R^{2c} or C₁₋₆ aliphatic groups;
   each R^{c} independently is selected from the group consisting of C₁₋₆ aliphatic, R^{2c}, R^{7c}, -T¹-R^{2c}, and -T¹-R^{7c};
   each R^{2c} independently is -halo, -NO₂, -CN, -C(R⁵)=C(R⁵)₂, -C≡C-R⁵, -OR⁵, -SR⁶, -S(O)R⁶, -SO₂R⁶, -SO₂N(R ⁴)₂, -N(R⁴)₂, -NR⁴C(O)R⁵, -NR⁴C(O)N(R⁴)₂ -NR⁴CO₂R⁶, -O-CO₂R⁵, -OC(O)N(R⁴)₂, -O-C(O)R⁵, -CO₂R⁵, -C(O)-C(O)R⁵, -C(O)R⁵, -C(O)N(R⁴)₂, -C(=NR⁴)-N(R⁴)₂, -C(=N R⁴)-OR⁵, -N(R⁴)-N(R⁴)₂, -N(R⁴)C(=NR⁴)-N(R⁴)₂, -N(R⁴)SO₂R⁶, -N(R⁴)SO₂N(R⁴)₂, -P( O)(R⁵)₂, or -P(O)(OR⁵)₂;
   each R^{7c} independently is an optionally substituted aryl, heterocyclyl, or heteroaryl group; T¹ is a C₁₋₆ alkylene chain optionally substituted with R³ or R^{3b}, wherein T¹ or a portion thereof optionally forms part of a 3- to 7-membered ring;
Ring **C** is substituted with 0-2 independently selected R^{d} and 0-3 independently selected R^{2d} or C₁₋₆ aliphatic groups;
   each R^{d} independently is selected from the group consisting of C₁₋₆ aliphatic, R^{2d}, R^{7d}, -T²-R^{2d}, -T²-R^{7d}, -V-T³-R^{2d}, and -V-T³-R^{7d};
   T² is a C₁₋₆ alkylene chain optionally substituted with R³ or R^{3b}, wherein the alkylene chain optionally is interrupted by -C(R⁵)=C(R⁵)-, -C≡C-, -O-, -S-, -S(O)-, -S(O)₂-, -SO₂N(R⁴)-, -N(R⁴)-, -N(R⁴)C(O) -, -NR⁴C(O)N(R⁴)-, -N(R⁴)CO₂-, -C(O)N(R⁴)-, -C(O)-, -C(O)-C(O)-, -CO₂-, -OC(O)-, -OC(O)O-, -OC(O)N(R⁴)-, -N(R⁴)-N(R⁴)-, -N(R⁴)SO₂-, or -SO₂N(R⁴)-, and wherein T² or a portion thereof optionally forms part of a 3-7 membered ring;
   T³ is a C₁₋₆ alkylene chain optionally substituted with R³ or R^{3b}, wherein the alkylene chain optionally is interrupted
      by -C(R⁵)=C(R⁵)-, -C≡C-, -O-, -S-, -S(O)-, -S(O)₂-, -SO₂N(R⁴)-, -N(R⁴)-, -N(R⁴)C(O) -, -NR⁴C(O)N(R⁴)-, -N(R⁴)CO₂-, -C(O)N(R⁴)-, -C(O)-, -C(O)-C(O)-, -CO₂-, -OC(O)-, -OC(O)O-, -OC(O)N(R⁴)-, -N(R⁴)-N(R⁴)-, -N(R⁴)SO₂-, or -SO₂N(R⁴)-, and wherein T³ or a portion thereof optionally forms part of a 3-7 membered ring;
   V
      is -C(R⁵)=C(R⁵)-, -C≡C-, -O-, -S-, -S(O)-, -S(O)₂-, -SO₂N(R⁴)-, -N(R⁴)-, -N(R⁴)C(O)-, -NR⁴C(O)N(R⁴)-, -N(R⁴)CO₂-, -C(O)N(R⁴)-, -C(O)-, -C(O)-C(O)-, -CO₂-, -OC(O)-, -OC(O)O-, -OC(O)N(R⁴)-, -C(NR⁴)=N-, -C(OR⁵)=N-, -N(R⁴)-N(R⁴)-, -N(R⁴)SO₂-, -N (R⁴)SO₂N(R⁴)-, -P(O)(R⁵)-, -P(O)(OR⁵)-O-, -P(O)-O-, or -P(O)(NR⁵)-N(R⁵)-;
   R^{2d}
      is -halo, -NO₂, -CN, -C(R⁵)=C(R⁵)₂, -C=C-R⁵, -OR⁵, -SR⁶, -S(O)R⁶, -SO₂R⁶, -SO₂N(R ⁴)₂, -N(R⁴)₂, -NR⁴C(O)R⁵, -NR⁴C(O)N(R⁴)₂, -NR⁴CO₂R⁶, -O-CO₂R⁵, -OC(O)N(R⁴)₂, - O-C(O)R⁵, -CO₂R⁵, -C(O)-C(O)R⁵, -C(O)R⁵, -C(O)N(R⁴)₂, -C(=NR⁴)-N(R⁴)₂, -C(=N R⁴)-OR⁵, -N(R⁴)-N(R⁴)₂, -N(R⁴)C(=NR⁴)-N(R⁴)₂, -N(R⁴)SO₂R⁶, -N(R⁴)SO₂N(R⁴)₂, -P( O)(R⁵)₂, or -P(O)(OR⁵)₂; and
   each R^{7d} independently is an optionally substituted aryl, heterocyclyl, or heteroaryl group. each R³ independently is selected from the group consisting
      of -halo, -OH, -O(C₁₋₃ alkyl), -CN, -N(R⁴)₂, -C(O)(C₁₋₃ alkyl), -CO₂H, -CO₂(C₁₋₃ alkyl), -C(O) NH₂, and -C(O)NH(C₁₋₃ alkyl);
each R^{3b} independently is a C₁₋₃ aliphatic optionally substituted with R³ or R⁷, or two substituents R^{3b} on the same carbon atom, taken together with the carbon atom to which they are attached, form a 3- to 6-membered carbocyclic ring;
each R⁴ independently is hydrogen or an optionally substituted aliphatic, aryl, heteroaryl, or heterocyclyl group; or two R⁴ on the same nitrogen atom, taken together with the nitrogen atom, form an optionally substituted 5- to 8-membered heteroaryl or heterocyclyl ring having, in addition to the nitrogen atom, 0-2 ring heteroatoms selected from N, O, and S;
each R⁵ independently is hydrogen or an optionally substituted aliphatic, aryl, heteroaryl, or heterocyclyl group;
each R⁶ independently is an optionally substituted aliphatic or aryl group; and
each R⁷ independently is an optionally substituted aryl, heterocyclyl, or heteroaryl group.

Table 1 provides the chemical names for specific examples of compounds of formula (*II*).

**Table 1. Examples of Compounds of Formula (II)**

| | |
|---|---|
| II-1 | 4-[9-Chloro-7-(2-fluoro-phenyl)-5*H*-benzo[c]pyrimido[4,5-e]azepin-2-ylamino]-*N*-(2-methylamino-ethyl)-benzamide |
| II-2 | *N*-(2-Amino-ethyl)-4-[9-chloro-7-(2-fluoro-phenyl)-5*H*-benzo[c]pyrimido-[4,5-e]azepin-2-ylamino]-*N*-methyl-benzamide |
| II-3 | 4-[9-Chloro-7-(2-fluoro-phenyl)-5*H*-benzo[c]pyrimido[4,5-e]azepin-2-ylamino]-*N*-methyl-*N*-(2-methylamino-ethyl)-benzamide |
| II-4 | 4-[9-Chloro-7-(2-fluoro-phenyl)-5*H*-benzo[c]pyrimido[4,5-e]azepin-2-ylamino]-*N*-(2-dimethylamino-ethyl)-benzamide |
| II-5 | 4-[9-Chloro-7-(2-fluoro-phenyl)-5*H*-benzo[c]pyrimido[4,5-e]azepin-2-ylamino]-*N*-(2-dimethylamino-ethyl)-*N*-methyl-benzamide |
| II-6 | 4-[9-Chloro-7-(2-fluoro-phenyl)-5*H*-benzo[c]pyrimido[4,5-e]azepin-2-ylamino]-*N*-(3-dimethylamino-propyl)-benzamide |
| II-7 | 4-[9-Chloro-7-(2-fluoro-phenyl)-5*H*-benzo[c]pyrimido[4,5-e]azepin-2-ylamino]-*N*-(3-dimethylamino-propyl)-*N*-methyl-benzamide |
| II-8 | {4-[9-Chloro-7-(2-fluoro-phenyl)-5*H*-benzo[c]pyrimido[4,5-e]azepin-2-ylamino]-phenyl}-piperazin-1-yl-methanone |
| II-9 | {4-[9-Chloro-7-(2-fluoro-phenyl)-5*H*-benzo[c]pyrimido[4,5-e]azepin-2-ylamino]-phenyl}-(4-methyl-piperazin-1-yl)-methanone |
| II-10 | {4-[9-Chloro-7-(2-chloro-phenyl)-5*H*-benzo[c]pyrimido[4,5-e]azepin-2-ylamino]-phenyl}-(4-methyl-piperazin-1-yl)-methanone |
| II-11 | [4-(9-Chloro-7-o-tolyl-5*H*-benzo[c]pyrimido[4,5-e]azepin-2-ylamino)-phenyl]-(4-methyl-piperazin-1-yl)-methanone |
| II-12 | {4-[9-Chloro-7-(2-methoxy-phenyl)-5*H*-benzo[c]pyrimido[4,5-e]azepin-2-ylamino]-phenyl}-(4-methyl-piperazin-1-yl)-methanone |
| II-13 | {4-[9-Chloro-7-(4-fluoro-phenyl)-5*H*-benzo[c]pyrimido[4,5-e]azepin-2-ylamino]-phenyl}-(4-methyl-piperazin-1-yl)-methanone |
| II-14 | {4-[7-(2-Fluoro-phenyl)-9-methyl-5*H*-benzo[c]pyrimido[4,5-e]azepin-2-ylamino]-phenyl}-(4-methyl-piperazin-1-yl)-methanone |
| II-15 | 2-{3-[9-Chloro-7-(2-fluoro-phenyl)-5*H*-benzo[c]pyrimido[4,5-e]azepin-2-ylamino]-phenyl}-1-(4-methyl-piperazin-1-yl)-ethanone |
| II-16 | 4-[9-Chloro-7-(2-fluoro-phenyl)-5*H*-benzo[c]pyrimido[4,5-e]azepin-2-ylamino]-*N*-piperidin-4-yl-benzamide |
| II-17 | (4-Amino-piperidin-1-yl)-{4-[9-chloro-7-(2-fluoro-phenyl)-5*H-*benzo[c] pyrimido [4,5-e]azepin-2-ylamino]-phenyl}-methanone |
| II-18 | {4-[9-Chloro-7-(2-fluoro-phenyl)-5*H*-benzo[c]pyrimido[4,5-e]azepin-2-ylamino]-phenyl}-(4-dimethylamino-piperidin-1-yl)-methanone |
| II-19 | 4-[9-Chloro-7-(2-fluoro-phenyl)-5*H*-benzo[c]pyrimido[4,5-e]azepin-2-ylamino]-*N*-[3-(4-methyl-piperazin-1-yl)-propyl]-benzamide |
| II-20 | 4-[9-Chloro-7-(2-chloro-phenyl)-5*H*-benzo[c]pyrimido[4,5-e]azepin-2-ylamino]-*N*-[3-(4-methyl-piperazin-1-yl)-propyl]-benzamide |
| II-21 | 4-(9-Chloro-7-o-tolyl-5*H*-benzo[c]pyrimido[4,5-e]azepin-2-ylamino)-*N*-[3-(4-methyl-piperazin-1-yl)-propyl]-benzamide |
| II-22 | 4-[9-Chloro-7-(2-methoxy-phenyl)-5*H*-benzo[c]pyrimido[4,5-e]azepin-2-ylamino]-N-[3-(4-methyl-piperazin-1-yl)-propyl]-benzamide |
| II-23 | 4-[9-Chloro-7-(4-fluoro-phenyl)-5*H*-benzo[c]pyrimido[4,5-e]azepin-2-ylamino]-*N*-[3-(4-methyl-piperazin-1-yl)-propyl]-benzamide |
| II-24 | 4-[7-(2-Fluoro-phenyl)-9-methyl-5*H*-benzo[c]pyrimido[4,5-e]azepin-2-ylamino]-*N-*[3-(4-methyl-piperazin-1-yl)-propyl]-benzamide |
| II-25 | 2-{3-[9-Chloro-7-(2-fluoro-phenyl)-5*H*-benzo[c]pyrimido[4,5-e]azepin-2-ylamino]-phenyl}-*N*-[3-(4-methyl-piperazin-1-yl)-propyl]-acetamide |
| II-26 | {4-[9-Chloro-7-(2-fluoro-phenyl)-5*H*-benzo[c]pyrimido[4,5-e]azepin-2-ylamino]-phenyl}-morpholin-4-yl-methanone |
| II-27 | 4-[9-Chloro-7-(2-fluoro-phenyl)-5*H*-benzo[c]pyrimido[4,5-e]azepin-2-ylamino]-*N,N*-bis-(2-hydroxy-ethyl)-benzamide |
| II-28 | {4-[9-Chloro-7-(2-chloro-phenyl)-5*H*-benzo[c]pyrimido[4,5-e]azepin-2-ylamino]-phenyl}-morpholin-4-yl-methanone |
| II-29 | 4-[9-Chloro-7-(2-fluoro-phenyl)-5*H*-benzo[c]pyrimido[4,5-e]azepin-2-ylamino]-*N*-(2-morpholin-4-yl-ethyl)-benzamide |
| II-30 | 4-[9-Chloro-7-(2-chloro-phenyl)-5*H*-benzo[c]pyrimido[4,5-e]azepin-2-ylamino]-*N*-(2-morpholin-4-yl-ethyl)-benzamide |
| II-31 | 4-(9-Chloro-7-o-tolyl-5*H*-benzo[c]pyrimido[4,5-e]azepin-2-ylamino)-*N*-(2-morpholin-4-yl-ethyl)-benzamide |
| II-32 | 4-[9-Chloro-7-(2-methoxy-phenyl)-5*H*-benzo[c]pyrimido[4,5-e]azepin-2-ylamino]-*N*-(3-morpholin-4-yl-propyl)-benzamide |
| II-33 | 4-[9-Chloro-7-(4-fluoro-phenyl)-5*H*-benzo[c]pyrimido[4,5-e]azepin-2-ylamino]-*N*-(2-morpholin-4-yl-ethyl)-benzamide |
| II-34 | 4-[9-Chloro-7-(2-chloro-phenyl)-5*H*-benzo[c]pyrimido[4,5-e]azepin-2-ylamino]-2-hydroxy-*N*-(2-morpholin-4-yl-ethyl)-benzamide |
| II-35 | [9-Chloro-7-(2-chloro-phenyl)-5*H*-benzo[c]pyrimido[4,5-e]azepin-2-yl]-pyridin-2-yl-amine |
| II-36 | [9-Chloro-7-(2-fluoro-phenyl)-5*H*-benzo[c]pyrimido[4,5-e]azepin-2-yl]-(3,5-dichloro-phenyl)-amine |
| II-37 | [9-Chloro-7-(2-fluoro-phenyl)-5*H*-benzo[c]pyrimido[4,5-e]azepin-2-yl]-(4-methoxy-phenyl)-amine |
| II-38 | [9-Chloro-7-(2-fluoro-phenyl)-5*H*-benzo[c]pyrimido[4,5-e]azepin-2-yl]-(4-ethoxy-phenyl)-amine |
| II-39 | [9-Chloro-7-(2-fluoro-phenyl)-5*H*-benzo[c]pyrimido[4,5-e]azepin-2-yl]-(3-methoxy-phenyl)-amine |
| II-40 | [9-Chloro-7-(2-fluoro-phenyl)-5*H*-benzo[c]pyrimido[4,5-e]azepin-2-yl]-(2-methoxy-phenyl)-amine |
| II-41 | [9-Chloro-7-(2-fluoro-phenyl)-5*H*-benzo[c]pyrimido[4,5-e]azepin-2-yl]-(4-chloro-phenyl)-amine |
| II-42 | [9-Chloro-7-(2-chloro-phenyl)-5*H*-benzo[c]pyrimido[4,5-e]azepin-2-yl]-(4-chloro-phenyl)-amine |
| II-43 | [9-Chloro-7-(2-fluoro-phenyl)-5*H*-benzo[c]pyrimido[4,5-e]azepin-2-yl]-(3-chloro-phenyl)-amine |
| II-44 | [9-Chloro-7-(2-fluoro-phenyl)-5*H*-benzo[c]pyrimido[4,5-e]azepin-2-yl]-(2-chloro-phenyl)-amine |
| II-45 | 4-[9-Chloro-7-(2-chloro-phenyl)-5*H*-benzo[c]pyrimido[4,5-e]azepin-2-ylamino]-phenol |
| II-46 | [9-Chloro-7-(2-fluoro-phenyl)-5*H*-benzo[c]pyrimido[4,5-e]azepin-2-yl]-(4-morpholin-4-yl-phenyl)-amine |
| II-47 | [9-Chloro-7-(2-fluoro-phenyl)-5*H*-benzo[c]pyrimido[4,5-e]azepin-2-yl]-[4-(4-methyl-piperazin-1-yl)-phenyl]-amine |
| II-48 | [9-Chloro-7-(2-fluoro-phenyl)-5*H*-benzo[c]pyrimido[4,5-e]azepin-2-yl]-(4-pyridin-4-ylmethyl-phenyl)-amine |
| II-49 | 4-[9-Chloro-7-(2-fluoro-phenyl)-5*H*-benzo[c]pyrimido[4,5-e]azepin-2-ylamino]-benzonitrile |
| II-50 | [9-Chloro-7-(2-fluoro-phenyl)-5*H*-benzo[c]pyrimido[4,5-e]azepin-2-yl]-(4-nitro-phenyl)-amine |
| II-51 | 4-[7-(2-Fluoro-phenyl)-5*H*-benzo[c]pyrimido[4,5-e]azepin-2-ylamino]-benzoic acid |
| II-52 | 4-[9-Chloro-7-(2-fluoro-phenyl)-5*H*-benzo[c]pyrimido[4,5-e]azepin-2-ylamino]-benzoic acid |
| II-53 | 4-[9-Chloro-7-(2-chloro-phenyl)-5*H*-benzo[c]pyrimido[4,5-e]azepin-2-ylamino]-benzoic acid |
| II-54 | 4-(9-Chloro-7-o-tolyl-5*H*-benzo[c]pyrimido[4,5-e]azepin-2-ylamino)-benzoic acid |
| II-55 | 4-[9-Chloro-7-(2-methoxy-phenyl)-5*H*-benzo[c]pyrimido[4,5-e]azepin-2-ylamino]-benzoic acid |
| II-56 | 4-[9-Chloro-7-(4-fluoro-phenyl)-5*H*-benzo[c]pyrimido[4,5-e]azepin-2-ylamino]-benzoic acid |
| II-57 | 4-[9-Fluoro-7-(2-fluoro-phenyl)-5*H*-benzo[c]pyrimido[4,5-e]azepin-2-ylamino]-benzoic acid |
| II-58 | 4-[7-(2-Fluoro-phenyl)-9-methyl-5*H*-benzo[c]pyrimido[4,5-e]azepin-2-ylamino]-benzoic acid |
| II-59 | 4-[10-Fluoro-7-(2-fluoro-phenyl)-5*H*-benzo[c]pyrimido[4,5-e]azepin-2-ylamino]-benzoic acid |
| II-60 | 4-[10-Chloro-7-(2-fluoro-phenyl)-5*H*-benzo[c]pyrimido[4,5-e]azepin-2-ylamino]-benzoic acid |
| II-61 | 4-[10-Bromo-7-(2-fluoro-phenyl)-5*H*-benzo[c]pyrimido[4,5-e]azepin-2-ylamino]-benzoic acid |
| II-62 | 4-[7-(2-Fluoro-phenyl)-10-methoxy-5*H*-benzo[c]pyrimido[4,5-e]azepin-2-ylamino]-benzoic acid |
| II-63 | 4-[9-Chloro-7-(2-chloro-phenyl)-5*H*-benzo[c]pyrimido[4,5-e]azepin-2-ylamino]-benzamide |
| II-64 | 3-[9-Chloro-7-(2-chloro-phenyl)-5*H*-benzo[c]pyrimido[4,5-e]azepin-2-ylamino]-benzamide |
| II-65 | {3-[9-Chloro-7-(2-chloro-phenyl)-5*H*-benzo[c]pyrimido[4,5-e]azepin-2-ylamino]-phenyl}-acetic acid |
| II-66 | 2-{3-[9-Chloro-7-(2-chloro-phenyl)-5*H*-benzo[c]pyrimido[4,5-e]azepin-2-ylamino]-phenyl}-acetamide |
| II-67 | 4-[9-Chloro-7-(2-fluoro-phenyl)-5*H*-benzo[c]pyrimido[4,5-e]azepin-2-ylamino]-benzenesulfonic acid |
| II-68 | 4-[9-Chloro-7-(2-fluoro-phenyl)-5*H*-benzo[c]pyrimido[4,5-e]azepin-2-ylamino]-benzenesulfonamide |
| II-69 | 4-[9-Chloro-7-(2-chloro-phenyl)-5*H*-benzo[c]pyrimido[4,5-e]azepin-2-ylamino]-*N*-(5-methyl-isoxazol-3-yl)-benzenesulfonamide |
| II-70 | [9-Chloro-7-(2-fluoro-phenyl)-5*H*-benzo[c]pyrimido[4,5-e]azepin-2-yl]-(4-trifluoromethanesulfonyl-phenyl)-amine |
| II-71 | [9-Chloro-7-(2-fluoro-phenyl)-5*H*-benzo[c]pyrimido[4,5-e]azepin-2-yl]-(3,4-dimethoxy-phenyl)-amine |
| II-72 | [9-Chloro-7-(2-fluoro-phenyl)-6,7-dihydro-5*H*-benzo[c]pyrimido[4,5-e]azepin-2-yl]-(3,4-dimethoxy-phenyl)-amine |
| II-73 | [9-Chloro-7-(2-chloro-phenyl)-5*H*-benzo[c]pyrimido[4,5-e]azepin-2-yl]-(3,4-dimethoxy-phenyl)-amine |
| II-74 | (9-Chloro-7-o-tolyl-5*H*-benzo[c]pyrimido[4,5-e]azepin-2-yl)-(3,4-dimethoxy-phenyl)-amine |
| II-75 | (3,4-Dimethoxy-phenyl)-[7-(2-fluoro-phenyl)-9-methyl-5*H*-benzo[c]pyrimido-[4,5-e]azepin-2-yl]-amine |
| II-76 | (3,4-Dimethoxy-phenyl)-[7-(2-fluoro-phenyl)-9-isopropyl-5*H-*benzo[c] pyrimido [4,5-e]azepin-2-yl]-amine |
| II-77 | (3,4-Dimethoxy-phenyl)-[10-fluoro-7-(2-fluoro-phenyl)-5*H*-benzo[c]pyrimido-[4,5-e]azepin-2-yl]-amine |
| II-78 | [10-Bromo-7-(2-fluoro-phenyl)-5*H*-benzo[c]pyrimido[4,5-e]azepin-2-yl]-(3,4-dimethoxy-phenyl)-amine |
| II-79 | (3,4-Dimethoxy-phenyl)-[7-(2-fluoro-phenyl)-10-trifluoromethyl-5*H-*benzo[c] pyrimido [4,5-e]azepin-2-yl]-amine |
| II-80 | (3,4-Dimethoxy-phenyl)-[7-(2-fluoro-phenyl)-10-methyl-5*H*-benzo[c]pyrimido-[4,5-e]azepin-2-yl]-amine |
| II-81 | (3,4-Dimethoxy-phenyl)-[7-(2-fluoro-phenyl)-10-methoxy-5*H-*benzo[c] pyrimido [4,5-e]azepin-2-yl]-amine |
| II-82 | (3,4-Dimethoxy-phenyl)-[7-(2-fluoro-phenyl)-11-methyl-5*H*-benzo[c]pyrimido-[4,5-e]azepin-2-yl]-amine |
| II-83 | [9-Chloro-7-(2-fluoro-phenyl)-5*H*-benzo[c]pyrimido[4,5-e]azepin-2-yl]-(2,3-dihydro-benzo[1,4]dioxin-6-yl)-amine |
| II-84 | [9-Chloro-7-(2-fluoro-phenyl)-5*H*-benzo[c]pyrimido[4,5-e]azepin-2-yl]-(4-fluoro-3-methoxy-phenyl)-amine |
| II-85 | 4-[9-Chloro-7-(2-fluoro-phenyl)-5*H*-benzo[c]pyrimido[4,5-e]azepin-2-ylamino]-2-hydroxy-benzoic acid |
| II-86 | 4-[9-Chloro-7-(2-chloro-phenyl)-5*H*-benzo[c]pyrimido[4,5-e]azepin-2-ylamino]-2-hydroxy-benzoic acid |
| II-87 | [9-Chloro-7-(2-fluoro-phenyl)-5*H*-benzo[c]pyrimido[4,5-e]azepin-2-yl]-(3,4-dichloro-phenyl)-amine |
| II-88 | [9-Chloro-7-(2-chloro-phenyl)-5*H*-benzo[c]pyrimido[4,5-e]azepin-2-yl]-(3,5-dimethoxy-phenyl)-amine |
| II-89 | [9-Chloro-7-(2-fluoro-phenyl)-5*H*-benzo[c]pyrimido[4,5-e]azepin-2-yl]-(3,5-dimethyl-phenyl)-amine |
| II-90 | [9-Chloro-7-(2-fluoro-phenyl)-5H-benzo[c]pyrimido[4,5-e]azepin-2-yl]-phenyl-amine |
| II-91 | 4-[9-Chloro-7-(2,5-difluoro-phenyl)-5H-benzo[c]pyrimido[4,5-e]azepin-2-ylamino]-benzoic acid |
| II-92 | 4-[9-Chloro-7-(2,3-difluoro-phenyl)-5H-benzo[c]pyrimido[4,5-e]azepin-2-ylamino]-benzoic acid |
| II-93 | (3-Dimethylamino-pyrrolidin-1-yl)-{4-[7-(2-fluoro-phenyl)-9-methoxy-5H-benzo[c] pyrimido [4,5-e]azepin-2-ylamino]-phenyl}-methanone |
| II-94 | 4-[9-Chloro-7-(2,5-dimethoxy-phenyl)-5H-benzo[c]pyrimido[4,5-e]azepin-2-ylamino]-benzoic acid |
| II-95 | 4-[7-(2-Fluoro-phenyl)-9-methoxy-5H-benzo[c]pyrimido[4,5-e]azepin-2-ylamino]-N,N-bis-(2-hydroxy-ethyl)-benzamide |
| II-96 | 4-[9-Chloro-7-(2,4-difluoro-phenyl)-5H-benzo[c]pyrimido[4,5-e]azepin-2-ylamino]-benzoic acid |
| II-97 | 4-[9-Chloro-7-(2,4-difluoro-phenyl)-7H-benzo[c]pyrimido[4,5-e]azepin-2-ylamino]-benzoic acid |
| II-98 | {4-[9-Chloro-7-(2-fluoro-phenyl)-5H-benzo[c]pyrimido[4,5-e]azepin-2-ylamino]-phenyl}-(3-dimethylamino-azetidin-1-yl)-methanone |
| II-99 | 4-[9-Chloro-7-(2-fluoro-phenyl)-5H-benzo[c]pyrimido[4,5-e]azepin-2-ylamino]-N-methyl-N-(1-methyl-pyrrolidin-3-yl)-benzamide |
| II-100 | {4-[9-Chloro-7-(2-fluoro-phenyl)-5H-benzo[c]pyrimido[4,5-e]azepin-2-ylamino]-phenyl}-(3-dimethylamino-pyrrolidin-1-yl)-methanone |
| II-101 | 4-[9-Chloro-7-(2,4-dimethoxy-phenyl)-5H-benzo[c]pyrimido[4,5-e]azepin-2-ylamino]-benzoic acid |
| II-102 | {4-[9-Chloro-7-(2-fluoro-phenyl)-5H-benzo[c]pyrimido[4,5-e]azepin-2-ylamino]-phenyl}-(3-methylamino-pyrrolidin-1-yl)-methanone |
| II-103 | (3-Amino-pyrrolidin-1-yl)-{4-[9-chloro-7-(2-fluoro-pheny1)-5H-benzo[c] pyrimido [4,5-e]azepin-2-ylamino]-phenyl}-methanone |
| II-104 | 4-[9-Chloro-7-(2,3-difluoro-phenyl)-5H-benzo[c]pyrimido[4,5-e]azepin-2-ylamino]-benzoic acid methyl ester |
| II-105 | 4-[9-Chloro-7-(2,5-difluoro-phenyl)-5H-benzo[c]pyrimido[4,5-e]azepin-2-ylamino]-benzoic acid methyl ester |
| II-106 | {4-[9-Chloro-7-(2-fluoro-phenyl)-5H-benzo[c]pyrimido[4,5-e]azepin-2-ylamino]-phenyl}-phosphonic acid |
| II-107 | N-{4-[9-Chloro-7-(2-fluoro-phenyl)-5H-benzo[c]pyrimido[4,5-e]azepin-2-ylamino]-phenyl}-methanesulfonamide |
| II-108 | N-{4-[9-Chloro-7-(2-fluoro-phenyl)-5H-benzo[c]pyrimido[4,5-e]azepin-2-ylamino]-phenyl}-N-methyl-acetamide |
| II-109 | 2-{4-[9-Chloro-7-(2-fluoro-phenyl)-5H-benzo[c]pyrimido[4,5-e]azepin-2-ylamino]-benzoylamino}-succinic acid |
| II-110 | [9-Chloro-7-(2-fluoro-phenyl)-4-methyl-5H-benzo[c]pyrimido[4,5-e]azepin-2-yl]-(3,4-dimethoxy-phenyl)-amine |
| II-111 | {4-[9-Chloro-7-(2-fluoro-phenyl)-5H-benzo[c]pyrimido[4,5-e]azepin-2-ylamino]-phenyl}-(3,5-dimethyl-piperazin-1-yl)-methanone |
| II-112 | 1-{4-[9-Chloro-7-(2-fluoro-phenyl)-5H-benzo[c]pyrimido[4,5-e]azepin-2-ylamino]-benzoyl}-pyrrolidine-2-carboxylic acid |
| II-113 | {4-[9-Chloro-7-(2-fluoro-phenyl)-5H-benzo[c]pyrimido[4,5-e]azepin-2-ylamino]-phenyl}-(3-methyl-piperazin-1-yl)-methanone |
| II-114 | [9-Chloro-7-(2-fluoro-phenyl)-5H-benzo[c]pyrimido[4,5-e]azepin-2-yl]-[4-(2H-tetrazol-5-yl)-phenyl]-amine |
| II-115 | N-{4-[9-Chloro-7-(2-fluoro-phenyl)-5H-benzo[c]pyrimido[4,5-e]azepin-2-ylamino] -phenyl}-acetamide |
| II-116 | 5-[9-Chloro-7-(2-fluoro-phenyl)-5H-benzo[c]pyrimido[4,5-e]azepin-2-ylamino]-2-fluoro-benzoic acid |
| II-117 | N-(3-Amino-propyl)-4-[9-chloro-7-(2-fluoro-phenyl)-5H-benzo[c]pyrimido-[4,5-e]azepin-2-ylamino]-N-methyl-benzamide |
| II-118 | 2-{4-[9-Chloro-7-(2-fluoro-phenyl)-5H-benzo[c]pyrimido[4,5-e]azepin-2-ylamino]-benzoylamino}-propionic acid |
| II-119 | 5-[9-Chloro-7-(2-fluoro-phenyl)-5H-benzo[c]pyrimido[4,5-e]azepin-2-ylamino]-pyridine-2-carboxylic acid |
| II-120 | 2-{4-[9-Chloro-7-(2-fluoro-phenyl)-5H-benzo[c]pyrimido[4,5-e]azepin-2-ylamino]-phenyl}-N-(2-morpholin-4-yl-ethyl)-acetamide |
| II-121 | 5-[9-Chloro-7-(2-fluoro-phenyl)-5H-benzo[c]pyrimido[4,5-e]azepin-2-ylamino]-2-methoxy-benzoic acid |
| II-122 | 5-[9-Chloro-7-(2-fluoro-phenyl)-5H-benzo[c]pyrimido[4,5-e]azepin-2-ylamino]-2-methyl-benzoic acid |
| II-123 | 6-[9-Chloro-7-(2-fluoro-phenyl)-5H-benzo[c]pyrimido[4,5-e]azepin-2-ylamino]-nicotinic acid |
| II-124 | 4-[9-Chloro-7-(2-fluoro-phenyl)-5H-benzo[c]pyrimido[4,5-e]azepin-2-ylamino]-N-(2-morpholin-4-yl-ethyl)-benzenesulfonamide |
| II-125 | 2-Chloro-5-[9-chloro-7-(2-fluoro-phenyl)-5H-benzo[c]pyrimido[4,5-e]azepin-2-ylamino]-benzoic acid |
| II-126 | {4-[9-Chloro-7-(2-fluoro-phenyl)-5H-benzo[c]pyrimido[4,5-e]azepin-2-ylamino]-phenyl}-acetic acid |
| II-127 | 4-[9-Chloro-7-(2-fluoro-phenyl)-5H-benzo[c]pyrimido[4,5-e]azepin-2-ylamino]-2-trifluoromethyl-benzoic acid |
| II-128 | 4-[9-Chloro-7-(2-fluoro-phenyl)-5H-benzo[c]pyrimido[4,5-e]azepin-2-ylamino]-N-methyl-N-(1-methyl-piperidin-4-yl)-benzamide |
| II-129 | N-(3-Amino-propyl)-4-[9-chloro-7-(2-fluoro-phenyl)-5H-benzo[c]pyrimido-[4,5-e]azepin-2-ylamino]-benzamide |
| II-130 | 4-[9-Chloro-7-(2-fluoro-phenyl)-5H-benzo[c]pyrimido[4,5-e]azepin-2-ylamino]-N-(3-methylamino-propyl)-benzamide |
| II-131 | N-(2-Amino-2-methyl-propyl)-4-[9-chloro-7-(2-fluoro-phenyl)-5H-benzo[c] pyrimido [4,5-e]azepin-2-ylamino]-benzamide |
| II-132 | 2-(3,4-Dimethoxy-phenylamino)-7-(2-fluoro-phenyl)-5H-benzo[c]pyrimido-[4,5-e]azepine-10-carboxylic acid |
| II-133 | 4-[9-Chloro-7-(2-fluoro-phenyl)-5H-benzo[c]pyrimido[4,5-e]azepin-2-ylamino]-2-methyl-benzoic acid |
| II-134 | 2-Chloro-4-[9-chloro-7-(2-fluoro-phenyl)-5H-benzo[c]pyrimido[4,5-e]azepin-2-ylamino]-benzoic acid |
| II-135 | 4-[9-Chloro-7-(2,6-difluoro-phenyl)-5H-benzo[c]pyrimido[4,5-e]azepin-2-ylamino]-benzoic acid |
| II-136 | 4-[9-Chloro-7-(2-fluoro-phenyl)-5H-benzo[c]pyrimido[4,5-e]azepin-2-ylamino]-2-fluoro-benzoic acid |
| II-137 | 4-[7-(2-Fluoro-phenyl)-9-methoxy-5H-benzo[c]pyrimido[4,5-e]azepin-2-ylamino]-benzoic acid |
| II-138 | (3,4-Dimethoxy-phenyl)-[7-(2-fluoro-phenyl)-9-methoxy-5H-benzo[c]pyrimido-[4,5-e]azepin-2-yl]-amine |
| II-139 | [9,10-Dichloro-7-(2-fluoro-phenyl)-5H-benzo[c]pyrimido[4,5-e]azepin-2-yl]-(3,4-dimethoxy-phenyl)-amine |
| II-140 | 4-[9,10-Dichloro-7-(2-fluoro-phenyl)-5H-benzo[c]pyrimido[4,5-e]azepin-2-ylamino]-benzoic acid |
| II-141 | 4-[9-Chloro-7-(2-fluoro-phenyl)-5H-benzo[c]pyrimido[4,5-e]azepin-2-ylamino]-2-methoxy-benzoic acid |
| II-142 | N-(2-Amino-ethyl)-4-[9-chloro-7-(2-fluoro-phenyl)-5H-benzo[c]pyrimido-[4,5-e]azepin-2-ylamino]-benzamide |
| II-143 | 4-(9-Chloro-7-phenyl-5H-benzo[c]pyrimido[4,5-e]azepin-2-ylamino)-benzoic acid |
| II-144 | [7-(2-Bromo-phenyl)-9-chloro-5H-benzo[c]pyrimido[4,5-e]azepin-2-yl]-(3,4-dimethoxy-phenyl)-amine |
| II-145 | 2-{4-[9-Chloro-7-(2-fluoro-phenyl)-5H-benzo[c]pyrimido[4,5-e]azepin-2-ylamino]-phenyl}-1-(4-methyl-piperazin-1-yl)-ethanone |
| II-146 | 3-[9-Chloro-7-(2-fluoro-phenyl)-5H-benzo[c]pyrimido[4,5-e]azepin-2-ylamino]-benzoic acid |
| II-147 | 4-[9-Chloro-7-(2-fluoro-phenyl)-5H-benzo[c]pyrimido[4,5-e]azepin-2-ylamino]-N-[2-(1H-imidazol-4-yl)-ethyl]-benzamide |
| II-148 | 4-[7-(2-Fluoro-phenyl)-9-methyl-5H-benzo[c]pyrimido[4,5-e]azepin-2-ylamino]-N-(2-morpholin-4-yl-ethyl)-benzamide |
| II-149 | {3-[9-Chloro-7-(2-fluoro-phenyl)-5H-benzo[c]pyrimido[4,5-e]azepin-2-ylamino]-phenyl}-acetic acid |
| II-150 | 4-[9-Chloro-7-(2-fluoro-phenyl)-5H-benzo[c]pyrimido[4,5-e]azepin-2-ylamino]-N-(2-pyridin-4-yl-ethyl)-benzamide |
| II-151 | 4-[9-Chloro-7-(2-fluoro-phenyl)-5H-benzo[c]pyrimido[4,5-e]azepin-2-ylamino]-N-(2-pyridin-3-yl-ethyl)-benzamide |
| II-152 | (9-Chloro-7-phenyl-5H-benzo[c]pyrimido[4,5-e]azepin-2-yl)-(3,4-dimethoxy-phenyl)-amine |
| II-153 | 4-[7-(2-Fluoro-phenyl)-10-methyl-5H-benzo[c]pyrimido[4,5-e]azepin-2-ylamino]-benzoic acid |
| II-154 | (3,4-Dimethoxy-phenyl)-[7-(2-fluoro-phenyl)-5H-benzo[c]pyrimido-[4,5-e]azepin-2-yl]-amine |
| II-155 | 4-[9-Chloro-7-(4-methoxy-phenyl)-5H-benzo[c]pyrimido[4,5-e]azepin-2-ylamino]-benzoic acid |
| II-156 | 4-[9-Chloro-7-(3-methoxy-phenyl)-5H-benzo[c]pyrimido[4,5-e]azepin-2-ylamino]-benzoic acid |
| II-157 | 4-[9-Chloro-7-(3-fluoro-phenyl)-5H-benzo[c]pyrimido[4,5-e]azepin-2-ylamino]-N-[3-(4-methyl-piperazin-1-yl)-propyl]-benzamide |
| II-158 | 4-[9-Chloro-7-(3-fluoro-phenyl)-5H-benzo[c]pyrimido[4,5-e]azepin-2-ylamino]-N-(2-morpholin-4-yl-ethyl)-benzamide |
| II-159 | {4-[9-Chloro-7-(3-fluoro-phenyl)-5H-benzo[c]pyrimido[4,5-e]azepin-2-ylamino]-phenyl}-(4-methyl-piperazin-1-yl)-methanone |
| II-160 | 4-[9-Chloro-7-(2-fluoro-phenyl)-5H-benzo[c]pyrimido[4,5-e]azepin-2-ylamino]-N-methyl-N-(2-pyridin-2-yl-ethyl)-benzamide |
| II-161 | 4-[9-Chloro-7-(2-fluoro-phenyl)-5H-benzo[c]pyrimido[4,5-e]azepin-2-ylamino]-N-(2-pyridin-2-yl-ethyl)-benzamide |
| II-162 | 4-[9-Chloro-7-(3-fluoro-phenyl)-5H-benzo[c]pyrimido[4,5-e]azepin-2-ylamino]-benzoic acid |
| II-163 | {3-[9-Chloro-7-(2-fluoro-phenyl)-5H-benzo[c]pyrimido[4,5-e]azepin-2-ylamino]-phenyl}-(4-methyl-piperazin-1-yl)-methanone |
| II-164 | 9-Chloro-7-(2-fluorophenyl)-*N*-{4-[(4-pyridin-2-ylpiperazin-1-yl)carbonyl]phenyl}-5*N*-pyrimido[5,4-*d*][2]benzazepin-2-amine |
| II-165 | 9-Chloro-7-(2-fluorophenyl)-*N*-(4-{[4-(2-morpholin-4-yl-2-oxoethyl)piperazin-1-yl]carbonyl}phenyl)-5*H*-pyrimido[5,4-*d*][2]benzazepin-2-amine |
| II-166 | 9-Chloro-7-(2-fluorophenyl-*N*-(4-{[4-(2-furoyl)piperazin-1-yl]carbonyl}phenyl)-5*H*-pyrimido[5,4-*d*][2]benzazepin-2-amine |
| II-167 | Benzyl-4-(4-{[9-chloro-7-(2-fluorophenyl)-5*H*-pyrimido[5,4-*d*][2]benzazepin-2-yl]amino}benzoyl)piperazine-1-carboxylate |
| II-168 | Ethyl-4-(4-{[9-chloro-7-(2-fluorophenyl)-5*H*-pyrimido[5,4-*d*][2]benzazepin-2-yl]amino}benzoyl)piperazine-1-carboxylate |
| II-169 | 2-[4-(4-{[9-Chloro-7-(2-fluorophenyl)-5*H*-pyrimido[5,4-*d*][2]benzazepin-2-yl]amino}benzoyl)piperazin-1-yl]benzoic acid |
| II-170 | 2-[4-(4-{[9-Chloro-7-(2-fluorophenyl)-5*H*-pyrimido[5,4-*d*][2]benzazepin-2-yl]amino}benzoyl)piperazin-1-yl]-*N*-isopropylacetamide |
| II-171 | 9-Chloro-7-(2-fluorophenyl)-*N*-(4-{[4-(2-pyrrolidin-1-ylethyl)piperazin-1-yl]carbonyl}phenyl)-5*H*-pyrimido[5,4-*d*] [2]benzazepin-2-amine |
| II-172 | *N*-[2-(aminocarbonyl)phenyl]-4-{[9-chloro-7-(2-fluorophenyl)-5*H*-pyrimido-[5,4-*d*][2]benzazepin-2-yl]amino}benzamide |
| II-173 | 9-Chloro-7-(2-fluorophenyl)-*N*-{4-[(4-pyrimidin-2-ylpiperazin-1-yl)carbonyl]phenyl}-5*H*-pyrimido[5,4-*d*][2]benzazepin-2-amine |
| II-174 | 4-{[9-Chloro-7-(2-chloro-6-fluorophenyl)-5*H*-pyrimido[5,4-*d*][2]benzazepin-2-yl]amino}benzoic acid |
| II-175 | 9-Chloro-7-(2,6-difluorophenyl)-*N*-{4-[(3,5-dimethylpiperazin-1-yl)carbonyl]phenyl}-5*H*-pyrimido[5,4-*d*][2]benzazepin-2-amine |
| II-176 | 9-Chloro-7-(2,6-difluorophenyl)-*N*-(4-{[3-(dimethylamino)pyrrolidin-1-yl]carbonyl}phenyl)-5*H*-pyrimido[5,4-*d*][2]benzazepin-2-amine |
| II-177 | 9-Chloro-N-{4-[(3,5-dimethylpiperazin-1-yl)carbonyl]phenyl}-7-(2-fluoro-6-methoxyphenyl)-5*H*-pyrimido[5,4-*d*][2]benzazepin-2-amine |
| II-178 | 9-Chloro-*N*-(4-{[3-(dimethylamino)pyrrolidin-1-yl]carbonyl}phenyl)-7-(2-fluoro-6-methoxyphenyl)-5*H*-pyrimido[5,4-*d*][2]benzazepin-2-amine |
| II-179 | 9-Chloro-*N*-(4-{[3-(dimethylamino)azetidin-1-yl]carbonyl}phenyl)-7-(2-fluoro-6-methoxyphenyl)-5*H*-pyrimido[5,4-*d*][2]benzazepin-2-amine |
| II-180 | 9-Chloro-7-(2,6-difluorophenyl)-*N*-(4-{[3-(dimethylamino)azetidin-1-yl]carbonyl}phenyl)-5*H*-pyrimido[5,4-*d*][2]benzazepin-2-amine |
| II-181 | {4-[9-Chloro-7-(2-fluoro-phenyl)-5H-benzo[c]pyrimido[4,5-e]azepin-2-ylamino]-phenyl}-[4-(3-piperidin-1-yl-propyl)-piperazin-1-yl]-methanone |
| II-182 | {4-[9-Chloro-7-(2-fluoro-phenyl)-5H-benzo[c]pyrimido[4,5-e]azepin-2-ylamino]-phenyl}-[4-(2-piperidin-1-yl-ethyl)-piperazin-1-yl]-methanone |
| II-183 | {4-[9-Chloro-7-(2,6-difluoro-phenyl)-5H-benzo[c]pyrimido[4,5-e]azepin-2-ylamino]-phenyl}-(4-dimethylamino-piperidin-1-yl)-methanone |
| II-184 | {4-[9-Chloro-7-(2,6-difluoro-phenyl)-5H-benzo[c]pyrimido[4,5-e]azepin-2-ylamino]-phenyl}-(4-methyl-piperazin-1-yl)-methanone |
| II-185 | 4-[9-Chloro-7-(2,6-difluoro-phenyl)-5H-benzo[c]pyrimido[4,5-e]azepin-2-ylamino]-N-(3-dimethylamino-propyl)-N-methyl-benzamide |
| II-186 | {4-[9-Chloro-7-(2-fluoro-6-methoxy-phenyl)-5H-benzo[c]pyrimido-[4,5-e]azepin-2-ylamino]-phenyl}-(4-dimethylamino-piperidin-1-yl)-methanone |
| II-187 | {4-[9-Chloro-7-(2-fluoro-phenyl)-5H-benzo[c]pyrimido[4,5-e]azepin-2-ylamino]-phenyl}-[4-(2-dipropylamino-ethyl)-piperazin-1-yl]-methanone |
| II-188 | {4-[9-Chloro-7-(2-fluoro-phenyl)-5H-benzo[c]pyrimido[4,5-e]azepin-2-ylamino]-phenyl}-[4-(3-pyrrolidin-1-yl-propyl)-piperazin-1-yl]-methanone |
| II-189 | {4-[9-Chloro-7-(2-fluoro-phenyl)-5H-benzo[c]pyrimido[4,5-e]azepin-2-ylamino]-phenyl}-[4-(2-morpholin-4-yl-ethyl)-piperazin-1-yl]-methanone |
| II-190 | 4-[9-Chloro-7-(2-fluoro-6-methoxy-phenyl)-5H-benzo[c]pyrimido[4,5-e]azepin-2-ylamino]-benzoic acid |
| II-191 | {4-[9-Chloro-7-(2,6-difluoro-phenyl)-5H-benzo[c]pyrimido[4,5-e]azepin-2-ylamino]-phenyl}-(3(S)-methyl-piperazin-1-yl)-methanone |
| II-192 | (3-Amino-azetidin-1-yl)-{4-[9-chloro-7-(2-fluoro-phenyl)-5H-benzo[c]pyrimido[4,5-e]azepin-2-ylamino]-phenyl}-methanone |
| II-193 | {4-[9-Chloro-7-(2-fluoro-phenyl)-5H-benzo[c]pyrimido[4,5-e]azepin-2-ylamino]-phenyl}-(3-dimethylaminomethyl-azetidin-1-yl)-methanone |
| II-194 | {4-[9-Chloro-7-(2,6-difluoro-phenyl)-5H-benzo[c]pyrimido[4,5-e]azepin-2-ylamino]-phenyl}-(3(R)-methyl-piperazin-1-yl)-methanone |
| II-195 | {4-[9-Chloro-7-(2,6-difluoro-phenyl)-5H-benzo[c]pyrimido[4,5-e]azepin-2-ylamino]-phenyl}-piperazin-1-yl-methanone |
| II-196 | (3-Amino-pyrrolidin-1-yl)-{4-[9-chloro-7-(2,6-difluoro-phenyl)-5H-benzo[c]pyrimido[4,5-e]azepin-2-ylamino]-phenyl}-methanone |
| II-197 | {4-[9-Chloro-7-(2,6-difluoro-phenyl)-5H-benzo[c]pyrimido[4,5-e]azepin-2-ylamino]-phenyl}-(3-methylamino-pyrrolidin-1-yl)-methanone |
| II-198 | 4-[9-Chloro-7-(2,6-difluoro-phenyl)-5H-benzo[c]pyrimido[4,5-e]azepin-2-ylamino]-N-methyl-N-(3-methylamino-propyl)-benzamide |
| II-199 | {4-[9-Chloro-7-(2-fluoro-6-methoxy-phenyl)-5H-benzo[c]pyrimido[4,5-e]azepin-2-ylamino]-phenyl}-(3-methylamino-pyrrolidin-1-yl)-methanone |
| II-200 | 4-[9-Chloro-7-(2-fluoro-phenyl)-5H-benzo[c]pyrimido[4,5-e]azepin-2-ylamino]-cyclohexanecarboxylic acid |
| II-201 | 9-chloro-*N*-(4-{[4-(2-ethoxyphenyl)piperazin-1-yl]carbonyl}phenyl)-7-(2-fluorophenyl)-5*H*-pyrimido[5,4-*d*][2]benzazepin-2-amine |
| II-202 | *N*-[amino(imino)methyl]-4-{[9-chloro-7-(2,6-difluorophenyl)-5*H*-pyrimido[5,4-*d*] [2]benzazepin-2-yl]amino}benzamide |
| II-203 | 3-{[9-chloro-7-(2,6-difluorophenyl)-5*H*-pyrimido[5,4-*d*][2]benzazepin-2-yl]amino}benzoic acid |
| II-204 | 9-chloro-7-(2,6-difluorophenyl)-*N*-(3-{[3-(dimethylamino)azetidin-1-yl]carbonyl}phenyl)-5*H*-pyrimido[5,4-*d*][2]benzazepin-2-amine |
| II-205 | 9-chloro-7-(2,6-difluorophenyl)-*N*-(3-{[4-(dimethylamino)piperidin-1-yl]carbonyl}phenyl)-5*H*-pyrimido[5,4-*d*][2]benzazepin-2-amine |
| II-206 | 9-chloro-7-(2,6-difluorophenyl)-*N*-(3-{[3-(dimethylamino)pyrrolidin-1-yl]carbonyl}phenyl)-5*H*-pyrimido[5,4-*d*][2]benzazepin-2-amine |
| II-207 | *N*-[2-(aminomethyl)-1,3-benzoxazol-5-yl]-9-chloro-7-(2,6-difluorophenyl)-5*H-*pyrimido[5,4-*d*][2]benzazepin-2-amine |
| II-208 | 9-chloro-*N*-[4-({4-[3-(diethylamino)propyl]piperazin-1-yl}carbonyl)phenyl]-7-(2-fluorophenyl)-5*H*-pyrimido[5,4-*d*][2]benzazepin-2-amine |
| II-209 | 9-chloro-*N*-[4-({4-[2-(diethylamino)ethyl]piperazin-1-yl}carbonyl)phenyl]-7-(2-fluorophenyl)-5*H*-pyrimido[5,4-*d*][2]benzazepin-2-amine |
| II-210 | 9-chloro-*N*-[4-({4-[3-(dimethylamino)propyl]piperazin-1-yl}carbonyl)phenyl]-7-(2-fluorophenyl)-5*H*-pyrimido[5,4-*d*][2]benzazepin-2-amine |
| II-211 | 9-chloro-7-(2-fluorophenyl)-*N*-[4-({4-[(1-methylpiperidin-3-yl)methyl]piperazin-1-yl}carbonyl)phenyl]-5*H*-pyrimido[5,4-*d*][2]benzazepin-2-amine |
| II-212 | 9-chloro-7-(2,6-difluorophenyl)-*N*-(4-nitrophenyl)-5*H*-pyrimido[5,4-*d*][2]benzazepin-2-amine |
| II-213 | 9-chloro-*N*-(3-chloro-4-{[4-(2-pyrrolidin-1-ylethyl)piperazin-1-yl]carbonyl}phenyl)-7-(2-fluorophenyl)-5*H*-pyrimido[5,4-*d*][2]benzazepin-2-amine |
| II-214 | 9-chloro-*N*-{3-chloro-4-[(3-methylpiperazin-1-yl)carbonyl]phenyl}-7-(2-fluorophenyl)-5*H*-pyrimido[5,4-*d*][2]benzazepin-2-amine |
| II-215 | 9-chloro-*N*-(3-chloro-4-{[3-(dimethylamino)pyrrolidin-1-yl]carbonyl}phenyl)-7-(2,6-difluorophenyl)-5*H*-pyrimido[5,4-*d*][2]benzazepin-2-amine |
| II-216 | 9-chloro-*N*-{3-chloro-4-[(3-methylpiperazin-1-yl)carbonyl]phenyl}-7-(2,6-difluorophenyl)-5*H*-pyrimido[5,4-*d*][2]benzazepin-2-amine |
| II-217 | *N*-[9-chloro-7-(2,6-difluorophenyl)-5*H*-pyrimido[5,4-*d*][2]benzazepin-2-yl]benzene-1,4-diamine |
| II-218 | methyl 2-chloro-4-{[9-chloro-7-(2,6-difluorophenyl)-5*H*-pyrimido[5,4-*d*] [2]benzazepin-2-yl]amino}benzoate |
| II-219 | 1-(4-{[9-chloro-7-(2,6-difluorophenyl)-5*H*-pyrimido[5,4-*d*][2]benzazepin-2-yl]amino}benzoyl)piperazine-2-carboxylic acid |
| II-220 | 9-chloro-7-(2,6-difluorophenyl)-*N*-(4-{[4-(methylamino)piperidin-1-yl]carbonyl}phenyl)-5*H*-pyrimido[5,4-*d*][2]benzazepin-2-amine |
| II-221 | *N*-{4-[(3-aminopiperidin-1-yl)carbonyl]pheny1}-9-chloro-7-(2,6-difluorophenyl)-5*H*-pyrimido[5,4-*d*][2]benzazepin-2-amine |
| II-222 | 9-chloro-7-(2,6-difluorophenyl)-*N*-{3-[(3,5-dimethylpiperazin-1-yl)carbonyl]phenyl}-5*H*-pyrimido[5,4-*d*][2]benzazepin-2-amine |
| II-223 | 4-{[9-chloro-7-(2,6-difluorophenyl)-5*H*-pyrimido[5,4-*d*][2]benzazepin-2-yl]amino}-*N*-[[4-(dimethylamino)piperidin-1-yl](imino)methyl]benzamide |
| II-224 | 4-{[9-chloro-7-(2,6-difluorophenyl)-5*H*-pyrimido[5,4-*d*][2]benzazepin-2-yl]amino}-*N*-[imino(piperazin-1-yl)methyl]benzamide |
| II-225 | 4-{[9-chloro-7-(2-fluoro-6-methoxyphenyl)-5*H*-pyrimido[5,4-*d*][2]benzazepin-2-yl]amino}-*N*-[3-(dimethylamino)propyl]-*N*-methylbenzamide |
| II-226 | 3-{[9-chloro-7-(2-fluoro-6-methoxyphenyl)-5*H*-pyrimido[5,4-*d*][2]benzazepin-2-yl]amino}-*N*-[3-(dimethylamino)propyl]-*N*-methylbenzamide |
| II-227 | 9-chloro-*N*-(3-{[3-(dimethylamino)azetidin-1-yl]carbonyl}phenyl)-7-(2-fluoro-6-methoxyphenyl)-5*H*-pyrimido[5,4-*d*][2]benzazepin-2-amine |
| II-228 | 9-chloro-*N*-{3-[(3,5-dimethylpiperazin-1-yl)carbonyl]phenyl}-7-(2-fluoro-6-methoxyphenyl)-5*H*-pyrimido[5,4-*d*][2]benzazepin-2-amine |
| II-229 | 9-chloro-*N*-(3-{[4-(dimethylamino)piperidin-1-yl]carbonyl}phenyl)-7-(2-fluoro-6-methoxyphenyl)-5*H*-pyrimido[5,4-*d*][2]benzazepin-2-amine |
| II-230 | *N*-(4-{[3-(aminomethyl)azetidin-1-yl]carbonyl}phenyl)-9-chloro-7-(2-fluorophenyl)-5*H*-pyrimido[5,4-*d*][2]benzazepin-2-amine |
| II-231 | 9-chloro-*N*-(3-{[3-(dimethylamino)pyrrolidin-1-yl]carbonyl}phenyl)-7-(2-fluoro-6-methoxyphenyl)-5*H*-pyrimido[5,4-*d*][2]benzazepin-2-amine |
| II-232 | 9-chloro-7-(2-fluoro-6-methoxyphenyl)-*N*-{4-[(3-methylpiperazin-1-yl)carbonyl]phenyl}-5*H*-pyrimido[5,4-*d*][2]benzazepin-2-amine |
| II-233 | 9-chloro-7-(2-fluoro-6-methoxyphenyl)-*N*-{4-[(4-methylpiperazin-1-yl)carbonyl]phenyl}-5*H*-pyrimido[5,4-*d*][2]benzazepin-2-amine |
| II-234 | 9-chloro-7-(2,6-difluorophenyl)-*N*-(4-{[3-(methylamino)azetidin-1-yl]carbonyl}phenyl)-5*H*-pyrimido[5,4-*d*][2]benzazepin-2-amine |
| II-235 | 9-chloro-7-(2-fluoro-6-methoxyphenyl)-*N*-(4-{[3-(methylamino)azetidin-1-yl]carbonyl}phenyl)-5*H*-pyrimido[5,4-*d*][2]benzazepin-2-amine |
| II-236 | 4-{[9-chloro-7-(2,6-difluorophenyl)-5*H*-pyrimido[5,4-*d*][2]benzazepin-2-yl]amino}benzonitrile |
| II-237 | 4-{[9-chloro-7-(2,6-difluorophenyl)-5*H*-pyrimido[5,4-*d*][2]benzazepin-2-yl]amino}-*N*-[[3-(dimethylamino)pyrrolidin-1-yl](imino)methyl]benzamide |
| II-238 | 4-{[9-chloro-7-(2,6-difluorophenyl)-5*H*-pyrimido[5,4-*d*][2]benzazepin-2-yl]amino}-*N*-[(3,5-dimethylpiperazin-1-yl)(imino)methyl]benzamide |
| II-239 | *N*-{4-[(4-aminopiperidin-1-yl)carbonyl]phenyl}-9-chloro-7-(2,6-difluorophenyl)-5*H*-pyrimido[5,4-*d*][2]benzazepin-2-amine |
| II-240 | *N*-{4-[(3-aminopyrrolidin-1-yl)carbonyl]phenyl}-9-chloro-7-(2-fluoro-6-methoxyphenyl)-5*H*-pyrimido[5,4-*d*][2]benzazepin-2-amine |
| II-241 | *N*-{4-[(4-aminopiperidin-1-yl)carbonyl]phenyl}-9-chloro-7-(2-fluoro-6-methoxyphenyl)-5*H*-pyrimido[5,4-*d*][2]benzazepin-2-amine |
| II-242 | 9-chloro-7-(2-fluoro-6-methoxyphenyl)-*N*-(4-{[4-(methylamino)piperidin-1-yl]carbonyl}phenyl)-5*H*-pyrimido[5,4-*d*][2]benzazepin-2-amine |
| II-243 | 9-chloro-7-(2-fluoro-6-methoxyphenyl)-*N*-[4-(piperazin-1-ylcarbonyl)phenyl]-5*H*-pyrimido[5,4-*d*][2]benzazepin-2-amine |
| II-244 | 9-chloro-7-(2,6-difluorophenyl)-*N*-{4-[[4-(dimethylamino)piperidin-1-yl](imino)methyl]phenyl}-5*H*-pyrimido[5,4-*d*][2]benzazepin-2-amine |
| II-245 | *N*-(4-{[9-chloro-7-(2,6-difluorophenyl)-5*H*-pyrimido[5,4-*d*][2]benzazepin-2-yl]amino}phenyl)guanidine |
| II-246 | 4-{[9-chloro-7-(2,6-difluorophenyl)-5*H*-pyrimido[5,4-*d*][2]benzazepin-2-yl]amino}-*N*-methyl-*N*-[2-(methylamino)ethyl]benzamide |
| II-247 | 4-{[9-chloro-7-(2,6-difluorophenyl)-5*H*-pyrimido[5,4-*d*][2]benzazepin-2-yl]amino}-*N*-[2-(dimethylamino)ethyl]-*N*-methylbenzamide |
| II-248 | methyl 4-(4-{[9-chloro-7-(2,6-difluorophenyl)-5*H*-pyrimido[5,4-*d*][2]benzazepin-2-yl]amino}benzoyl)piperazine-2-carboxylate |
| II-249 | 2-[(4-carboxyphenyl)amino]-7-(2-fluorophenyl)-5*H*-pyrimido[5,4-*d*] [2]benzazepine-9-carboxylic acid |
| II-250 | 9-chloro-7-(2,6-difluorophenyl)-*N*-{4-[[3-(dimethylamino)pyrrolidin-1-yl] (imino)methyl]phenyl}-5*H*-pyrimido[5,4-*d*][2]benzazepin-2-amine |
| II-251 | 9-chloro-7-(2,6-difluorophenyl)-*N*-{4-[(3,5-dimethylpiperazin-1-yl)(imino)methyl]phenyl}-5*H*-pyrimido[5,4-*d*][2]benzazepin-2-amine |
| II-252 | *N*-(2-aminoethyl)-4-{[9-chloro-7-(2,6-difluorophenyl)-5*H*-pyrimido[5,4-*d*][2]benzazepin-2-yl]amino}-*N*-methylbenzamide |
| II-253 | 9-chloro-7-(2,6-difluorophenyl)-*N*-(4-{[3-(methylamino)piperidin-1-yl]carbonyl}phenyl)-5*H*-pyrimido[5,4-*d*][2]benzazepin-2-amine |
| II-254 | 4-{[9-chloro-7-(2-fluoro-6-methoxyphenyl)-5*H*-pyrimido[5,4-*d*][2]benzazepin-2-yl]amino}-*N*-methyl-*N*-[2-(methylamino)ethyl]benzamide |
| II-255 | 4-{[9-chloro-7-(2-fluoro-6-methoxyphenyl)-5*H*-pyrimido[5,4-*d*][2]benzazepin-2-yl]amino}-*N*-[2-(dimethylamino)ethyl]-*N*-methylbenzamide |
| II-256 | 7-(2-fluorophenyl)-2-[(3-methoxyphenyl)amino]-5*H*-pyrimido[5,4-*d*] [2]benzazepine-9-carboxylic acid |
| II-257 | *N*-(3-aminopropyl)-4-{[9-chloro-7-(2,6-difluorophenyl)-5*H*-pyrimido[5,4-*d*] [2]benzazepin-2-yl]amino} -*N*-methylbenzamide |
| II-258 | 2-chloro-5-{[9-chloro-7-(2,6-difluorophenyl)-5*H*-pyrimido[5,4-*d*][2]benzazepin-2-yl]amino}benzoic acid |
| II-259 | 4-{[9-chloro-7-(2,6-difluorophenyl)-5*H*-pyrimido[5,4-*d*][2]benzazepin-2-yl]amino}-*N*-[[3-(dimethylamino)azetidin-1-yl](imino)methyl]benzamide |
| II-260 | *N*-(2-amino-2-methylpropyl)-4-{[9-chloro-7-(2,6-difluorophenyl)-5*H-*pyrimido[5,4-*d*][2]benzazepin-2-yl]amino}benzamide |
| II-261 | 4-{[9-chloro-7-(2-fluoro-6-methoxyphenyl)-5*H*-pyrimido[5,4-*d*][2]benzazepin-2-yl]amino}-*N*-methyl-*N*-[3-(methylamino)propyl]benzamide |
| II-262 | *N*-{4-[(3-aminopiperidin-1-yl)carbonyl]phenyl}-9-chloro-7-(2-fluoro-6-methoxyphenyl)-5*H*-pyrimido[5,4-*d*][2]benzazepin-2-amine |
| II-263 | 9-chloro-7-(2-fluoro-6-methoxyphenyl)-*N*-(4-{[3-(methylamino)piperidin-1-yl]carbonyl}phenyl)-5*H*-pyrimido[5,4-*d*][2]benzazepin-2-amine |
| II-264 | *N*-(3-aminopropyl)-4-{[9-chloro-7-(2-fluoro-6-methoxyphenyl)-5*H-*pyrimido[5,4-*d*][2]benzazepin-2-yl]amino}-*N*-methylbenzamide |
| II-265 | *N*-(2-aminoethyl)-4-{[9-chloro-7-(2-fluoro-6-methoxyphenyl)-5*H*-pyrimido[5,4-*d*] [2]benzazepin-2-yl]amino}-*N*-methylbenzamide |
| II-266 | 4-(4-{[9-chloro-7-(2,6-difluorophenyl)-5*H*-pyrimido[5,4-*d*][2]benzazepin-2-yl]amino}benzoyl)piperazine-2-carboxylic acid |
| II-267 | 9-chloro-7-(2,6-difluorophenyl)-*N*-{4-[[3-(dimethylamino)azetidin-1-yl] (imino)methyl]phenyl}-5*H*-pyrimido[5,4-*d*][2]benzazepin-2-amine |
| II-268 | 9-chloro-7-(2,6-difluorophenyl)-*N*-(4-{imino[3-(methylamino)pyrrolidin-1-yl]methyl}phenyl)-5*H*-pyrimido[5,4-*d*][2]benzazepin-2-amine |
| II-269 | 9-chloro-*N*-(4-chloro-3-{[4-(dimethylamino)piperidin-1-yl]carbonyl}phenyl)-7-(2,6-difluorophenyl)-5*H*-pyrimido[5,4-*d*][2]benzazepin-2-amine |
| II-270 | 9-chloro-7-(2,6-difluorophenyl)-*N*-[4-(5,5-dimethyl-4,5-dihydro-1*H*-imidazol-2-yl)phenyl]-5*H*-pyrimido[5,4-*d*] [2]benzazepin-2-amine |
| II-271 | *N*-[9-chloro-7-(2,6-difluorophenyl)-5*H*-pyrimido[5,4-*d*][2]benzazepin-2-yl]-*N*'-pyrimidin-2-ylbenzene-1,4-diamine |
| II-272 | 4-{[9-(3-aminoprop-1-yn-1-yl)-7-(2,6-difluorophenyl)-5*H*-pyrimido[5,4-*d*][2]benzazepin-2-yl]amino}benzoic acid |
| II-273 | 9-bromo-7-(2,6-difluorophenyl)-*N*-(3-methoxyphenyl)-5*H*-pyrimido[5,4-*d*][2]benzazepin-2-amine |
| II-274 | 4-{[9-bromo-7-(2,6-difluorophenyl)-5*H*-pyrimido[5,4-*d*][2]benzazepin-2-yl]amino}benzoic acid |
| II-275 | 7-(2,6-difluorophenyl)-*N*-(3-methoxyphenyl)-9-(3-pyrrolidin-1-ylprop-1-yn-1-yl)-5*H*-pyrimido[5,4-*d*][2]benzazepin-2-amine |
| II-276 | 9-(3-aminoprop-1-yn-1-yl)-7-(2,6-difluorophenyl)-*N*-(3-methoxyphenyl)-5*H-*pyrimido[5,4-*d*][2]benzazepin-2-amine |
| II-277 | 4-({9-chloro-7-[2-(trifluoromethyl)phenyl]-5*H*-pyrimido[5,4-*d*][2]benzazepin-2-yl}amino)benzoic acid |
| II-278 | *N*-{4-[(3-aminoazetidin-1-yl)carbonyl]phenyl}-9-chloro-7-(2,6-difluorophenyl)-5*H*-pyrimido[5,4-*d*][2]benzazepin-2-amine |
| II-279 | 4-[(9-chloro-7-pyridin-2-yl-5*H*-pyrimido[5,4-*d*][2]benzazepin-2-yl)amino]benzoic acid |
| II-280 | *N*-(4-{[9-chloro-7-(2,6-difluorophenyl)-5*H*-pyrimido[5,4-*d*][2]benzazepin-2-yl]amino}phenyl)-4-methylpiperazine-1-carboxamide |
| II-281 | 9-chloro-*N*-(4-chloro-3-{[3-(methylamino)pyrrolidin-1-yl]carbonyl}phenyl)-7-(2,6-difluorophenyl)-5*H*-pyrimido[5,4-*d*][2]benzazepin-2-amine |
| II-282 | 9-chloro-*N*-(4-chloro-3-{[4-(methylamino)piperidin-1-yl]carbonyl}phenyl)-7-(2,6-difluorophenyl)-5*H*-pyrimido[5,4-*d*][2]benzazepin-2-amine |
| II-283 | 2-chloro-5-{[9-chloro-7-(2,6-difluorophenyl)-5*H*-pyrimido[5,4-*d*][2]benzazepin-2-yl]amino}-*N*-methyl-*N*-[2-(methylamino)ethyl]benzamide |
| II-284 | *N*-{4-[(3-aminopyrrolidin-1-yl)(imino)methyl]phenyl}-9-chloro-7-(2,6-difluorophenyl)-5*H*-pyrimido[5,4-*d*][2]benzazepin-2-amine |
| II-285 | 2-(4-{[9-chloro-7-(2,6-difluorophenyl)-5*H*-pyrimido[5,4-*d*][2]benzazepin-2-yl]amino}phenyl)-1,4,5,6-tetrahydropyrimidin-5-ol |
| II-286 | *N*-{4-[(3-aminoazetidin-1-yl)carbonyl]phenyl}-9-chloro-7-(2-fluoro-6-methoxyphenyl)-5*H*-pyrimido[5,4-*d*][2]benzazepin-2-amine |
| II-287 | *N*-{4-[(4-aminopiperidin-1-yl)carbonyl]phenyl}-9-chloro-7-[2-(trifluoromethyl)phenyl]-5*H*-pyrimido[5,4-*d*][2]benzazepin-2-amine |
| II-288 | 9-chloro-*N*-(4-{[4-(methylamino)piperidin-1-yl]carbonyl}phenyl)-7-[2-(trifluoromethyl)phenyl]-5*H*-pyrimido[5,4-*d*][2]benzazepin-2-amine |
| II-289 | *N*-{4-[(3-aminopyrrolidin-1-yl)carbonyl]phenyl}-9-chloro-7-[2-(trifluoromethyl)phenyl]-5*H*-pyrimido[5,4-*d*][2]benzazepin-2-amine |
| II-290 | 9-chloro-*N*-(4-{[3-(methylamino)pyrrolidin-1-yl]carbonyl}phenyl)-7-[2-(trifluoromethyl)phenyl]-5*H*-pyrimido[5,4-*d*][2]benzazepin-2-amine |
| II-291 | 9-chloro-*N*-(4-chloro-3-{[3-(methylamino)azetidin-1l-yl]carbonyl}phenyl)-7-(2,6-difluorophenyl)-5*H*-pyrimido[5,4-*d*][2]benzazepin-2-amine |
| II-292 | *N*-{3-[(4-aminopiperidin-1-yl)carbonyl]-4-chlorophenyl}-9-chloro-7-(2,6-difluorophenyl)-5*H*-pyrimido[5,4-*d*][2]benzazepin-2-amine |
| II-293 | 9-chloro-7-(2,6-difluorophenyl)-*N*-(4-{[3-(dimethylamino)piperidin-1-yl]carbonyl}phenyl)-5*H*-pyrimido[5,4-*d*][2]benzazepin-2-amine |
| II-294 | methyl 4-amino-1-(4-{[9-chloro-7-(2,6-difluorophenyl)-5*H*-pyrimido[5,4-*d*][2]benzazepin-2-yl]amino}benzoyl)piperidine-4-carboxylate |
| II-295 | 4-amino-1-(4-{[9-chloro-7-(2,6-difluorophenyl)-5*H*-pyrimido[5,4-*d*][2]benzazepin-2-yl]amino}benzoyl)piperidine-4-carboxylic acid |
| II-296 | *N*-{4-[(3-aminoazetidin-1-yl)carbonyl]phenyl}-9-chloro-7-[2-(trifluoromethyl)phenyl]-5*H*-pyrimido[5,4-*d*][2]benzazepin-2-amine |
| II-297 | 9-chloro-*N*-(4-{[3-(methylamino)azetidin-1-yl]carbonyl}phenyl)-7-[2-(trifluoromethyl)phenyl]-5*H*-pyrimido[5,4-*d*][2]benzazepin-2-amine |
| II-298 | *N*-{4-[(4-aminopiperidin-1-yl)carbonyl]phenyl}-9-chloro-7-pyridin-2-yl-5*H-*pyrimido[5,4-*d*] [2]benzazepin-2-amine |
| II-299 | *N*-{4-[(3-aminopyrrolidin-1-yl)carbonyl]phenyl}-9-chloro-7-pyridin-2-yl-5*H-*pyrimido[5,4-*d*] [2]benzazepin-2-amine |
| II-300 | ethyl 2-amino-4-[(4-{[9-chloro-7-(2,6-difluorophenyl)-5*H*-pyrimido[5,4-*d*][2]benzazepin-2-yl]amino}benzoyl)amino]butanoate |
| II-301 | 4-{[9-chloro-7-(3-fluoropyridin-2-yl)-5*H*-pyrimido[5,4-*d*][2]benzazepin-2-yl]amino}benzoic acid |
| II-302 | 9-{[3-(dimethylamino)azetidin-1-yl]carbonyl}-7-(2-fluorophenyl)-*N*-(3-methoxyphenyl)-5*H*-pyrimido[5,4-*d*][2]benzazepin-2-amine |
| II-303 | 7-(2-fluorophenyl)-2-[(3-methoxyphenyl)amino]-*N*-methyl-*N*-[3-(methylamino)propyl]-5*H*-pyrimido[5,4-*d*][2]benzazepine-9-carboxamide |
| II-304 | *N*-{4-[(4-aminopiperidin-1-yl)carbonyl]phenyl}-9-chloro-7-(3-fluoropyridin-2-yl)-5*H*-pyrimido[5,4-*d*] [2]benzazepin-2-amine |
| II-305 | *N*-{4-[(3-aminopyrrolidin-1-yl)carbonyl]phenyl}-9-chloro-7-(3-fluoropyridin-2-yl)-5*H*-pyrimido[5,4-*d*][2]benzazepin-2-amine |
| II-306 | 2-(4-{[9-chloro-7-(2,6-difluorophenyl)-5*H*-pyrimido[5,4-*d*][2]benzazepin-2-yl]amino}phenyl)-4,5-dihydro-1*H*-imidazole-5-carboxylic acid |
| II-307 | *N*-(4-{[9-chloro-7-(2,6-difluorophenyl)-5*H*-pyrimido[5,4-*d*][2]benzazepin-2-yl]amino}phenyl)-2-(dimethylamino)acetamide |
| II-308 | 2-amino-*N*-(4-{[9-chloro-7-(2,6-difluorophenyl)-5*H*-pyrimido[5,4-*d*][2]benzazepin-2-yl]amino}phenyl)-2-methylpropanamide |
| II-309 | ethyl (2*R*)-4-amino-2-[(4-{[9-chloro-7-(2,6-difluorophenyl)-5*H*-pyrimido[5,4-*d*][2]benzazepin-2-yl]amino}benzoyl)amino]butanoate |
| II-310 | 4-(4-{[9-chloro-7-(2,6-difluorophenyl)-5*H*-pyrimido[5,4-*d*][2]benzazepin-2-yl]amino}benzoyl)-*N*-methylpiperazine-2-carboxamide |
| II-311 | 7-(2-fluorophenyl)-2-[(3-methoxyphenyl)amino]-*N*-(3-morpholin-4-ylpropyl)-5*H*-pyrimido[5,4-*d*][2]benzazepine-9-carboxamide |
| II-312 | 9-[(3,5-dimethylpiperazin-1-yl)carbonyl]-7-(2-fluorophenyl)-*N*-(3-methoxyphenyl)-5*H*-pyrimido[5,4-*d*][2]benzazepin-2-amine |
| II-313 | 9-chloro-*N*-(3-chloro-4-{[4-(dimethylamino)piperidin-1-yl]carbonyl}phenyl)-7-(2,6-difluorophenyl)-5*H*-pyrimido[5,4-*d*][2]benzazepin-2-amine |
| II-314 | ethyl 2-(4-{[9-chloro-7-(2,6-difluorophenyl)-5*H*-pyrimido[5,4-*d*][2]benzazepin-2-yl]amino}phenyl)-4,5-dihydro-1*H*-imidazole-5-carboxy late |
| II-315 | 9-chloro-*N*-(4-{[3-(methylamino)pyrrolidin-1-yl]carbonyl}phenyl)-7-pyridin-2-yl-5*H*-pyrimido[5,4-*d*][2]benzazepin-2-amine |
| II-316 | 9-chloro-*N*-(4-{[4-(methylamino)piperidin-1-yl]carbonyl}phenyl)-7-pyridin-2-yl-5*H*-pyrimido[5,4-*d*][2]benzazepin-2-amine |
| II-317 | 4-(4-{[9-chloro-7-(2,6-difluorophenyl)-5*H*-pyrimido[5,4-*d*][2]benzazepin-2-yl]amino}benzoyl)piperazine-2-carboxamide |
| II-318 | *N*-{4-[(3-aminopyrrolidin-1-yl)carbonyl]-3-chlorophenyl}-9-chloro-7-(2,6-difluorophenyl)-5*H*-pyrimido[5,4-*d*][2]benzazepin-2-amine |
| II-319 | *N*-(4-{[9-chloro-7-(2,6-difluorophenyl)-5*H*-pyrimido[5,4-*d*][2]benzazepin-2-yl]amino}phenyl)piperidine-4-carboxamide |
| II-320 | 4-{[9-chloro-7-(2-fluoro-6-{methyl[2-(methylamino)ethyl]amino}phenyl)-5*H-*pyrimido[5,4-*d*][2]benzazepin-2-yl]amino}benzoic acid |
| II-321 | 9-chloro-7-(2,4-difluorophenyl)-*N*-{4-[(3,5-dimethylpiperazin-1-yl)carbonyl]phenyl}-5*H*-pyrimido[5,4-*d*][2]benzazepin-2-amine |
| II-322 | 9-chloro-7-(2,4-dimethoxyphenyl)-*N*-{4-[(3,5-dimethylpiperazin-1-yl)carbonyl]phenyl}-5*H*-pyrimido[5,4-*d*][2]benzazepin-2-amine |
| II-323 | 9-chloro-7-(2-chloro-6-fluorophenyl)-*N*-{4-[(3-methylpiperazin-1-yl)carbonyl]phenyl}-5*H*-pyrimido[5,4-*d*][2]benzazepin-2-amine |
| II-324 | 9-chloro-7-(2-chloro-6-fluorophenyl)-*N*-{4-[(3,5-dimethylpiperazin-1-yl)carbonyl]phenyl}-5*H*-pyrimido[5,4-*d*][2]benzazepin-2-amine |
| II-325 | 9-chloro-7-(2-chloro-6-fluorophenyl)-*N*-(4-{[4-(methylamino)piperidin-1-yl]carbonyl}phenyl)-5*H*-pyrimido[5,4-*d*][2]benzazepin-2-amine |
| II-326 | 9-chloro-7-(2-chloro-6-fluorophenyl)-*N*-(4-{[3-(methylamino)piperidin-1-yl]carbonyl}phenyl)-5*H*-pyrimido[5,4-*d*][2]benzazepin-2-amine |
| II-327 | 9-chloro-7-(2-chloro-6-fluorophenyl)-*N*-(4-{[3-(methylamino)pyrrolidin-1-yl]carbonyl}phenyl)-5*H*-pyrimido[5,4-*d*][2]benzazepin-2-amine |
| II-328 | 9-chloro-*N*-(3,4-dimethoxyphenyl)-7-{2-[(dimethylamino)methyl]phenyl}-5*H-*pyrimido[5,4-*d*][2]benzazepin-2-amine |
| II-329 | 9-chloro-7-(2-methoxyphenyl)-*N*-{4-[(3-methylpiperazin-1-yl)carbonyl]phenyl}-5*H*-pyrimido[5,4-*d*][2]benzazepin-2-amine |
| II-330 | 9-chloro-*N*-{4-[(3,5-dimethylpiperazin-1-yl)carbonyl]phenyl}-7-(2-methoxyphenyl)-5*H*-pyrimido[5,4-*d*][2]benzazepin-2-amine |
| II-331 | 9-chloro-7-(2-methoxyphenyl)-*N*-(4-{[4-(methylamino)piperidin-1-yl]carbonyl}phenyl)-5*H*-pyrimido[5,4-*d*][2]benzazepin-2-amine |
| II-332 | 9-chloro-7-(2-methoxyphenyl)-*N*-(4-{[3-(methylamino)pyrrolidin-1-yl]carbonyl}phenyl)-5*H*-pyrimido[5,4-*d*][2]benzazepin-2-amine |
| II-333 | 9-chloro-7-(2-methoxyphenyl)-*N*-(4-{[3-(methylamino)piperidin-1-yl]carbonyl}phenyl)-5*H*-pyrimido[5,4-*d*][2]benzazepin-2-amine |
| II-334 | 4-{[9-chloro-7-(2,6-difluorophenyl)-5*H*-pyrimido[5,4-*d*][2]benzazepin-2-yl]amino}-*N*-methylbenzamide |
| II-335 | 4-{[9-chloro-7-(2-fluoro-6-{methyl[3-(methylamino)propyl]amino}phenyl)-5*H-*pyrimido[5,4-*d*][2]benzazepin-2-yl]amino}benzoic acid |
| II-336 | 4-{[9-chloro-7-(2-fluoro-6-{methyl[3-(methylamino)propyl]amino}phenyl)-5*H-*pyrimido[5,4-*d*][2]benzazepin-2-yl]amino}-*N*-methylbenzamide |
| II-337 | 1-(4-{[9-chloro-7-(2,6-difluorophenyl)-5*H*-pyrimido[5,4-*d*][2]benzazepin-2-yl]amino}phenyl)ethanone |
| II-338 | *N*-[3-(3-aminoprop-1-yn-1-yl)phenyl]-9-chloro-7-(2,6-difluorophenyl)-5*H-*pyrimido[5,4-*d*][2]benzazepin-2-amine |
| II-339 | 4-[(9-chloro-7-{2-fluoro-6-[(2-hydroxyethyl)amino]phenyl}-5*H*-pyrimido[5,4-*d*][2]benzazepin-2-yl)amino]-*N*-methylbenzamide |
| II-340 | 4-[(7-{2-[(2-aminoethyl)amino]-6-fluorophenyl}-9-chloro-5*H*-pyrimido[5,4-*d*][2]benzazepin-2-yl)amino]-*N*-methylbenzamide |
| II-341 | 4-amino-1-(4-{[9-chloro-7-(2,6-difluorophenyl)-5*H*-pyrimido[5,4-*d*][2]benzazepin-2-yl]amino}benzoyl)-*N*-methylpiperidine-4-carboxamide |
| II-342 | 4-[(9-chloro-7-{2-[4-(dimethylamino)piperidin-1-yl]-6-fluorophenyl}-5*H-*pyrimido[5,4-*d*][2]benzazepin-2-yl)amino]-*N*-methylbenzamide |
| II-343 | 9-chloro-7-(2,6-difluorophenyl)-*N*-{3-[3-(dimethylamino)prop-1-yn-1-yl]phenyl}-5*H*-pyrimido[5,4-*d*][2]benzazepin-2-amine |
| II-344 | 9-chloro-7-(2,6-difluorophenyl)-*N*-(3-iodophenyl)-5*H*-pyrimido[5,4-*d*][2]benzazepin-2-amine |
| II-345 | 4-{[9-chloro-7-(2-{[2-(dimethylamino)ethyl]amino}-6-fluorophenyl)-5*H-*pyrimido[5,4-*d*][2]benzazepin-2-yl]amino}-*N*-methylbenzamide |
| II-346 | 4-[(9-chloro-7-{2-[[2-(dimethylamino)ethyl](methyl)amino]-6-fluorophenyl}-5*H*-pyrimido[5,4-*d*][2]benzazepin-2-yl)amino]-*N*-methylbenzamide |
| II-347 | 4-{[9-chloro-7-(2-fluoro-6-{methyl[2-(methylamino)ethyl]amino}phenyl)-5*H-*pyrimido[5,4-*d*][2]benzazepin-2-yl]amino}-*N*-methylbenzamide |
| II-348 | 4-({7-[2-(4-aminopiperidin-1-yl)-6-fluorophenyl]-9-chloro-5*H*-pyrimido[5,4-*d*][2]benzazepin-2-yl}amino)-*N*-methylbenzamide |
| II-349 | 7-(2-fluorophenyl)-2-[(3-methoxyphenyl)amino]-*N*-methyl-N-[2-(methylamino)ethyl]-5*H*-pyrimido[5,4-*d*][2]benzazepine-9-carboxamide |
| II-350 | 4-amino-1-(4-{[9-chloro-7-(2,6-difluorophenyl)-5*H*-pyrimido[5,4-*d*][2]benzazepin-2-yl]amino}benzoyl)piperidme-4-carboxamide |
| II-351 | 9-chloro-7-(2-chloro-6-fluorophenyl)-*N*-(4-{[3-(methylamino)azetidin-1-yl]carbonyl}phenyl)-5*H*-pyrimido[5,4*-d*][2]benzazepin-2-amine |
| II-352 | 9-chloro-7-(2,6-difluorophenyl)-*N*-(4-methyl-1,3-thiazol-2-yl)-5*H*-pyrimido[5,4-*d*][2]benzazepin-2-amine |
| II-353 | 7-(2,6-difluorophenyl)-2-[(3-methoxyphenyl)amino]-5*H*-pyrimido[5,4-*d*] [2]benzazepine-9-carboxylicacid |
| II-354 | 4-({9-chloro-7-[2-fluoro-6-(methylamino)phenyl]-5*H*-pyrimido[5,4-*d*] [2]benzazepin-2-yl}amino)-*N*-methylbenzamide |
| II-355 | 2-{[9-chloro-7-(2,6-difluorophenyl)-5*H*-pyrimido[5,4-d][2]benzazepin-2-yl]amino}-*N*-methyl-1,3-thiazole-4-carboxamide |
| II-356 | N-1*H*-benzimidazol-2-yl-9-chloro-7-(2,6-difluorophenyl)-5*H*-pyrimido[5,4-*d*] [2]benzazepin-2-amine |
| II-357 | 7-(2,6-difluorophenyl)-2-[(4-methyl-1,3-thiazol-2-yl)amino]-5*H*-pyrimido[5,4-*d*][2]benzazepine-9-carboxylicacid |
| II-358 | 3-amino-1-(3-{[9-chloro-7-(2,6-difluorophenyl)-5*H*-pyrimido[5,4-*d*][2]benzazepin-2-yl]amino}phenyl)propan-1-one |
| II-359 | 1-(3-{[9-chloro-7-(2,6-difluorophenyl)-5*H*-pyrimido[5,4-d][2]benzazepin-2-yl]amino}phenyl)-3-(dimethylamino)propan-1-one |
| II-360 | 2-{[9-chloro-7-(2,6-difluorophenyl)-5*H*-pyrimido[5,4-d][2]benzazepin-2-yl]amino}-1,3-thiazole-4-carboxylic acid |
| II-361 | ethyl 2-{[9-chloro-7-(2,6-difluorophenyl)-5*H*-pyrimido[5,4-*d*][2]benzazepin-2-yl]amino}-1,3-thiazole-4-carboxylate |
| II-362 | 9-chloro-7-(2,6-difluorophenyl)-*N*-{4-[(3,5-dimethylpiperazin-1-yl)carbonyl]-1,3-thiazol-2-yl}-5*H*-pyrimido[5,4-*d*][2]benzazepin-2-amine |
| II-363 | ethyl 2-{[9-chloro-7-(2,6-difluorophenyl)-5*H*-pyrimido[5,4-*d*][2]benzazepin-2-yl]amino}-1,3-oxazole-5-carboxylate |
| II-364 | 2-{[9-chloro-7-(2,6-difluorophenyl)-5*H*-pyrimido[5,4-*d*][2]benzazepin-2-yl]amino}-1,3-oxazole-5-carboxylic acid |
| II-365 | 9-chloro-7-(2,6-difluorophenyl)-*N*-(4-{[(3*R*)-3-methylpiperazin-1-yl]carbonyl}-1,3-thiazol-2-yl)-5*H*-pyrimido[5,4-*d*][2]benzazepin-2-amine |
| II-366 | 9-chloro-7-(2,6-difluorophenyl)-*N*-(4-{[(2*R*)-2-methylpiperazin-1-yl]carbonyl}phenyl)-5*H*-pyrimido[5,4-*d*][2]benzazepin-2-amine |
| II-367 | 9-chloro-7-(2,6-difluorophenyl)-*N*-(4-{[3-(methylamino)pyrrolidin-1-yl]carbonyl}-1,3-thiazol-2-yl)-5*H*-pyrimido[5,4-*d*][2]benzazepin-2-amine |
| II-368 | 2-{[9-chloro-7-(2,6-difluorophenyl)-5*H*-pyrimido[5,4-*d*][2]benzazepin-2-yl]amino}-1,3-oxazole-4-carboxylic acid |
| II-369 | 9-chloro-7-(2,6-difluorophenyl)-*N*-{5-[(3,5-dimethylpiperazin-1-yl)carbonyl]-1,3-oxazol-2-yl}-5*H*-pyrimido[5,4-*d*][2]benzazepin-2-amine |
| II-370 | 9-chloro-7-(2,6-difluorophenyl)-*N*-(5-{[3-(methylamino)pyrrolidin-1-yl]carbonyl}-1,3-oxazol-2-yl)-5*H*-pyrimido[5,4-*d*][2]benzazepin-2-amine |
| II-371 | 4-{[9-chloro-7-(2,6-difluorophenyl)-5-methyl-5*H*-pyrimido[5,4-*d*] [2]benzazepin-2-yl]amino}benzoic acid |
| II-372 | 9-chloro-7-(2,6-difluorophenyl)-*N*-{3-[3-(dimethylamino)propyl]phenyl}-5*H-*pyrimido[5,4-*d*][2]benzazepin-2-amine |
| II-373 | *N*-[3-(3-aminopropyl)phenyl]-9-chloro-7-(2,6-difluorophenyl)-5*H*-pyrimido[5,4-*d*][2]benzazepin-2-amine |
| II-374 | 9-chloro-7-(2,6-difluorophenyl)-*N*-{4-[(3,5-dimethylpiperazin-1-yl)carbonyl]-1,3-oxazol-2-yl}-5*H*-pyrimido[5,4-*d*][2]benzazepin-2-amine |
| II-375 | 9-chloro-7-(2,6-difluorophenyl)-*N*-(4-{[3-(methylamino)pyrrolidin-1-yl]carbonyl}-1,3-oxazol-2-yl)-5*H*-pyrimido[5,4-*d*][2]benzazepin-2-amine |
| II-376 | 7-(2,6-difluorophenyl)-2-({4-[(3,5-dimethylpiperazin-1-yl)carbonyl]phenyl}amino)-*N*-methyl-5*H*-pyrimido[5,4-*d*][2]benzazepine-9-carboxamide |
| II-377 | 2-{[4-(aminocarbonyl)phenyl]amino}-7-(2,6-difluorophenyl)-5*H*-pyrimido[5,4-*d*] [2]benzazepine-9-carboxylic acid |
| II-378 | 1-(4-{[9-chloro-7-(2,6-difluorophenyl)-5*H*-pyrimido[5,4*d*][2]benzazepin-2-yl]amino}benzoyl)-*N*-methyl-4-(methylamino)piperidine-4-carboxamide |
| II-379 | *N*-{4-[(3-amino-3-methylpyrrolidin-1-yl)carbonyl]phenyl}-9-chloro-7-(2,6-difluorophenyl)-5*H*-pyrimido[5,4-*d*][2]benzazepin-2-amine |
| II-380 | 9-chloro-7-(2,6-difluorophenyl)-*N*-(4-{[3-methyl-3-(methylamino)pyrrolidin-1-yl]carbonyl}phenyl)-5*H*-pyrimido[5,4-*d*][2]benzazepin-2-amine |
| II-381 | 1-(4-{[9-chloro-7-(2,6-difluorophenyl)-5*H*-pyrimido[5,4-*d*][2]benzazepin-2-yl]amino}benzoyl)-4-(methylamino)piperidine-4-carboxamide |
| II-382 | 9-chloro-7-(2,6-difluorophenyl)-*N*-{4-[(3,3,5-trimethylpiperazin-1-yl)carbonyl]phenyl}-5*H*-pyrimido[5,4-*d*][2]benzazepin-2-amine |
| II-383 | *N*-1-azabicyclo[2.2.2]oct-3-yl-4-{[9-chloro-7-(2,6-difluorophenyl)-5*H-*pyrimido[5,4-*d*][2]benzazepin-2-y1]amino}-*N*-methylbenzamide |
| II-384 | *N*-1-azabicyclo[2-2.2]oct-3-yl-4-{[9-chloro-7-(2,6-difluoropheliyl)-5*H-*pyrimido[5,4-*d*][2]benzazepin-2-yl]amino}benzamide |
| II-385 | 4-{[9-chloro-7-(2,6-difluorophenyl)-5*H*-pyrimido[5,4-*d*][2]benzazepin-2-yl]amino}-*N*-hydroxybenzamide |
| II-386 | *N*-{4-[(aminooxy)carbonyl]phenyl}-9-chloro-7-(2,6-difluorophenyl)-5*H-*pyrimido[5 ,4-*d*] [2]benzazepin-2-amine |
| II-387 | 4-{[9-chloro-7-(2,6-difluorophenyl)-7*H*-pyrimido[5,4-*d*][2]benzazepin-2-yl]amino}benzoic acid |
| II-388 | 4-{[9-chloro-7-(2,3-difluorophenyl)-7*H*-pyrimido[5,4-*d*][2]benzazepin-2-yl]amino}benzoic acid |
| II-389 | 3-amino-1-(4-{[9-chloro-7-(2,6-difluorophenyl)-5*H*-pyrimido[5,4-*d*] [2]benzazepin-2-yl]amino }benzoyl)-*N*-methylpyrrolidine-3-Icarboxamide |
| II-390 | 3-amino-1-(2-chloro-4-{[9-chloro-7-(2,6-difluorophenyl)-5*H*-pyrimido[5,4-*d*][2]benzazepin-2-yl]amino}benzoyl)pyrrolidine-3-carboxamide |
| II-391 | 9-chloro-7-(2,6-difluorophenyl)-*N*-{4-[(3,3-dimethylpiperazin-1-yl)carbonyl]phenyl}-5*H*-pyrimido[5,4-*d*][2]benzazepin-2-amine |
| II-392 | 4-{[9-chloro-7-(2,6-difluorophenyl)-5*H*-pyrimido[5,4-*d*][2]benzazepin-2-yl]amino}-N-(8-methyl-8-azabicyclo[3.2.1]oct-3-yl)benzamide |
| II-393 | 9-chloro-7-(2,6-difluorophenyl)-N-(4-{[3-(dimethylamino)-3-methylpyrrolidin-1-yl]carbonyl}phenyl)-5H-pyrimido[5,4-d][2]benzazepin-2-amine |
| II-394 | 9-chloro-7-(2,6-difluorophenyl)-N-(3-methyl-1H-pyrazol-5-yl)-5H-pyrimido[5,4-d][2]benzazepin-2-amine |
| II-395 | 2-chloro-4-{[9-chloro-7-(2,6-difluorophenyl)-5H-pyrimido[5,4-d][2]benzazepin-2-yl]amino}benzoic acid |
| II-396 | 4-amino-1-(2-chloro-4-{[9-chloro-7-(2,6-difluorophenyl)-5H-pyrimido[5,4-d][2]benzazepin-2-yl]amino}benzoyl)-N-methylpiperidine-4-carboxamide |
| II-397 | 4-amino-1-(2-chloro-4-{[9-chloro-7-(2,6-difluorophenyl)-5H-pyrimido[5,4-d][2]benzazepin-2-yl]amino}benzoyl)-N,N-dimethylpiperidine-4-carboxamide |
| II-398 | 4-[(9-methoxy-7-oxo-6,7-dihydro-5H-pyrimido[5,4-d] [2]benzazepin-2-yl)amino]benzoic acid |
| II-399 | 2-({4-[(3,5-dimethylpiperazin-1-yl)carbonyl]phenyl}amino)-9-methoxy-5,6-dihydro-7H-pyrimido[5,4-d][2]benzazepin-7-one |
| II-400 | 9-methoxy-2-[(4-{[3-(methylamino)pyrrolidin-1-yl]carbonyl}phenyl)amino]-5,6-dihydro-7H-pyrimido[5,4-d][2]benzazepin-7-one |
| II-401 | 4-[(8-methyl-7-oxo-5,6,7,8-tetrahydropyrimido[5,4-c]pyrrolo[3,2-e]azepin-2-yl)amino]benzoic acid |
| II-402 | 2-({4-[(3,5-dimethylpiperazin-1-yl)carbonyl]phenyl}amino)-8-methyl-5,8-dihydropyrimido[5,4-c]pyrrolo[3,2-e]azepin-7(6H)-one |
| II-403 | 2-[(3-methoxyphenyl)amino]-8-methyl-5,8-dihydropyrimido[5,4-c]pyrrolo[3,2-e]azepin-7(6H)-one |
| II-404 | 9-chloro-2-[(3,4-dimethoxyphenyl)amino]-5,6-dihydro-7H-pyrimido[5,4-*d*][2]benzazepin-7-one |
| II-405 | 4-{[4-amino-9-chloro-7-(2,6-difluorophenyl)-5*H*-pyrimido[5,4-*d*][2]benzazepin-2-yl]amino}benzoic acid |
| II-406 | 9-chloro-*N*-(3-chloro-4-{[4-(methylamino)piperidin-1-yl]carbonyl}phenyl)-7-(2,6-difluorophenyl)-5*H*-pyrimido[5,4-*d*][2]benzazepin-2-amine |
| II-407 | 9-chloro-*N*-(3-chloro-4-{[4-(methylamino)piperidin-1-yl]carbonyl}phenyl)-7-(2-fluoro-6-methoxyphenyl)-5*H*-pyrimido[5,4-*d*][2]benzazepin-2-amine |
| II-408 | 4-{[9-chloro-7-(2-fluoro-6-hydroxyphenyl)-5*H*-pyrimido[5,4-*d*][2]benzazepin-2-yl]amino}benzoic acid |
| II-409 | 9-chloro-*N*-[4-(1,7-diazaspiro[4.4]non-7-ylcarbonyl)phenyl]-7-(2,6-difluorophenyl)-5*H*-pyrimido[5,4-*d*][2]benzazepin-2-amine |
| II-410 | 9-chloro-7-(2,6-difluorophenyl)-*N*-(4-{[2-(methylamino)-7-azabicyclo[2.2.1]hept-7-yl]carbonyl}phenyl)-5*H*-pyrimido[5,4-*d*][2]benzazepin-2-amine |
| II-411 | 1-(4-{[9-chloro-7-(2,6-difluorophenyl)-5*H*-pyrimido[5,4-*d*][2]benzazepin-2-yl]amino}benzoyl)-*N*-methyl-3-(methylamino)pyrrolidine-3-carboxamide |
| II-412 | 1-(4-{[9-chloro-7-(2,6-difluorophenyl)-5*H*-pyrimido[5,4-*d*][2]benzazepin-2-yl]amino}benzoyl)-3-(methylamino)pyrrolidine-3-carboxamide |
| II-413 | 1-(2-chloro-4-{[9-chloro-7-(2,6-difluorophenyl)-5*H*-pyrimido[5,4-*d*] [2]benzazepin-2-yl]amino}benzoy l)-*N*-methyl-3-(methylamino)piperidine-3-carboxamide |
| II-414 | 9-chloro-7-(2,6-difluorophenyl)-*N*-(4-{[3-methyl-3-(methylamino)piperidin-1-yl]carbonyl}phenyl)-5*H*-pyrimido[5,4-*d*][2]benzazepin-2-amine |
| II-415 | 9-chloro-7-(2-fluoro-6-methoxyphenyl)-*N*-(4-{[3-methyl-3-(methylamino)piperidin-1-yl]carbonyl}phenyl)-5*H*-pyrimido[5,4-*d*][2]benzazepin-2-amine |
| II-416 | {2-Chloro-4-[9-chloro-7-(2-fluoro-6-methoxy-phenyl)-5H-benzo[c] pyrimido [4,5-e]azepin-2-ylamino]-phenyl}-(3-methyl-3-methylamino-piperidin-1-yl)-methanone |
| II-417 | 9-chloro-7-(2,6-difluorophenyl)-*N*-(4-{[4-methyl-4-(methylamino)piperidin-1-yl]carbonyl}phenyl)-5*H*-pyrimido[5,4-*d*][2]benzazepin-2-amine |
| II-418 | 9-chloro-7-(2,6-difluorophenyl)-*N*-(4-{[4-(dimethylamino)-4-methylpiperidin-1-yl]carbonyl}phenyl)-5*H*-pyrimido[5,4-*d*][2]benzazepin-2-amine |
| II-419 | *N*-{4-[(4-amino-4-methylpiperidin-1-yl)carbonyl]phenyl}-9-chloro-7-(2,6-difluorophenyl)-5*H*-pyrimido[5,4-*d*][2]benzazepin-2-amine |
| II-420 | 9-chloro-*N*-(3-chloro-4-{[4-methyl-4-(methylamino)piperidin-1-yl]carbonyl}phenyl)-7-(2,6-difluorophenyl)-5*H*-pyrimido[5,4-*d*][2]benzazepin-2-amine |
| II-421 | 9-chloro-7-(2-fluoro-6-methoxyphenyl)-*N*-(4-{[4-methyl-4-(methylamino)piperidin-1-yl]carbonyl}phenyl)-5*H*-pyrimido[5,4-*d*] [2]benzazepin-2-amine |
| II-422 | 2-Chloro-4-[9-chloro-7-(2-fluoro-6-methoxy-phenyl)-5H-benzo[c]pyrimido[4,5-e]azepin-2-ylamino]-phenyl}-(4-methyl-4-methylamino-piperidin-1-yl)-methanone |
| II-423 | 9-chloro-7-(2-fluoro-6-methoxyphenyl)-*N*-(3-fluoro-4-{[4-methyl-4-(methylamino)piperidin-1-yl]carbonyl}phenyl)-5*H*-pyrimido[5,4-*d*][2]benzazepin-2-amine |
| II-424 | 9-chloro-*N*-{3-chloro-4-[(3,3,5,5-tetramethylpiperazin-1-yl)carbonyl]phenyl}-7-(2-fluoro-6-methoxyphenyl)-5*H*-pyrimido[5,4-*d*][2]benzazepin-2-amine |
| II-425 | *N*-1-azabicyclo[2.2.2]oct-3-yl-4-{[9-chloro-7-(2,6-difluorophenyl)-5*H-*pyrimido[5,4-*d*][2]benzazepin-2-yl]amino}-2-fluoro-*N*-methylbenzamide |
| II-426 | *N*-1-azabicyclo[2.2.2]oct-3-yl-4-{[9-chloro-7-(2-fluoro-6-methoxyphenyl)-5*H-*pyrimido[5,4-*d*][2]benzazepin-2-yl]amino}-*N*-methylbenzamide |
| II-427 | *N*-8-azabicyclo[3.2.1]oct-3-yl-4-{[9-chloro-7-(2,6-difluorophenyl)-5H-pyrimido[5,4-*d*][2]benzazepin-2-yl]amino}-*N*-methylbenzamide |
| II-428 | 9-chloro-7-(2,6-difluorophenyl)-*N*-(4-{[3-(methylamino)-8-azabicyclo[3.2.1]oct-8-yl]carbonyl}phenyl)-5*H*-pyrimido[5,4-*d*]\|2]benzazepin-2-amine |
| II-429 | 9-chloro-7-(2-fluoro-6-methoxyphenyl)-*N*-(4-{[3-(methylamino)-8-azabicyclo[3.2.1]oct-8-yl]carbonyl}phenyl)-5*H*-pyrimido[5,4-*d*][2]benzazepin-2-amine |
| II-430 | 4-{[7-(2,6-difluorophenyl)-9-methyl-5*H*-pyrimido[5,4-*c*]thieno[2,3-*e*]azepin-2-yl]amino}benzoic acid |
| II-431 | 7-(2,6-difluorophenyl)-*N*-{4-[(3,3,5,5-tetramethylpiperazin-1-yl)carbonyl]phenyl}-5*H*-pyrimido[5,4-*c*]thieno[2,3-*e*]azepin-2-amine |
| II-432 | *N*-{4-[(3-amino-3-methylpyrrolidin-1-yl)carbonyl]phenyl}-7-(2,6-difluorophenyl)-10-methyl-5,10-dihydropyrimido[5,4-*c*]pyrrolo[2,3-*e*]azepin-2-amine |
| II-433 | 7-(2,6-difluorophenyl)-9-methyl-*N*-(4-{[3-(methylamino)pyrroldin-1-yl]carbonyl}phenyl)-5*H*-furo[2,3-*c*]pyrimido[4,5-*e*]azepin-2-amine |
| II-434 | 4-(2,6-difluorophenyl)-2-methyl-*N*-(4-{[3-methyl-3-(methylamino)pyrrolidin-1-yl]carbonyl}phenyl)-6*H*-pyrimido[5,4-*c*][1,3]thiazolo[4,5-*e*]azepin-9-amine |
| II-435 | *N*-{4-[(3-amino-3-methylpyrrolidin-1-yl)carbonyl]phenyl}-7-(2-fluoro-6-methoxyphenyl)-5,9-dihydropyrimido[5,4-c]pyrrolo[3,4-e]azepin-2-amine |
| II-436 | 4-{[4-(2,6-difluorophenyl)-1-methyl-1,6-dihydropyrazolo[4,3-*c*]pyrimido[4,5-*e*]azepin-9-yl]amino}benzoic acid |
| II-437 | 1-{4-[4-(2,6-Difluoro-phenyl)-2-methyl-6H-3-thia-5,8,10-triaza-benzo[e]azulen-9-ylamino]-benzoyl}-4-dimethylamino-piperidine-4-carboxylic acid methylamide |
| II-438 | 4-(4-{[7-(2,6-difluorophenyl)-5*H*-furo[3,2-*c*]pyrimido[4,5-*e*]azepin-2-yl]amino}benzoyl)-*N*-methylpiperazine-2-carboxamide |
| II-439 | 4-(4-{[4-(2,6-difluorophenyl)-6*H*-isoxazolo[4,5-*c*]pyrimido[4,5-*e*]azepin-9-yl]amino}benzoyl)-*N*-methylpiperazine-2-carboxamide |
| II-440 | 4-(2,6-difluorophenyl)-9-[(4-{[3-methyl-3-(methylamino)pyrrolidin-1-yl]carbonyl}phenyl)amino]-3,6-dihydroimidazo[4,5-*c*]pyrimido[4,5-*e*]azepin-2(1*H*)-one |
| II-441 | 2-amino-*N*-(3-{[7-(2,6-difluorophenyl)-8,10-dimethyl-5*H*-pyrimido[5,4-*c*]thieno[3,4-*e*]azepin-2-yl]aminolphenyl)-*N*,2-dimethylpropanamide |
| II-442 | 9-chloro-7-(2,6-difluorophenyl)-*N*-{3-[(2,2,6,6-tetramethylpiperidin-4-yl)oxy]phenyl}-5*H*-pyrimido[5,4-*d*] [2]benzazepin-2-amine |
| II-443 | 4-(4-{[9-chloro-7-(2,6-difluorophenyl)-5*H*-pyrimido[5,4-*d*][2]benzazepin-2-yl]amino}phenyl)-*N*-methyl-1-(methylamino)cyclohexanecarboxamide |
| II-444 | 7-(3-{[7-(2-fluoro-6-methoxyphenyl)-9-methoxy-5*H*-pyrimido[5,4-*d*][2]benzazepin-2-yl]amino}phenyl)-1,7-diazaspiro[4.4]nonan-6-one |
| II-445 | 9-chloro-*N*-[4-(3,8-diazabicyclo[3.2.1]oct-3-ylcarbonyl)phenyl]-7-(2,6-difluorophenyl)-5*H*-pyrimido[5,4-*d*][2]benzazepin-2-amine |
| II-446 | 1-(3-{[9-chloro-7-(2,6-difluorophenyl)-5*H*-pyrimido[5,4-*d*][2]benzazepin-2-yl]amino}phenyl)-3,5,5-trimethylpiperazin-2-one |
| II-447 | 9-chloro-*N*-[4-(2,6-dimethylpiperidin-4-yl)phenyl]-7-(2-fluoro-6-methoxyphenyl)-5*H*-pyrimido[5,4-*d*][2]benzazepin-2-amine |
| II-448 | *N*-[4-(1-amino-1-methylethyl)phenyl]-9-chloro-7-(2,6-difluorophenyl)-5*H-*pyrimido[5,4-*d*] [2]benzazepin-2-amine |
| II-449 | *N*-[4-(2,5-diazaspiro[3.4]oct-2-ylcarbonyl)phenyl]-7-(2,6-difluorophenyl)-10-methyl-5*H*-isothiazolo[5,4-*c*]pyrimido[4,5-*e*]azepin-2-amine |
| II-450 | 4-(2,6-difluorophenyl)-1-methyl-9-[(4-{[4-methyl-4-(methylamino)piperidin-1-yl]carbonyl}phenyl)amino]-1,6-dihydro-2*H*-pyrimido[5,4-*c*][1,3]thiazolo[4,5-*e*]azepin-2-one |
| II-451 | 4-(2,6-difluorophenyl)-*N*-[4-(1*H*-imidazol-2-yl)phenyl]-1-methyl-1,6-dihydroimidazo[4,5-*c*]pyrimido[4,5-*e*]azepin-9-amine |
| II-452 | 4-{[7-(2,6-difluorophenyl)-5*H*-[1]benzofuro[2,3-*c*]pyrimido[4,5-*e*]azepin-2-yl]amino}benzoic acid |
| II-453 | 7-(2-fluorophenyl)-*N*-{4-[(3,3,5,5-tetramethylpiperazin-1-yl)carbonyl]phenyl}-8,9,10,11-tetrahydro-5*H*-pyrido[4',3':4,5]thieno[3,2-*c*]pyrimido[4,5-*e*]azepin-2-amine |
| II-454 | 9-bromo-7-(2-fluorophenyl)-*N*-(4-{[3-(methylamino)pyrrolidin-1-yl]carbonyl}phenyl)-5,8-dihydropyrimido[5,4-*c*]pyrrolo[3,2-*e*]azepin-2-amine |
| II-455 | 7-(2-fluorophenyl)-*N*-(3-methyl-1*H*-indazol-6-yl)-5,12-dihydropyrimido[4',5':5,6]azepino[4,3-*b*]indol-2-amine |
| II-456 | 1-(4-{[7-(2,6-difluorophenyl)-9,10-dimethyl-5,8-dihydropyrimido[5,4-*c*]pyrrolo[3,2-*e*]azepin-2-yl]amino}benzoyl)-3-(methylamino)pyrrolidine-3-carboxamide |
| II-457 | {3-[9-Chloro-7-(2-fluoro-6-methoxy-phenyl)-5H-benzo[c]pyrimido-[4,5-e]azepin-2-ylamino]-phenyl}-(4-methyl-piperazin-1-yl)-methanone |
| II-458 | [9-Chloro-7-(2,6-difluoro-phenyl)-5H-benzo[c]pyrimido[4,5-e]azepin-2-yl]-(2-methylaminomethyl-benzothiazol-6-yl)-amine |
| II-459 | 4-[9-Chloro-7-(2-isopropoxy-phenyl)-5*H*-benzo[c]pyrimido[4,5-e]azepin-2-ylamino]-benzoic acid |
| II-460 | 4-[9-Chloro-7-(2-fluoro-6-isopropoxy-phenyl)-5*H*-benzo[c]pyrimido-[4,5-e]azepin-2-ylamino]-benzoic acid |
| II-461 | 4-[9-Chloro-7-(2-ethoxy-phenyl)-5*H*-benzo[c]pyrimido[4,5-e]azepin-2-ylamino]-benzoic acid |
| II-462 | 4-[9-Chloro-7-(2-ethoxy-6-fluoro-phenyl)-5*H*-benzo[c]pyrimido[4,5-e]azepin-2-ylamino]-benzoic acid |
| II-463 | 4-[9-Chloro-7-(2-fluoro-6-methyl-phenyl)-5*H*-benzo[c]pyrimido[4,5-e]azepin-2-ylamino]-benzoic acid |
| II-464 | 4-[9-Chloro-7-(2-trifluoromethoxy-phenyl)-5*H*-benzo[c]pyrimido[4,5-e]azepin-2-ylamino]-benzoic acid |
| II-465 | 4-[9-Chloro-7-(2-fluoro-6-trifluoromethoxy-phenyl)-5*H*-benzo[c]pyrimido-[4,5-e]azepin-2-ylamino]-benzoic acid |
| II-466 | 4-[9-Chloro-7-(3-fluoro-2-trifluoromethoxy-phenyl)-5*H*-benzo[c]pyrimido-[4,5-e]azepin-2-ylamino]-benzoic acid |
| II-467 | 4-[9-Chloro-7-(2,3-dimethoxy-phenyl)-5*H*-benzo[c]pyrimido[4,5-e]azepin-2-ylamino]-benzoic acid |
| II-468 | 4-[9-Chloro-7-(2-isobutyl-phenyl)-5*H*-benzo[c]pyrimido[4,5-e]azepin-2-ylamino]-benzoic acid |
| II-469 | 4-(7-Benzofuran-2-yl-9-chloro-5*H*-benzo[c]pyrimido[4,5-e]azepin-2-ylamino)-benzoic acid |
| II-470 | 4-[9-Chloro-7-(1-methyl-1*H*-pyrrol-2-yl)-5*H*-benzo[c]pyrimido[4,5-e]azepin-2-ylamino]-benzoic acid |
| II-471 | 4-[9-Chloro-7-(1-methyl-1*H*-imidazol-2-yl)-5*H*-benzo[c]pyrimido[4,5-e]azepin-2-ylamino]-benzoic acid |
| II-472 | 4-(9-Chloro-7-thiophen-2-yl-5*H*-benzo[c]pyrimido[4,5-e]azepin-2-ylamino)-benzoic acid |
| II-473 | 4-[9-Chloro-7-(2*H*-pyrazol-3-yl)-5*H*-benzo[c]pyrimido[4,5-e]azepin-2-ylamino]-benzoic acid |
| II-474 | 4-[9-Chloro-7-(2-ethynyl-phenyl)-5*H*-benzo[c]pyrimido[4,5-e]azepin-2-ylamino]-benzoic acid |
| II-475 | 4-[7-(2-Aminomethyl-phenyl)-9-chloro-5*H*-benzo[c]pyrimido[4,5-e]azepin-2-ylamino]-benzoic acid |
| II-476 | 4-[9-Chloro-7-(5-fluoro-2-methoxy-phenyl)-5*H*-benzo[c]pyrimido[4,5-e]azepin-2-ylamino]-benzoic acid |
| II-477 | 4-[9-Chloro-7-(3-methoxy-pyridin-2-yl)-5*H*-benzo[c]pyrimido[4,5-e]azepin-2-ylamino]-benzoic acid |
| II-478 | 4-[8-Fluoro-7-(2-fluoro-phenyl)-5*H*-benzo[c]pyrimido[4,5-e]azepin-2-ylamino]-benzoic acid |
| II-479 | 4-[8-Chloro-7-(2-fluoro-phenyl)-5*H*-benzo[c]pyrimido[4,5-e]azepin-2-ylamino]-benzoic acid |
| II-480 | 4-[11-Fluoro-7-(2-fluoro-phenyl)-5*H*-benzo[c]pyrimido[4,5-e]azepin-2-ylamino]-benzoic acid |
| II-481 | 4-[11-Chloro-7-(2-fluoro-phenyl)-5*H-*benzo[c]pyrimido[4,5-e]azepin-2-ylamino]-benzoic acid |
| II-482 | 6-[9-Chloro-7-(2-fluoro-phenyl)-5*H*-benzo[c]pyrimido[4,5-e]azepin-2-ylamino]-pyridazine-3-carboxylic acid |
| II-483 | 2-[9-Chloro-7-(2-fluoro-phenyl)-5*H*-benzo[c]pyrimido[4,5-e]azepin-2-ylamino]-1*H*-imidazole-4-carboxylic acid |
| II-484 | 4-[9-Chloro-7-(2-fluoro-phenyl)-4-methyl-5*H*-benzo[c]pyrimido[4,5-e]azepin-2-ylamino]-benzoic acid |
| II-485 | 4-[4-Aminomethyl-9-chloro-7-(2-fluoro-phenyl)-5*H*-benzo[c]pyrimido-[4,5-e]azepin-2-ylamino]-benzoic acid |
| II-486 | 4-(9-Aminomethyl-7-phenyl-5*H*-benzo[c]pyrimido[4,5-e]azepin-2-ylamino)-benzoic acid |
| II-487 | 9-Chloro-7-(2-fluorophenyl)-*N*-{4-[(2-methylpiperazin-1-yl)carbonyl]phenyl}-5*H*-pyrimido[5,4-*d*][2]benzazepin-2-amine |
| II-488 | 4-{[9-Chloro-7-(2-fluorophenyl)-5*H*-pyrimido[5,4-*d*][2]benzazepin-2-yl]amino}-*N*-[{3-[(dimethylamino)methyl]azetidin-1-yl}(imino)methyl]benzamide |
| II-489 | 4-{[9-Chloro-7-(2-fluorophenyl)-5*H*-pyrimido[5,4-*d*][2]benzazepin-2-yl]amino}-*N*-[imino(piperazin-1-yl)methyl]benzamide |
| II-490 | 4-{[9-Chloro-7-(2-fluorophenyl)-5*H*-pyrimido[5,4-*d*][2]benzazepin-2-yl]amino}-*N*-[imino(3-methylpiperazin-1-yl)methyl]benzamide |
| II-491 | 4-{[9-Chloro-7-(2-fluorophenyl)-5*H*-pyrimido[5,4-*d*][2]benzazepin-2-yl]amino}-*N*-[[3-(dimethylamino)pyrrolidin-1-yl](imino)methyl]benzamide |
| II-492 | 4-{[9-Chloro-7-(2-fluorophenyl)-5*H*-pyrimido[5,4-*d*][2]benzazepin-2-yl]amino}-*N*-[imino(4-methylpiperazin-1-yl)methyl]benzamide |
| II-493 | 4-{[9-Chloro-7-(2-fluorophenyl)-5*H*-pyrimido[5,4-*d*][2]benzazepin-2-yl]amino}-*N*-[(3,5-dimethylpiperazin-1-yl)(imino)methyl]benzamide |
| II-494 | 1-[[(4-{[9-Chloro-7-(2-fluorophenyl)-5*H*-pyrimido[5,4-*d*][2]benzazepin-2-yl]amino}benzoyl)amino](imino)methyl]pyrrolidine-3-carboxamide |
| II-495 | 1-[[(4-{[9-Chloro-7-(2-fluorophenyl)-5*H*-pyrimido[5,4-*d*][2]benzazepin-2-yl]ammo}benzoyl)ammo](immo)methyl]piperidine-3-carboxamide |
| II-496 | 4-{[9-Chloro-7-(2-fluorophenyl)-5*H*-pyrimido[5,4-*d*][2]benzazepin-2-yl]amino}-*N*-[{4-[(cyclopropylcarbonyl)amino]piperidm-1-yl}(imino)methyl]benzamide |
| II-497 | 4-{[9-Chloro-7-(2-fluorophenyl)-5*H*-pyrimido[5,4-*d*][2]benzazepin-2-yl]amino}-*N*-[(dimethylamino)(imino)methyl]benzamide |
| II-498 | *N*-[[(4-{[9-Chloro-7-(2-fluorophenyl)-5*H*-pyrimido[5,4-*d*][2]benzazepin-2-yl]amino}phenyl)amino](imino)methyl]cyclopropanecarboxamide |
| II-499 | *N*-[[(4-{[9-Chloro-7-(2-fluorophenyl)-5*H*-pyrimido[5,4-*d*][2]benzazepin-2-yl]amino}phenyl)amino](imino)methyl]-3-(dimethylamino)cyclopentanecarboxamide |
| II-500 | 4-({9-Chloro-7-[2-fluoro-6-(trifluoromethyl)phenyl]-5*H*-pyrimido-[5,4-*d*][2]benzazepin-2-yl}amino)benzoic acid |
| II-501 | 4-{[9-Chloro-7-(2,6-dichlorophenyl)-5*H*>-pyrimido[5,4-d][2]benzazepin-2-yl]amino}benzoic acid |
| II-502 | 4-{[9-Chloro-7-(2-fluoro-6-methylphenyl)-5*H*-pyrimido[5,4-*d*][2]benzazepin-2-yl]amino}benzoic acid |
| II-503 | 4-{[7-(2-Bromo-6-chlorophenyl)-9-chloro-5*H*-pyrimido[5,4-*d*][2]benzazepin-2-yl]amino}benzoic acid |
| II-504 | 9-Chloro-7-(2,6-difluorophenyl)-*N*-{4-[(3,5-dimethylpiperazin-1-yl)carbonyl]-3-fluorophenyl}-5*H*-pyrimido[5,4-*d*][2]benzazepin-2-amine |
| II-505 | 4-{[9-Chloro-7-(2,6-difluorophenyl)-5*H*-pyrimido[5,4-*d*][2]benzazepin-2-yl]amino}-*N*-[(3,5-dimethylpiperazin-1-yl)(imino)methyl]-*N*-methylbenzamide |
| II-506 | 4-{[9-Chloro-7-(2,6-difluorophenyl)-5*H*-pyrimido[5,4-*d]*[2]benzazepin-2-yl]amino}-*N*-[[3-(dimethylamino)azetidin-1-yl](imino)methyl]-*N-*methy lbenzamide |
| II-507 | 3-{[9-Chloro-7-(2,6-difluorophenyl)-5*H*-pyrimido[5,4-*d*][2]benzazepin-2-yl]amino}-*N*-[(3,5-dimethylpiperazin-1-yl)(imino)methyl]benzamide |
| II-508 | 3-{[9-Chloro-7-(2,6-difluorophenyl)-5*H*-pyrimido[5,4-*d*][2]benzazepin-2-yl]amino}-*N*-[[3-(dimethylamino)pyrrolidin-1-yl](imino)methyl]benzamide |
| II-509 | 9-Chloro-7-(2,6-difluorophenyl)-*N*-{3-[(3,5-dimethylpiperazin-1-yl)carbonyl]-4-fluoropheny1}-5*H*-pyrimido[5,4-*d*][2]benzazepin-2-amine |
| II-510 | *N*-[[(4-{[9-Chloro-7-(2,6-difluorophenyl)-5*H*-pyrimido[5,4-*d*][2]benzazepin-2-yl]amino}phenyl)amino](imino)methy1]-3-(dimethylamino)cyclopentanecarboxamide |
| II-511 | *N*-[[(4-{[9-Chloro-7-(2,6-difluorophenyl)-5*H*-pyrimido[5,4-*d*][2]benzazepin-2-yl]amino}-2-fluorophenyl)amino](imino)methyl]-3-(dimethylamino)cyclopentanecarboxamide |
| II-512 | *N*-[[(5-{ [9-Chloro-7-(2,6-difluorophenyl)-5*H*-pyrimido[5,4-*d*][2]benzazepin-2-yl]amino}-2-fluorophenyl)amino] (imino)methyl]-3-(dimethylamino)cyclopentanecarboxamide |
| II-513 | *N*-(4-{[9-Chloro-7-(2,6-difluorophenyl)-5*H*-pyrimido[5,4-*d*][2]benzazepin-2-yl]amino}phenyl)-3,5-dimethylpiperazme-1-carboximidamide |
| II-514 | 4-{[9-Chloro-7-(2,6-difluorophenyl)-5*H*-pyrimido[5,4-*d*][2]benzazepin-2-yl]amino}-*N*-[[3-(dimethylamino)pyrrolidin-1-yl](imino)methyl]-*N-*methy lbenzamide |
| II-515 | *N*-(3-{[9-Chloro-7-(2,6-difluorophenyl)-5*H*-pyrimido[5,4-*d*][2]benzazepin-2-yl]amino}phenyl)-3,5-dimethylpiperazme-1-carboximidamide |
| II-516 | *N*-(3-{[9-Chloro-7-(2,6-difluorophenyl)-5*H*-pyrimido[5,4-*d*][2]benzazepin-2-yl]amino}phenyl)-*N*,3,5-trimethylpiperazine-1-carboximidamide |
| II-517 | 3-{[9-Chloro-7-(2,6-difluorophenyl)-5*H*-pyrimido[5,4-*d*][2]benzazepin-2-yl]amino}-*N*-[[3-(diniethylamino)azetdin-1-yl](imino)methyl]benzamide |
| II-518 | *N*-(5-{[9-Chloro-7-(2,6-difluorophenyl)-5*H*-pyrimido[5,4-*d*][2]benzazepin-2-yl]amino}-2-fluorophenyl)-*N*,3,5-trimethylpiperazine-1-carboximidamide |
| II-519 | *N*-[[(3-{ [9-Chloro-7-(2,6-difluorophenyl)-5*H*-pyrimido[5,4-*d*][2]benzazepin-2-yl] amino}phenyl)amino](imino)methyl]-3-(dimethylamino)cyclopentanecarboxamide |
| II-520 | 9-Chloro-7-(2,6-difluorophenyl)-*N*-{3-[(3,5-dimethylpiperazin-1-yl)(imino)methyl]phenyl}-5*H*-pyrimido[5,4-*d*][2]benzazepin-2-amine |
| II-521 | *N*-(4-{[9-Chloro-7-(2,6-difluorophenyl)-5*H*-pyrimido[5,4-*d]*[2]benzazepin-2-yl]amino}phenyl)-*N*,3,5-trimethylpiperazine-1-carboximidamide |
| II-522 | *N*-(4-{[9-Chloro-7-(2,6-difluorophenyl)-5*H*-pyrimido[5,4-*d*][2]benzazepin-2-yl]amino}-2-fluorophenyl)-3,5-dimethylpiperazine-1-carboximidamide |
| II-523 | 9-Chloro-7-(2,6-difluoropheny{4-[(3,5-dimethylpiperazin-1-yl)(imino)methyl]-3-fluorophenyl}-5*H*-pyrimido[5,4-*d*][2]benzazepin-2-amine |
| II-524 | 5-{[9-Chloro-7-(2,6-difluorophenyl)-5*H*-pyrimido[5,4-*d*][2]benzazepin-2-yl]amino}-2-(2,6-dimethylpiperidin-4-yl)-1*H*-isoindole-1,3(2*H*)-dione |
| II-525 | *N*-[2-(Aminomethyl)-1*H*-benzimidazol-6-yl]-9-chloro-7-(2-fluorophenyl)-5*H-*pyrimido[5,4-*d*] [2]benzazepin-2-amine |
| II-526 | 9-Chloro-7-(2-fluorophenyl)-*N*-{2-[(methylamino)methyl]-1*H*-benzimidazol-6-yl}-5*H*-pyrimido[5,4-*d*][2]benzazepin-2-amine |
| II-527 | 9-Chloro-*N*-{2-[(dimethylamino)methyl]-1*H*-benzimidazol-6-yl}-7-(2-fluorophenyl)-5*H*-pyrimido[5,4-*d*][2]benzazepin-2-amine |
| II-528 | 9-Chloro-7-(2-fluorophenyl)-*N*-{2-[(methylamino)methyl]-1,3-benzothiazol-6-yl}-5*H*-pyrimido[5,4-*d*][2]benzazepin-2-amine |
| II-529 | 9-Chloro-7-(2,6-difluorophenyl)-*N*-{2-[(methylamino)methyl]-1*H-*benzimidazol-6-yl}-5*H*-pyrimido[5,4-*d*][2]benzazepin-2-amine |
| II-530 | 9-Chloro-7-(2,6-difluorophenyl)-*N*-{2-[(methylamino)methyl]-1,3-benzoxazol-6-yl}-5*H*-pyrimido[5,4-*d*][2]benzazepin-2-amine |
| II-531 | 9-Chloro-7-(2-fluorophenyl)-*N*-{2-[(methylammo)methyl]-1,3-benzoxazol-6-yl}-5*H*-pyrimido[5,4-*d*][2]benzazepin-2-amine |
| II-532 | 9-Chloro-7-(2,6-difluorophenyl)-*N*-{3-[(3,5-dimethylpiperazin-1-yl)(imino)methyl]-4-fluorophenyl}-5*H*-pyrimido[5,4-*d*][2]benzazepin-2-amine |
| II-533 | 9-Chloro-7-(2,6-difluorophenyl)-*N*-{2-[(methylamino)methyl]-1,3-benzothiazol-6-yl}-5*H*-pyrimido[5,4-*d*][2]benzazepin-2-amine |
| II-534 | {3-[9-Chloro-7-(2,6-difluorophenyl)-5H-benzo[c]pyrimido[4,5-e]azepin-2-ylamino]-phenyl}-(4-methyl-piperazin-1-yl)-methanone |
| II-535 | 3-[9-Chloro-7-(2,6-difluoro-phenyl)-5H-benzo[c]pyrimido[4,5-e]azepin-2-ylamino]-N-methyl-N-(4-methyl-pentyl)-benzamide |

In some embodiments, the selective Aurora A kinase inhibitor is represented by formula (***III***): 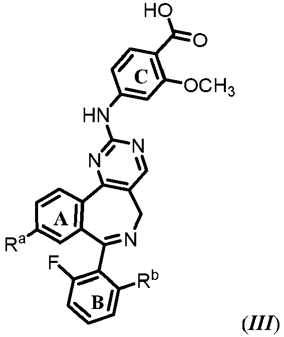 or a pharmaceutically acceptable salt thereof;
wherein:
R^{a} is selected from the group consisting of C₁₋₃ aliphatic, C₁₋₃ fluoroaliphatic, -R¹, -T-R¹, -R², and -T-R²;
   T is a C₁₋₃ alkylene chain optionally substituted with fluoro;
   R¹ is an optionally substituted aryl, heteroaryl, or heterocyclyl group;
   R² is selected from the group consisting of halo, -C≡C-R³, -CH=CH-R³, -N(R⁴)₂, and -OR⁵;
   R³ is hydrogen or an optionally substituted aliphatic, aryl, heteroaryl, or heterocyclyl group;
   each R⁴ independently is hydrogen or an optionally substituted aliphatic, aryl, heteroaryl, or heterocyclyl group; or two R⁴ on the same nitrogen atom, taken together with the nitrogen atom form an optionally substituted 5- to 6-membered heteroaryl or 4- to 8-membered heterocyclyl ring having, in addition to the nitrogen atom, 0-2 ring heteroatoms selected from N, O, and S;
   R⁵ is hydrogen or an optionally substituted aliphatic, aryl, heteroaryl, or heterocyclyl group; and
R^{b} is selected from the group consisting of fluoro, chloro, -CH₃, -CF₃, -OH, -OCH₃, -OCF₃, -OCH₂CH₃, and -OCH₂CF₃.

In some embodiments, R¹ is a 5- or 6-membered aryl, heteroaryl, or heterocyclyl ring optionally substituted with one or two substituents independently selected from the group consisting of halo, C₁₋₃ aliphatic, and C₁₋₃ fluoroaliphatic. In certain embodiments, R¹ is a phenyl, furyl, pyrrolidinyl, or thienyl ring optionally substituted with one or two substituents independently selected from the group consisting of halo, C₁₋₃ aliphatic, and C₁₋₃ fluoroaliphatic.

In some embodiments, R³ is hydrogen, C₁₋₃ aliphatic, C₁₋₃ fluoroaliphatic, or -CH₂-OCH₃.

In some embodiments, R⁵ is hydrogen, C₁₋₃ aliphatic, or C₁₋₃ fluoroaliphatic.

In certain embodiments, R^{a} is halo, C₁₋₃ aliphatic,
C₁₋₃ fluoroaliphatic, -OH, -O(C₁₋₃ aliphatic), -O(C₁₋₃ fluoroaliphatic), -C≡C-R³, -CH=CH-R³, or an optionally substituted pyrrolidinyl, thienyl, furyl, or phenyl ring, wherein R³ is hydrogen,
C₁₋₃ aliphatic, C₁₋₃ fluoroaliphatic, or -CH₂-OCH₃. In certain particular embodiments, R^{a} is selected from the group consisting of chloro, fluoro, C₁₋₃ aliphatic,
C₁₋₃ fluoroaliphatic, -OCH₃, -OCF₃, -C≡C-H, -C≡C-CH₃, -C≡C-CH₂OCH₃, -CH=CH₂, -CH=CHCH₃, *N*-methylpyrrolidinyl, thienyl, methylthienyl, furyl, methylfuryl, phenyl, fluorophenyl, and tolyl.

Table 2 provides the chemical names for specific examples of compounds of formula (***III***).

**Table 2. Examples of Compounds of Formula (III)**

| | **Chemical Name** |
|---|---|
| III-I | 4-{[9-chloro-7-(2-fluoro-6-methoxyphenyl)-5*H*-pyrimido[5,4-*d*][2]benzazepin-2-yl]amino}-2-methoxybenzoic acid |
| III-2 | 4-{[9-ethynyl-7-(2-fluoro-6-methoxyphenyl)-5*H*-pyrimido[5,4-*d*][2]benzazepin-2-yl]amino}-2-methoxybenzoic acid |
| III-3 | 4-({9-chloro-7-[2-fluoro-6-(trifluoromethoxy)phenyl]-5*H*-pyrimido[5,4-*d*] [2]benzazepin-2-yl}amino)-2-methoxybenzoic acid |
| III-4 | 4-{[7-(2-fluoro-6-methoxyphenyl)-9-(1-methyl-1*H*-pyrrol-2-yl)-5*H*-pyrimido[5,4-*d*] [2]benzazepin-2-yl]amino}-2-methoxybenzoic acid |
| III-5 | 4-{[7-(2-fluoro-6-methoxyphenyl)-9-(4-methyl-3-thienyl)-5*H*-pyrimido[5,4-*d*][2]benzazepin-2-yl]amino}-2-methoxybenzoic acid |
| III-6 | 4-{[7-(2-fluoro-6-methoxyphenyl)-9-(3-methyl-2-furyl)-5*H*-pyrimido[5,4-*d*][2]benzazepin-2-yl]amino}-2-methoxybenzoic acid |
| III-7 | 4-({9-ethynyl-7-[2-fluoro-6-(2,2,2-trifluoroethoxy)phenyl]-5*H*-pyrimido[5,4-*d*][2]benzazepin-2-yl}amino)-2-methoxybenzoic acid |
| III-8 | 4-{[9-chloro-7-(2,6-difluorophenyl)-5*H*-pyrimido[5,4-*d*][2]benzazepin-2-yl]amino}-2-methoxybenzoic acid |
| III-9 | 4-{[7-(2-fluoro-6-methoxyphenyl)-9-(2-methylphenyl)-5*H*-pyrimido[5,4-*d*][2]benzazepin-2-yl]amino}-2-methoxybenzoic acid |
| III-10 | 4-{[7-(2-fluoro-6-methoxyphenyl)-9-prop-1-yn-1-yl-5*H*-pyrimido[5,4-*d*] [2]benzazepin-2-yl]amino}-2-methoxybenzoic acid |
| III-11 | 4-{[7-(2-fluoro-6-methoxyphenyl)-9-vinyl-5*H*-pyrimido[5,4-*d*][2]benzazepin-2-yl]amino}-2-methoxybenzoic acid |
| III-12 | 4-{[7-(2-fluoro-6-methoxyphenyl)-9-(2-fluorophenyl)-5*H*-pyrimido[5,4-*d*] [2]benzazepin-2-yl]amino}-2-methoxybenzoic acid |
| III-13 | 4-{[7-(2-fluoro-6-methoxyphenyl)-9-(3-methoxyprop-1-yn-1-yl)-5*H*-pyrimido[5,4-*d*] [2]benzazepin-2-yl]amino}-2-methoxybenzoic acid |
| III-14 | 4-({7-(2-fluoro-6-methoxyphenyl)-9-[(1*E*)-prop-1-en-1-yl]-5*H*-pyrimido[5,4-*d*] [2]benzazepin-2-yl}amino)-2-methoxybenzoic acid |
| III-15 | 4-({9-chloro-7-[2-fluoro-6-(2,2,2-trifluoroethoxy)phenyl]-5*H*-pyrimido[5,4-*d*][2]benzazepin-2-yl}amino)-2-methoxybenzoic acid |
| III-16 | 4-{[7-(2-fluoro-6-methoxyphenyl)-9-(2-furyl)-5*H*-pyrimido[5,4-*d*][2]benzazepin-2-yl]amino}-2-methoxybenzoic acid |
| III-17 | 4-{[9-chloro-7-(2-fluoro-6-hydroxyphenyl)-5*H*-pyrimido[5,4-*d*][2]benzazepin-2-yl]amino}-2-methoxybenzoic acid |
| III-18 | 4-{[7-(2-fluoro-6-methoxyphenyl)-9-phenyl-5*H*-pyrimido[5,4-*d*][2]benzazepin-2-yl]amino}-2-methoxybenzoic acid |

In one embodiment, the compound of formula (***III***) is 4-{[9-chloro-7-(2-fluoro-6-methoxyphenyl)-5*H*-pyrimido[5,4-*d*][2]benzazepin-2-yl]amino}-2-methoxybenzoic acid (alisertib (MLN8237)), or a pharmaceutically acceptable salt thereof. In a particular embodiment, the compound of formula (***I***) is sodium 4-{[9-chloro-7-(2-fluoro-6-methoxyphenyl)-5*H*-pyrimido[5,4-*d*][2]benzazepin-2-yl]amino}-2-methoxybenzoate. In another embodiment, the compound of formula (***III***) is sodium 4-{[9-chloro-7-(2-fluoro-6-methoxyphenyl)-5*H*-pyrimido[5,4-d][2]benzazepin-2-yl]amino}-2-methoxybenzoate monohydrate. In another embodiment, the compound of formula (***III***) is sodium 4-{[9-chloro-7-(2-fluoro-6-methoxyphenyl)-5*H*-pyrimido[5,4-d][2]benzazepin-2-yl]amino}-2-methoxybenzoate or a crystakline form thereof, as described in US Publication No. 2008/0167292, US Patent No. 8,026,246, and US Publication No. 2011/0245234.

The present disclosure provides a method of treating cancer, comprising administering to a patient in need thereof a therapeutically effective amount of anyone of the compounds of formulas (***I***), (***II***) or (***III***), or a pharmaceutically acceptable salt thereof.

In some embodiments, the disclosure provides anyone of the compounds of formulas (***I***), (***II***) or (***III***), or a pharmaceutically acceptable salt thereof for use in treating cancer. In some embodiments, the disclosure provides the use of anyone of the compounds of formulas (***I***), (***II***) or (***III***), or a pharmaceutically acceptable salt thereof, for the preparation of a pharmaceutical composition (as described herein) for the treatment of cancer. In some embodiments, the disclosure provides the use of a therapeutically effective amount of anyone of the compounds of formulas (***I***), (***II***) or (***III***), or a pharmaceutically acceptable salt thereof, for the treatment of cancer.

In some embodiments, the disclosure provides a method of determining whether to treat a patient with cancer with a therapeutically effective amount of anyone of the compounds of formulas (***I***), (***II***) or (***III***), or a pharmaceutically acceptable salt thereof, based on identifying the patient with cancer as being likely to respond to the treatment based upon the presence of mutations in the patient's cell sample(s). In some embodiments, the disclosure provides a method of determining whether to treat a patient with cancer with a therapeutically effective amount of an Aurora A Kinase inhibitor, e.g., alisertib, or a pharmaceutically acceptable salt thereof based on identifying the patient with cancer as being likely to respond to the treatment based upon the presence of mutations in the patient's cell sample(s). In some embodiments, the mutations in the patient's cell samples are mutations of genes in the Wnt/β-catenin signaling pathway or the Hippo signaling pathway.

In some embodiments, the present disclosure provides a method of treating cancer, comprising administering a therapeutically effective amount of an Aurora A Kinase inhibitor, e.g., alisertib, or a pharmaceutically acceptable salt or pharmaceutical composition thereof, to a cancer patient whose tumor sample is characterized by having a mutation in a gene selected from the group consisting of LEF1, MAP3K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWTR1 and YAP1.

In some embodiments, the disclosure provides a compound of anyone of formulas (***I***), (***II***) or (***III***), or a pharmaceutically acceptable salt or a pharmaceutical composition thereof, for use in treating cancer in a patient with a mutation in a gene selected from the group consisting of LEF1, MAP3K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWTR1 and YAP1.

Described herein is the assessment of outcome for treatment of a tumor through measurement of the amount of pharmacogenomic markers. Also described herein is the assessment of the treatment outcome by noninvasive, convenient or low-cost means, for example, from blood samples. The disclosure provides methods for determining, assessing, advising or providing an appropriate therapy regimen for treating a tumor or managing disease in a patient. Monitoring a treatment using the kits and methods disclosed herein can identify the potential for unfavorable outcome and allow their prevention, and thus a savings in morbidity, mortality and treatment costs through adjustment in the therapeutic regimen, cessation of therapy or use of alternative therapy.

The term "biological sample" is intended to include a patient sample, e.g, tissue, cells, biological fluids and isolates thereof, isolated from a subject, as well as tissues, cells and fluids present within a subject and can be obtained from a patient or a normal subject. In hematological tumors of the bone marrow, *e.g.*, myeloma tumors, primary analysis of the tumor can be performed on bone marrow samples. However, some tumor cells, (*e.g.*, clonotypic tumor cells, circulating endothelial cells), are a percentage of the cell population in whole blood. These cells also can be mobilized into the blood during treatment of the patient with granulocyte-colony stimulating factor (G-CSF) in preparation for a bone marrow transplant, a standard treatment for hematological tumors, e.g., leukemias, lymphomas and myelomas. Examples of circulating tumor cells in multiple myeloma have been studied e.g., by Pilarski et al. (2000) Blood 95:1056-65 and Rigolin et al. (2006) Blood 107:2531-5. Thus, noninvasive samples, *e.g.,* for *in vitro* measurement of markers to determine outcome of treatment, can include peripheral blood samples. Accordingly, cells within peripheral blood can be tested for marker amount. For patients with hematological tumors, a control, reference sample for normal characteristic, e.g., size, sequence, composition or amount can be obtained from skin or a buccal swab of the patient. For solid tumors, a typical tumor sample is a biopsy of the tumor. For solid tumors, a control reference sample for normal characteristic, e.g., size, sequence, composition or amount can be obtained from blood of the patient.

Blood collection containers can comprise an anti-coagulant, *e.g.*, heparin or ethylenediaminetetraacetic acid (EDTA), sodium citrate or citrate solutions with additives to preserve blood integrity, such as dextrose or albumin or buffers, *e.g.*, phosphate. If the amount of marker is being measured by measuring the level of its DNA in the sample, a DNA stabilizer, *e.g.*, an agent that inhibits DNAse, can be added to the sample. If the amount of marker is being measured by measuring the level of its RNA in the sample, an RNA stabilizer, *e.g.*, an agent that inhibits RNAse, can be added to the sample. If the amount of marker is being measured by measuring the level of its protein in the sample, a protein stabilizer, *e.g.*, an agent that inhibits proteases, can be added to the sample. An example of a blood collection container is PAXGENE® tubes (PREANALYTIX, Valencia, CA), useful for RNA stabilization upon blood collection. Peripheral blood samples can be modified, *e.g.*, fractionated, sorted or concentrated (*e.g.*, to result in samples enriched with tumor or depleted of tumor (e.g., for a reference sample)). Examples of modified samples include clonotypic myeloma cells, which can be collected by *e.g.*, negative selection, *e.g.*, separation of white blood cells from red blood cells (*e.g.*, differential centrifugation through a dense sugar or polymer solution (*e.g.*, FICOLL® solution (Amersham Biosciences division of GE healthcare, Piscataway, NJ) or HISTOPAQUE®-1077 solution, Sigma-Aldrich Biotechnology LP and Sigma-Aldrich Co., St. Louis, MO)) and/or positive selection by binding B cells to a selection agent (*e.g.*, a reagent which binds to a tumor cell or myeloid progenitor marker, such as CD34, CD38, CD138, or CD133, for direct isolation (*e.g.*, the application of a magnetic field to solutions of cells comprising magnetic beads (*e.g.*, from Miltenyi Biotec, Auburn, CA) which bind to the B cell markers) or fluorescent-activated cell sorting).

Alternatively, a tumor cell line, *e.g.*, OCI-Ly3, OCI-Ly 10 cell (Alizadeh et al. (2000) Nature 403:503-511), a RPMI 6666 cell, a SUP-B15 cell, a KG-1 cell, a CCRF-SB cell, an 8ES cell, a Kasumi-1 cell, a Kasumi-3 cell, a BDCM cell, an HL-60 cell, a Mo-B cell, a JM1 cell, a GA-10 cell or a B-cell lymphoma (*e.g*., BC-3) or a cell line or a collection of tumor cell lines (see e.g., McDermott et al. (2007) PNAS 104:19936-19941 or ONCOPANEL™ anti-cancer tumor cell profiling screen (Ricerca Biosciences, Bothell, WA)) can be assayed. A skilled artisan readily can select and obtain the appropriate cells (*e.g.*, from American Type Culture Collection (ATCC®), Manassas, VA) that are used in the present method. If the compositions or methods are being used to predict outcome of treatment in a patient or monitor the effectiveness of a therapeutic protocol, then a tissue or blood sample having been obtained from the patient being treated is a useful source of cells or marker gene or gene products for an assay.

The sample, *e.g.*, tumor, e.g., biopsy or bone marrow, blood or modified blood, (*e.g.*, comprising tumor cells) and/or the reference, e.g., matched control (e.g., germline), sample can be subjected to a variety of well-known post-collection preparative and storage techniques (*e.g.*, nucleic acid and/or protein extraction, fixation, storage, freezing, ultrafiltration, concentration, evaporation, centrifugation, *etc.*) prior to assessing the amount of the marker in the sample.

In an embodiment, a mutation in a marker can be identified by sequencing a nucleic acid, e.g., a DNA, RNA, cDNA or a protein correlated with the marker gene. There are several sequencing methods known in the art to sequence nucleic acids. A primer can be designed to bind to a region comprising a potential mutation site or can be designed to complement the mutated sequence rather than the wild type sequence. Primer pairs can be designed to bracket a region comprising a potential mutation in a marker gene. A primer or primer pair can be used for sequencing one or both strands of DNA corresponding to the marker gene. A primer can be used in conjunction with a probe to amplify a region of interest prior to sequencing to boost sequence amounts for detection of a mutation in a marker gene. Examples of regions which can be sequenced include an entire gene, transcripts of the gene and a fragment of the gene or the transcript, e.g., one or more of exons or untranslated regions. Examples of mutations to target for primer selection and sequence or composition analysis can be found in public databases which collect mutation information, such as COSMIC and dbGaP. Some mutations of marker genes such as LEF1, MAP2K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWC1, WWTR1 and YAP1, etc. are listed in Tables 9-10 in the Examples as examples of mutations that can be associated with sensitivity to Aurora A Kinase inhibition, e.g., inhibition by alisertib.

Sequencing methods are known to one skilled in the art. Examples of methods include the Sanger method, the SEQUENOM™ method and Next Generation Sequencing (NGS) methods. The Sanger method, comprising using electrophoresis, e.g., capillary electrophoresis to separate primer-elongated labeled DNA fragments, can be automated for high-throughput applications. The primer extension sequencing can be performed after PCR amplification of regions of interest. Software can assist with sequence base calling and with mutation identification. SEQUENOM™ MASSARRAY® sequencing analysis (San Diego, CA) is a mass-spectrometry method which compares actual mass to expected mass of particular fragments of interest to identify mutations. NGS technology (also called "massively parallel sequencing" and "second generation sequencing") in general provides for much higher throughput than previous methods and uses a variety of approaches (reviewed in Zhang et al. (2011) J. Genet. Genomics 38:95-109 and Shendure and Hanlee (2008) Nature Biotech. 26:1135-1145). NGS methods can identify low frequency mutations in a marker in a sample. Some NGS methods (see, e.g., GS-FLX Genome Sequencer (Roche Applied Science, Branford, CT), Genome analyzer (Illumina, Inc. San Diego, CA) SOLID™ analyzer (Applied Biosystems, Carlsbad, CA), Polonator G.007 (Dover Systems, Salem, NH), HELISCOPE™ (Helicos Biosciences Corp., Cambridge, MA)) use cyclic array sequencing, with or without clonal amplification of PCR products spatially separated in a flow cell and various schemes to detect the labeled modified nucleotide that is incorporated by the sequencing enzyme (e.g., polymerase or ligase). In one NGS method, primer pairs can be used in PCR reactions to amplify regions of interest. Amplified regions can be ligated into a concatenated product. Clonal libraries are generated in the flow cell from the PCR or ligated products and further amplified ("bridge" or "cluster" PCR) for single-end sequencing as the polymerase adds a labeled, reversibly terminated base that is imaged in one of four channels, depending on the identity of the labeled base and then removed for the next cycle. Software can aid in the comparison to genomic sequences to identify mutations.

Composition of proteins and nucleic acids can be determined by many ways known in the art, such as by treating them in ways that cleave, degrade or digest them and then analyzing the components. Mass spectrometry, electrophoresis and chromatography can separate and define components for comparison. Mutations which cause deletions or insertions can be identified by size or charge differences in these methods. Protein digestion or restriction enzyme nucleic acid digestion can reveal different fragment patterns after some mutations. Antibodies that recognize particular mutant amino acids in their structural contexts can identify and detect these mutations in samples (see below).

In an embodiment, DNA, e.g., genomic DNA corresponding to the wild type or mutated marker can be analyzed both by *in situ* and by *in vitro* formats in a biological sample using methods known in the art. DNA can be directly isolated from the sample or isolated after isolating another cellular component, e.g., RNA or protein. Kits are available for DNA isolation, e.g., QIAAMP® DNA Micro Kit (Qiagen, Valencia, CA). DNA also can be amplified using such kits.

In another embodiment, mRNA corresponding to the marker can be analyzed both by *in situ* and by *in vitro* formats in a biological sample using methods known in the art. Many expression detection methods use isolated RNA. For *in vitro* methods, any RNA isolation technique that does not select against the isolation of mRNA can be utilized for the purification of RNA from tumor cells (see, *e.g.,* Ausubel et al., ed., Current Protocols in Molecular Biology, John Wiley & Sons, New York 1987-1999). Additionally, large numbers of tissue samples can readily be processed using techniques well known to those of skill in the art, such as, for example, the single-step RNA isolation process of Chomczynski (1989, U.S. Patent No. 4,843,155). RNA can be isolated using standard procedures (see *e.g.,* Chomczynski and Sacchi (1987) Anal. Biochem.162:156-159), solutions (*e.g.*, trizol, TRI REAGENT® (Molecular Research Center, Inc., Cincinnati, OH; see U.S. Patent No. 5,346,994) or kits (*e.g*., a QIAGEN® Group RNEASY® isolation kit (Valencia, CA) or LEUKOLOCK™ Total RNA Isolation System, Ambion division of Applied Biosystems, Austin, TX).

Additional steps may be employed to remove DNA from RNA samples. Cell lysis can be accomplished with a nonionic detergent, followed by microcentrifugation to remove the nuclei and hence the bulk of the cellular DNA. DNA subsequently can be isolated from the nuclei for DNA analysis. In one embodiment, RNA is extracted from cells of the various types of interest using guanidinium thiocyanate lysis followed by CsCl centrifugation to separate the RNA from DNA (Chirgwin et al. (1979) Biochemistry 18:5294-99). Poly(A)+RNA is selected by selection with oligo-dT cellulose (see Sambrook et al. (1989) Molecular Cloning--A Laboratory Manual (2nd ed.), Cold Spring Harbor Laboratory, Cold Spring Harbor, N.Y.). Alternatively, separation of RNA from DNA can be accomplished by organic extraction, for example, with hot phenol or phenol/chloroform/isoamyl alcohol. If desired, RNAse inhibitors may be added to the lysis buffer. Likewise, for certain cell types, it may be desirable to add a protein denaturation/digestion step to the protocol. For many applications, it is desirable to enrich mRNA with respect to other cellular RNAs, such as transfer RNA (tRNA) and ribosomal RNA (rRNA). Most mRNAs contain a poly(A) tail at their 3' end. This allows them to be enriched by affinity chromatography, for example, using oligo(dT) or poly(U) coupled to a solid support, such as cellulose or SEPHADEX.R™. medium (see Ausubel et al. (1994) Current Protocols In Molecular Biology, vol. 2, Current Protocols Publishing, New York). Once bound, poly(A)+mRNA is eluted from the affinity column using 2 mM EDTA/0.1% SDS.

The characteristic of a marker of the disclosure in a biological sample involves obtaining a biological sample (*e.g.*, a bone marrow sample, a tumor biopsy or a reference sample) from a test subject may be assessed by any of a wide variety of well known methods for detecting or measuring the characteristic, e.g., of a nucleic acid (*e.g*., RNA, mRNA, genomic DNA, or cDNA) and/or translated protein. Non-limiting examples of such methods include immunological methods for detection of secreted, cell-surface, cytoplasmic, or nuclear proteins, protein purification methods, protein function or activity assays, nucleic acid hybridization methods, nucleic acid reverse transcription methods, and nucleic acid amplification methods. These methods include gene array/chip technology, RT-PCR, TAQMAN® gene expression assays (Applied Biosystems, Foster City, CA), e.g., under GLP approved laboratory conditions, *in situ* hybridization, immunohistochemistry, immunoblotting, FISH (flourescence *in situ* hybridization), FACS analyses, northern blot, southern blot, INFINIUM® DNA analysis Bead Chips (Illumina, Inc., San Diego, CA), quantitative PCR, bacterial artificial chromosome arrays, single nucleotide polymorphism (SNP) arrays (Affymetrix, Santa Clara, CA) or cytogenetic analyses. The detection methods of the disclosure can thus be used to detect RNA, mRNA, protein, cDNA, or genomic DNA, for example, in a biological sample *in vitro* as well as *in vivo.* Furthermore, *in vivo* techniques for detection of a polypeptide or nucleic acid corresponding to a marker of the disclosure include introducing into a subject a labeled probe to detect the biomarker, e.g., a nucleic acid complementary to the transcript of a biomarker or a labeled antibody, Fc receptor or antigen directed against the polypeptide, e.g., wild type or mutant marker. For example, the antibody can be labeled with a radioactive isotope whose presence and location in a subject can be detected by standard imaging techniques. These assays can be conducted in a variety of ways. A skilled artisan can select from these or other appropriate and available methods based on the nature of the marker(s), tissue sample and mutation in question. Some methods are described in more detail in later sections. Different methods or combinations of methods could be appropriate in different cases or, for instance in different types of tumors or patient populations.

*In vitro* techniques for detection of a polypeptide corresponding to a marker of the disclosure include enzyme linked immunosorbent assays (ELISAs), Western blots, protein array, immunoprecipitations and immunofluorescence. In such examples, expression of a marker is assessed using an antibody (*e.g*., a radio-labeled, chromophore-labeled, fluorophore-labeled, or enzyme-labeled antibody), an antibody derivative (*e.g*., an antibody conjugated with a substrate or with the protein or ligand of a protein-ligand pair (*e.g.,* biotin-streptavidin)), or an antibody fragment (*e.g*., a single-chain antibody, an isolated antibody hypervariable domain, *etc*.) which binds specifically with a marker protein or fragment thereof, e.g., a protein or fragment comprising a region which can be mutated or a portion comprising a mutated sequence, or a mutated residue in its structural context, including a marker protein which has undergone all or a portion of its normal post-translational modification. An antibody can detect a protein with an amino acid sequence selected from the group of proteins disclosed by GenPept Accession numbers within Tables 9 and 10 herein. Alternatively, an antibody can detect a mutated protein with a variant amino acid sequence selected from the group of proteins disclosed by GenPept Accession numbers within Tables 9 and 10 herein. Residues listed as mutated in public databases such as COSMIC of dbGaP can be prepared in immunogenic compositions for generation of antibodies that will specifically recognize and bind to the mutant residues. Another method can employ pairs of antibodies, wherein one of the pair would bind a marker protein upstream, i.e. N-terminal to the region of expected mutation, e.g., nonsense or deletion and the other of the pair would bind the protein downstream. Wild type protein would bind both antibodies of the pair, but a protein with a nonsense or deletion mutation would bind only the N-terminal antibody of the pair. An assay such as a sandwich ELISA assay could detect a loss of quantity of the wild type protein in the tumor sample, e.g., in comparison to the reference sample, or a standard ELISA would comparison of the levels of binding of the antibodies to infer that a mutation is present in a tumor sample.

Indirect methods for determining the amount or functionality of a protein marker also include measurement of the activity of the protein. For example, a sample, or a protein isolated from the sample or expressed from nucleic acid isolated, cloned or amplified from the sample can be assessed for marker protein activity. Biomarker activity can be measured by its ability to associate with binding partners, e.g., in a cell-free assay or in a cell-based assay. Alternatively, biomarker activity can be measured by its activity in signal transduction, e.g., in a cell-free assay or in a cell-based assay.

In one embodiment, expression of a marker is assessed by preparing mRNA/cDNA (*i.e*., a transcribed polynucleotide) from cells in a patient sample, and by hybridizing the mRNA/cDNA with a reference polynucleotide which is a complement of a marker nucleic acid, or a fragment thereof. cDNA can, optionally, be amplified using any of a variety of polymerase chain reaction methods prior to hybridization with the reference polynucleotide. Expression of one or more markers likewise can be detected using quantitative PCR to assess the level of expression of the marker(s). An example of the use of measuring mRNA levels is that an inactivating mutation in a marker gene can result in an altered level of mRNA in a cell. The level can be upregulated due to feedback signaling protein production in view of nonfunctional or absent protein or downregulated due to instability of an altered mRNA sequence. Alternatively, any of the many known methods of detecting mutations or variants (*e.g.* single nucleotide polymorphisms, deletions, etc., discussed above) of a marker of the disclosure may be used to detect occurrence of a mutation in a marker gene in a patient.

An example of direct measurement is quantification of transcripts. As used herein, the level or amount of expression refers to the absolute amount of expression of an mRNA encoded by the marker or the absolute amount of expression of the protein encoded by the marker. As an alternative to making determinations based on the absolute expression amount of selected markers, determinations may be based on normalized expression amounts. Expression amount can be normalized by correcting the absolute expression level of a marker upon comparing its expression to the expression of a control marker that is not a marker, *e.g*., in a housekeeping role that is constitutively expressed. Suitable markers for normalization also include housekeeping genes, such as the actin gene or beta-2 microglobulin. Reference markers for data normalization purposes include markers which are ubiquitously expressed and/or whose expression is not regulated by oncogenes. Constitutively expressed genes are known in the art and can be identified and selected according to the relevant tissue and/or situation of the patient and the analysis methods. Such normalization allows one to compare the expression level in one sample, to another sample, *e.g*., between samples from different times or different subjects. Further, the expression level can be provided as a relative expression level. The baseline of a genomic DNA sample, *e.g*., diploid copy number, can be determined by measuring amounts in cells from subjects without a tumor or in non-tumor cells from the patient. To determine a relative amount of a marker or marker set, the amount of the marker or marker set is determined for at least 1, or 2, 3, 4, 5, or more samples, *e.g*., 7, 10, 15, 20 or 50 or more samples in order to establish a baseline, prior to the determination of the expression level for the sample in question. To establish a baseline measurement, the mean amount or level of each of the markers or marker sets assayed in the larger number of samples is determined and this is used as a baseline expression level for the biomarkers or biomarker sets in question. The amount of the marker or marker set determined for the test sample (*e.g*., absolute level of expression) is then divided by the baseline value obtained for that marker or marker set. This provides a relative amount and aids in identifying abnormal levels of marker protein activity.

Probes based on the sequence of a nucleic acid molecule of the disclosure can be used to detect transcripts or genomic sequences corresponding to one or more markers of the disclosure. The probe can comprise a label group attached thereto, *e.g.,* a radioisotope, a fluorescent compound, an enzyme, or an enzyme co-factor. Such probes can be used as part of a diagnostic test kit for identifying cells or tissues which express the protein, such as by measuring levels of a nucleic acid molecule encoding the protein in a sample of cells from a subject, *e.g*., detecting mRNA levels or determining whether a gene encoding the protein has been mutated or deleted.

In addition to the nucleotide sequences described in the database records described herein, it will be appreciated by those skilled in the art that DNA sequence polymorphisms that lead to changes in the amino acid sequence can exist within a population (*e.g*., the human population). Such genetic polymorphisms can exist among individuals within a population due to naturally occuring allelic variation. An allele is one of a group of genes which occur alternatively at a given genetic locus. In addition, it will be appreciated that DNA polymorphisms that affect RNA expression levels can also exist that may affect the overall expression level of that gene (*e.g*., by affecting regulation or degradation).

Primers or nucleic acid probes comprise a nucleotide sequence complementary to a specific marker or a mutated region thereof and are of sufficient length to selectively hybridize with a marker gene or nucleic acid associated with a marker gene. Primers and probes can be used to aid in the isolation and sequencing of marker nucleic acids. In one embodiment, the primer or nucleic acid probe, *e.g*., a substantially purified oligonucleotide, comprises a region having a nucleotide sequence which hybridizes under stringent conditions to about 6, 8, 10, 12, or 15, 20, 25, 30, 40, 50, 60, 75, 100 or more consecutive nucleotides of a marker gene. In another embodiment, the primer or nucleic acid probe is capable of hybridizing to a marker nucleic acid comprising a nucleotide sequence disclosed by GenBank Accession number within Tables 9 and 10 herein, or a complement of any of the foregoing. For example, a primer or nucleic acid probe comprising a nucleotide sequence of at least about 15 consecutive nucleotides, at least about 25 nucleotides or having from about 15 to about 20 nucleotides, 10 to 50 consecutive nucleotides, 12 to 35 consecutive nucleotides, 15 to 50 consecutive nucleotides, 20 to 100 consecutive nucleotides set forth in any of the nucleotide sequences disclosed by GenBank Accession number within Tables 9 and 10 herein, or a complement of any of the foregoing are provided by the disclosure. Primers or nucleic acid probes having a sequence of more than about 25 nucleotides are also within the scope of the disclosure. In another embodiment, a primer or nucleic acid probe can have a sequence at least 70%, at least 75%, 80% or 85%, or at least, 90%, 95% or 97% identical to the nucleotide sequence of any nucleotide sequence disclosed by GenBank Accession number within Tables 9 and 10 herein, or a complement of any of the foregoing. Nucleic acid analogs can be used as binding sites for hybridization. An example of a suitable nucleic acid analogue is peptide nucleic acid (see, *e.g.,* Egholm et al., Nature 363:566 568 (1993); U.S. Pat. No. 5,539,083).

Primers or nucleic acid probes can be selected using an algorithm that takes into account binding energies, base composition, sequence complexity, cross-hybridization binding energies, and secondary structure (see Friend et al., International Patent Publication WO 01/05935, published Jan. 25, 2001; Hughes et al., Nat. Biotech. 19:342-7 (2001). Useful primers or nucleic acid probes of the disclosure bind sequences which are unique for each transcript, e.g., target mutated regions and can be used in PCR for amplifying, detecting and sequencing only that particular nucleic acid, e.g., transcript or mutated transcript. Examples of some mutations of marker genes, e.g., LEF1, MAP2K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWC1, WWTR1 and YAP1, etc... are found in Tables in the Examples (Tables 9-10). Other mutations are described in reference articles cited herein and in public databases described herein. One of skill in the art can design primers and nucleic acid probes for the markers disclosed herein or related markers with similar characteristics, e.g., markers on the chromosome loci, or mutations in different regions of the same marker gene described herein, using the skill in the art, e.g., adjusting the potential for primer or nucleic acid probe binding to standard sequences, mutants or allelic variants by manipulating degeneracy or GC content in the primer or nucleic acid probe. Computer programs that are well known in the art are useful in the design of primers with the required specificity and optimal amplification properties, such as Oligo version 5.0 (National Biosciences, Plymouth, MN). While perfectly complementary nucleic acid probes and primers can be used for detecting the markers described herein and mutants, polymorphisms or alleles thereof, departures from complete complementarity are contemplated where such departures do not prevent the molecule from specifically hybridizing to the target region. For example, an oligonucleotide primer may have a non-complementary fragment at its 5' end, with the remainder of the primer being complementary to the target region. Alternatively, non-complementary nucleotides may be interspersed into the nucleic acid probe or primer as long as the resulting probe or primer is still capable of specifically hybridizing to the target region.

An indication of treatment outcome can be assessed by studying the amount of 1 marker, 2 markers, 3 markers or 4 markers, or more, *e.g*., 5, 6, 7, 8, 9, 10, 15, 20, or 25 markers, or mutated portions thereof e.g., marker genes which participate in or interact with the Wnt/β-catenin signaling pathway, marker genes which participate in or interact with the Hippo pathway, or marker genes which participate in or interact with tumor suppressors. Markers can be studied in combination with another measure of treatment outcome, *e.g.,* biochemical markers (*e.g*., M protein, proteinuria) or histology markers (e.g., blast count, number of mitotic figures per unit area).

Any marker, *e.g.,* marker gene or combination of marker, *e.g*., marker genes of the disclosure, or mutations thereof as well as any known markers in combination with the markers, *e.g*., marker genes of the disclosure, may be used in the compositions, kits, and methods of the present disclosure. In general, markers are selected for as great as possible difference between the characteristic, e.g., size, sequence, composition or amount of the marker in samples comprising tumor cells and the characteristic, e.g., size, sequence, composition or amount of the same marker in control cells. Although this difference can be as small as the limit of detection of the method for assessing the amount of the marker, in another embodiment, the difference can be at least greater than the standard error of the assessment method. In the case of RNA or protein amount, a difference can be at least 1.5-, 2-, 3-, 4-, 5-, 6-, 7-, 8-, 9-, 10-, 15-, 20-, 25-, 100-, 500-, 1000-fold or greater. "Low" RNA or protein amount can be that expression relative to the overall mean across tumor samples (e.g., hematological tumor, e.g., myeloma) is low. In the case of amount of DNA, e.g., copy number, the amount is 0, 1, 2, 3, 4, 5, 6, or more copies. A deletion causes the copy number to be 0 or 1; an amplification causes the copy number to be greater than 2. The difference can be qualified by a confidence level, e.g., p < 0.05, p < 0.02, p < 0.01 or lower p-value.

Measurement of more than one marker, *e.g.,* a set of 2, 3, 4, 5, 6, 7, 8, 9, 10, 12, 15, 20, or 25 or more markers can provide an expression profile or a trend indicative of treatment outcome. In some embodiments, the marker set comprises no more than 2, 3, 4, 5, 6, 7, 8, 9, 10, 12, 15, 20, or 25 markers. In some embodiments, the marker set comprises 1-5, 1-10, 1-15, 1-20, 1-25, 2-5, 2-10, 2-15, 2-20, 2-25, 3-5, 3-10, 3-15, 3-20, 3-25, 4-10, 4-15, 4-20, 4-25, 5-10, 5-15, 5-20, 5-25, 6-10, 6-15, 6-20, 6-25, 7-10, 7-15, 7-20, 7-25, 8-10, 8-15, 8-20, 8-25, 9-15, 9-20, 9-25, 10-15, 10-20, 10-25, 11-15, 11-20, 11-25, 12-15, 12-20, 12-25, 13-15, 13-20, 13-20, 14-20, 14-25, 15-20, 15-25, 16-20, 16-25, 17-20, 17-25, 18-20, 18-25, 19-25, 20-25, 21-25, 22-25, 23-25 or 24-25 markers. In some embodiments, the marker set includes a plurality of chromosome loci, a plurality of marker genes, or a plurality of markers of one or more marker genes (e.g., nucleic acid and protein, genomic DNA and mRNA, or various combinations of markers described herein). Analysis of treatment outcome through assessing the amount of markers in a set can be accompanied by a statistical method, *e.g.*, a weighted voting analysis which accounts for variables which can affect the contribution of the amount of a marker in the set to the class or trend of treatment outcome, *e.g*., the signal-to-noise ratio of the measurement or hybridization efficiency for each marker. A marker set, *e.g.,* a set of 2, 3, 4, 5, 6, 7, 8, 9, 10, 12, 15, 20, or 25 or more markers, can comprise a primer, probe or primers to analyze at least one marker DNA or RNA described herein, *e.g*., LEF1, MAP3K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWTR1 and/or YAP1, or a complement of any of the foregoing. A marker set, e.g., a set of 2, 3, 4, 5, 6, 7, 8, 9, 10, 12, 15, 20, or 25 or more markers, can comprise a primer, probe or primers to detect at least one or at least two or more markers, or at least 2, 3, 4, 5, 6, 7, 8, 9, 10, 12, 15, 20, or 25 or more mutations on the markers *e.g*., LEF1, MAP3K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWTR1 and/or YAP1. In another embodiment, a marker set can comprise LEF1, MAP3K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWTR1 and/or YAP1. In an embodiment, a marker set for breast cancer comprises LEF1, MAP3K7, FZD2, LATS1 and/or WWC1. In an embodiment, a marker set for gastric cancer comprises FZD2 and/or LATS2. In an embodiment, a marker set for head and neck cancer comprises MAP3K7, JUN, ROR2, CCND1, LATS1, MOB1B and/or NPHP4. In an embodiment, a marker set for non-small cell lung cancer comprises XPO1 and/or TJP1. In an embodiment, a marker set for small cell lung cancer comprises LEF1, APC, PRKCA, RORA, CAMK2G, CTNNB1, AMOT, DVL2, TJP1, TJP2, WWTR1 and/or YAP1. Selected marker sets can be assembled from the markers provided herein or selected from among markers using methods provided herein and analogous methods known in the art. A way to qualify a new marker for use in an assay of the disclosure is to correlate DNA copy number in a sample comprising tumor cells with differences in expression (e.g., fold-change from baseline) of a marker, *e.g.,* a marker gene. A useful way to judge the relationship is to calculate the coefficient of determination r2, after solving for r, the Pearson product moment correlation coefficient and/or preparing a least squares plot, using standard statistical methods. A correlation can analyze DNA copy number versus the level of expression of marker, *e.g.,* a marker gene. A gene product can be selected as a marker if the result of the correlation (r2, e.g., the linear slope of the data in this analysis), is at least 0.1- 0.2, at least 0.3-0.5, or at least 0.6-0.8 or more. Markers can vary with a positive correlation to response, TTP or survival (i.e., change expression levels in the same manner as copy number, e.g., decrease when copy number is decreased). Markers which vary with a negative correlation to copy number (i.e., change expression levels in the opposite manner as copy number levels, e.g., increase when copy number is decreased) provide inconsistent determination of outcome.

Another way to qualify a new marker for use in the assay would be to assay the expression of large numbers of markers in a number of subjects before and after treatment with a test agent. The expression results allow identification of the markers which show large changes in a given direction after treatment relative to the pre-treatment samples. One can build a repeated-measures linear regression model to identify the genes that show statistically significant changes or differences. To then rank these significant genes, one can calculate the area under the change from *e.g*., baseline vs time curve. This can result in a list of genes that would show the largest statistically significant changes. Then several markers can be combined together in a set by using such methods as principle component analysis, clustering methods (*e.g*., k-means, hierarchical), multivariate analysis of variance (MANOVA), or linear regression techniques. To use such a gene (or group of genes) as a marker, genes which show 2-, 2.5-, 3-, 3.5-, 4-, 4.5-, 5-, 7-,10- fold, or more differences of expression from baseline would be included in the marker set. An expression profile, *e.g.,* a composite of the expression level differences from baseline or reference of the aggregate marker set would indicate at trend, *e.g.,* if a majority of markers show a particular result, *e.g.,* a significant difference from baseline or reference, e.g., 60%, 70%, 80%, 90%, 95% or more markers; or more markers, *e.g*., 10% more, 20% more, 30% more, 40% more, show a significant result in one direction than the other direction.

In an embodiment, a probe set can comprise probes for assessing characteristics of markers selected from the group consisting of LEF1, MAP3K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWTR1 and YAP1. In an embodiment, a probe set for breast cancer comprises probes for assessing characteristics of LEF1, MAP3K7, FZD2, LATS1 and/or WWC1. In an embodiment, a probe set for gastric cancer comprises probes for assessing characteristics of FZD2 and/or LATS2. In an embodiment, a probe set for head and neck cancer comprises probes for assessing characteristics of MAP3K7, JUN, ROR2, CCND1, LATS1, MOB1B and/or NPHP4. In an embodiment, a probe set for non-small cell lung cancer comprises probes for assessing characteristics XPO1 and/or TJP1. In an embodiment, a probe set for small cell lung cancer comprises probes for assessing characteristics of LEF1, APC, PRKCA, RORA, CAMK2G, CTNNB1, AMOT, DVL2, TJP1, TJP2, WWTR1 and/or YAP1.

In embodiments when the compositions, kits, and methods of the disclosure are used for characterizing treatment outcome in a patient, the marker or set of markers of the disclosure is selected such that a significant result is obtained in at least about 20%, at least about 40%, 60%, or 80%, or in substantially all patients treated with the test agent. The marker or set of markers of the disclosure can be selected such that a positive predictive value (PPV) of greater than about 10% is obtained for the general population and additional confidence in a marker can be inferred when the PPV is coupled with an assay specificity greater than 80%.

### Detection Methods

A general principle of prognostic assays involves preparing a sample or reaction mixture that may contain a marker, and a probe, under appropriate conditions and for a time sufficient to allow the marker and probe to interact and bind, thus forming a complex that can be removed and/or detected in the reaction mixture. These assays can be conducted in a variety of ways.

For example, one method to conduct such an assay would involve anchoring the marker or probe onto a solid phase support, also referred to as a substrate, and detecting target marker/probe complexes anchored on the solid phase at the end of the reaction. In one embodiment of such a method, a sample from a subject, which is to be assayed for presence and/or concentration of marker, can be anchored onto a carrier or solid phase support. In another embodiment, the reverse situation is possible, in which the probe can be anchored to a solid phase and a sample from a subject can be allowed to react as an unanchored component of the assay. One example of such an embodiment includes use of an array or chip which contains a predictive marker or marker set anchored for expression analysis of the sample.

There are many established methods for anchoring assay components to a solid phase. These include, without limitation, marker or probe molecules which are immobilized through conjugation of biotin and streptavidin. Such biotinylated assay components can be prepared from biotin-NHS (N-hydroxy-succinimide) using techniques known in the art (*e.g*., biotinylation kit, Pierce Chemicals, Rockford, IL), and immobilized in the wells of streptavidin-coated 96 well plates (Pierce Chemical). In certain embodiments, the surfaces with immobilized assay components can be prepared in advance and stored.

Other suitable carriers or solid phase supports for such assays include any material capable of binding the class of molecule to which the marker or probe belongs. Well-known supports or carriers include, but are not limited to, glass, polystyrene, nylon, polypropylene, nylon, polyethylene, dextran, amylases, natural and modified celluloses, polyacrylamides, gabbros, and magnetite. One skilled in the art will know many other suitable carriers for binding antibody or antigen, and will be able to adapt such support for use with the present disclosure. For example, protein isolated from cells can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

In order to conduct assays with the above mentioned approaches, the non-immobilized component is added to the solid phase upon which the second component is anchored. After the reaction is complete, uncomplexed components may be removed (*e.g*., by washing) under conditions such that any complexes formed will remain immobilized upon the solid phase. The detection of marker/probe complexes anchored to the solid phase can be accomplished in a number of methods outlined herein.

In an embodiment, the probe, when it is the unanchored assay component, can be labeled for the purpose of detection and readout of the assay, either directly or indirectly, with detectable labels discussed herein and which are well-known to one skilled in the art. The term "labeled", with regard to the probe (*e.g*., nucleic acid or antibody), is intended to encompass direct labeling of the probe by coupling (*i.e*., physically linking) a detectable substance to the probe, as well as indirect labeling of the probe by reactivity with another reagent that is directly labeled. An example of indirect labeling includes detection of a primary antibody using a fluorescently labeled secondary antibody. It is also possible to directly detect marker/probe complex formation without further manipulation or labeling of either component (marker or probe), for example by utilizing the technique of fluorescence energy transfer (FET, see, for example, Lakowicz et al., U.S. Patent No. 5,631,169; Stavrianopoulos, et al., U.S. Patent No. 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (*e.g*., using a fluorimeter).

In another embodiment, determination of the ability of a probe to recognize a marker can be accomplished without labeling either assay component (probe or marker) by utilizing a technology such as real-time Biomolecular Interaction Analysis (BIA) (see, *e.g*., Sjolander, S. and Urbaniczky, C. (1991) Anal. Chem. 63:2338-2345 and Szabo et al. (1995) Curr. Opin. Struct. Biol. 5:699-705). As used herein, "BIA" or "surface plasmon resonance" is a technology for studying biospecific interactions in real time, without labeling any of the interactants (*e.g*., BIACORE™). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, in another embodiment, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas, G., and Minton, A.P. (1993) Trends Biochem Sci. 18:284-7). Standard chromatographic techniques also can be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, *e.g.,* Heegaard, N.H. (1998) J. Mol. Recognit. 11:141-8; Hage, D.S., and Tweed, S.A. (1997) J. Chromatogr. B. Biomed. Sci. Appl. 699:499-525). Gel electrophoresis may also be employed to separate complexed assay components from unbound components (see, *e.g.,* Ausubel et al., ed., Current Protocols in Molecular Biology, John Wiley & Sons, New York, 1987-1999). In this technique, protein or nucleic acid complexes are separated based on size or charge, for example. In some embodiments, non-denaturing gel matrix materials and conditions in the absence of reducing agent are used in order to maintain the binding interaction during the electrophoretic process. Appropriate conditions to the particular assay and components thereof will be well known to one skilled in the art.

The isolated mRNA can be used in hybridization or amplification assays that include, but are not limited to, Southern or Northern analyses, polymerase chain reaction and TAQMAN® gene expression assays (Applied Biosystems, Foster City, CA) and probe arrays. One diagnostic method for the detection of mRNA levels involves contacting the isolated mRNA with a nucleic acid molecule (probe) that can hybridize to the mRNA encoded by the gene being detected. Nucleic acids comprising mutations of marker genes can be used as probes or primers. The nucleic acid probes or primers of the disclosure can be single stranded DNA (*e.g*., an oligonucleotide), double stranded DNA (*e.g*., double stranded oligonucleotide) or RNA. Primers of the disclosure refer to nucleic acids which hybridize to a nucleic acid sequence which is adjacent to the region of interest and is extended or which covers the region of interest. A nucleic acid probe can be, for example, a full-length cDNA, or a portion thereof, such as an oligonucleotide of 10 to 50 consecutive nucleotides, 15 to 45 consecutive nucleotides, 15 to 75 consecutive nucleotides, 20 to 100 consecutive nucleotides, 25 to 250 consecutive nucleotides, or at least 7, 15, 20, 25, 30, 50, 75, 100, 125, 150, 175, 200, 250 or 500 or more consecutive nucleotides of the marker and sufficient to specifically hybridize under stringent conditions to a mRNA or genomic DNA encoding a marker of the present disclosure. The exact length of the nucleic acid probe will depend on many factors that are routinely considered and practiced by the skilled artisan. Nucleic acid probes of the disclosure may be prepared by chemical synthesis using any suitable methodology known in the art, may be produced by recombinant technology, or may be derived from a biological sample, for example, by restriction digestion. Other suitable probes for use in the diagnostic assays of the disclosure are described herein. The probe can comprise a label group attached thereto, *e.g.,* a radioisotope, a fluorescent compound, an enzyme, an enzyme co-factor, a hapten, a sequence tag, a protein or an antibody. The nucleic acids can be modified at the base moiety, at the sugar moiety, or at the phosphate backbone. An example of a nucleic acid label is incorporated using SUPER™ Modified Base Technology (Nanogen, Bothell, WA, see U.S. Patent No. 7,045,610). The level of expression can be measured as general nucleic acid levels, *e.g.,* after measuring the amplified DNA levels (e.g. using a DNA intercalating dye, *e.g.,* the SYBR green dye (Qiagen Inc., Valencia, CA) or as specific nucleic acids, *e.g*., using a probe based design, with the probes labeled. TAQMAN® assay formats can use the probe-based design to increase specificity and signal-to-noise ratio.

Such primers or probes can be used as part of a diagnostic test kit for identifying cells or tissues which express the protein, such as by measuring amounts of a nucleic acid molecule transcribed in a sample of cells from a subject, *e.g*., detecting transcript, mRNA levels or determining whether a gene encoding the protein has been mutated or deleted. Hybridization of an RNA or a cDNA with the nucleic acid probe can indicate that the marker in question is being expressed. The disclosure further encompasses detecting nucleic acid molecules that differ, due to degeneracy of the genetic code, from the nucleotide sequence of nucleic acids encoding a marker protein (*e.g*., protein having the sequence disclosed by GenPept Accession number within Tables 9 and 10 herein), and thus encode the same protein. It will be appreciated by those skilled in the art that DNA sequence polymorphisms that lead to changes in the amino acid sequence can exist within a population (*e.g*., the human population). Such genetic polymorphisms can exist among individuals within a population due to natural allelic variation. An allele is one of a group of genes which occur alternatively at a given genetic locus. Such natural allelic variations can typically result in 1-5% variance in the nucleotide sequence of a given gene. Alternative alleles can be identified by sequencing the gene of interest in a number of different individuals, e.g., normal samples from individuals. This can be readily carried out by using hybridization probes to identify the same genetic locus in a variety of individuals. Detecting any and all such nucleotide variations and resulting amino acid polymorphisms or variations that are the result of natural allelic variation and that do not alter the functional activity are intended to be within the scope of the disclosure. In addition, it will be appreciated that DNA polymorphisms that affect RNA expression levels can also exist that may affect the overall expression level of that gene (*e.g*., by affecting regulation or degradation).

As used herein, the term "hybridizes" is intended to describe conditions for hybridization and washing under which nucleotide sequences that are significantly identical or homologous to each other remain hybridized to each other. In some embodiments, the conditions are such that sequences at least about 70%, at least about 80%, at least about 85%, 90% or 95% identical to each other remain hybridized to each other for subsequent amplification and/or detection. Stringent conditions vary according to the length of the involved nucleotide sequence but are known to those skilled in the art and can be found or determined based on teachings in Current Protocols in Molecular Biology, Ausubel et al., eds., John Wiley & Sons, Inc. (1995), sections 2, 4 and 6. Additional stringent conditions and formulas for determining such conditions can be found in Molecular Cloning: A Laboratory Manual, Sambrook et al., Cold Spring Harbor Press, Cold Spring Harbor, NY (1989), chapters 7, 9 and 11. A non-limiting example of stringent hybridization conditions for hybrids that are at least 10 basepairs in length includes hybridization in 4X sodium chloride/sodium citrate (SSC), at about 65-70°C (or hybridization in 4X SSC plus 50% formamide at about 42-50°C) followed by one or more washes in 1X SSC, at about 65-70°C. A non-limiting example of highly stringent hybridization conditions for such hybrids includes hybridization in 1X SSC, at about 65-70°C (or hybridization in 1X SSC plus 50% formamide at about 42-50°C) followed by one or more washes in 0.3X SSC, at about 65-70°C. A non-limiting example of reduced stringency hybridization conditions for such hybrids includes hybridization in 4X SSC, at about 50-60°C (or alternatively hybridization in 6X SSC plus 50% formamide at about 40-45°C) followed by one or more washes in 2X SSC, at about 50-60°C. Ranges intermediate to the above-recited values, *e.g*., at 65-70°C or at 42-50°C are also intended to be encompassed by the present disclosure. Another example of stringent hybridization conditions are hybridization in 6X sodium chloride/sodium citrate (SSC) at about 45°C, followed by one or more washes in 0.2X SSC, 0.1% SDS at 50-65°C. A further example of stringent hybridization buffer is hybridization in 1 M NaCl, 50 mM 2-(N-morpholino)ethanesulfonic acid (MES) buffer (pH 6.5), 0.5% sodium sarcosine and 30% formamide. SSPE (1xSSPE is 0.15M NaCl, 10mM NaH₂PO₄, and 1.25mM EDTA, pH 7.4) can be substituted for SSC (1xSSC is 0.15M NaCl and 15mM sodium citrate) in the hybridization and wash buffers; washes are performed for 15 minutes each after hybridization is complete The hybridization temperature for hybrids anticipated to be less than 50 base pairs in length should be 5-10°C less than the melting temperature (Tₘ) of the hybrid, where Tₘ is determined according to the following equations. For hybrids less than 18 base pairs in length, Tₘ(°C) = 2(# of A + T bases) + 4(# of G + C bases). For hybrids between 18 and 49 base pairs in length, Tₘ(°C) = 81.5 + 16.6(log₁₀[Na⁺]) + 0.41(%G+C) - (600/N), where N is the number of bases in the hybrid, and [Na⁺] is the concentration of sodium ions in the hybridization buffer ([Na⁺] for 1xSSC = 0.165 M). It will also be recognized by the skilled practitioner that additional reagents may be added to hybridization and/or wash buffers to decrease non-specific hybridization of nucleic acid molecules to membranes, for example, nitrocellulose or nylon membranes, including but not limited to blocking agents (*e.g*., BSA or salmon or herring sperm carrier DNA), detergents (*e.g*., SDS), chelating agents (*e.g*., EDTA), Ficoll, polyvinylpyrrolidone (PVP) and the like. When using nylon membranes, in particular, an additional non-limiting example of stringent hybridization conditions is hybridization in 0.25-0.5M NaH₂PO₄, 7% SDS at about 65°C, followed by one or more washes at 0.02M NaH₂PO₄, 1% SDS at 65°C, see *e.g.,* Church and Gilbert (1984) Proc. Natl. Acad. Sci. USA 81:1991-1995, (or alternatively 0.2X SSC, 1% SDS). A primer or nucleic acid probe can be used alone in a detection method, or a primer can be used together with at least one other primer or nucleic acid probe in a detection method. Primers can also be used to amplify at least a portion of a nucleic acid. Nucleic acid probes of the disclosure refer to nucleic acids which hybridize to the region of interest and which are not further extended. For example, a nucleic acid probe is a nucleic acid which specifically hybridizes to a mutant region of a biomarker, and which by hybridization or absence of hybridization to the DNA of a patient or the type of hybrid formed can be indicative of the presence or identity of the mutation of the biomarker or the amount of marker activity.

In one format, the RNA is immobilized on a solid surface and contacted with a probe, for example by running the isolated RNA on an agarose gel and transferring the RNA from the gel to a membrane, such as nitrocellulose. In an alternative format, the nucleic acid probe(s) are immobilized on a solid surface and the RNA is contacted with the probe(s), for example, in an AFFYMETRIX® gene chip array or a SNP chip (Santa Clara, CA) or customized array using a marker set comprising at least one marker indicative of treatment outcome. A skilled artisan can readily adapt known RNA and DNA detection methods for use in detecting the amount of the markers of the present disclosure. For example, the high density microarray or branched DNA assay can benefit from a higher concentration of tumor cell in the sample, such as a sample which had been modified to isolate tumor cells as described in earlier sections. In a related embodiment, a mixture of transcribed polynucleotides obtained from the sample is contacted with a substrate having fixed thereto a polynucleotide complementary to or homologous with at least a portion (*e.g*., at least 7, 10, 15, 20, 25, 30, 40, 50, 100, 500, or more nucleotide residues) of a marker nucleic acid. If polynucleotides complementary to or homologous with the marker are differentially detectable on the substrate (*e.g*., detectable using different chromophores or fluorophores, or fixed to different selected positions), then the levels of expression of a plurality of markers can be assessed simultaneously using a single substrate (*e.g*., a "gene chip" microarray of polynucleotides fixed at selected positions). In an embodiment when a method of assessing marker expression is used which involves hybridization of one nucleic acid with another, the hybridization can be performed under stringent hybridization conditions.

An alternative method for determining the amount of RNA corresponding to a marker of the present disclosure in a sample involves the process of nucleic acid amplification, *e.g*., by RT-PCR (the experimental embodiment set forth in Mullis, 1987, U.S. Patent No. 4,683,202), ligase chain reaction (Barany, 1991, Proc. Natl. Acad. Sci. USA, 88:189-193), self sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technology 6:1197), rolling circle replication (Lizardi et al., U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to about 30 nucleotides in length and flank a region from about 50 to about 200 nucleotides in length. Under appropriate conditions and with appropriate reagents, such primers permit the amplification of a nucleic acid molecule comprising the nucleotide sequence flanked by the primers.

For *in situ* methods, RNA does not need to be isolated from the cells prior to detection. In such methods, a cell or tissue sample is prepared/processed using known histological methods. The sample is then immobilized on a support, typically a glass slide, and then contacted with a probe that can hybridize to RNA that encodes the marker.

In another embodiment of the present disclosure, a polypeptide corresponding to a marker is detected. In some embodiments, an agent for detecting a polypeptide of the disclosure is an antibody capable of binding to a polypeptide corresponding to a marker of the disclosure. In related embodiments, the antibody has a detectable label. Antibodies can be polyclonal, or monoclonal. An intact antibody, or a fragment thereof (*e.g*., Fab or F(ab')₂) can be used.

A variety of formats can be employed to determine whether a sample contains a protein that binds to a given antibody. Examples of such formats include, but are not limited to, enzyme immunoassay (EIA), radioimmunoassay (RIA), Western blot analysis and enzyme linked immunoabsorbant assay (ELISA). A skilled artisan can readily adapt known protein/antibody detection methods for use in determining whether B cells express a marker of the present disclosure.

Another method for determining the level of a polypeptide corresponding to a marker is mass spectrometry. For example, intact proteins or peptides, e.g., tryptic peptides can be analyzed from a sample, *e.g.,* a blood sample, a lymph sample or other sample, containing one or more polypeptide markers. The method can further include treating the sample to lower the amounts of abundant proteins, *e.g*., serum albumin, to increase the sensitivity of the method. For example, liquid chromatography can be used to fractionate the sample so portions of the sample can be analyzed separately by mass spectrometry. The steps can be performed in separate systems or in a combined liquid chromatography/mass spectrometry system (LC/MS, see for example, Liao, et al. (2004) Arthritis Rheum. 50:3792-3803). The mass spectrometry system also can be in tandem (MS/MS) mode. The charge state distribution of the protein or peptide mixture can be acquired over one or multiple scans and analyzed by statistical methods, e.g. using the retention time and mass-to-charge ratio (m/z) in the LC/MS system, to identify proteins expressed at statistically significant levels differentially in samples from patients responsive or non-responsive to Aurora A Kinase inhibition therapy. Examples of mass spectrometers which can be used are an ion trap system (ThermoFinnigan, San Jose, CA) or a quadrupole time-of-flight mass spectrometer (Applied Biosystems, Foster City, CA). The method can further include the step of peptide mass fingerprinting, e.g. in a matrix-assisted laser desorption ionization with time-of-flight (MALDI-TOF) mass spectrometry method. The method can further include the step of sequencing one or more of the tryptic peptides. Results of this method can be used to identify proteins from primary sequence databases, *e.g*., maintained by the National Center for Biotechnology Information, Bethesda, MD, or the Swiss Institute for Bioinformatics, Geneva, Switzerland, and based on mass spectrometry tryptic peptide m/z base peaks.

### Electronic Apparatus Readable Arrays

Electronic apparatus, including readable arrays comprising at least one predictive marker of the present disclosure is also contemplated for use in conjunction with the methods of the disclosure. As used herein, "electronic apparatus readable media" refers to any suitable medium for storing, holding or containing data or information that can be read and accessed directly by an electronic apparatus. As used herein, the term "electronic apparatus" is intended to include any suitable computing or processing apparatus or other device configured or adapted for storing data or information. Examples of electronic apparatus suitable for use with the present disclosure and monitoring of the recorded information include stand-alone computing apparatus; networks, including a local area network (LAN), a wide area network (WAN) Internet, Intranet, and Extranet; electronic appliances such as personal digital assistants (PDAs), cellular phone, pager and the like; and local and distributed processing systems. As used herein, "recorded" refers to a process for storing or encoding information on the electronic apparatus readable medium. Those skilled in the art can readily adopt any of the presently known methods for recording information on known media to generate manufactures comprising the markers of the present disclosure.

For example, microarray systems are well known and used in the art for assessment of samples, whether by assessment gene expression (e.g., DNA detection, RNA detection, protein detection), or metabolite production, for example. Microarrays for use according to the disclosure include one or more probes of predictive marker(s) of the disclosure characteristic of response and/or non-response to a therapeutic regimen as described herein. In one embodiment, the microarray comprises one or more probes corresponding to one or more of markers selected from the group consisting of markers which demonstrate increased expression in short term survivors, and genes which demonstrate increased expression in long term survivors in patients. A number of different microarray configurations and methods for their production are known to those of skill in the art and are disclosed, for example, in U.S. Pat. Nos: 5,242,974; 5,384,261; 5,405,783; 5,412,087; 5,424,186; 5,429,807; 5,436,327; 5,445,934; 5,556,752; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,624,711; 5,700,637; 5,744,305; 5,770,456; 5,770,722; 5,837,832; 5,856,101; 5,874,219; 5,885,837; 5,919,523; 5981185; 6,022,963; 6,077,674; 6,156,501; 6261776; 6346413; 6440677; 6451536; 6576424; 6610482; 5,143,854; 5,288,644; 5,324,633; 5,432,049; 5,470,710; 5,492,806; 5,503,980; 5,510,270; 5,525,464; 5,547,839; 5,580,732; 5,661,028; 5,848,659; and 5,874,219; Shena, et al. (1998), Tibtech 16:301; Duggan et a/. (1999) Nat. Genet. 21:10; Bowtell et al. (1999) Nat. Genet. 21:25; Lipshutz et al. (1999) Nature Genet. 21:20-24, 1999; Blanchard, et al. (1996) Biosensors and Bioelectronics, 11:687-90; Maskos, et al., (1993) Nucleic Acids Res. 21:4663-69; Hughes, et al. (2001) Nat. Biotechol. 19:342, 2001. A tissue microarray can be used for protein identification (see Hans et al. (2004)Blood 103:275-282). A phage-epitope microarray can be used to identify one or more proteins in a sample based on whether the protein or proteins induce auto-antibodies in the patient (Bradford et al. (2006) Urol. Oncol. 24:237-242).

A microarray thus comprises one or more probes corresponding to one or more markers identified herein, e.g., those indicative of treatment outcome, e.g., to identify wild type marker genes, normal allelic variants and mutations of marker genes. The microarray can comprise probes corresponding to, for example, at least 2, at least 3, at least 4, at least 5, at least 10, at least 15, at least 20, at least 25, at least 30, at least 35, at least 40, at least 45, at least 50, at least 75, or at least 100, biomarkers and/or mutations thereof indicative of treatment outcome. The microarray can comprise probes corresponding to one or more biomarkers as set forth herein. Still further, the microarray may comprise complete marker sets as set forth herein and which may be selected and compiled according to the methods set forth herein. The microarray can be used to assay expression of one or more predictive markers or predictive marker sets in the array. In one example, the array can be used to assay more than one predictive marker or marker set expression in a sample to ascertain an expression profile of markers in the array. In this manner, up to about 44,000 markers can be simultaneously assayed for expression. This allows an expression profile to be developed showing a battery of markers specifically expressed in one or more samples. Still further, this allows an expression profile to be developed to assess treatment outcome.

The array is also useful for ascertaining differential expression patterns of one or more markers in normal and abnormal (*e.g*., sample, *e.g*., tumor) cells. This provides a battery of markers that could serve as a tool for ease of identification of treatment outcome of patients. Further, the array is useful for ascertaining expression of reference markers for reference expression levels. In another example, the array can be used to monitor the time course of expression of one or more markers in the array.

In addition to such qualitative determination, the disclosure allows the quantification of marker expression. Thus, predictive markers can be grouped on the basis of marker sets or outcome indications by the amount of the marker in the sample. This is useful, for example, in ascertaining the outcome of the sample by virtue of scoring the amounts according to the methods provided herein.

The array is also useful for ascertaining the effect of the expression of a marker on the expression of other predictive markers in the same cell or in different cells. This provides, for example, a selection of alternate molecular targets for therapeutic intervention if patient is predicted to have an unfavorable outcome.

### Therapeutic Agents

The markers and marker sets of the present disclosure assess the likelihood of favorable outcome in cancer patients. Using this prediction, cancer therapies can be evaluated to design a therapy regimen best suitable for patients in either category.

Therapeutic agents for use in the methods of the disclosure include a class of therapeutic agents known as Aurora A Kinase inhibitors, as described herein.

The agents disclosed herein may be administered by any route, including intradermally, subcutaneously, orally, intraarterially or intravenously. In one embodiment, administration will be by the intravenous route. Parenteral administration can be provided in a bolus or by infusion.

The concentration of a disclosed compound in a pharmaceutically acceptable mixture will vary depending on several factors, including the dosage of the compound to be administered, the pharmacokinetic characteristics of the compound(s) employed, and the route of administration. The agent may be administered in a single dose or in repeat doses. Treatments may be administered daily or more frequently depending upon a number of factors, including the overall health of a patient, and the formulation and route of administration of the selected compound(s).

If a pharmaceutically acceptable salt of Aurora A kinase is utilized in these compositions, the salt preferably is derived from an inorganic or organic acid or base. For reviews of suitable salts, see, e.g., Berge et al, J. Pharm. Sci. 66:1-19 (1977) and Remington: The Science and Practice of Pharmacy, 20th Ed., ed. A. Gennaro, Lippincott Williams & Wilkins, 2000.

Nonlimiting examples of suitable acid addition salts include the following: acetate, adipate, alginate, aspartate, benzoate, benzene sulfonate, bisulfate, butyrate, citrate, camphorate, camphor sulfonate, cyclopentanepropionate, digluconate, dodecylsulfate, ethanesulfonate, fumarate, lucoheptanoate, glycerophosphate, hemisulfate, heptanoate, hexanoate, hydrochloride, hydrobromide, hydroiodide, 2-hydroxyethanesulfonate, lactate, maleate, methane sulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate and undecanoate.

Suitable base addition salts include, without limitation, ammonium salts, alkali metal salts, such as sodium and potassium salts, alkaline earth metal salts, such as calcium and magnesium salts, salts with organic bases, such as dicyclohexylamine, *N*-methyl-D-glucamine, *t*-butylamine, ethylene diamine, ethanolamine, and choline, and salts with amino acids such as arginine, lysine, and so forth.

Also, basic nitrogen-containing groups may be quaternized with such agents as lower alkyl halides, such as methyl, ethyl, propyl, and butyl chlorides, bromides and iodides; dialkyl sulfates, such as dimethyl, diethyl, dibutyl and diamyl sulfates, long chain halides such as decyl, lauryl, myristyl and stearyl chlorides, bromides and iodides, aralkyl halides, such as benzyl and phenethyl bromides and others. Water or oil-soluble or dispersible products are thereby obtained.

The term "pharmaceutically acceptable carrier" is used herein to refer to a material that is compatible with a recipient subject, preferably a mammal, more preferably a human, and is suitable for delivering an active agent to the target site without terminating the activity of the agent. The toxicity or adverse effects, if any, associated with the carrier preferably are commensurate with a reasonable risk/benefit ratio for the intended use of the active agent.

The terms "carrier", "adjuvant", or "vehicle" are used interchangeably herein, and include any and all solvents, diluents, and other liquid vehicles, dispersion or suspension aids, surface active agents, isotonic agents, thickening or emulsifying agents, preservatives, solid binders, lubricants and the like, as suited to the particular dosage form desired. Remington: The Science and Practice of Pharmacy, 20th F.d., ed. A. Gennaro, Lippincott Williams & Wilkins, 2000 discloses various carriers used in formulating pharmaceutically acceptable compositions and known techniques for the preparation thereof. Except insofar as any conventional carrier medium is incompatible with the compounds of the disclosure, such as by producing any undesirable biological effect or otherwise interacting in a deleterious manner with any other component(s) of the pharmaceutically acceptable composition, its use is contemplated to be within the scope of this disclosure. Some examples of materials which can serve as pharmaceutically acceptable carriers include, but are not limited to, ion exchangers, alumina, aluminum stearate, lecithin, serum proteins, such as human serum albumin, buffer substances such as disodium hydrogen phosphate, potassium hydrogen phosphate, sodium carbonate, sodium bicarbonate, potassium carbonate, potassium bicarbonate, magnesium hydroxide and aluminum hydroxide, glycine, sorbic acid, or potassium sorbate, partial glyceride mixtures of saturated vegetable fatty acids, water, pyrogen-free water, salts or electrolytes such as protamine sulfate, disodium hydrogen phosphate, potassium hydrogen phosphate, sodium chloride, and zinc salts, colloidal silica, magnesium trisilicate, polyvinyl pyrrolidone, polyacrylates, waxes, polyethylene-polyoxypropylene-block polymers, wool fat, sugars such as lactose, glucose, sucrose, starches such as corn starch and potato starch, cellulose and its derivatives such as sodium carboxymethyl cellulose, ethyl cellulose and cellulose acetate, powdered tragacanth; malt, gelatin, talc, excipients such as cocoa butter and suppository waxes, oils such as peanut oil, cottonseed oil, safflower oil, sesame oil, olive oil, corn oil and soybean oil, glycols such as propylene glycol and polyethylene glycol, esters such as ethyl oleate and ethyl laurate, agar, alginic acid, isotonic saline, Ringer's solution, alcohols such as ethanol, isopropyl alcohol, hexadecyl alcohol, and glycerol, cyclodextrins, lubricants such as sodium lauryl sulfate and magnesium stearate, petroleum hydrocarbons such as mineral oil and petrolatum. Coloring agents, releasing agents, coating agents, sweetening, flavoring and perfuming agents, preservatives and antioxidants can also be present in the composition, according to the judgment of the formulator.

The pharmaceutical compositions of the disclosure can be manufactured by methods well known in the art such as conventional granulating, mixing, dissolving, encapsulating, lyophilizing, or emulsifying processes, among others. Compositions may be produced in various forms, including granules, precipitates, or particulates, powders, including freeze dried, rotary dried or spray dried powders, amorphous powders, tablets, capsules, syrup, suppositories, injections, emulsions, elixirs, suspensions or solutions. Formulations may optionally contain solvents, diluents, and other liquid vehicles, dispersion or suspension aids, surface active agents, pH modifiers, isotonic agents, thickening or emulsifying agents, stabilizers and preservatives, solid binders, lubricants and the like, as suited to the particular dosage form desired.

According to a preferred embodiment, the compositions of this disclosure are formulated for pharmaceutical administration to a mammal, preferably a human being. Such pharmaceutical compositions of the present disclosure may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intravenously, or subcutaneously. The formulations of the disclosure may be designed to be short-acting, fast-releasing, or long-acting. Still further, compounds can be administered in a local rather than systemic means, such as administration (e.g., by injection) at a tumor site.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, cyclodextrins, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. Compositions formulated for parenteral administration may be injected by bolus injection or by timed push, or may be administered by continuous infusion.

In order to prolong the effect of a compound of the present disclosure, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this disclosure with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents such as phosphates or carbonates.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this disclosure include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this disclosure. Additionally, the present disclosure contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

The selective inhibitor of Aurora A kinase can be administered by any method known to one skilled in the art. For example, the selective inhibitor of Aurora A kinase can be administered in the form of a composition, in one embodiment a pharmaceutical composition of the selective inhibitor of Aurora A kinase and a pharmaceutically acceptable carrier, such as those described herein. Preferably, the pharmaceutical composition is suitable for oral administration. In some embodiments, the pharmaceutical composition is a tablet for oral administration, such as an enteric coated tablet. Such tablets are described in US Publication No. 2010/0310651. In some other embodiments, the pharmaceutical composition is a liquid dosage form for oral administration. Such liquid dosage forms are described in US Publication No. 2011/0039826. In certain embodiments, these compositions optionally further comprise one or more additional therapeutic agents.

The expressions "therapeutically effective" and "therapeutic effect" refer to a benefit including, but not limited to, the treatment or prophylaxis or amelioration of symptoms of a proliferative disorder discussed herein. It will be appreciated that the therapeutically effective amount or the amount of agent required to provide a therapeutic effect will vary depending upon the intended application (*in vitro* or *in vivo*), or the subject and disease condition being treated (e.g., nature of the severity of the condition to be treated, the particular inhibitor, the route of administration and the age, weight, general health, and response of the individual patient), which can be readily determined by a person of skill in the art. For example, an amount of a selective inhibitor of Aurora A kinase is therapeutically effective if it is sufficient to effect the treatment or prophylaxis or amelioration of symptoms of a proliferative disorder discussed herein.

Compositions for use in the method of the disclosure may be formulated in unit dosage form for ease of administration and uniformity of dosage. The expression "unit dosage form" as used herein refers to a physically discrete unit of agent appropriate for the patient to be treated. It will be understood, however, that the total daily usage of the compounds and compositions of the present disclosure will be decided by the attending physician within the scope of sound medical judgment. A unit dosage form for parenteral administration may be in ampoules or in multi-dose containers.

In some embodiments, the treatment period during which an agent is administered is then followed by a non-treatment period of particular time duration, during which the therapeutic agents are not administered to the patient. This non-treatment period can then be followed by a series of subsequent treatment and non-treatment periods of the same or different frequencies for the same or different lengths of time. In some embodiments, the treatment and non-treatment periods are alternated. It will be understood that the period of treatment in cycling therapy may continue until the patient has achieved a complete response or a partial response, at which point the treatment may be stopped. Alternatively, the period of treatment in cycling therapy may continue until the patient has achieved a complete response or a partial response, at which point the period of treatment may continue for a particular number of cycles. In some embodiments, the length of the period of treatment may be a particular number of cycles, regardless of patient response. In some other embodiments, the length of the period of treatment may continue until the patient relapses.

It will be appreciated that the frequency with which any of these therapeutic agents can be administered can be once or more than once over a period of about 2 days, about 3 days, about 4 days, about 5 days, about 6 days, about 7 days, about 8 days, about 9 days, about 10 days, about 20 days, about 28 days, about a week, about 2 weeks, about 3 weeks, about 4 weeks, about a month, about every 2 months, about every 3 months, about every 4 months, about every 5 months, about every 6 months, about every 7 months, about every 8 months, about every 9 months, about every 10 months, about every 11 months, about every year, about every 2 years, about every 3 years, about every 4 years, or about every 5 years.

For example, an agent may be administered daily, weekly, biweekly, or monthly for a particular period of time. An agent may be dosed daily over a 14 day time period, or twice daily over a seven day time period. In some embodiments, a certain amount of the selective Aurora A kinase can be administered daily for 7 days. Alternatively, an agent may be administered daily, weekly, biweekly, or monthly for a particular period of time followed by a particular period of non-treatment. In some embodiments, a certain amount of the Aurora A kinase inhibitor can be administered daily for 14 days followed by seven days of non-treatment, and repeated for two more cycles of daily administration for 14 days followed by seven days of non-treatment. In some embodiments, a certain amount of the selective Aurora A kinase inhibitor can be administered twice daily for seven days followed by 14 days of non-treatment, which may be repeated for one or two more cycles of twice daily administration for seven days followed by 14 days of non-treatment.

In one embodiment, a certain amount of the selective Aurora A kinase inhibitor is administered daily over a period of seven days. In another embodiment, a certain amount of the Aurora A inhibitor is administered daily over a period of six days, or five days, or four days, or three days. In another embodiment, a certain amount of the selective Aurora A kinase inhibitor is administered twice daily over a period of seven days, followed by a treatment-free period of 7, 14 or 21 days. In another embodiment, alisertib is administered twice daily at a dose of 50mg for 7 days, followed by a 14 day treatment-free interval, in 21-day cycles.

Suitable daily dosages of selective inhibitors of Aurora A kinase can generally range, in single or divided or multiple doses, from about 10% to about 120% of the maximum tolerated dose as a single agent. In certain embodiments, the suitable dosages are from about 20% to about 100% of the maximum tolerated dose as a single agent. In some other embodiments, the suitable dosages are from about 25% to about 90% of the maximum tolerated dose as a single agent. In some other embodiments, the suitable dosages are from about 30% to about 80% of the maximum tolerated dose as a single agent. In some other embodiments, the suitable dosages are from about 40% to about 75% of the maximum tolerated dose as a single agent. In some other embodiments, the suitable dosages are from about 45% to about 60% of the maximum tolerated dose as a single agent. In other embodiments, suitable dosages are about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 105%, about 110%, about 115%, or about 120% of the maximum tolerated dose as a single agent.

Suitable daily dosages of alisertib can generally range, in single or divided or multiple doses, from about 20 mg to about 120 mg per day. Other suitable daily dosages of alisertib can generally range, in single or divided or multiple doses, from about 30 mg to about 90 mg per day. Other suitable daily dosages of alisertib can generally range, in single or divided or multiple doses, from about 40 mg to about 80 mg per day. In some embodiments, the suitable dosages are from about 10 mg twice daily to about 50 mg twice daily. In some other embodiments, the suitable dosages are from about 15 mg twice daily to about 45 mg twice daily. In some other embodiments, the suitable dosages are from about 20 mg twice daily to about 40 mg twice daily. In some other embodiments, the suitable dosages are from about 25 mg twice daily to about 40 mg twice daily. In some embodiments, suitable dosages are about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, or about 120 mg per day. In certain other embodiments, suitable dosages are about 10 mg, about 15 mg, about 20 mg, about 25 mg, about 30 mg, about 35 mg, about 40 mg, about 45 mg, about 50 mg, about 55 mg, or about 60 mg twice daily. In some embodiments, the suitable dosage of alisertib is about 30 mg twice daily. In some embodiments, the suitable dosage of alisertib is about 35 mg twice daily. In some embodiments, the suitable dosage of alisertib is about 40 mg twice daily. In some embodiments, the suitable dosage of alisertib is about 50 mg twice daily.

It will be understood that a suitable dosage of a selective inhibitor of Aurora A kinase may be taken at any time of the day or night. In some embodiments, a suitable dosage of a selective inhibitor of Aurora A kinase is taken in the morning. In some other embodiments, a suitable dosage of a selective inhibitor of Aurora A kinase is taken in the evening. In some other embodiments, a suitable dosage of a selective inhibitor of Aurora A kinase is taken both in the morning and the evening. It will be understood that a suitable dosage of a selective inhibitor of Aurora A kinase may be taken with or without food. In some embodiments a suitable dosage of a selective inhibitor of Aurora A kinase is taken with a meal. In some embodiments a suitable dosage of a selective inhibitor of Aurora A kinase is taken while fasting.

In some embodiments, a first treatment period in which a first amount of the selective inhibitor of Aurora A kinase is administered can be followed by another treatment period in which a same or different amount of the same or a different selective inhibitor of Aurora A kinase is administered. A wide variety of therapeutic agents may have a therapeutically relevant added benefit in combination with the Aurora A kinase of the present disclosure. Combination therapies that comprise the Aurora A kinase of the present disclosure with one or more other therapeutic agents can be used, for example, to: 1) enhance the therapeutic effect(s) of the methods of the present disclosure and/or the one or more other therapeutic agents; 2) reduce the side effects exhibited by the methods of the present disclosure and/or the one or more other therapeutic agents; and/or 3) reduce the effective dose of the Aurora A kinase of the present disclosure and/or the one or more other therapeutic agents. For example, such therapeutic agents may combine with the Aurora A kinase of the present disclosure to inhibit undesirable cell growth, such as inappropriate cell growth resulting in undesirable benign conditions or tumor growth.

### Reagents and Kits

The disclosure also encompasses kits for detecting the presence of a polypeptide or nucleic acid corresponding to a marker of the disclosure in a biological sample (*e.g.* a bone marrow sample, tumor biopsy or a reference sample). Such kits can be used to assess treatment outcome, e.g., determine if a subject can have a favorable outcome, e.g., after Aurora A Kinase inhibitor treatment. For example, the kit can comprise a labeled compound or agent capable of detecting a genomic DNA segment, a polypeptide or a transcribed RNA corresponding to a marker of the disclosure or a mutation of a marker gene in a biological sample and means for determining the amount of the genomic DNA segment, the polypeptide or RNA in the sample. Suitable reagents for binding with a marker protein include antibodies, antibody derivatives, antibody fragments, and the like. Suitable reagents for binding with a marker nucleic acid (*e.g*., a genomic DNA, an mRNA, a spliced mRNA, a cDNA, or the like) include complementary nucleic acids. The kit can also contain a control or reference sample or a series of control or reference samples which can be assayed and compared to the test sample. For example, the kit may have a positive control sample, e.g., including one or more markers or mutations described herein, or reference markers, *e.g*. housekeeping markers to standardize the assay among samples or timepoints or reference genomes, *e.g*., form subjects without tumor *e.g*., to establish diploid copy number baseline or reference expression level of a marker. By way of example, the kit may comprise fluids (*e.g*., buffer) suitable for annealing complementary nucleic acids or for binding an antibody with a protein with which it specifically binds and one or more sample compartments. The kit of the disclosure may optionally comprise additional components useful for performing the methods of the disclosure, *e.g.,* a sample collection vessel, *e.g*., a tube, and optionally, means for optimizing the amount of marker detected, for example if there may be time or adverse storage and handling conditions between the time of sampling and the time of analysis. For example, the kit can contain means for increasing the number of tumor cells in the sample, as described above, a buffering agent, a preservative, a stabilizing agent or additional reagents for preparation of cellular material or probes for use in the methods provided; and detectable label, alone or conjugated to or incorporated within the provided probe(s). In one exemplary embodiment, a kit comprising a sample collection vessel can comprise *e.g*., a tube comprising anti-coagulant and/or stabilizer, as described above, or known to those skilled in the art. The kit can further comprise components necessary for detecting the detectable label (*e.g*., an enzyme or a substrate). For marker sets, the kit can comprise a marker set array or chip for use in detecting the biomarkers. Kits also can include instructions for interpreting the results obtained using the kit. The kit can contain reagents for detecting one or more biomarkers, *e.g*., 2, 3, 4, 5, or more biomarkers described herein.

In one embodiment, the kit comprises a probe to detect at least one biomarker, e.g., a marker indicative of treatment outcome (*e.g*., upon Aurora A Kinase inhibitor treatment). In an exemplary embodiment, the kit comprises a nucleic acid probe to detect a marker gene selected from the group consisting of the marker genes disclosed in Tables 9 and 10 herein. In some embodiments, the kit comprises a probe to detect a marker selected from the group consisting of LEF1, MAP2K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWC1, WWTR1 and YAP1. In an embodiment, a kit comprises probes to detect a marker set comprising two or more markers from the group consisting of LEF1, MAP2K7, APC, FZD2, PRKCA, RORA, CAMK2G, JUN, XPO1, ROR2, CCND1, CTNNB1, AMOT, DVL2, LATS1, LATS2, MOB1B, NPHP4, TJP1, TJP2, WWC1, WWC1, WWTR1 and YAP1. In related embodiments, the kit comprises a nucleic acid probe comprising or derived from (*e.g*., a fragment, mutant or variant (*e.g*., homologous or complementary) thereof) a nucleic acid sequence selected from the group consisting of the nucleotide sequences disclosed by GenBank Accession numbers within Tables 9 and 10 herein. For kits comprising nucleic acid probes, e.g., oligonucleotide-based kits, the kit can comprise, for example: one or more nucleic acid reagents such as an oligonucleotide (labeled or non-labeled) which hybridizes to a nucleic acid sequence corresponding to a marker of the disclosure, optionally fixed to a substrate; labeled oligonucleotides not bound with a substrate, a pair of PCR primers, useful for amplifying a nucleic acid molecule corresponding to a marker of the disclosure, molecular beacon probes, a marker set comprising oligonucleotides which hybridize to at least two nucleic acid sequences corresponding to markers of the disclosure, and the like. The kit can contain an RNA-stabilizing agent.

For kits comprising protein probes, *e.g*., antibody-based kits, the kit can comprise, for example: (1) a first antibody (*e.g*., attached to a solid support) which binds to a polypeptide corresponding to a marker of the disclosure; and, optionally, (2) a second, different antibody which binds to either the polypeptide or the first antibody and is conjugated to a detectable label. The kit can contain a protein stabilizing agent. The kit can contain reagents to reduce the amount of non-specific binding of non-biomarker material from the sample to the probe. Examples of reagents include nonioinic detergents, non-specific protein containing solutions, such as those containing albumin or casein, or other substances known to those skilled in the art.

An isolated polypeptide corresponding to a predictive marker of the disclosure, or a fragment or mutant thereof, can be used as an immunogen to generate antibodies using standard techniques for polyclonal and monoclonal antibody preparation. For example, an immunogen typically is used to prepare antibodies by immunizing a suitable (*i.e*., immunocompetent) subject such as a rabbit, goat, mouse, or other mammal or vertebrate. In still a further aspect, the disclosure provides monoclonal antibodies or antigen binding fragments thereof, which antibodies or fragments specifically bind to a polypeptide comprising an amino acid sequence selected from the group consisting of the amino acid sequences of the present disclosure, an amino acid sequence encoded by the cDNA of the present disclosure, a fragment of at least 8, 10, 12, 15, 20 or 25 amino acid residues of an amino acid sequence of the present disclosure, an amino acid sequence which is at least 95%, 96%, 97%, 98% or 99% identical to an amino acid sequence of the present disclosure (wherein the percent identity is determined using the ALIGN program of the GCG software package with a PAM120 weight residue table, a gap length penalty of 12, and a gap penalty of 4) and an amino acid sequence which is encoded by a nucleic acid molecule which hybridizes to a nucleic acid molecule consisting of the nucleic acid molecules of the present disclosure, or a complement thereof, under conditions of hybridization of 6X SSC at 45°C and washing in 0.2 X SSC, 0.1% SDS at 65°C. The monoclonal antibodies can be human, humanized, chimeric and/or non-human antibodies. An appropriate immunogenic preparation can contain, for example, recombinantly-expressed or chemically-synthesized polypeptide. The preparation can further include an adjuvant, such as Freund's complete or incomplete adjuvant, or a similar immunostimulatory agent.

Methods for making human antibodies are known in the art. One method for making human antibodies employs the use of transgenic animals, such as a transgenic mouse. These transgenic animals contain a substantial portion of the human antibody producing genome inserted into their own genome and the animal's own endogenous antibody production is rendered deficient in the production of antibodies. Methods for making such transgenic animals are known in the art. Such transgenic animals can be made using XENOMOUSE ™ technology or by using a "minilocus" approach. Methods for making XENOMICE™ are described in U.S. Pat. Nos. 6,162,963, 6,150,584, 6,114,598 and 6,075,181. Methods for making transgenic animals using the "minilocus" approach are described in U.S. Pat. Nos. 5,545,807, 5,545,806 and 5,625,825; also see International Publication No. WO93/12227.

Antibodies include immunoglobulin molecules and immunologically active portions of immunoglobulin molecules, *i.e*., molecules that contain an antigen binding site which specifically binds an antigen, such as a polypeptide of the disclosure, *e.g*., an epitope of a polypeptide of the disclosure. A molecule which specifically binds to a given polypeptide of the disclosure is a molecule which binds the polypeptide, but does not substantially bind other molecules in a sample, *e.g.,* a biological sample, which naturally contains the polypeptide. For example, antigen-binding fragments, as well as full-length monomeric, dimeric or trimeric polypeptides derived from the above-described antibodies are themselves useful. Useful antibody homologs of this type include (i) a Fab fragment, a monovalent fragment consisting of the VL, VH, CL and CHI domains; (ii) a F(ab')₂ fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., Nature 341:544-546 (1989)), which consists of a VH domain; (vii) a single domain functional heavy chain antibody, which consists of a VHH domain (known as a nanobody) see *e.g.,* Cortez-Retamozo, et al., Cancer Res. 64: 2853-2857(2004), and references cited therein; and (vii) an isolated complementarity determining region (CDR), e.g., one or more isolated CDRs together with sufficient framework to provide an antigen binding fragment. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. Science 242:423-426 (1988); and Huston et al. Proc. Natl. Acad. Sci. USA 85:5879-5883 (1988). Such single chain antibodies are also intended to be encompassed within the term "antigen-binding fragment" of an antibody. These antibody fragments are obtained using conventional techniques known to those with skill in the art, and the fragments are screened for utility in the same manner as are intact antibodies. Antibody fragments, such as Fv, F(ab')₂ and Fab may be prepared by cleavage of the intact protein, e.g. by protease or chemical cleavage. The disclosure provides polyclonal and monoclonal antibodies. Synthetic and genetically engineered variants (See U.S. Pat. No. 6,331,415) of any of the foregoing are also contemplated by the present disclosure. Polyclonal and monoclonal antibodies can be produced by a variety of techniques, including conventional murine monoclonal antibody methodology e.g., the standard somatic cell hybridization technique of Kohler and Milstein, Nature 256: 495 (1975) the human B cell hybridoma technique (see Kozbor et al., 1983, Immunol. Today 4:72), the EBV-hybridoma technique (see Cole et al., pp. 77-96 In Monoclonal Antibodies and Cancer Therapy, Alan R. Liss, Inc., 1985) or trioma techniques. See generally, Harlow, E. and Lane, D. (1988) Antibodies: A Laboratory Manual, Cold Spring Harbor Laboratory Press, Cold Spring Harbor, NY; and Current Protocols in Immunology, Coligan et al. ed., John Wiley & Sons, New York, 1994. For diagnostic applications, the antibodies can be monoclonal antibodies, e.g., generated in mouse, rat, or rabbit. Additionally, for use in *in vivo* applications the antibodies of the present disclosure can be human or humanized antibodies. Hybridoma cells producing a monoclonal antibody of the disclosure are detected by screening the hybridoma culture supernatants for antibodies that bind the polypeptide of interest, *e.g*., using a standard ELISA assay.

If desired, the antibody molecules can be harvested or isolated from the subject (*e.g*., from the blood or serum of the subject) and further purified by well-known techniques, such as protein A chromatography to obtain the IgG fraction. Alternatively, antibodies specific for a protein or polypeptide of the disclosure can be selected or (*e.g*., partially purified) or purified by, *e.g.,* affinity chromatography to obtain substantially purified and purified antibody. By a substantially purified antibody composition is meant, in this context, that the antibody sample contains at most only 30% (by dry weight) of contaminating antibodies directed against epitopes other than those of the desired protein or polypeptide of the disclosure, and at most 20%, at most 10%, or at most 5% (by dry weight) of the sample is contaminating antibodies. A purified antibody composition means that at least 99% of the antibodies in the composition are directed against the desired protein or polypeptide of the disclosure.

An antibody directed against a polypeptide corresponding to a marker of the disclosure (*e.g*., a monoclonal antibody) can be used to detect the marker (*e.g*., in a cellular sample) in order to evaluate the level and pattern of expression of the marker. The antibodies can also be used diagnostically to monitor protein levels in tissues or body fluids (*e.g*. in a blood sample) as part of a clinical testing procedure, *e.g*., to, for example, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and 125 131 35 3 aequorin, and examples of suitable radioactive material include I, I, S or H.

Accordingly, in one aspect, the disclosure provides substantially purified antibodies or fragments thereof, and non-human antibodies or fragments thereof, which antibodies or fragments specifically bind to a polypeptide comprising an amino acid sequence encoded by a marker identified herein. The substantially purified antibodies of the disclosure, or fragments thereof, can be human, non-human, chimeric and/or humanized antibodies.

In another aspect, the disclosure provides non-human antibodies or fragments thereof, which antibodies or fragments specifically bind to a polypeptide comprising an amino acid sequence which is encoded by a nucleic acid molecule of a predictive marker of the disclosure. Such non-human antibodies can be goat, mouse, sheep, horse, chicken, rabbit, or rat antibodies. Alternatively, the non-human antibodies of the disclosure can be chimeric and/or humanized antibodies. In addition, the non-human antibodies of the disclosure can be polyclonal antibodies or monoclonal antibodies.

The substantially purified antibodies or fragments thereof may specifically bind to a signal peptide, a secreted sequence, an extracellular domain, a transmembrane or a cytoplasmic domain or cytoplasmic loop of a polypeptide of the disclosure. The substantially purified antibodies or fragments thereof, the non-human antibodies or fragments thereof, and/or the monoclonal antibodies or fragments thereof, of the disclosure specifically bind to a secreted sequence or an extracellular domain of the amino acid sequences of the present disclosure.

The disclosure also provides a kit containing an antibody of the disclosure conjugated to a detectable substance, and instructions for use. Still another aspect of the disclosure is a diagnostic composition comprising a probe of the disclosure and a pharmaceutically acceptable carrier. In one embodiment, the diagnostic composition contains an antibody of the disclosure, a detectable moiety, and a pharmaceutically acceptable carrier.

### Sensitivity Assays

A sample of cancerous cells is obtained from a patient. An expression level is measured in the sample for a marker corresponding to at least one of the markers described herein. A marker set comprising markers as described herein can be put together using the methods described herein. Such analysis is used to obtain an expression profile of the tumor in the patient. Evaluation of the expression profile is then used to determine whether the patient is expected to have a favorable outcome and would benefit from treatment with, *e.g.,* Aurora A Kinase inhibittion therapy (e.g., treatment with an Aurora A Kinase inhibitor (e.g., alisertib) alone, or in combination with additional agents)), or with an alternative agent expected to have a similar effect on survival. Evaluation of the expression profile can also be used to determine whether a patient is expected to have an unfavorable outcome and would benefit from a cancer therapy other than Aurora A Kinase inhibition therapy or would benefit from an altered Aurora A Kinase inhibition therapy regimen. Evaluation can include use of one marker set prepared using any of the methods provided or other similar scoring methods known in the art (e.g., weighted voting, combination of threshold features (CTF), Cox proportional hazards analysis, principal components scoring, linear predictive score, K-nearest neighbor, etc), e.g., using expression values deposited with the Gene Expresion Omnibus (GEO) program at the National Center for Biotechnology Information (NCBI, Bethesda, MD). Still further, evaluation can comprise use of more than one prepared marker set. An Aurora A Kinase inhibition therapy will be identified as appropriate to treat the cancer when the outcome of the evaluation demonstrates a favorable outcome or a more aggressive therapy regimen will be identified for a patient with an expected unfavorable outcome.

In one aspect, the disclosure features a method of evaluating a patient, e.g., a patient with cancer, e.g. a hematological cancer or solid tumor cancer for treatment outcome. The method includes i) evaluating the expression of the markers in a marker set in the patient sample, wherein the marker set has the following properties: a) it includes a plurality of genes, each of which is differentially expressed between patients with identified outcome and non-afflicted subjects; b) it contains a sufficient number of differentially expressed markers, such that differential amount (e.g., as compared to a level in a non-afflicted reference sample) of each of the markers in the marker set in a subject is predictive of treatment outcome with no more than about 15%, about 10%, about 5%, about 2.5%, or about 1% false positives (wherein false positive means incorrectly predicting whether a patient is responsive or non-responsive); and ii) comparing the amount of each of the markers in the set from the patient to a reference value, thereby evaluating the patient.

By examining the amount of one or more of the identified markers or marker sets in a tumor sample taken from a patient during the course of Aurora A Kinase inhibition therapy, it is also possible to determine whether the therapeutic agent is continuing to work or whether the cancer has become non-responsive (refractory) to the treatment protocol. For example, a patient receiving a treatment regiment comprising alisertib would have tumor cells removed and monitored for the expression of a marker or marker set. If the profile of one or more markers as disclosed herein typifies favorable outcome in the presence of the agent, e.g., the Aurora A Kinase inhibitor (alisertib), the treatment would continue. However, if the profile of the one or more markers identified herein typifies unfavorable outcome in the presence of the agent, then the cancer may have become resistant to therapy, e.g., Aurora A Kinase inhibition (alisertib) therapy, and another treatment protocol should be initiated to treat the patient.

Importantly, these determinations can be made on a patient-by-patient basis or on an agent-by-agent (or combinations of agents). Thus, one can determine whether or not a particular Aurora A Kinase inhibition therapy is likely to benefit a particular patient or group/class of patients, or whether a particular treatment should be continued.

### Use of Information

In one method, information, e.g., about the patient's marker(s) characteristic, e.g., size, sequence, composition or amount (e.g., the result of evaluating a marker or marker set described herein), or about whether a patient is expected to have a favorable outcome, is provided (e.g., communicated, e.g., electronically communicated) to a third party, e.g., a hospital, clinic, a government entity, reimbursing party or insurance company (e.g., a life insurance company). For example, choice of medical procedure, payment for a medical procedure, payment by a reimbursing party, or cost for a service or insurance can be function of the information. E.g., the third party receives the information, makes a determination based at least in part on the information, and optionally communicates the information or makes a choice of procedure, payment, level of payment, coverage, etc. based on the information. In the method, informative expression level of a marker or a marker set selected from or derived from Table 8 and/or described herein is determined.

In one embodiment, a premium for insurance (e.g., life or medical) is evaluated as a function of information about one or more marker expression levels, e.g., a marker or marker set, e.g., a level of expression associated with treatment outcome (e.g., the informative amount). For example, premiums can be increased (e.g., by a certain percentage) if the marker genes of a patient or a patient's marker set described herein have different characteristic, e.g., size, sequence, composition or amount between an insured candidate (or a candidate seeking insurance coverage) and a reference value (e.g., a non-afflicted person) or a reference sample, e.g., matched control. Premiums can also be scaled depending on the result of evaluating a marker or marker set described herein. For example, premiums can be assessed to distribute risk, e.g., as a function of marker, e.g., the result of evaluating a marker or marker set described herein. In another example, premiums are assessed as a function of actuarial data that is obtained from patients that have known treatment outcomes.

Information about marker characteristic, e.g., size, sequence, composition or amount, e.g., the result of evaluating a marker or marker set described herein (e.g., the informative amount), can be used, e.g., in an underwriting process for life insurance. The information can be incorporated into a profile about a subject. Other information in the profile can include, for example, date of birth, gender, marital status, banking information, credit information, children, and so forth. An insurance policy can be recommended as a function of the information on marker characteristic, e.g., size, sequence, composition or amount, e.g., the result of evaluating a marker or marker set described herein, along with one or more other items of information in the profile. An insurance premium or risk assessment can also be evaluated as function of the marker or marker set information. In one implementation, points are assigned on the basis of expected treatment outcome.

In one embodiment, information about marker characteristic, e.g., size, sequence, composition or amount, e.g., the result of evaluating a marker or marker set described herein, is analyzed by a function that determines whether to authorize the transfer of funds to pay for a service or treatment provided to a subject (or make another decision referred to herein). For example, the results of analyzing a characteristic, e.g., size, sequence, composition or amount of a marker or marker set described herein may indicate that a subject is expected to have a favorable outcome, suggesting that a treatment course is needed, thereby triggering an result that indicates or causes authorization to pay for a service or treatment provided to a subject. In one example, informative characteristic, e.g., size, sequence, composition or amount of a marker or a marker set selected from or derived from Tables 9-10 and/or described herein is determined and payment is authorized if the informative amount identifies a favorable outcome. For example, an entity, e.g., a hospital, care giver, government entity, or an insurance company or other entity which pays for, or reimburses medical expenses, can use the result of a method described herein to determine whether a party, e.g., a party other than the subject patient, will pay for services (e.g., a particular therapy) or treatment provided to the patient. For example, a first entity, e.g., an insurance company, can use the outcome of a method described herein to determine whether to provide financial payment to, or on behalf of, a patient, e.g., whether to reimburse a third party, e.g., a vendor of goods or services, a hospital, physician, or other care-giver, for a service or treatment provided to a patient. For example, a first entity, e.g., an insurance company, can use the outcome of a method described herein to determine whether to continue, discontinue, enroll an individual in an insurance plan or program, e.g., a health insurance or life insurance plan or program.

In one aspect, the disclosure features a method of providing data. The method includes providing data described herein, e.g., generated by a method described herein, to provide a record, e.g., a record described herein, for determining if a payment will be provided. In some embodiments, the data is provided by computer, compact disc, telephone, facsimile, email, or letter. In some embodiments, the data is provided by a first party to a second party. In some embodiments, the first party is selected from the subject, a healthcare provider, a treating physician, a health maintenance organization (HMO), a hospital, a governmental entity, or an entity which sells or supplies the drug. In some embodiments, the second party is a third party payor, an insurance company, employer, employer sponsored health plan, HMO, or governmental entity. In some embodiments, the first party is selected from the subject, a healthcare provider, a treating physician, an HMO, a hospital, an insurance company, or an entity which sells or supplies the drug and the second party is a governmental entity. In some embodiments, the first party is selected from the subject, a healthcare provider, a treating physician, an HMO, a hospital, an insurance company, or an entity which sells or supplies the drug and the second party is an insurance company.

In another aspect, the disclosure features a record (e.g., computer readable record) which includes a list and value of characteristic, e.g., size, sequence, composition or amount for the marker or marker set for a patient. In some embodiments, the record includes more than one value for each marker.

Unless defined otherwise, all technical and scientific terms used herein have the same meanings as commonly understood by one of ordinary skill in the art to which this disclosure belongs. Although any methods and materials similar or equivalent to those described herein can be used in the practice or testing of the present disclosure, the preferred methods, devices and materials are herein described.

The present disclosure will now be illustrated by the following Examples, which are not intended to be limiting in any way.

### EXAMPLES

### Clinical Trial

In a multicenter phase 1/2 clinical trial of single agent alisertib in patients with five predefined advanced solid tumor types [relapsed/refractory breast cancer (BC), small cell lung cancer (SCLC), non-small cell lung cancer (NSCLC), head and neck squamous cell carcinoma (HNSCC) and gastroesophageal (GE) adenocarcinoma], alisertib demonstrated single agent clinically meaningful antitumor activity in four of the five solid tumor types testes (all except NSCLC) (Melichar B. et al., Safety and activity of alisertib, an investigational aurora kinase A inhibitor, in patients with breast cancer, small-cell lung cancer, non-small-cell lung cancer, head and neck squamous cell carcinoma, and gastro-oesophageal adenocarcinoma: a five-arm pase 2 study. Lancet Oncology Vol 16, pages 395-405, April 2015). Archived tumor biopsies and normal blood samples were obtained from 47 patients enrolled in this study. Table 3 below provides the distribution of the tumor types of these 47 patients.

**Table 3:**

| **Cancer indication** | **Number of patients** |
|---|---|
| Breast (BC) | 14 |
| Head and neck squamous cell (HNSCC) | 19 |
| Small cell lung (SCLC) | 4 |
| Non-small cell lung (NSCLC) | 4 |
| Gastroesophageal (GE) | 6 |

### Efficacy endpoint and clinical covariates:

The best tumor size change and progression-free survival (PFS) of the patients were used as the efficacy response endpoints. Since the tumor type showed moderate association with the efficacy endpoints among several clinical variables; the tumor type was included as a covariate in the statistical model for association analysis to correct its effect.

### Whole exome sequencing:

The genomic DNA was isolated from 47 patients with 2-4 slides of 5micron thickness from archived tumor biopsies with Qiagen FFPE kit (Qiagen, Germany). Genomic DNA from blood samples were extracted utilizing blood DNA kit (Qiagen, Germany). Tumor-normal (blood) paired DNAs were sequenced by whole exome DNA sequencing employing Agilent Sure Select exome capture and Illumina Hi-Seq™ technologies. The average sequencing depth of the whole exome sequencing was achieved at ∼100X per bp.

### Bioinformatics Analysis Pipeline

The analysis pipeline for whole exome sequencing was designed to identify mutations from raw sequence reads and evaluate their potential correlation with clinical response. For this purpose, three major analytical units, the upstream, middle-stream, and downstream modules, were devised to process DNA-Seq data. The upstream analysis module runs preprocessing for DNA-Seq, including raw sequence read quality control (QC), read alignment/mapping, and raw somatic mutation calling from the tumor and germline genomes of the patients. Raw mutation calls are evaluated in the middle-stream analysis based on quality statistics and mutation annotations in order to deliver high-confidence mutation calls for the downstream analysis. In the downstream analysis, efforts were made for identifying individual mutated genes or groups of mutated genes that are significantly associated with the response to alisertib.

### Upstream Analysis

The upstream analysis focuses on processing raw DNA-Seq files and calling raw mutations. Short sequence reads of 75bp produced by next-generation sequencing (NGS) are electronically present in the FASTQ or BAM file format. The quality of sequence reads was assessed using fastQC (ver. 0.10.1) to determine if each sample has enough quality for mutation calling. Next, raw sequence reads were mapped to the human reference genome (hg19) using BWA (ver. 0.6.2-r126). After reads were mapped to the reference genome, raw somatic mutations and germline variants, including SNVs and small indels, were called using a VarScan2(ver. 2.3.4) and GATK (ver. 2.3.9), respectively.

### Middle-stream analysis:

The raw mutations were processed in the middle-stream analysis in order to identify high-confidence somatic mutations. Germline variants were also processed similarly. The middle-stream analysis is composed of variant annotation and filtering based on the annotation and variant quality. The annotation sources are summarized in Table 4. RefSeq (hg19), dbSNP, COSMIC, 1000 genome, and The Cancer Genome Atlas (TCGA), and Thomson Reuters Genetic Variant Database (GVDB) were selected as public and proprietary annotation sources.

**Table 4. Annotation sources for raw mutation calls from the upstream analysis:**

| | **Database** | **Note** |
|---|---|---|
| 1 | **RefSeq** | Genomic regions (e.g. coding vs. non-coding), chromosomal coordinate, and gene symbol. Hg19 was used. |
| 2 | **dbSNP** | Reported human SNPs. dbSNP137 was used. |
| 3 | **COSMIC** | Reported somatic mutations in cancers. v68 was used. |
| 4 | **1000 genome** | Low coverage and exome studies. |
| 5 | **TCGA** | Reported somatic mutations in TCGA samples. |
| 6 | **GVDB** | Thomson Reuters Genetic Variant Database (GVDB). |
| 7 | **Prediction of functional impact on protein** | Bioinformatics tools for functional impact on protein (FATHMM, MutationTaster). |

The filtering criteria are summarized in Table 5. Through the filtering steps, only coding-changing somatic mutations were kept (Table 5 row number 1) and mutations found in the matched germline genome were eliminated (Table 5 row number 2). Sequence depth denotes the number of sequence reads piled up at a variant site (Table 5 row number 3). A higher sequence depth better supports an identified variant because NGS is known to have a relatively high error rate. For example, if many reads support the same sequence variation, the variant would be more likely to be a real one, not a sequencing artifact. Similarly, minor allele fraction (MAF) was considered to reduce the chance of including sequencing artifacts and subclonal mutations (Table 5 row number 4). Next, filters about sequencing quality and mapping quality were applied to raw mutations (Table 5 row number 5-7). In order to eliminate potential germline variants, variants overlapped by dbSNP and 1000 genome were discarded, but if they were reported by TCGA, they were kept (Table 5 row number 8). Finally, an optional filtering step was added so that only variants with functional impact on protein coding will remain (Table 5 row number 9). The functional consequences of variants were predicted by publicly available bioinformatics tools, FATHMM (ver. 2.3) and MutationTaster and the results were used as supplementary information to assess the functional impact of each coding-mutation. This filter was used in pathway-level association tests because the use of the filter may reduce the influence of potential passenger genes in each pathway.

**Table 5. Variant filtering criteria for high-confidence somatic mutations:**

| | **QC criterion** | **Note** |
|---|---|---|
| 1 | **Non-synonymous SNVs and small insertions/deletions in coding regions.** | Only coding change mutations are kept. |
| 2 | **VarScan2 somatic p value <= 0.01** | This p value cut-off roughly corresponds to 10% FDR. |
| 3 | **Total read depth >=20, variant read depth >=2 in the tumor BAM.** | Variants must be supported by an enough number of reads. |
| 4 | **MAF >= 0.1** | This cut-off was adjusted by tumor purity. For example, if tumor purity = 80%, then the MAF cut-off becomes 0.1^{∗}0.8=0.08. |
| 5 | **Variant MAPQ >=45, BaseQ >= 25** | The median MAPQ and BaseQ of variant reads must be high enough. |
| 6 | **Strand bias (ratio of + strand reads and - strand reads)** | Ratio <= 0.9 (less than 90% of reads supporting a variant are mapped on one strand. |
| 7 | **Variants overlapped by dbSNP and 1000 genome were removed; however, if they had been reported by COSMIC as somatic mutations, they were kept.** | Any potential germline variants were removed. |
| 8 | **Fpfilter.pl provided by the VarScan2 authors were applied** | Sequence read quality was considered. |
| 9 | **Functional consequences of coding sequence change** | FATHMM and MutationTaster were used to predict the functional consequences of variants. |

### Downstream analysis for single genes:

The downstream analysis was designed to identify potential biomarkers predictive of drug response using the high-confidence mutations obtained from the middle-stream analysis. A linear regression model and Cox proportional hazard model were used for best tumor size change and PFS as endpoints, respectively. Tumor type was considered as a covariate in both models to adjust its confounding effect. In case of linear regression for best tumor size change as an endpoint, baseline tumor size was added as an additional covariate since baseline tumor size is weakly correlated with the endpoint. In the following linear regression equation, ***y* = *β*₀ + *β*₁*x* + *β*_{*c*₁}*c*₁ + *β*_{*c*₁}*c*₂ + *ε*, *y*, *x*,** *c₁*, *c₂*, and *ε* represent the best tumor size change, a single gene mutation status, tumor type, baseline tumor size, and random error, respectively. When the gene harbors high-confidence mutation(s), *x* becomes 1; otherwise, it is 0. The 1^{st} covariate *c*₁ was converted into five binary variables to represent the five different tumor types and the 2^{nd} covariate *c*₂ is a continuous variable. As a result, the coefficient *β₁* indicates the contribution of the mutated gene to sensitivity or resistance to alisertib treatment after adjusting the potential confounding effects of the covariates. A p-value was computed for testing the null hypothesis *β*₁ = 0. Since multiple genes were tested using these models and the p-values were corrected for multiple hypothesis testing using false discovery rate (FDR).

### Downstream analysis for pathways:

A similar approach was used for the pathway association analysis. A pathway can be defined as a group of genes that are known to be interacting together for a common biological function. As can be seen in Table 6 below, six public and proprietary pathway databases were combined in the pathway association analysis to maximize our search ability. Two types of statistical tests were selected in order to test the degree of association of mutations on the pathway level and alisertib treatment outcome, particularly best tumor size change. First, a binary variable was set to 1 (mutated) if any of the genes belonging to a pathway were found to be somatically mutated. A linear regression model was built using this binary variable as an independent variable and tumor type and baseline tumor size were used as covariates, similar to the equation used for gene-level association. Secondly, sequence kernel association tests (SKAT) were run to account for cases where individual mutated genes in a pathway contribute differently to drug sensitivity or resistance. The two tests resulted in a pair of p-values for each of the pathways in Table 6. As the two methods, linear regression and SKAT, provide different angles in terms of association between pathways and alisertib response, the smaller p-value (the best of the two tests, BOT from here on) was selected as a representative p-value for each pathway. The p-values of the pathways were subjected to multiplicity adjustments in order to control the type I error of the statistical tests.

**Table 6. Pathways tested in terms of association with alisertib response. A total of 3,505 pathways were tested.**

| **Pathway DB** | **Number of pathways** |
|---|---|
| MetaCore™ | 912 |
| BIOCYC | 33 |
| KEGG | 794 |
| REACTOME | 1358 |
| Wiki Pathways | 225 |
| Pathway interaction DB | 183 |

### Association between mutated genes/pathwavs and progression-free survival:

The Cox proportional hazard models were used to see potential association between mutated genes/pathways and progression-free survival (PFS). This analysis was done as a supplementary study in order to confirm that the genes and pathways identified by the aforementioned analyses also showed significant differences in PFS between mutant and WT groups.

### Multiplicity adjustment:

For single gene-level association, the Benjamini & Hochberg (BH) method was used to compute FDR (also known as q-value). For pathway-level association, as multiple statistical association analyses (linear regression, SKAT and BOT) were conducted for each pathway, and pathways are often correlated through commonly present genes, multiplicity adjustment becomes more complicated. To address these challenges, a re-sampling-based multiplicity adjustment was implemented. The null distributions of p-values from pathway association tests were simulated using parametric bootstrapping. The adjusted p-values were obtained by comparing the observed p-values to the simulated null distributions as reference distributions.

### High-confidence somatic mutations:

After the middle-stream analysis, a total of 6,410 genes had high-confidence somatic mutations in their coding exons from the 47 patient whole exome sequencing data. When the last filter in Table 5 was also applied, 4,400 mutated genes remained as high-confidence and also functional variants. Figure 2 provides a visual representation of the mutation landscape of the 47 patients in the study (6,410 genes).

### The results of single gene association:

The linear regression model of single gene mutation status (x) and tumor type (*c₁*) and baseline tumor size (*c₂*) as covariates was run to identify mutated genes that are correlated with sensitivity or resistance to alisertib treatment. The 6,410 genes were tested and their associated p-values were corrected using BH adjustment. Four genes were selected among the top genes in p-values based on Aurora A Kinase biology (Table 7). One gene (FAT1) was identified to be significantly associated with tumor reduction when a raw p-value cut-off of 0.05 was applied. Three other genes (MLL3, EP300, FBXW7) were determined to be associated with tumor reduction when a more relaxed cut-off, 0.1 was used. MLL3 was identified by both best tumor size change and PFS and EP300 showed a correlation with only longer PFS. These two genes are known to play an important role in chromatin modification and cell division, which is closely related to the key functions of Aurora A Kinase. Interestingly, FBXW7 was correlated with tumor progression while other genes are associated with tumor reduction or longer PFS.

**Table 7. The single genes, when mutated, showing association with drug response and backed by Aurora A Kinase biology**

| Genes associated with best tumor size changes across all five tumors tested, namely breast cancer, small cell lung cancer, non-small cell lung cancer, head and neck squamous cell carcinoma and gastroesophageal adenocarcinoma. Changes when adjusted by tumor indication and tumor baseline size. | | | | | |
|---|---|---|---|---|---|
| g | P | q | est* | cilow | ciup |
| FAT1 | 0.0403 | 0.302 | -39.70 | -77.6 | -1.84 |
| MLL3 | 0.0651 | 0.302 | -28.84 | -59.6 | 1.89 |
| FBXW7 | 0.0697 | 0.302 | 41.96 | -3.55 | 87.5 |

| Genes associated with progression free survival (PFS) when adjusted by baseline and patients were stratified by indication. | | | | | |
|---|---|---|---|---|---|
| g | P | q | est** | cilow | ciup |
| MLL3 | 0.055 | 0.592 | 0.129 | 0.016 | 1.049 |
| EP300 | 0.091 | 0.592 | 0.271 | 0.0599 | 1.232 |

| | | | | | |
|---|---|---|---|---|---|
| Notations: P & q: raw p-value and BH adjusted p-values or FDR. *est: estimated coefficient in the model (est is the mean % best tumor size change between mutant and WT groups). ** est: estimated coefficient in the model (est in the bottom table indicates HR (hazard ratio)). Cilow/ciup: 95% confidence interval for the estimation. | | | | | |

### The results of pathway-level association:

Two pathways were identified as pathways associated with responsiveness to treatment with alisertib (Table 8). The 1^{st} pathway is the WNT/β-catenin signaling pathway in Thomson Reuters MetaCore™. The WNT/ β-catenin signaling pathway is known to interact with Aurora A Kinase in many diseases including multiple myeloma and glioma. Also, silencing Aurora A Kinase leads to the down-regulation of WNT/ β-catenin signaling. This WNT/ β-catenin pathway is composed of 23 genes (Table 8) and 12 of them, namely; LEF1, MAP3K7, APC, FZD2, PRKCA, RORA, CAMK2G, JUN, XPO1, ROR2, CCND1, CTNNB1, were identified to be somatically mutated in the whole exome sequencing data. The mutation patterns of these 12 genes are illustrated in the heatmap in Figure 3 (top). The other pathway is the Hippo signaling pathway in REACTOME. The Hippo signaling pathway is composed of 22 genes (Table 8) and 11 out of the 22 genes were mutated in the patient samples of the clinical trial. The 11 mutated genes are LOC646561, YWHAEP5, AMOTL1, SAV1, MOB1A, AMOTL2, YWHAE, YWHAB, STK4, STK3 and CASP3. This pathway is known to be related to cell proliferation and apoptosis, particularly tetraploidy-induced cell cycle arrest. This implies a functional link to alisertib since inhibition of Aurora A Kinase leads cells to death by causing mitotic defects leading to aneuploidy and finally cell cycle arrest. The mutation patterns of the Hippo signaling pathway are shown in Figure 3 (bottom). As can be seen in Figures 3 and 4, patients with the mutated WNT or Hippo signaling pathways tend to respond more favorably to alisertib treatment.

**Table 8. The pathways identified to be significantly associated with sensitivity to alisertib treatment. These pathways were selected based on the adjusted p-values from the association tests and Aurora A Kinase /alisertib biology**

| **Pathway name** | **Pathway source** | **Adjusted p-value (raw pvalue)** | **Genes** |
|---|---|---|---|
| Inhibition of WNT5A dependent non-canonical pathway in colorectal cancer | MetaCore™ | 0.0454 (3.43E-05) | LEF1, MAP3K7, APC, FZD2, PRKCA, RORA, CAMK2G, JUN, XPO1, ROR2, CCND1, CTNNB1, CAMK2A, CAMK2B, CAMK2D, CALM1, CALM2, CALM3, NLK, SIAH2, TCF7, WNT5A, MYC |
| Signaling by Hippo | REACTOME | 0.0163 (1.10E-05) | AMOT, DVL2, LATS1, LATS2, MOB1B, NPHP4, TJP1, TJP2, WWC1, WWTR1, YAP1, LOC646561 , YWHAEP5, AMOTL1, SAV1, MOB1A, AMOTL2, YWHAE, YWHAB, STK4, STK3, CASP3 |

**Table 9 Marker Genes of the WNT/β -catenin signaling pathway for Aurora A Kinase Inhibitor Treatment**

| Gene symbol | Marker Gene Name | Entrez | chromosome | start | end | GenBank Accession No. / SEQ ID NO: | GenPept Accession No. / SEQ ID NO: |
|---|---|---|---|---|---|---|---|
| LEF1 | lymphoid enhancer-binding factor 1 | 51176 | chr4 | 108968700 | 109090112 | NM_ 016269.4 / SEQ ID NO: 1 | NP_ 057353.1 / SEQ ID NO:2 |
| MAP3 K7 | mitogen-activated protein kinase kinase kinase 7 | 6885 | chr6 | 91223291 | 91297020 | NM_145331.2 / SEQ ID NO:3 | NP_ 663304.1 / SEQ ID NO:4 |
| APC | adenomat ous polyposis coli | 324 | chr5 | 112073555 | 112181936 | NM_ 000038.5 / SEQ ID NO:5 | NP_000029.2 / SEQ ID NO:6 |
| FZD2 | frizzled class receptor 2 | 2535 | chr17 | 42634811 | 42638630 | NM_ 001466.3 / SEQ ID NO:7 | NP_ 001457.1 / SEQ ID NO:8 |
| PRKCA | protein kinase C, alpha | 5578 | chr17 | 64298925 | 64806862 | XM_ 01152499 0.1 / SEQ ID NO:9 | XP_ 01152329 2.1/ SEQ ID NO:10 |
| RORA | RAR-related orphan receptor A | 6095 | chr15 | 60780482 | 60919729 | NM_ 002943.3 / SEQ ID NO:11 | NP_ 002934.1 / SEQ ID NO:12 |
| CAMK 2G | calcium/c almodulin dependent protein kinase II gamma | 818 | chr10 | 75572258 | 75634349 | NM_172171.2 / SEQ ID NO:13 | NP_ 751911.1 / SEQ ID NO:14 |
| JUN | jun proto-oncogene | 3725 | chr1 | 59246462 | 59249785 | NM_ 002228.3 / SEQ ID NO: 15 | NP_002219.1 / SEQ ID NO:16 |
| XPO1 | exportin 1 | 7514 | chr2 | 61705068 | 61765418 | NM_ 003400.3 / SEQ ID NO:17 | NP_ 003391.1 / SEQ ID NO:18 |
| ROR2 | receptor tyrosine kinase-like orphan receptor 2 | 4920 | chr9 | 94484877 | 94712444 | NM_ 004560.3 / SEQ ID NO:19 | NP_ 004551.2 / SEQ ID NO:20 |
| CCND1 | cyclin D | 595 | chr11 | 69455872 | 69469242 | NM_ 053056.2 / SEQ ID NO:21 | NP_ 444284.1 / SEQ ID NO:22 |
| CTNN B1 | catenin (cadherin-associated protein), beta 1 | 1499 | chr3 | 41240941 | 41281939 | NM_00109820 9.1 / SEQ ID NO:23 | NP_00109167 9.1/ SEQ ID NO:24 |

**Table 10 Marker Genes of the Hippo signaling pathway for Aurora A Kinase Inhibitor Treatment**

| Gene symbol | Marker Gene Name | Entrez | chrom osome | start | end | GenBank Accession No. / SEQ ID NO: | GenPept Accession No. / SEQ ID NO: |
|---|---|---|---|---|---|---|---|
| AMOT | angiomoti n | 154796 | chrX | 112018104 | 112066372 | NM_0011134 90.1 / SEQ ID NO:25 | NP_00110696 2.1/ SEQ ID NO:26 |
| DVL2 | dishevelle d segment polarity protein 2 | 1856 | chr17 | 7128660 | 7137863 | NM_ 004422. 2 / SEQ ID NO:27 | NP_004413.1 / SEQ ID NO:28 |
| LATS1 | large tumor suppressor kinase 1 | 9113 | chr6 | 149979288 | 150039392 | NM_ 004690. 3 / SEQ ID NO:29 | NP_004681.1 / SEQ ID NO:30 |
| LATS2 | large tumor suppressor kinase 2 | 26524 | chr13 | 21547175 | 21635722 | XM_ 0052663 42.1 / SEQ ID NO:31 | XP_ 00526639 9.1 / SEQ ID NO:32 |
| MOB1 B | MOB kinase activator 1B | 92597 | chr4 | 71768056 | 71853891 | NM_ 0012447 66.1 / SEQ ID NO:33 | NP_ 00123169 5.1 / SEQ ID NO:34 |
| NPHP4 | nephronop hthisis 4 | 261734 | chr1 | 5922869 | 6052533 | NM_ 015102. 4 / SEQ ID NO:35 | NP_055917.1 / SEQ ID NO:36 |
| TJP1 | tight junction protein 1 | 7082 | chr15 | 29992356 | 30114706 | NM_ 003257. 4 / SEQ ID NO:37 | NP_003248.3 / SEQ ID NO:38 |
| TJP2 | tight junction protein 2 | 9414 | chr9 | 71820077 | 71870124 | NM_ 004817. 3 / SEQ ID NO:39 | NP_004808.2 / SEQ ID NO:40 |
| WWC1 | WW and C2 domain containing 1 | 23286 | chr5 | 167719064 | 167899308 | NM_0011616 61.1 / SEQ ID NO:41 | NP_ 00115513 3.1 / SEQ ID NO:42 |
| WWTR 1 | WW domain containing transcripti on regulator 1 | 25937 | chr3 | 149235021 | 149375812 | NM_0011682 78.1 / SEQ ID NO:43 | NP_ 00116175 0.1 / SEQ ID NO:44 |
| YAP1 | Yes-associated protein 1 | 10413 | chr11 | 101981191 | 102104154 | NM_0012821 01.1 / SEQ ID NO:45 | NP_ 00126903 0.1 / SEQ ID NO:46 |

## Claims

1. A method for determining whether to treat a patient having cancer with an Aurora A kinase inhibitor, wherein the Aurora A kinase inhibitor is 4-{[9-chloro-7-(2-fluoro-6-methoxyphenyl)-5*H*-pyrimido[5,4-*d*][2]benzazepin-2-yl]amino}-2-methoxybenzoic acid, or a pharmaceutically acceptable salt thereof, the method comprising the steps of:
a) determining in a cancer cell sample obtained from the patient whether any of the WNT pathway genes listed in Table 9 and/or any of the Hippo pathway genes listed in Table 10 contain mutations in comparison to each of the genes' respective wild type sequence; and
b) determining whether to treat the patient with the Aurora A kinase inhibitor based on the mutation analysis in step a), wherein if at least one gene from Table 9 and/or 10 is found to be mutated, the patient may favorably respond to the Aurora A kinase inhibitor.

2. The method of claim 1, further comprising determining to treat the patient if the comparison predicts sensitivity of the cancer cell sample to the Aurora A kinase inhibitor.

3. The method of claim 1, further comprising determining not to treat the patient if the comparison predicts resistance of the cancer cell sample to the Aurora A kinase inhibitor.

4. A method for identifying a patient having cancer as a candidate for treatment with an Aurora A kinase inhibitor, wherein the Aurora A kinase inhibitor is 4-{[9-chloro-7-(2-fluoro-6-methoxyphenyl)-5*H*-pyrimido[5,4-*d*][2]benzazepin-2-yl]amino}-2-methoxybenzoic acid, or a pharmaceutically acceptable salt thereof, the method comprising the steps of:
a) determining in a cancer cell sample obtained from the patient whether any of the WNT pathway genes listed in Table 9 and/or any of the Hippo pathway genes listed in Table 10 contain mutations in comparison to each of the genes' respective wild type sequence; and
b) identifying the patient as a candidate for treatment with the Aurora A kinase inhibitor if the mutation analysis in step a) indicates the presence of a mutation or the presence of several mutations in at least one gene from Table 9 and/or 10.

5. An Aurora A Kinase inhibitor for use in a method for treating a patient having cancer, wherein the Aurora A kinase inhibitor is 4-{[9-chloro-7-(2-fluoro-6-methoxyphenyl)-5*H-*pyrimido[5,4-*d*][2]benzazepin-2-yl]amino}-2-methoxybenzoic acid, or a pharmaceutically acceptable salt thereof, the method comprising the step of:
a) obtaining a cancer cell sample from the patient;
b) determining whether any of the WNT pathway genes listed in Table 9 and/or any of the Hippo pathway genes listed in Table 10 contain mutations in comparison to each of the genes' respective wild type sequence; and
c) treating the subject with the Aurora A Kinase inhibitor if the mutation analysis in b) indicates the presence of a mutation or the presence of several mutations in at least one gene from Table 9 and/or 10.

6. The method of any one of claims 1 to 4 or the Aurora A Kinase inhibitor for use of claim 5, wherein the mutational analysis of the candidate marker genes is determined by a method selected from the group consisting of microarray, PCR and next generation sequencing.

7. The method of any one of claims 1 to 4, 6 or the Aurora A Kinase inhibitor for use of claim 5 or claim 6, wherein the cancer is a solid tumor or a hematological malignancy.

8. The method of claim 7 or the Aurora A Kinase inhibitor for use of claim 7, wherein the solid tumor is selected from the group consisting of breast cancer, head and neck squamous cell cancer, small cell lung cancer, non-small cell lung cancer and gastroesophageal cancer.

9. The method of any one of claims 1 to 4, 6 to 8 or the Aurora A Kinase inhibitor for use of any one of claims 5 to 8, wherein the cancer cell sample is a blood sample or a sample taken from a tumor biopsy.

10. The method of claim 1 to 4 or 6 to 9, or the Aurora A Kinase inhibitor for use of any one of claims 5 to 9, wherein the Aurora A Kinase inhibitor is sodium 4-{[9-chloro-7-(2-fluoro-6-methoxyphenyl)-5*H*-pyrimido[5,4-*d*][2]benzazepin-2-yl]amino}-2-methoxybenzoate or a pharmaceutical composition thereof.

## Patentansprüche

1. Verfahren zur Bestimmung, ob ein an Krebs leidender Patient mit einem Aurora-A-Kinase-Hemmer behandelt werden soll, wobei es sich bei dem Aurora-A-Kinase-Hemmer um 4-{[9-Chlor-7-(2-fluor-6-methoxyphenyl)-5*H*-pyrimido[5,4-*d*][2]benzazepin-2-yl]amino}-2-methoxybenzoesäure oder ein pharmazeutisch unbedenkliches Salz davon handelt, wobei das Verfahren die folgenden Schritte umfasst:
a) Bestimmen, ob in einer vom Patienten erhaltenen Krebszellprobe eines der in Tabelle 9 aufgeführten WNT-Weg-Gene und/oder eines der in Tabelle 10 aufgeführten Hippo-Weg-Gene jeweils im Vergleich zur jeweiligen Wildtypsequenz der Gene Mutationen enthalten; und
b) Bestimmen anhand der Mutationsanalyse in Schritt a), ob der Patient mit dem Aurora-A-Kinase-Hemmer behandelt werden soll, wobei, falls sich herausstellt, dass wenigstens ein Gen aus Tabelle 9 und/oder 10 mutiert ist, der Patient möglicherweise positiv auf den Aurora-A-Kinase-Hemmer anspricht.

2. Verfahren nach Anspruch 1, ferner umfassend Bestimmen, dass der Patient behandelt wird, falls der Vergleich Empfindlichkeit der Krebszellprobe gegenüber dem Aurora-A-Kinase-Hemmer vorhersagt.

3. Verfahren nach Anspruch 1, ferner umfassend Bestimmen, dass der Patient nicht behandelt wird, falls der Vergleich Resistenz der Krebszellprobe gegenüber dem Aurora-A-Kinase-Hemmer vorhersagt.

4. Verfahren zur Identifizierung eines an Krebs leidenden Patienten als Kandidaten für eine Behandlung mit einem Aurora-A-Kinase-Hemmer, wobei es sich bei dem Aurora-A-Kinase-Hemmer um 4-{[9-Chlor-7-(2-fluor-6-methoxyphenyl)-5*H*-pyrimido[5,4-*d*][2]benz-azepin-2-yl]amino}-2-methoxybenzoesäure oder ein pharmazeutisch unbedenkliches Salz davon handelt, wobei das Verfahren die folgenden Schritte umfasst:
a) Bestimmen, ob in einer vom Patienten erhaltenen Krebszellprobe eines der in Tabelle 9 aufgeführten WNT-Weg-Gene und/oder eines der in Tabelle 10 aufgeführten Hippo-Weg-Gene jeweils im Vergleich zur jeweiligen Wildtypsequenz der Gene Mutationen enthalten; und
b) Identifizieren des an Krebs leidenden Patienten als Kandidaten für eine Behandlung mit dem Aurora-A-Kinase-Hemmer, falls die Mutationsanalyse in Schritt a) das Vorliegen einer Mutation oder das Vorliegen mehrerer Mutationen in wenigstens einem Gen aus Tabelle 9 und/oder 10 anzeigt.

5. Aurora-A-Kinase-Hemmer zur Verwendung bei einem Verfahren zur Behandlung eines an Krebs leidenden Patienten, wobei es sich bei dem Aurora-A-Kinase-Hemmer um 4-{[9-Chlor-7-(2-fluor-6-methoxyphenyl)-5*H*-pyrimido[5,4-*d*][2]benzazepin-2-yl]amino}-2-methoxybenzoesäure oder ein pharmazeutisch unbedenkliches Salz davon handelt, wobei das Verfahren die folgenden Schritte umfasst:
a) Erhalten einer Krebszellprobe vom Patienten;
b) Bestimmen, ob eines der in Tabelle 9 aufgeführten WNT-Weg-Gene und/oder eines der in Tabelle 10 aufgeführten Hippo-Weg-Gene jeweils im Vergleich zur jeweiligen Wildtypsequenz der Gene Mutationen enthalten; und
c) Behandeln des Individuums mit dem Aurora-A-Kinase-Hemmer, falls die Mutationsanalyse in b) das Vorliegen einer Mutation oder das Vorliegen mehrerer Mutationen in wenigstens einem Gen aus Tabelle 9 und/oder 10 anzeigt.

6. Verfahren nach einem der Ansprüche 1 bis 4 oder Aurora-A-Kinase-Hemmer zur Verwendung nach Anspruch 5, wobei die Mutationsanalyse der Kandidatenmarkergene mit einem Verfahren ausgewählt aus der Gruppe bestehend aus Mikroarray, PCR und NGS (Next Generation Sequencing) bestimmt wird.

7. Verfahren nach einem der Ansprüche 1 bis 4, 6 oder Aurora-A-Kinase-Hemmer zur Verwendung nach Anspruch 5 oder Anspruch 6, wobei es sich bei dem Krebs um einen soliden Tumor oder ein hämatologisches Malignom handelt.

8. Verfahren nach Anspruch 7 oder Aurora-A-Kinase-Hemmer zur Verwendung nach Anspruch 7, wobei der solide Tumor ausgewählt ist aus der Gruppe bestehend aus Brustkrebs, Plattenepithelkarzinom an Kopf und Hals, kleinzelligem Lungenkrebs, nichtkleinzelligem Lungenkrebs und gastroösophagealem Karzinom.

9. Verfahren nach einem der Ansprüche 1 bis 4, 6 bis 8 oder Aurora-A-Kinase-Hemmer zur Verwendung nach einem der Ansprüche 5 bis 8, wobei es sich bei der Krebszellprobe um eine Blutprobe oder eine einer Tumorbiopsie entnommene Probe handelt.

10. Verfahren nach Anspruch 1 bis 4 oder 6 bis 9 oder Aurora-A-Kinase-Hemmer zur Verwendung nach einem der Ansprüche 5 bis 9, wobei es sich bei dem Aurora-A-Kinase-Hemmer um Natrium-4-{ [9-chlor-7-(2-fluor-6-methoxyphenyl)-5H-pyrimido[5,4-d][2]benzazepin-2-yl]amino}-2-methoxybenzoat oder eine pharmazeutische Zusammensetzung davon handelt.

## Revendications

1. Procédé pour déterminer si un patient ayant un cancer doit être traité avec un inhibiteur de kinase Aurora A, dans lequel l'inhibiteur de kinase Aurora A est l'acide 4-{[9-chloro-7-(2-fluoro-6-méthoxyphényl)-5*H-*pyrimido[5,4-d][2]benzazépin-2-yl]amino}-2-méthoxybenzoïque, ou un sel pharmaceutiquement acceptable de celui-ci, le procédé comprenant les étapes consistant à :
a) déterminer si un échantillon de cellules cancéreuses obtenu à partir du patient du fait que l'un quelconque des gènes de la voie WNT répertoriés dans le tableau 9 et/ou l'un quelconque des gènes de voie Hippo répertoriés dans le tableau 10 contiennent des mutations par rapport à la séquence de type sauvage respective de chacun des gènes ; et
b) déterminer si le patient doit être traité avec l'inhibiteur de kinase Aurora A sur la base de l'analyse de mutations dans l'étape a), dans lequel, s'il est observé qu'au moins un gène du tableau 9 et/ou 10 est muté, le patient peut répondre favorablement à l'inhibiteur de kinase Aurora A.

2. Procédé selon la revendication 1, comprenant en outre la détermination du fait que le patient doit être traité si la comparaison prédit la sensibilité de l'échantillon de cellules cancéreuses à l'inhibiteur de kinase Aurora A.

3. Procédé selon la revendication 1, comprenant en outre la détermination du fait que le patient ne doit pas être traité si la comparaison prédit une résistance de l'échantillon de cellules cancéreuses à l'inhibiteur de kinase Aurora A.

4. Procédé d'identification d'un patient ayant un cancer en tant que candidat pour traitement avec un inhibiteur de kinase Aurora A, dans lequel l'inhibiteur de kinase Aurora A est l'acide 4-{[9-chloro-7-(2-fluoro-6-méthoxyphényl)-5H-pyrimido[5,4-d][2]benzazépin-2-yl]amino}-2-méthoxybenzoïque, ou un sel pharmaceutiquement acceptable de celui-ci, le procédé comprenant les étapes consistant à :
a) déterminer dans un échantillon de cellules cancéreuses obtenu à partir du patient si l'un quelconque des gènes de la voie WNT répertoriés dans le tableau 9 et/ou l'un quelconque des gènes de voie Hippo répertoriés dans le tableau 10 contiennent des mutations par rapport à la séquence de type sauvage respective de chacun des gènes ; et
b) identifier le patient en tant que candidat pour traitement avec l'inhibiteur de kinase Aurora A si l'analyse de mutation dans l'étape a) indique la présence d'une mutation ou la présence de plusieurs mutations dans au moins un gène des tableaux 9 et/ou 10.

5. Inhibiteur de kinase Aurora A pour utilisation dans un procédé de traitement d'un patient ayant un cancer, où l'inhibiteur de kinase Aurora A est l'acide 4-{[9-chloro-7-(2-fluoro-6-méthoxyphényl)-5H-pyrimido[5,4-*d*][2]benzazépin-2-yl]amino}-2-méthoxybenzoïque, ou un sel pharmaceutiquement acceptable de celui-ci, le procédé comprenant l'étape consistant à :
a) obtenir un échantillon de cellules cancéreuses à partir du patient ;
b) déterminer si l'un quelconque des gènes de la voie WNT répertoriés dans le tableau 9 et/ou l'un quelconque des gènes de voie Hippo répertoriés dans le tableau 10 contiennent des mutations par rapport à la séquence de type sauvage respective de chacun des gènes ; et
c) traiter le sujet avec l'inhibiteur de kinase Aurora A si l'analyse de mutation dans b) indique la présence d'une mutation ou la présence de plusieurs mutations dans au moins un gène des tableaux 9 et/ou 10.

6. Procédé selon l'une quelconque des revendications 1 à 4 ou l'inhibiteur de kinase Aurora A pour utilisation selon la revendication 5, dans lequel l'analyse de mutations des gènes marqueurs candidats est déterminée par un procédé choisi dans le groupe constitué d'un microréseau, une PCR et un séquençage de nouvelle génération.

7. Procédé selon l'une quelconque des revendications 1 à 4, 6 ou l'inhibiteur de kinase Aurora A pour utilisation selon la revendication 5 ou la revendication 6, dans lequel le cancer est une tumeur solide ou une tumeur maligne hématologique.

8. Procédé selon la revendication 7 ou inhibiteur de kinase Aurora A pour utilisation selon la revendication 7, dans lequel la tumeur solide est choisie dans le groupe constitué d'un cancer du sein, d'un cancer épidermoïde de la tête et du cou, d'un cancer du poumon à petites cellules, d'un cancer du poumon non à petites cellules et d'un cancer gastro-œsophagien.

9. Procédé selon l'une quelconque des revendications 1 à 4, 6 à 8 ou inhibiteur de kinase Aurora A pour utilisation selon l'une quelconque des revendications 5 à 8, où l'échantillon de cellules cancéreuses est un échantillon de sang ou un échantillon prélevé à partir d'une biopsie de tumeur.

10. Procédé selon la revendication 1 à 4 ou 6 à 9, ou inhibiteur de kinase Aurora A pour utilisation selon l'une quelconque des revendications 5 à 9, où l'inhibiteur de kinase Aurora A est le 4-{ [9-chloro-7-(2-fluoro-6-méthoxyphényl)-5H-pyrimido[5,4-d] [2]benzazépin-2-yl]amino}-2-méthoxybenzoate de sodium ou une composition pharmaceutique de celui-ci.
